# EUROPEAN PATENT APPLICATION

(11) **EP 3 604 548 A1**
(43) Date of publication of application: **05.02.2020**
(21) Application number: 17901776.9
(22) Date of filing: 04.08.2017
(51) Int. Cl.: C12N 15/861, C12N 15/00, C12N 7/01, C12N 7/00, A61K 48/00, A61K 47/46, A61K 47/00, A61K 35/761, A61K 35/00, A61P 35/00

(54) **PROGRAMMABLE ONCOLYTIC VIRUS VACCINE SYSTEM AND APPLICATION THEREOF**

(30) Priority: 24.03.2017 CN 201710184736
(71) Applicant: Tsinghua University, Beijing 100084 (CN); Beijing Syngentech Co., Ltd., Beijing 102206 (CN)
(72) Inventor: XIE, Zhen, Beijing 100084 (CN); HUANG, Huiya, Beijing 100084 (CN); LIU, Yiqi, Beijing 100084 (CN); LIAO, Weixi, Beijing 100084 (CN)
(74) Representative: Schwarz & Partner Patentanwälte OG
(86) International application number: PCT/CN2017/096043
(87) International publication number: WO 2018/171103

(57) **Abstract**

An expression system, comprising: a first nucleic acid molecule having a cell-specific promoter; a second nucleic acid molecule which encodes a transcriptional activator; a third nucleic acid molecule having a first recognition sequence of the transcriptional activator; a fourth nucleic acid molecule having a first promoter and a first regulatory element; a fifth nucleic acid molecule which encodes a first regulatory protein; a sixth nucleic acid molecule having a second recognition sequence of the transcriptional activator; a seventh nucleic acid molecule having a second promoter and a second regulatory element; an eighth nucleic acid molecule which encodes a second regulatory protein; as well as a ninth nucleic acid molecule which is configured to conditionally inhibit the expression of the first regulatory protein, and a tenth nucleic acid molecule which is configured to conditionally inhibit the expression of the second regulatory protein; the first regulatory element is adapted to inhibit the function of the first promoter by means of binding to the second regulatory protein, and the second regulatory element is adapted to inhibit the function of the second promoter by means of binding to the first regulatory protein.

## Description

### PRIORITY INFORMATION

This application claims priority to and the benefit of the patent application Serial No. 201710184736.2 filed with the National Intellectual Property Administration of PRC on March 24, 2017, the entire content of which is incorporated herein by reference.

### FIELD

The present disclosure relates to the field of biomedicine. In particular, the present disclosure relates to an expression system and use thereof, and more particularly, to an expression system, a recombinant virus, a recombinant cell, and use of the gene expression system, the recombinant virus and the recombinant cell in the preparation of a medicament. Further, the present disclosure also relates to a method of expressing a protein of interest by using the expression system.

### BACKGROUND

Oncolytic viruses refer to a type of viruses that have the ability of replication and packaging so as to achieve tumor killing. At present, most studies have showed that some naturally occurring attenuated viruses after engineering modification can be specifically expressed and packaged in tumor cells, thus realizing oncolytic effect. There are two main principles for recognizing tumor cells via oncolytic viruses. Firstly, the oncolytic viruses selectively infect tumor cells due to inactivation or defect of tumor suppressor genes in target cells. Secondly, tumor-specific promoters are selected to regulate the expression of key genes of oncolytic viruses such that the oncolytic viruses replicate in large amounts and express toxic proteins in tumor cells to destroy the tumor cells, and/or simultaneously secrete cytokines to stimulate the autoimmune system, thus attacking tumor cells. Corresponding oncolytic virus cannot replicate in normal cells of an organism, thereby having no killing ability, thus the oncolytic virus has a higher anti-tumor effect and a lower side effect. In recent decades, oncolytic viral treatment has gained extensive attentions, and relevant researches have made great progress. Currently, adenovirus, Herpes Simplex Virus-1 (HSV-1), Newcastle disease virus and the like have been engineered to oncolytic viruses. In 2006, oncolytic adenovirus products (Gendicine and Oncorine) have been used in clinical treatment in China, mainly for the treatment of head and neck cancer, sinus cancer and the like. Gendicine and Oncorine have a similar mechanism, that is, deleting the EIB-55kD region of human type-5 adenovirus, such that the human type-5 adenovirus can reproduce in p53 gene-mutated cancer cells and kill tumor cells, thereby generating therapeutic oncolytic effects. However, clinical data shows that the therapeutic effect of these two oncolytic adenoviruses based on the single p53 gene mutation are not ideal. Further, JX-594 of the US biotherapeutic company Jennerex is an engineered vaccinia virus. It is shown that primary liver cancer patients have an extended median life-span reaching 14.1 months after high-dose injection of the engineered vaccinia virus, compared to 6.7 months of life-span after low-dose injection in the phase II clinical trial completed in 2013. Furthermore, the genetically engineered herpes simplex virus OncoVEX GM-CSF developed by biotechnology company BioVex, which is the first oncolytic virus product on market in the US and Europe approved by the FDA in October 2015, can selectively kill tumor cells while expressing and secreting GM-CSF to initiate the generation of systematic immune response in body, thus killing residual tumor cells and their metastatic sites. The clinical results of metastatic melanoma in phase II trial published by BioVex in 2009 show that 26% of 50 patients exhibit improvement after treatment and 8 patients are totally recovered. Agmen, acquiring the company BioVex at $1 billion in 2011 for promoting the research of phase III clinical trial, published the efficacy data of OncoVEX in March 2013, which demonstrates the drug OncoVEX is capable of decreasing the tumor size of advanced patients successfully, and exhibits stronger efficacy than other similar drugs in the phase III clinical trial involving more than 400 patients.

The oncolytic virus indeed exhibits efficacy on treatment of tumors based on the above research results, however, some deficiencies still exist.

Currently, there are 52 known human adenoviruses, named as Adl∼Ad52, where Ad2 is the most common. The transcriptional pattern of adenovirus has a very distinct feature, where adenoviral genome has at least five known transcription units, including the E1 region involved in cell transformation and located in the left side of the adenoviral genome, which can be subdivided into E1A and E1B; the E2 region, encoding DNA binding protein and involved in viral replication; the E3 region, encoding a glycoprotein that appears on the surface of host cells; the E4 region, located at the right end of the Ad2 genome and regulated by the DNA binding protein encoded by the E2 region; and the fifth transcription unit, synthesizing the Ad2 protein IV in the metaphase of viral infection. After the adenoviral genome enters cell nucleus, cellular transcription factor firstly binds to the enhancer upstream of the E1A region, expressing the E1A protein, which regulates cellular metabolism and allows viral DNA to be replicated in the cell easily, as well as activates promoters of other early genes (E1B, E2A, E2B, E3 and E4), where E2B drives the expression of three additional transcriptional units of early genes involving in viral replication (i.e. precursor terminal protein (pTP), single-stranded DNA binding protein (ssDBP) and DNA polymerase (DNA pol)), these three gene expression products tightly binding to a complex, interacting with at least three cellular proteins to initiate replication of the viral genome. The following engineering strategies can be made according to the analysis of adenoviral genome and transcriptional pattern: regulating E1A expression to regulate the replication and packaging of adenovirus in target cells; removing some non-essential genes relating to viral packaging to reduce toxicity of virus on non-target cells while increasing the packaging capacity of virus (such as, E3, E4 and the like); replacing the coat protein of adenovirus, thus changing the targeting of adenovirus to specific cells and tissues.

However, the existing research results of oncolytic adenovirus show the presence of deficiencies of the current regulation system such as monotony, poor specificity, low safety and the like as well as the cumbersome method for engineering adenovirus. Therefore, how to design a more rigorous and safer regulation system and how to realize the assembly of oncolytic adenovirus in a quick and effective way is needed to be solved urgently in the aspect of design and construction of oncolytic adenovirus.

### SUMMARY

The present disclosure aims at solving several of the aforementioned technical problems to at least some extent.

Based on the research achievements in inventors' laboratory, the present inventors have designed and constructed a gene circuit that is suitable for adenovirus regulation and responsive to different microenvironments. In this gene circuit, the present inventors utilize multiple levels of biomarkers. Specifically, first, a specific promoter is used as a master switch for regulating the gene circuit, that is, employing the specific promoter to regulate the expression of a master activator, thus further regulating the expression of the adenovirus E1A gene; second, microRNA target sequence is used to respond to the expression characteristics of microRNA under different microenvironments, which acts as a secondary switch for regulating the gene circuit; third, a close-loop gene circuit is employed to construct a mutually repressive switch, such a mutually repressive close-loop switch can repression respond to changes of microenvironment more efficiently while effectively avoiding leakage; fourth, the E1B gene is removed, thus the engineered adenovirus has an improved ability of distinguishing P53 deficient tumor cells from normal cells due to the absence of E1B gene; fifth, the E3 gene is removed, thus reducing the toxicity of oncolytic adenovirus on normal cells while increasing the packaging capacity of adenoviral vectors.

In general, type-2 and type-5 human adenoviruses have a genome length of 36 K, thus it will be very difficult and time consuming to genetically modify the adenovirus by using traditional plasmid construction methods like cleavage, ligation and the like. In order to solve this problem, the present inventors designed a method for rapidly constructing the adenovirus constructed by the present gene circuit, comprising the following three steps. In the first step, a primary element library is constructed, where the required components are constructed into corresponding plasmids. Such a primary element library comprises three gene parts, including a repression element library A, mainly expressing the repression components in one side of the gene circuit, the E1A gene of adenovirus and effector genes co-expressed with the adenovirus E1A gene (such as an immune factor gene or a killer gene); a repression element library B, mainly expressing the repression gene in the other side of the gene circuit, configured to regulate the effective reversal of the gene circuit, thus enhancing the safety of the switch; and a specific promoter library, being the master switch which regulates the downstream gene of the gene circuit via tissue or tumor-specific promoters. In the second step, after determination of biomarkers and effectors to be expressed, corresponding plasmids selected from the primary element library are assembled into a gene circuit rapidly through the Golden Gate method. In the third step, the gene circuit is loaded into the engineered adenoviral vector (removal of the E1 gene to facilitate the artificial regulation of adenovirus; and removal of part E3 sequence to enlarge the packaging capacity).

Carrying and simultaneously expressing multiple genes is another remarkable feature of oncolytic adenovirus. In the present disclosure, one or more cellular immune-related genes like IL-2, GM-CSF, anti- PD-1 scFv, anti-PD-Ll scFv, and fusion proteins between these genes and the like are simultaneously expressed by oncolytic adenovirus through the disclosure by the present inventors. The oncolytic adenovirus will trigger systematic immune responses when attacking tumor cells, while having a risk of causing an immune overreaction due to the presence of immune-related genes. Thus, the therapeutic effect of the oncolytic viruses will be strongly influenced by the immune-related genes carried, the corresponding administration dose and the administration routes. In the present disclosure, the inventors have attempted to model the infecting process of adenovirus against target cells by means of bioinformatics method, and studied the properties of oncolytic adenovirus at killing tumor cells, so as to improve the efficacy of oncolytic adenovirus as much as possible.

In a first aspect, the present disclosure in embodiments provides an expression system. According to an embodiment of the present disclosure, the expression system comprises: a first nucleic acid molecule, incorporating a cell-specific promoter; a second nucleic acid molecule, operably linked to the first nucleic acid molecule and encoding a transcriptional activator; a third nucleic acid molecule, incorporating a first recognition sequence of the transcriptional activator; a fourth nucleic acid molecule, operably linked to the third nucleic acid molecule and incorporating a first promoter and a first regulatory element; a fifth nucleic acid molecule, operably linked to the fourth nucleic acid molecule and encoding a first regulatory protein; a sixth nucleic acid molecule, incorporating a second recognition sequence of the transcriptional activator; a seventh nucleic acid molecule, operably linked to the sixth nucleic acid molecule and incorporating a second promoter and a second regulatory element; an eighth nucleic acid molecule, operably linked to the seventh nucleic acid and encoding a second regulatory protein; and at least one selected from the group consisting of: a ninth nucleic acid molecule, operably linked to the fifth nucleic acid molecule and configured to conditionally inhibit expression of the first regulatory protein; a tenth nucleic acid molecule, operably linked to the eighth nucleic acid molecule and configured to conditionally inhibit expression of the second regulatory protein, wherein the first regulatory element is adapted to inhibit the function of the first promoter by binding to the second regulatory protein, and the second regulatory element is adapted to inhibit the function of the second promoter by binding to the first regulatory protein. With the expression system according to the embodiment of the present disclosure, the gene of interest can be specifically expressed under a specific cellular environment while being regulated in a mutual repression way, with strong control, high efficiency and specificity.

In embodiments of the present disclosure, the expression system may further comprise at least one of additional technical features as follows.

In embodiments of the present disclosure, the cell-specific promoter is a tumor cell-specific promoter. The tumor cell-specific promoter is at least one selected from the group consisting of an alpha-fetoprotein-specific promoter, a Survivin promoter, a human telomerase reverse transcriptase gene promoter, a cholecystokinin A receptor gene promoter, a carcinoembryonic antigen promoter, a proto-oncogene human epidermal growth factor receptor 2 promoter, a prostaglandin endoxygenase reductase 2 promoter, a chemokine receptor-4, an E2F-1 gene promoter, a mucin promoter, a prostate specific antigen, a human tyrosinase-related protein 1, and a tyrosinase promoter. The expression system in the embodiments can be initiated in the microenvironment of specific tumor cells under the control of the tumor cell-specific promoters described as above, thereby further enhancing the specificity of gene expression and regulation.

In embodiments of the present disclosure, the transcriptional activator is at least one selected from the group consisting of Gal4VP16, Gal4VP64, Gal4esn, dCas9-VP16, dCas9-VP64, dCas9-VPR, dCas9-VTR and rtTA.

In embodiments of the present disclosure, the first recognition sequence and the second recognition sequence are each independently selected from at least one of 5× UAS, 7 × tetO and a target sequence of dCas9.

In embodiments of the present disclosure, the first promoter and the second promoter are each independently selected from a miniCMV and a TATA box.

In embodiments of the present disclosure, the first regulatory protein and the second regulatory protein are each independently selected from at least one of LacI, tetR, zinc finger, KRAB, tetR-KRAB and dCas9-KRAB.

In embodiments of the present disclosure, the first regulatory element and the second regulatory element are each independently selected from at least one of tetO, LacO, zinc finger target site and a target sequence of dCas9.

In an embodiment of the present disclosure, the first regulatory protein is LacI, and the second regulatory element comprises a plurality of repeated LacO sequences, wherein at least one of the pluralities of repeated LacO sequences is set downstream of the second promoter. The LacI after expressed can specifically binds to the LacO sequence, thereby inhibiting the function of the second promoter. The LacI/LacO repression system according to the embodiment is capable of effectively inhibiting the expression of downstream genes of the second promoter as demonstrated by experiments.

In an embodiment of the present disclosure, the second regulatory protein is tetR-KRAB, and the first regulatory element comprises a plurality of repeated tetO sequences, wherein at least one of the pluralities of repeated tetO sequences is set downstream of the first promoter. The tetR-KRAB/tetO repression system according to the embodiment is capable of effectively inhibiting the expression of downstream genes of the first promoter.

In an embodiment of the present disclosure, at least one of the fifth nucleic acid molecule and the ninth nucleic acid molecule further comprises a sequence encoding a protein of interest. With the expression system according to the embodiment of the present disclosure, the protein of interest can be specifically expressed in a specific cellular microenvironment, while being regulated in a mutual repression way. According to a specific embodiment of the present disclosure, the expression system exhibits a significant increased specificity and regulation on the expression of the protein of interest.

In an embodiment of the present disclosure, the fifth nucleic acid molecule comprises a sequence encoding the protein of interest, and the protein of interest comprises at least one selected from a viral replication and packaging protein and an immune effector. Optionally, the viral replication and packaging protein and the immune effector may be present in the form of a fusion protein. The viral replication and packaging protein can effectively ensure the survival and replication of the expression system vector in a host. The expression of the immune effector can effectively activate the immune system in body, thereby promoting the immune killing to specific cells such as tumor cells.

In an embodiment of the present disclosure, the virus replication and packaging protein comprises at least one selected from the group consisting of an adenovirus E1 gene, an adenovirus E1A gene, an adenovirus E1B gene, an adenovirus E2 gene, and an adenovirus E4 gene.

In an embodiment of the present disclosure, the immune effector comprises at least one sequence selected from the group consisting of an inhibitory sequence that antagonizes PD-1 gene, an inhibitory sequence that antagonizes PD-L1 gene, an inhibitory sequence that antagonizes CTLA4 gene, an inhibitory sequence that antagonizes Tim-3 gene, GM-CSF, IL-2, IL-12 and IL-15. Optionally, the immune effector as described above may be present in the form of a fusion protein.

In an embodiment of the present disclosure, the protein of interest and the first regulatory protein are expressed in the form of a fusion protein, and the protein of interest and the first regulatory protein are linked by a cleavable linker peptide. The protein of interest and the first regulatory protein are regulated and expressed by a same promoter, and are cleaved at the linker peptide after expressed, thus the protein of interest and the first regulatory protein are separated, and function independently of each other.

In an embodiment of the present disclosure, the ninth nucleic acid molecule and the tenth nucleic acid molecule independently inhibit expression of the first regulatory protein or the second regulatory protein via RNA interference, wherein microRNA is a specific microRNA expressed in different cellular microenvironments, and the ninth or tenth nucleic acid molecule is a specific target sequence of the microRNA. The microRNA expressed in a specific microenvironment can specifically act on the target sequence via RNA interference, thus the expression of the first regulatory protein or the second regulatory protein can be specifically regulated. In the embodiment of the present disclosure, the ninth nucleic acid molecule comprises a nucleic acid sequence specifically recognized by a first microRNA, the first microRNA being a normal cell-specific microRNA; and the tenth nucleic acid molecule comprises a nucleic acid sequence specifically recognized by a second microRNA, the second microRNA being an abnormal cell-specific microRNA. Further, the first regulatory protein is expressed in abnormal cells and is not expressed or low-expressed in normal cells, while the second regulatory protein is expressed in normal cells and is not expressed or low-expressed in abnormal cells.

In a specific embodiment of the present disclosure, the first microRNA comprises at least one selected from the group consisting of miR199a, miR95, miR125, miR25b, Let-7, miR143, miR145 and miR200C. The microRNA as described above is expressed in normal liver cells.

In a specific embodiment of the present disclosure, the second microRNA comprises at least one selected from the group consisting of miR21, miR223, miR224, miR221, miR18, miR214, miR146a and miR1792. The second microRNA as described above is a microRNA specifically expressed in hepatoma cells (such as HepG2, Huh7, and PLC). Furthermore, the first regulatory protein is expressed in hepatoma cells and is not expressed or low-expressed in normal cells, while the second regulatory protein is expressed in normal cells and is not expressed or low-expressed in hepatoma cells.

In an embodiment of the present disclosure, the first nucleic acid molecule and the second nucleic acid molecule are loaded in a first expression vector; the third nucleic acid molecule, the fourth nucleic acid molecule, the fifth nucleic acid molecule and optionally the ninth nucleic acid molecule are loaded in a second expression vector; and the sixth nucleic acid molecule, the seventh nucleic acid molecule, the eighth nucleic acid molecule and optionally the tenth nucleic acid molecules are loaded in a third expression vector. The first, second and third expression vectors serve as a loading vector for the expression system so as to regulate the specific expression of the gene of interest in a suitable microenvironment, such as a cell.

The selection of the expression vector is not particularly limited as long as the expression system can exhibit its function in a suitable microenvironment. According to a specific embodiment of the present disclosure, the first expression vector, the second expression vector and the third expression vector are each independently selected from at least one of plasmids, viruses, stable cell lines, and other carriers such as nanomaterials, liposomes, molecularly coupled vectors, naked DNAs, chromosomal vectors and polymers.

In an embodiment of the present disclosure, the virus comprises at least one selected from the group consisting of an adenovirus, a vaccinia virus, a herpes virus and a retrovirus.

In an embodiment of the present disclosure, the first expression vector, the second expression vector and the third expression vector are loaded in one same vector. It should be noted that the connection order of the first expression vector, the second expression vector, and the third expression vector is not particularly limited as long as it does not affect the realization of the biological function of the expression system. According to a specific embodiment of the present disclosure, the problem of extremely low co-transfection efficiency of multiple large fragment vectors can be effectively solved by loading on one same expression vector.

In an embodiment of the present disclosure, the one same vector is an adenoviral vector. As a gene therapy vector, adenovirus has advantages of a wide range of hosts, low pathogenicity to humans, infection and gene expression in proliferating and non-proliferating cells, high titer, homology with human genes, no mutagenicity after insertion, as well as amplifying in a suspension and simultaneously expressing multiple genes.

In a specific embodiment of the present disclosure, the first expression vector comprises: BsaI, AFP III, Gal4VP16 and BsaI from the 5' end to the 3' end (BsaI-AFP III-Gal4VP16-BsaI).

In an embodiment of the present disclosure, the first expression vector carries a nucleic acid having the nucleotide sequence shown in SEQ ID NO: 1.

GGTCTCTGCTCCAGATTGAATTATTTGCCTGTCATACAGCTAATAATTGACCATAAG
ACAATTAGATTTAAATTAGTTTTGAATCTTTCTAATACCAAAGTTCAGTTTACTGTTCCA
TGTTGCTTCTGAGTGGCTTCACAGACTTATGAAAAAGTAAACGGAATCAGAATTACAT
CAATGCAAAAGCATTGCTGTGAACTCTGTACTTAGGACTAAACTTTGAGCAATAACAC
ATATAGATTGAGGATTGTTTGCTGTTAGTATACAAACTCTGGTTCAAAGCTCCTCTTTAT
TGCTTGTCTTGGAAAATTTGCTGTTCTTCATGGTTTCTCTTTTCACTGCTATCTATTTTTC
TCAACCACTCACATGGCTACAAAAGCTTCCTGATTAATAATTACACTAAGTCAATAGGC
ATAGAGCCAGGACTGTTTGGGTAAACTGGTCACTTTATCTTAAACTAAATATATCCAAA
ACTGAACATGTACTTAGTTACTAAGTCTTTGACTTTATCTCATTCATACCACTCAGCTTT
ATCCAGGCCACTTATTTGACAGTATTATTGCGAAAACTTCCTATCTAGAAGTTTGAGGA
GAATATTTGTTATATTTGCAAAATAAAATAAGTTTGCAAGTTTTTTTTTCTGCCCCAAA

GAGCTCTGTGTCCTTGAACATAAAATACAAATAACCGCTATGCTGTTAATTATTGACAA
ATGTCCCATTTTCAACCTAAGGAAATACCATAAAGTAACAGATATACCAACAAAAGGTT
ACTAGTTAACAGGCATTGCCTGAAAAGAGTATAAAAGAATTTCAGCATGATTTTCCATA
TTGTGCTTCCACCACTGCCAATAACAAAATAACTAGCAAGGATCCGGCCACCATGGCC
CCCCCGACCGATGTCAGCCTGGGGGACGAGCTCCACTTAGACGGCGAGGACGTGGCG
ATGGCGCATGCCGACGCGCTAGACGATTTCGATCTGGACATGTTGGGGGACGGGGATT
CCCCGGGTCCGGGATTTACCCCCCACGACTCCGCCCCCTACGGCGCTCTGGATATGGCC
GACTTCGAGTTTGAGCAGATGTTTACCGATGCCCTTGGAATTGACGAGTACGGTGGGA
CGCGTATGAAGCTACTGTCTTCTATCGAACAAGCATGCGATATTTGCCGACTTAAAAAG
CTCAAGTGCTCCAAAGAAAAACCGAAGTGCGCCAAGTGTCTGAAGAACAACTGGGA
GTGTCGCTACTCTCCCAAAACCAAAAGGTCTCCGCTGACTAGGGCACATCTGACAGAA
GTGGAATCAAGGCTAGAAAGACTGGAACAGCTATTTCTACTGATTTTTCCTCGAGAAG
ACCTTGACATGATTTTGAAAATGGATTCTTTACAGGATATAAAAGCATTGTTAACAGGA
TTATTTGTACAAGATAATGTGAATAAAGATGCCGTCACAGATAGATTGGCTTCAGTGGA
GACTGATATGCCTCTAACATTGAGACAGCATAGAATAAGTGCGACATCATCATCGGAAG
AGAGTAGTAACAAAGGTCAAAGACAGTTGACTGTATAATTCACTCCTCAGGTGCAGGC
TGCCTATCAGAAGGTGGTGGCTGGTGTGGCCAATGCCCTGGCTCACAAATACCACTGA
GATCTTTTTCCCTCTGCCAAAAATTATGGGGACATCATGAAGCCCCTTGAGCATCTGAC
TTCTGGCTAATAAAGGAAATTTATTTTCATTGCAATAGTGTGTTGGAATTTTTTGTGTCT
CTCACTCGGAAGGACATATGGGAGGGCAAATCATTTAAAACATCAGAATGAGTATTTG
GTTTAGAGTTTGGCAACATATGCCCATATGCTGGCTGCCATGAACAAAGGTTGGCTATA
AAGAGGTCATCAGTATATGAAACAGCCCCCTGCTGTCCATTCCTTATTCCATAGAAAAG
CCTTGACTTGAGGTTAGATTTTTTTTATATTTTGTTTTGTGTTATTTTTTTCTTTAACATCC
CTAAAATTTTCCTTACATGTTTTACTAGCCAGATTTTTCCTCCTCTCCTGACTACTCCCA
GTCATAGCTGTCCCTCTTCTCTTATGGAGATCGGAGAAAGAGGTAATTTAATTAAGTCG
ATGAGACC (SEQ ID NO:1).

In a specific embodiment of the present disclosure, the second expression vector comprises: BsaI, 5 × UAS, tetO, miniCMV, tetO, E1A, 2A, an immune effect factor (Effector), LacI, microRNA199a specific recognition sequence (target site) and BsaI from the 5' end to the 3' end (BsaI-5 × UAS-tetO-miniCMV-tetO-E1A-2A-Effector-LacI-miR199a target site-BsaI). In a specific embodiment of the present disclosure, the effector specifically comprises IL-2, hGM-CSF, mGM-CSF, anti-PD-1 scFv, anti-PD-Ll scFv, IL-2 -anti-PD-1 scfv fusion protein, hGM-CSF-anti- PD-1scfv fusion protein, mGM-CSF -anti-PD-lscfv fusion protein, IL-2 - anti-PD-Llscfv fusion protein, hGM-CSF - anti-PD-Llscfv fusion protein, mGM-CSF - anti-PD-Llscfv fusion protein.

In an embodiment of the present disclosure, the second expression vector carries a nucleic acid having the nucleotide sequence selected from the group consisting of SEQ ID NOs: 2-7. 
GGTCTCTCTATTTAATTAAGTAACTATAACGGTCGCTCCGAATTTCTCGAGTTAATTA
AGATTACGCCAAGCTACGGGCGGAGTACTGTCCTCCGAGCGGAGTACTGTCCTCCGAG
CGGAGTACTGTCCTCCGAGCGGAGTACTGTCCTCCGAGCGGAGTTCTGTCCTCCGAGC
GGAGACTCTAGACTCCCTATCAGTGATAGAGATAGGCGTGTACGGTGGAGGCCTATAT
AAGCAGAGCTCGTTTAGTGAACCGTCAGATCGCTCCCTATCAGTGATAGAGAGAATTC
GACCGCCACCATGTCGGAATTCATGAGACATATTATCTGCCACGGAGGTGTTATTACCG
AAGAAATGGCCGCCAGTCTTTTGGACCAGCTGATCGAAGAGGTACTGGCTGATAATCT
TCCACCTCCTAGCCATTTTGAACCACCTACCCTTCACGAACTGTATGATTTAGACGTGA
CGGCCCCCGAAGATCCCAACGAGGAGGCGGTTTCGCAGATTTTTCCCGACTCTGTAAT
GTTGGCGGTGCAGGAAGGGATTGACTTACTCACTTTTCCGCCGGCGCCCGGTTCTCCG
GAGCCGCCTCACCTTTCCCGGCAGCCCGAGCAGCCGGAGCAGAGAGCCTTGGGTCCG
GTTTCTATGCCAAACCTTGTACCGGAGGTGATCGATCTTACCTGCCACGAGGCTGGCTT
TCCACCCAGTGACGACGAGGATGAAGAGGGTGAGGAGTTTGTGTTAGATTATGTGGAG
CACCCCGGGCACGGTTGCAGGTCTTGTCATTATCACCGGAGGAATACGGGGGACCCAG
ATATTATGTGTTCGCTTTGCTATATGAGGACCTGTGGCATGTTTGTCTACAGTAAGTGAA
AATTATGGGCAGTGGGTGATAGAGTGGTGGGTTTGGTGTGGTAATTTTTTTTTAATTTT
TACAGTTTTGTGGTTTAAAGAATTTTGTATTGTGATTTTTTTAAAAGGTCCTGTGTCTGA
ACCTGAGCCTGAGCCCGAGCCAGAACCGGAGCCTGCAAGACCTACCCGCCGTCCTAA
AATGGCGCCTGCTATCCTGAGACGCCCGACATCACCTGTGTCTAGAGAATGCAATAGTA
GTACGGATAGCTGTGACTCCGGTCCTTCTAACACACCTCCTGAGATACACCCGGTGGT
CCCGCTGTGCCCCATTAAACCAGTTGCCGTGAGAGTTGGTGGGCGTCGCCAGGCTGTG
GAATGTATCGAGGACTTGCTTAACGAGCCTGGGCAACCTTTGGACTTGAGCTGTAAAC
GCCCCAGGCCACTCGAGGGTACCGGGTCCGGAGCTACAAATTTTTCCCTCCTCAAACA
GGCTGGAGATGTCGAAGAAAATCCTGGGCCTACCGGTCCCATGGTGAGCAAGGGCGA
GGAGCTGTTCACCGGGGTGGTGCCCATCCTGGTCGAGCTGGACGGCGACGTAAACGG
CCACAAGTTCAGCGTGAGGGGCGAGGGCGAGGGCGATGCCACCAACGGCAAGCTGA
CCCTGAAGTTCATCTGCACCACCGGCAAGCTGCCCGTGCCCTGGCCCACCCTCGTGAC
 
CACCCTGAGCCACGGCGTGCAGTGCTTCGCCCGCTACCCCGACCACATGAAGCAGCA
CGACTTCTTCAAGTCCGCCATGCCCGAAGGCTACGTCCAGGAGCGCACCATCTTCTTC
AAGGACGACGGCACCTACAAGACCCGCGCCGAGGTGAAGTTCGAGGGCGACACCCT
AGTGAACCGCATCGAGCTGAAGGGCGTCGACTTCAAGGAGGACGGCAACATCCTGGG
GCACAAGCTGGAGTACAACTTCAACAGCCACAACATCTATATCATGGCCGTCAAGCAG
AAGAACGGCATCAAGGTGAACTTCAAGATCCGCCACAACGTGGAGGACGGCAGCGTG
CAGCTCGCCGACCACTACCAGCAGAACACCCCCATCGGCGACGGCCCCGTGCTGCTG
CCCGACAGCCACTACCTGAGCACCCAGTCCGTGCTGAGCAAAGACCCCAACGAGAAG
CGCGATCACATGGTCCTGCTGGAGTTCCGCACCGCCGCCGGGATCACTCTCGGCATGG
ACGAGCTGTACAAGAGATCTGAGGGCCGCGGCAGCCTGCTGACCTGCGGCGACGTGG
AGGAAAACCCCGGCCCCGGATCCATGAAACCAGTAACGTTATACGATGTCGCAGAGTA
TGCCGGTGTCTCTTATCAGACCGTTTCCCGCGTGGTGAACCAGGCCAGCCACGTTTCT
GCGAAAACGCGGGAAAAAGTGGAAGCGGCGATGGCGGAGCTGAATTACATTCCCAAC
CGCGTGGCACAACAACTGGCGGGCAAACAGTCGTTGCTGATTGGCGTTGCCACCTCC
AGTCTGGCCCTGCACGCGCCGTCGCAAATTGTCGCGGCGATTAAATCTCGCGCCGATC
AACTGGGTGCCAGCGTGGTGGTGTCGATGGTAGAACGAAGCGGCGTCGAAGCCTGTA
AAGCGGCGGTGCACAATCTTCTCGCGCAACGCGTCAGTGGGCTGATCATTAACTATCC
GCTGGATGACCAGGATGCCATTGCTGTGGAAGCTGCCTGCACTAATGTTCCGGCGTTAT
TTCTTGATGTCTCTGACCAGACACCCATCAACAGTATTATTTTCTCCCATGAAGACGGT
ACGCGACTGGGCGTGGAGCATCTGGTCGCATTGGGTCACCAGCAAATCGCGCTGTTAG
CGGGCCCATTAAGTTCTGTCTCGGCGCGTCTGCGTCTGGCTGGCTGGCATAAATATCTC
ACTCGCAATCAAATTCAGCCGATAGCGGAACGGGAAGGCGACTGGAGTGCCATGTCC
GGTTTTCAACAAACCATGCAAATGCTGAATGAGGGCATCGTTCCCACTGCGATGCTGG
TTGCCAACGATCAGATGGCGCTGGGCGCAATGCGCGCCATTACCGAGTCCGGGCTGCG
CGTTGGTGCGGATATCTCGGTAGTGGGATACGACGATACCGAAGACAGCTCATGTTATA
TCCCGCCGTTAACCACCATCAAACAGGATTTTCGCCTGCTGGGGCAAACCAGCGTGGA
CCGCTTGCTGCAACTCTCTCAGGGCCAGGCGGTGAAGGGCAATCAGCTGTTGCCCGTC
TCACTGGTGAAAAGAAAAACCACCCTGGCGCCCAATACGCAAACCGCCTCTCCCCGC
GCGTTGGCCGATTCATTAATGCAGCTGGCACGACAGGTTTCCCGACTGGAAAGCGGGC
AGAGCAGCCTGAGGCCTCCTAAGAAGAAGAGGAAGGTTGCGGCCGCTAACCAATGTG
CAGACTACTGTTAACCAATGTGCAGACTACTGTGAATTCTAACCAATGTGCAGACTACT
GTTAACCAATGTGCAGACTACTGTAAGCTTCGAATTGAGTAGTGCTTTCTACTTTATGA
 
GTAGTGCTTTCTACTTTATGAGTAGTGCTTTCTACTTTATGAGTAGTGCTTTCTACTTTAT
GGTCGACAATACTAGTTAAGAAATGAGACC (SEQ ID NO:2).
GGTCTCTCTATTTAATTAAGTAACTATAACGGTCGCTCCGAATTTCTCGAGTTAATTA
AGATTACGCCAAGCTACGGGCGGAGTACTGTCCTCCGAGCGGAGTACTGTCCTCCGAG
CGGAGTACTGTCCTCCGAGCGGAGTACTGTCCTCCGAGCGGAGTTCTGTCCTCCGAGC
GGAGACTCTAGACTCCCTATCAGTGATAGAGATAGGCGTGTACGGTGGGAGGCCTATAT
AAGCAGAGCTCGTTTAGTGAACCGTCAGATCGCTCCCTATCAGTGATAGAGAGAATTC
GACCGCCACCATGTCGGAATTCATGAGACATATTATCTGCCACGGAGGTGTTATTACCG
AAGAAATGGCCGCCAGTCTTTTGGACCAGCTGATCGAAGAGGTACTGGCTGATAATCT
TCCACCTCCTAGCCATTTTGAACCACCTACCCTTCACGAACTGTATGATTTAGACGTGA
CGGCCCCCGAAGATCCCAACGAGGAGGCGGTTTCGCAGATTTTTCCCGACTCTGTAAT
GTTGGCGGTGCAGGAAGGGATTGACTTACTCACTTTTCCGCCGGCGCCCGGTTCTCCG
GAGCCGCCTCACCTTTCCCGGCAGCCCGAGCAGCCGGAGCAGAGAGCCTTGGGTCCG
GTTTCTATGCCAAACCTTGTACCGGAGGTGATCGATCTTACCTGCCACGAGGCTGGCTT
TCCACCCAGTGACGACGAGGATGAAGAGGGTGAGGAGTTTGTGTTAGATTATGTGGAG
CACCCCGGGCACGGTTGCAGGTCTTGTCATTATCACCGGAGGAATACGGGGGACCCAG
ATATTATGTGTTCGCTTTGCTATATGAGGACCTGTGGCATGTTTGTCTACAGTAAGTGAA
AATTATGGGCAGTGGGTGATAGAGTGGTGGGTTTGGTGTGGTAATTTTTTTTTAATTTT
TACAGTTTTGTGGTTTAAAGAATTTTGTATTGTGATTTTTTTAAAAGGTCCTGTGTCTGA
ACCTGAGCCTGAGCCCGAGCCAGAACCGGAGCCTGCAAGACCTACCCGCCGTCCTAA
AATGGCGCCTGCTATCCTGAGACGCCCGACATCACCTGTGTCTAGAGAATGCAATAGTA
GTACGGATAGCTGTGACTCCGGTCCTTCTAACACACCTCCTGAGATACACCCGGTGGT
CCCGCTGTGCCCCATTAAACCAGTTGCCGTGAGAGTTGGTGGGCGTCGCCAGGCTGTG
GAATGTATCGAGGACTTGCTTAACGAGCCTGGGCAACCTTTGGACTTGAGCTGTAAAC
GCCCCAGGCCACTCGAGGAGGGCCGCGGCAGCCTGCTGACCTGCGGCGACGTGGAG
GAAAACCCCGGCCCCACCGGTCACCATGGATGTACAGGATGCAACTCCTGTCTTGCAT
TGCACTAAGTCTTGCACTTGTCACAAACAGTGCACCTACTTCAAGTTCTACAAAGAAA
ACACAGCTACAACTGGAGCATTTACTGCTGGATTTACAGATGATTTTGAATGGAATTAA
TAATTACAAGAATCCCAAACTCACCGCGATGCTCACAGCTAAGTTTGCCATGCCCAAG
AAGGCCACAGAACTGAAACATCTTCAGTGTCTAGAAGAAGCACTCAAACCTCTGGAG
GAAGTGCTAAATTTAGCTCAAAGCAAAAACTTTCACTTAAGACCCAGGGACTTAATCA
GCAATATCAACGTAATAGTTCTGGAACTAAAGGGATCTGAAACAACATTCATGTGTGAA
 
TATGCTGATGAGACAGCAACCATTGTAGAATTTCTGAACAGATGGATTACCTTTTGTCA
AAGCATCATCTCAACACTGACTTGAAGATCTGAGGGCCGCGGCAGCCTGCTGACCTGC
GGCGACGTGGAGGAAAACCCCGGCCCCGGATCCATGAAACCAGTAACGTTATACGATG
TCGCAGAGTATGCCGGTGTCTCTTATCAGACCGTTTCCCGCGTGGTGAACCAGGCCAG
CCACGTTTCTGCGAAAACGCGGGAAAAAGTGGAAGCGGCGATGGCGGAGCTGAATTA
CATTCCCAACCGCGTGGCACAACAACTGGCGGGCAAACAGTCGTTGCTGATTGGCGTT
GCCACCTCCAGTCTGGCCCTGCACGCGCCGTCGCAAATTGTCGCGGCGATTAAATCTC
GCGCCGATCAACTGGGTGCCAGCGTGGTGGTGTCGATGGTAGAACGAAGCGGCGTCG
AAGCCTGTAAAGCGGCGGTGCACAATCTTCTCGCGCAACGCGTCAGTGGGCTGATCAT
TAACTATCCGCTGGATGACCAGGATGCCATTGCTGTGGAAGCTGCCTGCACTAATGTTC
CGGCGTTATTTCTTGATGTCTCTGACCAGACACCCATCAACAGTATTATTTTCTCCCATG
AAGACGGTACGCGACTGGGCGTGGAGCATCTGGTCGCATTGGGTCACCAGCAAATCG
CGCTGTTAGCGGGCCCATTAAGTTCTGTCTCGGCGCGTCTGCGTCTGGCTGGCTGGCAT
AAATATCTCACTCGCAATCAAATTCAGCCGATAGCGGAACGGGAAGGCGACTGGAGTG
CCATGTCCGGTTTTCAACAAACCATGCAAATGCTGAATGAGGGCATCGTTCCCACTGC
GATGCTGGTTGCCAACGATCAGATGGCGCTGGGCGCAATGCGCGCCATTACCGAGTCC
GGGCTGCGCGTTGGTGCGGATATCTCGGTAGTGGGATACGACGATACCGAAGACAGCT
CATGTTATATCCCGCCGTTAACCACCATCAAACAGGATTTTCGCCTGCTGGGGCAAACC
AGCGTGGACCGCTTGCTGCAACTCTCTCAGGGCCAGGCGGTGAAGGGCAATCAGCTG
TTGCCCGTCTCACTGGTGAAAAGAAAAACCACCCTGGCGCCCAATACGCAAACCGCC
TCTCCCCGCGCGTTGGCCGATTCATTAATGCAGCTGGCACGACAGGTTTCCCGACTGG
AAAGCGGGCAGAGCAGCCTGAGGCCTCCTAAGAAGAAGAGGAAGGTTGCGGCCGCT
AACCAATGTGCAGACTACTGTTAACCAATGTGCAGACTACTGTGAATTCTAACCAATGT
GCAGACTACTGTTAACCAATGTGCAGACTACTGTAAGCTTCGAATTGAGTAGTGCTTTC
TACTTTATGAGTAGTGCTTTCTACTTTATGAGTAGTGCTTTCTACTTTATGAGTAGTGCTT
TCTACTTTATGGTCGACAATACTAGTTAAGAAATGAGACC (SEQ ID NO:3).
GGTCTCTCTATTTAATTAAGTAACTATAACGGTCGCTCCGAATTTCTCGAGTTAATTA
AGATTACGCCAAGCTACGGGCGGAGTACTGTCCTCCGAGCGGAGTACTGTCCTCCGAG
CGGAGTACTGTCCTCCGAGCGGAGTACTGTCCTCCGAGCGGAGTTCTGTCCTCCGAGC
GGAGACTCTAGACTCCCTATCAGTGATAGAGATAGGCGTGTACGGTGGGAGGCCTATAT
AAGCAGAGCTCGTTTAGTGAACCGTCAGATCGCTCCCTATCAGTGATAGAGAGAATTC
GACCGCCACCATGTCGGAATTCATGAGACATATTATCTGCCACGGAGGTGTTATTACCG
 
AAGAAATGGCCGCCAGTCTTTTGGACCAGCTGATCGAAGAGGTACTGGCTGATAATCT
TCCACCTCCTAGCCATTTTGAACCACCTACCCTTCACGAACTGTATGATTTAGACGTGA
CGGCCCCCGAAGATCCCAACGAGGAGGCGGTTTCGCAGATTTTTCCCGACTCTGTAAT
GTTGGCGGTGCAGGAAGGGATTGACTTACTCACTTTTCCGCCGGCGCCCGGTTCTCCG
GAGCCGCCTCACCTTTCCCGGCAGCCCGAGCAGCCGGAGCAGAGAGCCTTGGGTCCG
GTTTCTATGCCAAACCTTGTACCGGAGGTGATCGATCTTACCTGCCACGAGGCTGGCTT
TCCACCCAGTGACGACGAGGATGAAGAGGGTGAGGAGTTTGTGTTAGATTATGTGGAG
CACCCCGGGCACGGTTGCAGGTCTTGTCATTATCACCGGAGGAATACGGGGGACCCAG
ATATTATGTGTTCGCTTTGCTATATGAGGACCTGTGGCATGTTTGTCTACAGTAAGTGAA
AATTATGGGCAGTGGGTGATAGAGTGGTGGGTTTGGTGTGGTAATTTTTTTTTAATTTT
TACAGTTTTGTGGTTTAAAGAATTTTGTATTGTGATTTTTTTAAAAGGTCCTGTGTCTGA
ACCTGAGCCTGAGCCCGAGCCAGAACCGGAGCCTGCAAGACCTACCCGCCGTCCTAA
AATGGCGCCTGCTATCCTGAGACGCCCGACATCACCTGTGTCTAGAGAATGCAATAGTA
GTACGGATAGCTGTGACTCCGGTCCTTCTAACACACCTCCTGAGATACACCCGGTGGT
CCCGCTGTGCCCCATTAAACCAGTTGCCGTGAGAGTTGGTGGGCGTCGCCAGGCTGTG
GAATGTATCGAGGACTTGCTTAACGAGCCTGGGCAACCTTTGGACTTGAGCTGTAAAC
GCCCCAGGCCACTCGAGGGTACCGGGTCCGGAGCTACAAATTTTCCCTCCTCAAACA
GGCTGGAGATGTCGAAGAAAATCCTGGGCCTACCGGTCACCATGGATGTGGCTGCAGA
GCCTGCTGCTCTTGGGCACTGTGGCCTGCAGCATCTCTGCACCCGCCCGCTCGCCCAG
CCCCAGCACGCAGCCCTGGGAGCATGTGAATGCCATCCAGGAGGCCCGGCGTCTCCTG
AACCTGAGTAGAGACACTGCTGCTGAGATGAATGAAACAGTAGAAGTCATCTCAGAA
ATGTTTGACCTCCAGGAGCCGACCTGCCTACAGACCCGCCTGGAGCTGTACAAGCAG
GGCCTGCGGGGCAGCCTCACCAAGCTCAAGGGCCCCTTGACCATGATGGCCAGCCAC
TACAAGCAGCACTGCCCTCCAACCCCGGAAACTTCCTGTGCAACCCAGATTATCACCT
TTGAAAGTTTCAAAGAGAACCTGAAGGACTTTCTGCTTGTCATCCCCTTTGACTGCTG
GGAGCCAGTCCAGGAGTGAAGATCTGAGGGCCGCGGCAGCCTGCTGACCTGCGGCGA
CGTGGAGGAAAACCCCGGCCCCGGATCCATGAAACCAGTAACGTTATACGATGTCGCA
GAGTATGCCGGTGTCTCTTATCAGACCGTTTCCCGCGTGGTGAACCAGGCCAGCCACG
TTTCTGCGAAAACGCGGGAAAAAGTGGAAGCGGCGATGGCGGAGCTGAATTACATTC
CCAACCGCGTGGCACAACAACTGGCGGGCAAACAGTCGTTGCTGATTGGCGTTGCCA
CCTCCAGTCTGGCCCTGCACGCGCCGTCGCAAATTGTCGCGGCGATTAAATCTCGCGC
CGATCAACTGGGTGCCAGCGTGGTGGTGTCGATGGTAGAACGAAGCGGCGTCGAAGC
 
CTGTAAAGCGGCGGTGCACAATCTTCTCGCGCAACGCGTCAGTGGGCTGATCATTAAC
TATCCGCTGGATGACCAGGATGCCATTGCTGTGGAAGCTGCCTGCACTAATGTTCCGGC
GTTATTTCTTGATGTCTCTGACCAGACACCCATCAACAGTATTATTTTCTCCCATGAAGA
CGGTACGCGACTGGGCGTGGAGCATCTGGTCGCATTGGGTCACCAGCAAATCGCGCTG
TTAGCGGGCCCATTAAGTTCTGTCTCGGCGCGTCTGCGTCTGGCTGGCTGGCATAAATA
TCTCACTCGCAATCAAATTCAGCCGATAGCGGAACGGGAAGGCGACTGGAGTGCCATG
TCCGGTTTTCAACAAACCATGCAAATGCTGAATGAGGGCATCGTTCCCACTGCGATGC
TGGTTGCCAACGATCAGATGGCGCTGGGCGCAATGCGCGCCATTACCGAGTCCGGGCT
GCGCGTTGGTGCGGATATCTCGGTAGTGGGATACGACGATACCGAAGACAGCTCATGTT
ATATCCCGCCGTTAACCACCATCAAACAGGATTTTCGCCTGCTGGGGCAAACCAGCGT
GGACCGCTTGCTGCAACTCTCTCAGGGCCAGGCGGTGAAGGGCAATCAGCTGTTGCC
CGTCTCACTGGTGAAAAGAAAAACCACCCTGGCGCCCAATACGCAAACCGCCTCTCC
CCGCGCGTTGGCCGATTCATTAATGCAGCTGGCACGACAGGTTTCCCGACTGGAAAGC
GGGCAGAGCAGCCTGAGGCCTCCTAAGAAGAGGAAGGTTGCGGCCGCTAACCA
ATGTGCAGACTACTGTTAACCAATGTGCAGACTACTGTGAATTCTAACCAATGTGCAGA
CTACTGTTAACCAATGTGCAGACTACTGTAAGCTTCGAATTGAGTAGTGCTTTCTACTTT
ATGAGTAGTGCTTTCTACTTTATGAGTAGTGCTTTCTACTTTATGAGTAGTGCTTTCTAC
TTTATGGTCGACAATACTAGTTAAGAAATGAGACC (SEQ ID NO:4).
GGTCTCTCTATTTAATTAAGTAACTATAACGGTCGCTCCGAATTTCTCGAGTTAATTA
AGATTACGCCAAGCTACGGGCGGAGTACTGTCCTCCGAGCGGAGTACTGTCCTCCGAG
CGGAGTACTGTCCTCCGAGCGGAGTACTGTCCTCCGAGCGGAGTTCTGTCCTCCGAGC
GGAGACTCTAGACTCCCTATCAGTGATAGAGATAGGCGTGTACGGTGGGAGGCCTATAT
AAGCAGAGCTCGTTTAGTGAACCGTCAGATCGCTCCCTATCAGTGATAGAGAGAATTC
GACCGCCACCATGTCGGAATTCATGAGACATATTATCTGCCACGGAGGTGTTATTACCG
AAGAAATGGCCGCCAGTCTTTTGGACCAGCTGATCGAAGAGGTACTGGCTGATAATCT
TCCACCTCCTAGCCATTTTGAACCACCTACCCTTCACGAACTGTATGATTTAGACGTGA
CGGCCCCCGAAGATCCCAACGAGGAGGCGGTTTCGCAGATTTTTCCCGACTCTGTAAT
GTTGGCGGTGCAGGAAGGGATTGACTTACTCACTTTTCCGCCGGCGCCCGGTTCTCCG
GAGCCGCCTCACCTTTCCCGGCAGCCCGAGCAGCCGGAGCAGAGAGCCTTGGGTCCG
GTTTCTATGCCAAACCTTGTACCGGAGGTGATCGATCTTACCTGCCACGAGGCTGGCTT
TCCACCCAGTGACGACGAGGATGAAGAGGGTGAGGAGTTTGTGTTAGATTATGTGGAG
CACCCCGGGCACGGTTGCAGGTCTTGTCATTATCACCGGAGGAATACGGGGGACCCAG
 
ATATTATGTGTTCGCTTTGCTATATGAGGACCTGTGGCATGTTTGTCTACAGTAAGTGAA
AATTATGGGCAGTGGGTGATAGAGTGGTGGGTTTGGTGTGGTAATTTTTTTTTAATTTT
TACAGTTTTGTGGTTTAAAGAATTTTGTATTGTGATTTTTTTAAAAGGTCCTGTGTCTGA
ACCTGAGCCTGAGCCCGAGCCAGAACCGGAGCCTGCAAGACCTACCCGCCGTCCTAA
AATGGCGCCTGCTATCCTGAGACGCCCGACATCACCTGTGTCTAGAGAATGCAATAGTA
GTACGGATAGCTGTGACTCCGGTCCTTCTAACACACCTCCTGAGATACACCCGGTGGT
CCCGCTGTGCCCCATTAAACCAGTTGCCGTGAGAGTTGGTGGGCGTCGCCAGGCTGTG
GAATGTATCGAGGACTTGCTTAACGAGCCTGGGCAACCTTTGGACTTGAGCTGTAAAC
GCCCCAGGCCACTCGAGGGTACCGGGTCCGGAGCTACAAATTTTCCCTCCTCAAACA
GGCTGGAGATGTCGAAGAAAATCCTGGGCCTACCGGTCACCATGGATGTGGCTGCAGA
ATTTACTTTTCCTGGGCATTGTGGTCTACAGCCTCTCAGCACCCACCCGCTCACCCATC
ACTGTCACCCGGCCTTGGAAGCATGTAGAGGCCATCAAAGAAGCCCTGAACCTCCTG
GATGACATGCCTGTCACGTTGAATGAAGAGGTAGAAGTCGTCTCTAACGAGTTCTCCT
TCAAGAAGCTAACATGTGTGCAGACCCGCCTGAAGATATTCGAGCAGGGTCTACGGGG
CAATTTCACCAAACTCAAGGGCGCCTTGAACATGACAGCCAGCTACTACCAGACATAC
TGCCCCCCAACTCCGGAAACGGACTGTGAAACACAAGTTACCACCTATGCGGATTTCA
TAGACAGCCTTAAAACCTTTCTGACTGATATCCCCTTTGAATGCAAAAAACCAGGCCA
AAAATGAAGATCTGAGGGCCGCGGCAGCCTGCTGACCTGCGGCGACGTGGAGGAAAA
CCCCGGCCCCGGATCCATGAAACCAGTAACGTTATACGATGTCGCAGAGTATGCCGGT
GTCTCTTATCAGACCGTTTCCCGCGTGGTGAACCAGGCCAGCCACGTTTCTGCGAAAA
CGCGGGAAAAAGTGGAAGCGGCGATGGCGGAGCTGAATTACATTCCCAACCGCGTGG
CACAACAACTGGCGGGCAAACAGTCGTTGCTGATTGGCGTTGCCACCTCCAGTCTGGC
CCTGCACGCGCCGTCGCAAATTGTCGCGGCGATTAAATCTCGCGCCGATCAACTGGGT
GCCAGCGTGGTGGTGTCGATGGTAGAACGAAGCGGCGTCGAAGCCTGTAAAGCGGCG
GTGCACAATCTTCTCGCGCAACGCGTCAGTGGGCTGATCATTAACTATCCGCTGGATGA
CCAGGATGCCATTGCTGTGGAAGCTGCCTGCACTAATGTTCCGGCGTTATTTCTTGATG
TCTCTGACCAGACACCCATCAACAGTATTATTTTCTCCCATGAAGACGGTACGCGACTG
GGCGTGGAGCATCTGGTCGCATTGGGTCACCAGCAAATCGCGCTGTTAGCGGGCCCAT
TAAGTTCTGTCTCGGCGCGTCTGCGTCTGGCTGGCTGGCATAAATATCTCACTCGCAAT
CAAATTCAGCCGATAGCGGAACGGGAAGGCGACTGGAGTGCCATGTCCGGTTTTCAA
CAAACCATGCAAATGCTGAATGAGGGCATCGTTCCCACTGCGATGCTGGTTGCCAACG
ATCAGATGGCGCTGGGCGCAATGCGCGCCATTACCGAGTCCGGGCTGCGCGTTGGTGC
 
GGATATCTCGGTAGTGGGATACGACGATACCGAAGACAGCTCATGTTATATCCCGCCGT
TAACCACCATCAAACAGGATTTTCGCCTGCTGGGGCAAACCAGCGTGGACCGCTTGCT
GCAACTCTCTCAGGGCCAGGCGGTGAAGGGCAATCAGCTGTTGCCCGTCTCACTGGT
GAAAAGAAAAACCACCCTGGCGCCCAATACGCAAACCGCCTCTCCCCGCGCGTTGGC
CGATTCATTAATGCAGCTGGCACGACAGGTTTCCCGACTGGAAAGCGGGCAGAGCAG
CCTGAGGCCTCCTAAGAAGAAGAGGAAGGTTGCGGCCGCTAACCAATGTGCAGACTA
CTGTTAACCAATGTGCAGACTACTGTGAATTCTAACCAATGTGCAGACTACTGTTAACC
AATGTGCAGACTACTGTAAGCTTCGAATTGAGTAGTGCTTTCTACTTTATGAGTAGTGC
TTTCTACTTTATGAGTAGTGCTTTCTACTTTATGAGTAGTGCTTTCTACTTTATGGTCGAC
AATACTAGTTAAGAAATGAGACC (SEQ ID NO:5).
GGTCTCTCTATTTAATTAAGTAACTATAACGGTCGCTCCGAATTTCTCGAGTTAATTA
AGATTACGCCAAGCTACGGGCGGAGTACTGTCCTCCGAGCGGAGTACTGTCCTCCGAG
CGGAGTACTGTCCTCCGAGCGGAGTACTGTCCTCCGAGCGGAGTTCTGTCCTCCGAGC
GGAGACTCTAGACTCCCTATCAGTGATAGAGATAGGCGTGTACGGTGGGAGGCCTATAT
AAGCAGAGCTCGTTTAGTGAACCGTCAGATCGCTCCCTATCAGTGATAGAGAGAATTC
GACCGCCACCATGTCGGAATTCATGAGACATATTATCTGCCACGGAGGTGTTATTACCG
AAGAAATGGCCGCCAGTCTTTTGGACCAGCTGATCGAAGAGGTACTGGCTGATAATCT
TCCACCTCCTAGCCATTTTGAACCACCTACCCTTCACGAACTGTATGATTTAGACGTGA
CGGCCCCCGAAGATCCCAACGAGGAGGCGGTTTCGCAGATTTTTCCCGACTCTGTAAT
GTTGGCGGTGCAGGAAGGGATTGACTTACTCACTTTTCCGCCGGCGCCCGGTTCTCCG
GAGCCGCCTCACCTTTCCCGGCAGCCCGAGCAGCCGGAGCAGAGAGCCTTGGGTCCG
GTTTCTATGCCAAACCTTGTACCGGAGGTGATCGATCTTACCTGCCACGAGGCTGGCTT
TCCACCCAGTGACGACGAGGATGAAGAGGGTGAGGAGTTTGTGTTAGATTATGTGGAG
CACCCCGGGCACGGTTGCAGGTCTTGTCATTATCACCGGAGGAATACGGGGGACCCAG
ATATTATGTGTTCGCTTTGCTATATGAGGACCTGTGGCATGTTTGTCTACAGTAAGTGAA
AATTATGGGCAGTGGGTGATAGAGTGGTGGGTTTGGTGTGGTAATTTTTTTTTAATTTT
TACAGTTTTGTGGTTTAAAGAATTTTGTATTGTGATTTTTTTAAAAGGTCCTGTGTCTGA
ACCTGAGCCTGAGCCCGAGCCAGAACCGGAGCCTGCAAGACCTACCCGCCGTCCTAA
AATGGCGCCTGCTATCCTGAGACGCCCGACATCACCTGTGTCTAGAGAATGCAATAGTA
GTACGGATAGCTGTGACTCCGGTCCTTCTAACACACCTCCTGAGATACACCCGGTGGT
CCCGCTGTGCCCCATTAAACCAGTTGCCGTGAGAGTTGGTGGGCGTCGCCAGGCTGTG
GAATGTATCGAGGACTTGCTTAACGAGCCTGGGCAACCTTTGGACTTGAGCTGTAAAC
 
GCCCCAGGCCACTCGAGGGTACCGGGTCCGGAGCTACAAATTTTTCCCTCCTCAAACA
GGCTGGAGATGTCGAAGAAAATCCTGGGCCTACCGGTCCCATGAGTGTGCCCACTCAG
GTCCTGGGGTTGCTGCTGCTGTGGCTTACAGACGCTCGCTGCCAGGTGCAGCTGGTGG
AGAGCGGCGGCGGCGTGGTGCAGCCCGGCAGGAGCCTGAGGCTGGACTGCAAGGCC
AGCGGCATCACCTTCAGCAACAGCGGCATGCACTGGGTGAGGCAGGCCCCCGGCAAG
GGCCTGGAGTGGGTGGCCGTGATCTGGTACGACGGCAGCAAGAGGTACTACGCCGAC
AGCGTGAAGGGCAGGTTCACCATCAGCAGGGACAACAGCAAGAACACCCTGTTCCTG
CAGATGAACAGCCTGAGGGCCGAGGACACCGCCGTGTACTACTGCGCCACCAACGAC
GACTACTGGGGCCAGGGCACCCTGGTGACCGTGAGCAGCGGTGGAGGCGGTTCAGGC
GGAGGTGGCTCTGGCGGTGGCGGATCGGAGATCGTGCTGACCCAGAGCCCCGCCACC
CTGAGCCTGAGCCCCGGCGAGAGGGCCACCCTGAGCTGCAGGGCCAGCCAGAGCGT
GAGCAGCTACCTGGCCTGGTACCAGCAGAAGCCCGGCCAGGCCCCCAGGCTGCTGAT
CTACGACGCCAGCAACAGGGCCACCGGCATCCCCGCCAGGTTCAGCGGCAGCGGCAG
CGGCACCGACTTCACCCTGACCATCAGCAGCCTGGAGCCCGAGGACTTCGCCGTGTAC
TACTGCCAGCAGAGCAGCAACTGGCCCAGGACCTTCGGCCAGGGCACCAAGGTGGAG
ATCAAGAGATCTGAGGGCCGCGGCAGCCTGCTGACCTGCGGCGACGTGGAGGAAAAC
CCCGGCCCCGGATCCATGAAACCAGTAACGTTATACGATGTCGCAGAGTATGCCGGTG
TCTCTTATCAGACCGTTTCCCGCGTGGTGAACCAGGCCAGCCACGTTTCTGCGAAAAC
GCGGGAAAAAGTGGAAGCGGCGATGGCGGAGCTGAATTACATTCCCAACCGCGTGGC
ACAACAACTGGCGGGCAAACAGTCGTTGCTGATTGGCGTTGCCACCTCCAGTCTGGCC
CTGCACGCGCCGTCGCAAATTGTCGCGGCGATTAAATCTCGCGCCGATCAACTGGGTG
CCAGCGTGGTGGTGTCGATGGTAGAACGAAGCGGCGTCGAAGCCTGTAAAGCGGCGG
TGCACAATCTTCTCGCGCAACGCGTCAGTGGGCTGATCATTAACTATCCGCTGGATGAC
CAGGATGCCATTGCTGTGGAAGCTGCCTGCACTAATGTTCCGGCGTTATTTCTTGATGT
CTCTGACCAGACACCCATCAACAGTATTATTTTCTCCCATGAAGACGGTACGCGACTGG
GCGTGGAGCATCTGGTCGCATTGGGTCACCAGCAAATCGCGCTGTTAGCGGGCCCATT
AAGTTCTGTCTCGGCGCGTCTGCGTCTGGCTGGCTGGCATAAATATCTCACTCGCAATC
AAATTCAGCCGATAGCGGAACGGGAAGGCGACTGGAGTGCCATGTCCGGTTTTCAAC
AAACCATGCAAATGCTGAATGAGGGCATCGTTCCCACTGCGATGCTGGTTGCCAACGA
TCAGATGGCGCTGGGCGCAATGCGCGCCATTACCGAGTCCGGGCTGCGCGTTGGTGCG
GATATCTCGGTAGTGGGATACGACGATACCGAAGACAGCTCATGTTATATCCCGCCGTT
AACCACCATCAAACAGGATTTTCGCCTGCTGGGGCAAACCAGCGTGGACCGCTTGCTG
 
CAACTCTCTCAGGGCCAGGCGGTGAAGGGCAATCAGCTGTTGCCCGTCTCACTGGTG
AAAAGAAAAACCACCCTGGCGCCCAATACGCAAACCGCCTCTCCCCGCGCGTTGGCC
GATTCATTAATGCAGCTGGCACGACAGGTTTCCCGACTGGAAAGCGGGCAGAGCAGC
CTGAGGCCTCCTAAGAAGAGGAAGGTTGCGGCCGCTAACCAATGTGCAGACTAC
TGTTAACCAATGTGCAGACTACTGTGAATTCTAACCAATGTGCAGACTACTGTTAACCA
ATGTGCAGACTACTGTAAGCTTCGAATTGAGTAGTGCTTTCTACTTTATGAGTAGTGCT
TTCTACTTTATGAGTAGTGCTTTCTACTTTATGAGTAGTGCTTTCTACTTTATGGTCGACA
ATACTAGTTAAGAAATGAGACC (SEQ ID NO:6).
GGTCTCTCTATTTAATTAAGTAACTATAACGGTCGCTCCGAATTTCTCGAGTTAATTA
AGATTACGCCAAGCTACGGGCGGAGTACTGTCCTCCGAGCGGAGTACTGTCCTCCGAG
CGGAGTACTGTCCTCCGAGCGGAGTACTGTCCTCCGAGCGGAGTTCTGTCCTCCGAGC
GGAGACTCTAGACTCCCTATCAGTGATAGAGATAGGCGTGTACGGTGGGAGGCCTATAT
AAGCAGAGCTCGTTTAGTGAACCGTCAGATCGCTCCCTATCAGTGATAGAGAGAATTC
GACCGCCACCATGTCGGAATTCATGAGACATATTATCTGCCACGGAGGTGTTATTACCG
AAGAAATGGCCGCCAGTCTTTTGGACCAGCTGATCGAAGAGGTACTGGCTGATAATCT
TCCACCTCCTAGCCATTTTGAACCACCTACCCTTCACGAACTGTATGATTTAGACGTGA
CGGCCCCCGAAGATCCCAACGAGGAGGCGGTTTCGCAGATTTTTCCCGACTCTGTAAT
GTTGGCGGTGCAGGAAGGGATTGACTTACTCACTTTTCCGCCGGCGCCCGGTTCTCCG
GAGCCGCCTCACCTTTCCCGGCAGCCCGAGCAGCCGGAGCAGAGAGCCTTGGGTCCG
GTTTCTATGCCAAACCTTGTACCGGAGGTGATCGATCTTACCTGCCACGAGGCTGGCTT
TCCACCCAGTGACGACGAGGATGAAGAGGGTGAGGAGTTTGTGTTAGATTATGTGGAG
CACCCCGGGCACGGTTGCAGGTCTTGTCATTATCACCGGAGGAATACGGGGGACCCAG
ATATTATGTGTTCGCTTTGCTATATGAGGACCTGTGGCATGTTTGTCTACAGTAAGTGAA
AATTATGGGCAGTGGGTGATAGAGTGGTGGGTTTGGTGTGGTAATTTTTTTTTAATTTT
TACAGTTTTGTGGTTTAAAGAATTTTGTATTGTGATTTTTTTAAAAGGTCCTGTGTCTGA
ACCTGAGCCTGAGCCCGAGCCAGAACCGGAGCCTGCAAGACCTACCCGCCGTCCTAA
AATGGCGCCTGCTATCCTGAGACGCCCGACATCACCTGTGTCTAGAGAATGCAATAGTA
GTACGGATAGCTGTGACTCCGGTCCTTCTAACACACCTCCTGAGATACACCCGGTGGT
CCCGCTGTGCCCCATTAAACCAGTTGCCGTGAGAGTTGGTGGGCGTCGCCAGGCTGTG
GAATGTATCGAGGACTTGCTTAACGAGCCTGGGCAACCTTTGGACTTGAGCTGTAAAC
GCCCCAGGCCACTCGAGGGTACCGGGTCCGGAGCTACAAATTTTTCCCTCCTCAAACA
GGCTGGAGATGTCGAAGAAAATCCTGGGCCTACCGGTCCCATGAGTGTGCCCACTCAG
 
GTCCTGGGGTTGCTGCTGCTGTGGCTTACAGACGCTCGCTGCGAGGTGCAGCTGGTGG
AGAGCGGCGGCGGCCTGGTGCAGCCCGGCGGCAGCCTGAGGCTGAGCTGCGCCGCC
AGCGGCTTCACCTTCAGCGACAGCTGGATCCACTGGGTGAGGCAGGCCCCCGGCAAG
GGCCTGGAGTGGGTGGCCTGGATCAGCCCCTACGGCGGCAGCACCTACTACGCCGAC
AGCGTGAAGGGCAGGTTCACCATCAGCGCCGACACCAGCAAGAACACCGCCTACCTG
CAGATGAACAGCCTGAGGGCCGAGGACACCGCCGTGTACTACTGCGCCAGGAGGCAC
TGGCCCGGCGGCTTCGACTACTGGGGCCAGGGCACCCTGGTGACCGTGAGCAGCGGT
GGAGGCGGTTCAGGCGGAGGTGGCTCTGGCGGTGGCGGATCGGACATCCAGATGACC
CAGAGCCCCAGCAGCCTGAGCGCCAGCGTGGGCGACAGGGTGACCATCACCTGCAGG
GCCAGCCAGGACGTGAGCACCGCCGTGGCCTGGTACCAGCAGAAGCCCGGCAAGGC
CCCCAAGCTGCTGATCTACAGCGCCAGCTTCCTGTACAGCGGCGTGCCCAGCAGGTTC
AGCGGCAGCGGCAGCGGCACCGACTTCACCCTGACCATCAGCAGCCTGCAGCCCGAG
GACTTCGCCACCTACTACTGCCAGCAGTACCTGTACCACCCCGCCACCTTCGGCCAGG
GCACCAAGGTGGAGATCAAGAGATCTGAGGGCCGCGGCAGCCTGCTGACCTGCGGCG
ACGTGGAGGAAAACCCCGGCCCCGGATCCATGAAACCAGTAACGTTATACGATGTCGC
AGAGTATGCCGGTGTCTCTTATCAGACCGTTTCCCGCGTGGTGAACCAGGCCAGCCAC
GTTTCTGCGAAAACGCGGGAAAAAGTGGAAGCGGCGATGGCGGAGCTGAATTACATT
CCCAACCGCGTGGCACAACAACTGGCGGGCAAACAGTCGTTGCTGATTGGCGTTGCC
ACCTCCAGTCTGGCCCTGCACGCGCCGTCGCAAATTGTCGCGGCGATTAAATCTCGCG
CCGATCAACTGGGTGCCAGCGTGGTGGTGTCGATGGTAGAACGAAGCGGCGTCGAAG
CCTGTAAAGCGGCGGTGCACAATCTTCTCGCGCAACGCGTCAGTGGGCTGATCATTAA
CTATCCGCTGGATGACCAGGATGCCATTGCTGTGGAAGCTGCCTGCACTAATGTTCCGG
CGTTATTTCTTGATGTCTCTGACCAGACACCCATCAACAGTATTATTTTCTCCCATGAAG
ACGGTACGCGACTGGGCGTGGAGCATCTGGTCGCATTGGGTCACCAGCAAATCGCGCT
GTTAGCGGGCCCATTAAGTTCTGTCTCGGCGCGTCTGCGTCTGGCTGGCTGGCATAAAT
ATCTCACTCGCAATCAAATTCAGCCGATAGCGGAACGGGAAGGCGACTGGAGTGCCAT
GTCCGGTTTTCAACAAACCATGCAAATGCTGAATGAGGGCATCGTTCCCACTGCGATG
CTGGTTGCCAACGATCAGATGGCGCTGGGCGCAATGCGCGCCATTACCGAGTCCGGGC
TGCGCGTTGGTGCGGATATCTCGGTAGTGGGATACGACGATACCGAAGACAGCTCATG
TTATATCCCGCCGTTAACCACCATCAAACAGGATTTTCGCCTGCTGGGGCAAACCAGCG
TGGACCGCTTGCTGCAACTCTCTCAGGGCCAGGCGGTGAAGGGCAATCAGCTGTTGC
CCGTCTCACTGGTGAAAAGAAAAACCACCCTGGCGCCCAATACGCAAACCGCCTCTC
 
CCCGCGCGTTGGCCGATTCATTAATGCAGCTGGCACGACAGGTTTCCCGACTGGAAAG
CGGGCAGAGCAGCCTGAGGCCTCCTAAGAAGAGGAAGGTTGCGGCCGCTAACC
AATGTGCAGACTACTGTTAACCAATGTGCAGACTACTGTGAATTCTAACCAATGTGCAG
ACTACTGTTAACCAATGTGCAGACTACTGTAAGCTTCGAATTGAGTAGTGCTTTCTACT
TTATGAGTAGTGCTTTCTACTTTATGAGTAGTGCTTTCTACTTTATGAGTAGTGCTTTCTA
CTTTATGGTCGACAATACTAGTTAAGAAATGAGACC (SEQ ID NO:7).
 wherein SEQ ID NO: 2 is the sequence of BsaI-5 ×
UAS-tetO-miniCMV-tetO-E1A-2A-EBFP-2A-LacI-miR199a target site-BsaI; SEQ ID NO: 3 is the sequence of BsaI-5 ×
UAS-tetO-miniCMV-tetO-E1A-2A-hIL-2-2A-LacI-miR199a target site-BsaI; SEQ ID NO: 4 is the sequence of BsaI-5 ×
UAS-tetO-miniCMV-tetO-E1A-2A-hGM-CSF-2A-LacI-miR199a target site-BsaI; SEQ ID NO: 5 is the sequence of BsaI-5 ×
UAS-tetO-miniCMV-tetO-E1A-2A-mGM-CSF-2A-LacI-miR199a target site-BsaI; SEQ ID NO: 6 is the sequence of BsaI-5 ×
UAS-tetO-miniCMV-tetO-E1A-2A-anti-PD-1scFv-2A-LacI-miR199a target site-BsaI; and SEQ ID NO: 7 is the sequence of BsaI-5 ×
UAS-tetO-miniCMV-tetO-E1A-2A-anti-PD-L1scFv-2A-LacI-miR199a target site-BsaI.

In a specific embodiment of the present disclosure, the third expression vector comprises: Bsal, 5 × UAS, LacO, miniCMV, LacO, tetR-KRAB, microRNA21 specific recognition sequence (target site) and BsaI from the 5' end to the 3' end (BsaI-5 × UAS-LacO-miniCMV-LacO-tetR-KRAB-microRNA21 target site-BsaI).

In an embodiment of the present disclosure, the third expression vector carries a nucleic acid having the nucleotide sequence set forth in SEQ ID NO: 8.

GGTCTCCGAAATCGCCAAGCTACGGGCGGAGTACTGTCCTCCGAGCGGAGTACTG
TCCTCCGAGCGGAGTACTGTCCTCCGAGCGGAGTACTGTCCTCCGAGCGGAGTACTGT
CCTCCGAGCGGAGACTCTAGAAATTGTGAGCGGATAACAATTAGGCGTGTACGGTGG
GAGGCCTATATAAGCAGAGCTCGTTTAGTGAACCGTCAGATCGCCAGCTGACAAAATT
GTGAGCGCTCACAATTACTAGAAGATCTTGAATTCGCCACCATGGCTAGATTAGATAAA
AGTAAAGTGATTAACAGCGCATTAGAGCTGCTTAATGAGGTCGGAATCGAAGGTTTAA
CAACCCGTAAACTCGCCCAGAAGCTAGGTGTAGAGCAGCCTACATTGTATTGGCATGT
AAAAAATAAGCGGGCTTTGCTCGACGCCTTAGCCATTGAGATGTTAGATAGGCACCATA

CTCACTTTTGCCCTTTAGAAGGGGAAAGCTGGCAAGATTTTTTACGTAATAACGCTAAA
AGTTTTAGATGTGCTTTACTAAGTCATCGCGATGGAGCAAAAGTACATTTAGGTACACG
GCCTACAGAAAAACAGTATGAAACTCTCGAAAATCAATTAGCCTTTTTATGCCAACAA
GGTTTTTCACTAGAGAATGCATTATATGCACTCAGCGCTGTGGGGCATTTTACTTTAGGT
TGCGTATTGGAAGATCAAGAGCATCAAGTCGCTAAAGAAGAAAGGGAAACACCTACT
ACTGATAGTATGCCGCCATTATTACGACAAGCTATCGAATTATTTGATCACCAAGGTGCA
GAGCCAGCCTTCTTATTCGGCCTTGAATTGATCATATGCGGATTAGAAAAACAACTTAA
ATGTGAAAGTGGGTCGCCAAAAAAGAAGAGAAAGGTCGACGGCGGTGGTGCTTTGTC
TCCTCAGCACTCTGCTGTCACTCAAGGAAGTATCATCAAGAACAAGGAGGGCATGGAT
GCTAAGTCACTAACTGCCTGGTCCCGGACACTGGTGACCTTCAAGGATGTATTTGTGG
ACTTCACCAGGGAGGAGTGGAAGCTGCTGGACACTGCTCAGCAGATCGTGTACAGAA
ATGTGATGCTGGAGAACTATAAGAACCTGGTTTCCTTGGGTTATCAGCTTACTAAGCCA
GATGTGATCCTCCGGTTGGAGAAGGGAGAAGAGCCCTGGCTGGTGGAGAGAGAAATT
CACCAAGAGACCCATCCTGATTCAGAGACTGCATTTGAAATCAAATCATCAGTTTAATA
CAAGGCGGCCGCAAATCAACATCAGTCTGATAAGCTATCAACATCAGTCTGATAAGCTA
TCAACATCAGTCTGATAAGCTATCAACATCAGTCTGATAAGCTAAAGCTTCGAATTCTG
ATAATCAGCCATACCACATTTGTAGAGGTTTTACTTGCTTTAAAAAACCTCCCACACCT
CCCCCTGAACCTGAAACATAAAATGAATGCAATTGTTGTTGTTAACTTGTTTATTGCAG
CTTATAATGGTTACAAATAAAGCAATAGCATCACAAATTTCACAAATAAAGCATTTTTTT
CACTGCAGCTCTGAGACC (SEQ ID NO:8).

In a specific embodiment of the present disclosure, the adenovirus comprises: a first inverted terminal repeat sequence (ITR), a packaging signal, AFPIII, Gal4VP16, 5 × UAS, tetO, miniCMV, tetO, E1A, 2A, Effector, 2A, LacI, miR 199a target site, 5 × UAS, LacO, miniCMV, LacO, tetR-KRAB, miR21 target site, an adenovirus E2 gene region, an adenovirus E3 gene region (removal of region 28922-30801), an adenovirus E4 gene region, and a second inverted terminal repeat sequence (ITR) from the 5' end to the 3' end. The above adenovirus when injected into a tumor mouse model by the present inventors can significantly inhibit the growth of tumor in mouse. The above adenovirus can be used as a safe and effective oncolytic virus vaccine to specifically kill related tumors in a safe and effective way.

In an embodiment of the present disclosure, the adenoviral vector carries a nucleic acid having the nucleotide sequences selected from the group consisting of SEQ ID NOs: 9-14. GTAACTATAACGGTCGCTCCGAATTTCTCGAGTTAATTAAGATTACGCCAAGCTACG 
GGCGGAGTACTGTCCTCCGAGCGGAGTACTGTCCTCCGAGCGGAGTACTGTCCTCCGA
GCGGAGTACTGTCCTCCGAGCGGAGTTCTGTCCTCCGAGCGGAGACTCTAGACTCCCT
ATCAGTGATAGAGATAGGCGTGTACGGTGGGAGGCCTATATAAGCAGAGCTCGTTTAGT
GAACCGTCAGATCGCTCCCTATCAGTGATAGAGAATTCGACCGCCACCATGTCGGA
ATTCATGAGACATATTATCTGCCACGGAGGTGTTATTACCGAAGAAATGGCCGCCAGTC
TTTTGGACCAGCTGATCGAAGAGGTACTGGCTGATAATCTTCCACCTCCTAGCCATTTT
GAACCACCTACCCTTCACGAACTGTATGATTTAGACGTGACGGCCCCCGAAGATCCCA
ACGAGGAGGCGGTTTCGCAGATTTTTCCCGACTCTGTAATGTTGGCGGTGCAGGAAGG
GATTGACTTACTCACTTTTCCGCCGGCGCCCGGTTCTCCGGAGCCGCCTCACCTTTCCC
GGCAGCCCGAGCAGCCGGAGCAGAGAGCCTTGGGTCCGGTTTCTATGCCAAACCTTG
TACCGGAGGTGATCGATCTTACCTGCCACGAGGCTGGCTTTCCACCCAGTGACGACGA
GGATGAAGAGGGTGAGGAGTTTGTGTTAGATTATGTGGAGCACCCCGGGCACGGTTGC
AGGTCTTGTCATTATCACCGGAGGAATACGGGGGACCCAGATATTATGTGTTCGCTTTG
CTATATGAGGACCTGTGGCATGTTTGTCTACAGTAAGTGAAAATTATGGGCAGTGGGTG
ATAGAGTGGTGGGTTTGGTGTGGTAATTTTTTTTTAATTTTTACAGTTTTGTGGTTTAA
AGAATTTTGTATTGTGATTTTTTTAAAAGGTCCTGTGTCTGAACCTGAGCCTGAGCCCG
AGCCAGAACCGGAGCCTGCAAGACCTACCCGCCGTCCTAAAATGGCGCCTGCTATCCT
GAGACGCCCGACATCACCTGTGTCTAGAGAATGCAATAGTAGTACGGATAGCTGTGAC
TCCGGTCCTTCTAACACACCTCCTGAGATACACCCGGTGGTCCCGCTGTGCCCCATTAA
ACCAGTTGCCGTGAGAGTTGGTGGGCGTCGCCAGGCTGTGGAATGTATCGAGGACTTG
CTTAACGAGCCTGGGCAACCTTTGGACTTGAGCTGTAAACGCCCCAGGCCACTCGAG
GGTACCGGGTCCGGAGCTACAAATTTTTCCCTCCTCAAACAGGCTGGAGATGTCGAAG
AAAATCCTGGGCCTACCGGTCCCATGGTGAGCAAGGGCGAGGAGCTGTTCACCGGGG
TGGTGCCCATCCTGGTCGAGCTGGACGGCGACGTAAACGGCCACAAGTTCAGCGTGA
GGGGCGAGGGCGAGGGCGATGCCACCAACGGCAAGCTGACCCTGAAGTTCATCTGCA
CCACCGGCAAGCTGCCCGTGCCCTGGCCCACCCTCGTGACCACCCTGAGCCACGGCG
TGCAGTGCTTCGCCCGCTACCCCGACCACATGAAGCAGCACGACTTCTTCAAGTCCGC
CATGCCCGAAGGCTACGTCCAGGAGCGCACCATCTTCTTCAAGGACGACGGCACCTAC
AAGACCCGCGCCGAGGTGAAGTTCGAGGGCGACACCCTAGTGAACCGCATCGAGCTG
AAGGGCGTCGACTTCAAGGAGGACGGCAACATCCTGGGGCACAAGCTGGAGTACAAC
TTCAACAGCCACAACATCTATATCATGGCCGTCAAGCAGAAGAACGGCATCAAGGTGA
ACTTCAAGATCCGCCACAACGTGGAGGACGGCAGCGTGCAGCTCGCCGACCACTACC
 
AGCAGAACACCCCCATCGGCGACGGCCCCGTGCTGCTGCCCGACAGCCACTACCTGA
GCACCCAGTCCGTGCTGAGCAAAGACCCCAACGAGAAGCGCGATCACATGGTCCTGC
TGGAGTTCCGCACCGCCGCCGGGATCACTCTCGGCATGGACGAGCTGTACAAGAGATC
TGAGGGCCGCGGCAGCCTGCTGACCTGCGGCGACGTGGAGGAAAACCCCGGCCCCG
GATCCATGAAACCAGTAACGTTATACGATGTCGCAGAGTATGCCGGTGTCTCTTATCAG
ACCGTTTCCCGCGTGGTGAACCAGGCCAGCCACGTTTCTGCGAAAACGCGGGAAAAA
GTGGAAGCGGCGATGGCGGAGCTGAATTACATTCCCAACCGCGTGGCACAACAACTG
GCGGGCAAACAGTCGTTGCTGATTGGCGTTGCCACCTCCAGTCTGGCCCTGCACGCGC
CGTCGCAAATTGTCGCGGCGATTAAATCTCGCGCCGATCAACTGGGTGCCAGCGTGGT
GGTGTCGATGGTAGAACGAAGCGGCGTCGAAGCCTGTAAAGCGGCGGTGCACAATCT
TCTCGCGCAACGCGTCAGTGGGCTGATCATTAACTATCCGCTGGATGACCAGGATGCCA
TTGCTGTGGAAGCTGCCTGCACTAATGTTCCGGCGTTATTTCTTGATGTCTCTGACCAG
ACACCCATCAACAGTATTATTTTCTCCCATGAAGACGGTACGCGACTGGGCGTGGAGC
ATCTGGTCGCATTGGGTCACCAGCAAATCGCGCTGTTAGCGGGCCCATTAAGTTCTGTC
TCGGCGCGTCTGCGTCTGGCTGGCTGGCATAAATATCTCACTCGCAATCAAATTCAGCC
GATAGCGGAACGGGAAGGCGACTGGAGTGCCATGTCCGGTTTTCAACAAACCATGCA
AATGCTGAATGAGGGCATCGTTCCCACTGCGATGCTGGTTGCCAACGATCAGATGGCG
CTGGGCGCAATGCGCGCCATTACCGAGTCCGGGCTGCGCGTTGGTGCGGATATCTCGG
TAGTGGGATACGACGATACCGAAGACAGCTCATGTTATATCCCGCCGTTAACCACCATC
AAACAGGATTTTCGCCTGCTGGGGCAAACCAGCGTGGACCGCTTGCTGCAACTCTCTC
AGGGCCAGGCGGTGAAGGGCAATCAGCTGTTGCCCGTCTCACTGGTGAAAAGAAAAA
CCACCCTGGCGCCCAATACGCAAACCGCCTCTCCCCGCGCGTTGGCCGATTCATTAATG
CAGCTGGCACGACAGGTTTCCCGACTGGAAAGCGGGCAGAGCAGCCTGAGGCCTCCT
AAGAAGAAGAGGAAGGTTGCGGCCGCTAACCAATGTGCAGACTACTGTTAACCAATG
TGCAGACTACTGTGAATTCTAACCAATGTGCAGACTACTGTTAACCAATGTGCAGACTA
CTGTAAGCTTCGAATTGAGTAGTGCTTTCTACTTTATGAGTAGTGCTTTCTACTTTATGA
GTAGTGCTTTCTACTTTATGAGTAGTGCTTTCTACTTTATGGTCGACAATACTAGTTAAG
AAATCGCCAAGCTACGGGCGGAGTACTGTCCTCCGAGCGGAGTACTGTCCTCCGAGCG
GAGTACTGTCCTCCGAGCGGAGTACTGTCCTCCGAGCGGAGTACTGTCCTCCGAGCGG
AGACTCTAGAAATTGTGAGCGGATAACAATTTAGGCGTGTACGGTGGGAGGCCTATATA
AGCAGAGCTCGTTTAGTGAACCGTCAGATCGCCAGCTGACAAAATTGTGAGCGCTCAC
AATTACTAGAAGATCTTGAATTCGCCACCATGGCTAGATTAGATAAAAGTAAAGTGATT
 
AACAGCGCATTAGAGCTGCTTAATGAGGTCGGAATCGAAGGTTTAACAACCCGTAAAC
TCGCCCAGAAGCTAGGTGTAGAGCAGCCTACATTGTATTGGCATGTAAAAAATAAGCG
GGCTTTGCTCGACGCCTTAGCCATTGAGATGTTAGATAGGCACCATACTCACTTTTGCC
CTTTAGAAGGGGAAAGCTGGCAAGATTTTTTACGTAATAACGCTAAAAGTTTTAGATGT
GCTTTACTAAGTCATCGCGATGGAGCAAAAGTACATTTAGGTACACGGCCTACAGAAA
AACAGTATGAAACTCTCGAAAATCAATTAGCCTTTTTATGCCAACAAGGTTTTTCACTA
GAGAATGCATTATATGCACTCAGCGCTGTGGGGCATTTTACTTTAGGTTGCGTATTGGA
AGATCAAGAGCATCAAGTCGCTAAAGAAGAAAGGGAAACACCTACTACTGATAGTATG
CCGCCATTATTACGACAAGCTATCGAATTATTTGATCACCAAGGTGCAGAGCCAGCCTT
CTTATTCGGCCTTGAATTGATCATATGCGGATTAGAAAAACAACTTAAATGTGAAAGTG
GGTCGCCAAAAAAGAAGAGAAAGGTCGACGGCGGTGGTGCTTTGTCTCCTCAGCACT
CTGCTGTCACTCAAGGAAGTATCATCAAGAACAAGGAGGGCATGGATGCTAAGTCACT
AACTGCCTGGTCCCGGACACTGGTGACCTTCAAGGATGTATTTGTGGACTTCACCAGG
GAGGAGTGGAAGCTGCTGGACACTGCTCAGCAGATCGTGTACAGAAATGTGATGCTG
GAGAACTATAAGAACCTGGTTTCCTTGGGTTATCAGCTTACTAAGCCAGATGTGATCCT
CCGGTTGGAGAAGGGAGAAGAGCCCTGGCTGGTGGAGAGAGAAATTCACCAAGAGA
CCCATCCTGATTCAGAGACTGCATTTGAAATCAAATCATCAGTTTAATACAAGGCGGCC
GCAAATCAACATCAGTCTGATAAGCTATCAACATCAGTCTGATAAGCTATCAACATCAG
TCTGATAAGCTATCAACATCAGTCTGATAAGCTAAAGCTTCGAATTCTGATAATCAGCCA
TACCACATTTGTAGAGGTTTTACTTGCTTTAAAAAACCTCCCACACCTCCCCCTGAACC
TGAAACATAAAATGAATGCAATTGTTGTTGTTAACTTGTTTATTGCAGCTTATAATGGTT
ACAAATAAAGCAATAGCATCACAAATTTCACAAATAAAGCATTTTTTTCACTGCAGCTC
CAGATTGAATTATTTGCCTGTCATACAGCTAATAATTGACCATAAGACAATTAGATTTAA
ATTAGTTTTGAATCTTTCTAATACCAAAGTTCAGTTTACTGTTCCATGTTGCTTCTGAGT
GGCTTCACAGACTTATGAAAAAGTAAACGGAATCAGAATTACATCAATGCAAAAGCAT
TGCTGTGAACTCTGTACTTAGGACTAAACTTTGAGCAATAACACATATAGATTGAGGAT
TGTTTGCTGTTAGTATACAAACTCTGGTTCAAAGCTCCTCTTTATTGCTTGTCTTGGAAA
ATTTGCTGTTCTTCATGGTTTCTCTTTTCACTGCTATCTATTTTTCTCAACCACTCACATG
GCTACAAAAGCTTCCTGATTAATAATTACACTAAGTCAATAGGCATAGAGCCAGGACTG
TTTGGGTAAACTGGTCACTTTATCTTAAACTAAATATATCCAAAACTGAACATGTACTTA
GTTACTAAGTCTTTGACTTTATCTCATTCATACCACTCAGCTTTATCCAGGCCACTTATTT
GACAGTATTATTGCGAAAACTTCCTATCTAGAAGTTTGAGGAGAATATTTGTTATATTTG
 
CAAAATAAAATAAGTTTGCAAGTTTTTTTTTCTGCCCCAAAGAGCTCTGTGTCCTTGA
ACATAAAATACAAATAACCGCTATGCTGTTAATTATTGACAAATGTCCCATTTTCAACCT
AAGGAAATACCATAAAGTAACAGATATACCAACAAAAGGTTACTAGTTAACAGGCATT
GCCTGAAAAGAGTATAAAAGAATTTCAGCATGATTTTCCATATTGTGCTTCCACCACTG
CCAATAACAAAATAACTAGCAAGGATCCGGCCACCATGGCCCCCCCGACCGATGTCAG
CCTGGGGGACGAGCTCCACTTAGACGGCGAGGACGTGGCGATGGCGCATGCCGACGC
GCTAGACGATTTCGATCTGGACATGTTGGGGGACGGGGATTCCCCGGGTCCGGGATTT
ACCCCCCACGACTCCGCCCCCTACGGCGCTCTGGATATGGCCGACTTCGAGTTTGAGC
AGATGTTTACCGATGCCCTTGGAATTGACGAGTACGGTGGGACGCGTATGAAGCTACT
GTCTTCTATCGAACAAGCATGCGATATTTGCCGACTTAAAAAGCTCAAGTGCTCCAAA
GAAAAACCGAAGTGCGCCAAGTGTCTGAAGAACAACTGGGAGTGTCGCTACTCTCCC
AAAACCAAAAGGTCTCCGCTGACTAGGGCACATCTGACAGAAGTGGAATCAAGGCTA
GAAAGACTGGAACAGCTATTTCTACTGATTTTTCCTCGAGAAGACCTTGACATGATTTT
GAAAATGGATTCTTTACAGGATATAAAAGCATTGTTAACAGGATTATTTGTACAAGATAA
TGTGAATAAAGATGCCGTCACAGATAGATTGGCTTCAGTGGAGACTGATATGCCTCTAA
CATTGAGACAGCATAGAATAAGTGCGACATCATCATCGGAAGAGAGTAGTAACAAAGG
TCAAAGACAGTTGACTGTATAATTCACTCCTCAGGTGCAGGCTGCCTATCAGAAGGTG
GTGGCTGGTGTGGCCAATGCCCTGGCTCACAAATACCACTGAGATCTTTTTCCCTCTGC
CAAAAATTATGGGGACATCATGAAGCCCCTTGAGCATCTGACTTCTGGCTAATAAAGG
AAATTTATTTTCATTGCAATAGTGTGTTGGAATTTTTTGTGTCTCTCACTCGGAAGGACA
TATGGGAGGGCAAATCATTTAAAACATCAGAATGAGTATTTGGTTTAGAGTTTGGCAAC
ATATGCCCATATGCTGGCTGCCATGAACAAAGGTTGGCTATAAAGAGGTCATCAGTATA
TGAAACAGCCCCCTGCTGTCCATTCCTTATTCCATAGAAAAGCCTTGACTTGAGGTTAG
ATTTTTTTTATATTTTGTTTTGTGTTATTTTTTTCTTTAACATCCCTAAAATTTTCCTTACA
TGTTTTACTAGCCAGATTTTTCCTCCTCTCCTGACTACTCCCAGTCATAGCTGTCCCTCT
TCTCTTATGGAGATCGGAGAAAGAGGTAAT (SEQ ID NO:9).
GTAACTATAACGGTCGCTCCGAATTTCTCGAGTTAATTAAGATTACGCCAAGCTACG
GGCGGAGTACTGTCCTCCGAGCGGAGTACTGTCCTCCGAGCGGAGTACTGTCCTCCGA
GCGGAGTACTGTCCTCCGAGCGGAGTTCTGTCCTCCGAGCGGAGACTCTAGACTCCCT
ATCAGTGATAGAGATAGGCGTGTACGGTGGGAGGCCTATATAAGCAGAGCTCGTTTAGT
GAACCGTCAGATCGCTCCCTATCAGTGATAGAGAGAATTCGACCGCCACCATGTCGGA
ATTCATGAGACATATTATCTGCCACGGAGGTGTTATTACCGAAGAAATGGCCGCCAGTC
 
TTTTGGACCAGCTGATCGAAGAGGTACTGGCTGATAATCTTCCACCTCCTAGCCATTTT
GAACCACCTACCCTTCACGAACTGTATGATTTAGACGTGACGGCCCCCGAAGATCCCA
ACGAGGAGGCGGTTTCGCAGATTTTCCCGACTCTGTAATGTTGGCGGTGCAGGAAGG
GATTGACTTACTCACTTTTCCGCCGGCGCCCGGTTCTCCGGAGCCGCCTCACCTTTCCC
GGCAGCCCGAGCAGCCGGAGCAGAGAGCCTTGGGTCCGGTTTCTATGCCAAACCTTG
TACCGGAGGTGATCGATCTTACCTGCCACGAGGCTGGCTTTCCACCCAGTGACGACGA
GGATGAAGAGGGTGAGGAGTTTGTGTTAGATTATGTGGAGCACCCCGGGCACGGTTGC
AGGTCTTGTCATTATCACCGGAGGAATACGGGGGACCCAGATATTATGTGTTCGCTTTG
CTATATGAGGACCTGTGGCATGTTTGTCTACAGTAAGTGAAAATTATGGGCAGTGGGTG
ATAGAGTGGTGGGTTTGGTGTGGTAATTTTTTTTTAATTTTTACAGTTTTGTGGTTTAA
AGAATTTTGTATTGTGATTTTTTTAAAAGGTCCTGTGTCTGAACCTGAGCCTGAGCCCG
AGCCAGAACCGGAGCCTGCAAGACCTACCCGCCGTCCTAAAATGGCGCCTGCTATCCT
GAGACGCCCGACATCACCTGTGTCTAGAGAATGCAATAGTAGTACGGATAGCTGTGAC
TCCGGTCCTTCTAACACACCTCCTGAGATACACCCGGTGGTCCCGCTGTGCCCCATTAA
ACCAGTTGCCGTGAGAGTTGGTGGGCGTCGCCAGGCTGTGGAATGTATCGAGGACTTG
CTTAACGAGCCTGGGCAACCTTTGGACTTGAGCTGTAAACGCCCCAGGCCACTCGAG
GGTACCGGGTCCGGAGCTACAAATTTTCCCTCCTCAAACAGGCTGGAGATGTCGAAG
AAAATCCTGGGCCTACCGGTCCCATGTACAGGATGCAACTCCTGTCTTGCATTGCACTA
AGTCTTGCACTTGTCACAAACAGTGCACCTACTTCAAGTTCTACAAAGAAAACACAGC
TACAACTGGAGCATTTACTGCTGGATTTACAGATGATTTTGAATGGAATTAATAATTACA
AGAATCCCAAACTCACCGCGATGCTCACAGCTAAGTTTGCCATGCCCAAGAAGGCCAC
AGAACTGAAACATCTTCAGTGTCTAGAAGAAGCACTCAAACCTCTGGAGGAAGTGCT
AAATTTAGCTCAAAGCAAAAACTTTCACTTAAGACCCAGGGACTTAATCAGCAATATC
AACGTAATAGTTCTGGAACTAAAGGGATCTGAAACAACATTCATGTGTGAATATGCTGA
TGAGACAGCAACCATTGTAGAATTTCTGAACAGATGGATTACCTTTTGTCAAAGCATCA
TCTCAACACTGACTTGAAGATCTGAGGGCCGCGGCAGCCTGCTGACCTGCGGCGACG
TGGAGGAAAACCCCGGCCCCGGATCCATGAAACCAGTAACGTTATACGATGTCGCAGA
GTATGCCGGTGTCTCTTATCAGACCGTTTCCCGCGTGGTGAACCAGGCCAGCCACGTT
TCTGCGAAAACGCGGGAAAAAGTGGAAGCGGCGATGGCGGAGCTGAATTACATTCCC
AACCGCGTGGCACAACAACTGGCGGGCAAACAGTCGTTGCTGATTGGCGTTGCCACC
TCCAGTCTGGCCCTGCACGCGCCGTCGCAAATTGTCGCGGCGATTAAATCTCGCGCCG
ATCAACTGGGTGCCAGCGTGGTGGTGTCGATGGTAGAACGAAGCGGCGTCGAAGCCT
 
GTAAAGCGGCGGTGCACAATCTTCTCGCGCAACGCGTCAGTGGGCTGATCATTAACTA
TCCGCTGGATGACCAGGATGCCATTGCTGTGGAAGCTGCCTGCACTAATGTTCCGGCG
TTATTTCTTGATGTCTCTGACCAGACACCCATCAACAGTATTATTTTCTCCCATGAAGAC
GGTACGCGACTGGGCGTGGAGCATCTGGTCGCATTGGGTCACCAGCAAATCGCGCTGT
TAGCGGGCCCATTAAGTTCTGTCTCGGCGCGTCTGCGTCTGGCTGGCTGGCATAAATAT
CTCACTCGCAATCAAATTCAGCCGATAGCGGAACGGGAAGGCGACTGGAGTGCCATGT
CCGGTTTTCAACAAACCATGCAAATGCTGAATGAGGGCATCGTTCCCACTGCGATGCT
GGTTGCCAACGATCAGATGGCGCTGGGCGCAATGCGCGCCATTACCGAGTCCGGGCTG
CGCGTTGGTGCGGATATCTCGGTAGTGGGATACGACGATACCGAAGACAGCTCATGTTA
TATCCCGCCGTTAACCACCATCAAACAGGATTTTCGCCTGCTGGGGCAAACCAGCGTG
GACCGCTTGCTGCAACTCTCTCAGGGCCAGGCGGTGAAGGGCAATCAGCTGTTGCCC
GTCTCACTGGTGAAAAGAAAAACCACCCTGGCGCCCAATACGCAAACCGCCTCTCCC
CGCGCGTTGGCCGATTCATTAATGCAGCTGGCACGACAGGTTTCCCGACTGGAAAGCG
GGCAGAGCAGCCTGAGGCCTCCTAAGAAGAGGAAGGTTGCGGCCGCTAACCAAT
GTGCAGACTACTGTTAACCAATGTGCAGACTACTGTGAATTCTAACCAATGTGCAGACT
ACTGTTAACCAATGTGCAGACTACTGTAAGCTTCGAATTGAGTAGTGCTTTCTACTTTA
TGAGTAGTGCTTTCTACTTTATGAGTAGTGCTTTCTACTTTATGAGTAGTGCTTTCTACT
TTATGGTCGACAATACTAGTTAAGAAATCGCCAAGCTACGGGCGGAGTACTGTCCTCC
GAGCGGAGTACTGTCCTCCGAGCGGAGTACTGTCCTCCGAGCGGAGTACTGTCCTCCG
AGCGGAGTACTGTCCTCCGAGCGGAGACTCTAGAAATTGTGAGCGGATAACAATTTAG
GCGTGTACGGTGGGAGGCCTATATAAGCAGAGCTCGTTTAGTGAACCGTCAGATCGCC
AGCTGACAAAATTGTGAGCGCTCACAATTACTAGAAGATCTTGAATTCGCCACCATGG
CTAGATTAGATAAAAGTAAAGTGATTAACAGCGCATTAGAGCTGCTTAATGAGGTCGGA
ATCGAAGGTTTAACAACCCGTAAACTCGCCCAGAAGCTAGGTGTAGAGCAGCCTACAT
TGTATTGGCATGTAAAAAATAAGCGGGCTTTGCTCGACGCCTTAGCCATTGAGATGTTA
GATAGGCACCATACTCACTTTTGCCCTTTAGAAGGGGAAAGCTGGCAAGATTTTTACG
TAATAACGCTAAAAGTTTTAGATGTGCTTTACTAAGTCATCGCGATGGAGCAAAAGTAC
ATTTAGGTACACGGCCTACAGAAAAACAGTATGAAACTCTCGAAAATCAATTAGCCTTT
TTATGCCAACAAGGTTTTTCACTAGAGAATGCATTATATGCACTCAGCGCTGTGGGGCA
TTTTACTTTAGGTTGCGTATTGGAAGATCAAGAGCATCAAGTCGCTAAAGAAGAAAGG
GAAACACCTACTACTGATAGTATGCCGCCATTATTACGACAAGCTATCGAATTATTTGAT
CACCAAGGTGCAGAGCCAGCCTTCTTATTCGGCCTTGAATTGATCATATGCGGATTAGA
 
AAAACAACTTAAATGTGAAAGTGGGTCGCCAAAAAAGAAGAGAAAGGTCGACGGCG
GTGGTGCTTTGTCTCCTCAGCACTCTGCTGTCACTCAAGGAAGTATCATCAAGAACAA
GGAGGGCATGGATGCTAAGTCACTAACTGCCTGGTCCCGGACACTGGTGACCTTCAAG
GATGTATTTGTGGACTTCACCAGGGAGGAGTGGAAGCTGCTGGACACTGCTCAGCAGA
TCGTGTACAGAAATGTGATGCTGGAGAACTATAAGAACCTGGTTTCCTTGGGTTATCAG
CTTACTAAGCCAGATGTGATCCTCCGGTTGGAGAAGGGAGAAGAGCCCTGGCTGGTG
GAGAGAGAAATTCACCAAGAGACCCATCCTGATTCAGAGACTGCATTTGAAATCAAAT
CATCAGTTTAATACAAGGCGGCCGCAAATCAACATCAGTCTGATAAGCTATCAACATCA
GTCTGATAAGCTATCAACATCAGTCTGATAAGCTATCAACATCAGTCTGATAAGCTAAA
GCTTCGAATTCTGATAATCAGCCATACCACATTTGTAGAGGTTTTACTTGCTTTAAAAAA
CCTCCCACACCTCCCCCTGAACCTGAAACATAAAATGAATGCAATTGTTGTTGTTAACT
TGTTTATTGCAGCTTATAATGGTTACAAATAAAGCAATAGCATCACAAATTTCACAAATA
AAGCATTTTTTTCACTGCAGCTCCAGATTGAATTATTTGCCTGTCATACAGCTAATAATT
GACCATAAGACAATTAGATTTAAATTAGTTTTGAATCTTTCTAATACCAAAGTTCAGTTT
ACTGTTCCATGTTGCTTCTGAGTGGCTTCACAGACTTATGAAAAAGTAAACGGAATCA
GAATTACATCAATGCAAAAGCATTGCTGTGAACTCTGTACTTAGGACTAAACTTTGAGC
AATAACACATATAGATTGAGGATTGTTTGCTGTTAGTATACAAACTCTGGTTCAAAGCT
CCTCTTTATTGCTTGTCTTGGAAAATTTGCTGTTCTTCATGGTTTCTCTTTTCACTGCTAT
CTATTTTTCTCAACCACTCACATGGCTACAAAAGCTTCCTGATTAATAATTACACTAAGT
CAATAGGCATAGAGCCAGGACTGTTTGGGTAAACTGGTCACTTTATCTTAAACTAAATA
TATCCAAAACTGAACATGTACTTAGTTACTAAGTCTTTGACTTTATCTCATTCATACCAC
TCAGCTTTATCCAGGCCACTTATTTGACAGTATTATTGCGAAAACTTCCTATCTAGAAGT
TTGAGGAGAATATTTGTTATATTTGCAAAATAAAATAAGTTTGCAAGTTTTTTTTTTCTG
CCCCAAAGAGCTCTGTGTCCTTGAACATAAAATACAAATAACCGCTATGCTGTTAATTA
TTGACAAATGTCCCATTTTCAACCTAAGGAAATACCATAAAGTAACAGATATACCAACA
AAAGGTTACTAGTTAACAGGCATTGCCTGAAAAGAGTATAAAAGAATTTCAGCATGAT
TTTCCATATTGTGCTTCCACCACTGCCAATAACAAAATAACTAGCAAGGATCCGGCCAC
CATGGCCCCCCCGACCGATGTCAGCCTGGGGGACGAGCTCCACTTAGACGGCGAGGA
CGTGGCGATGGCGCATGCCGACGCGCTAGACGATTTCGATCTGGACATGTTGGGGGAC
GGGGATTCCCCGGGTCCGGGATTTACCCCCCACGACTCCGCCCCCTACGGCGCTCTGG
ATATGGCCGACTTCGAGTTTGAGCAGATGTTTACCGATGCCCTTGGAATTGACGAGTAC
GGTGGGACGCGTATGAAGCTACTGTCTTCTATCGAACAAGCATGCGATATTTGCCGACT
 
TAAAAAGCTCAAGTGCTCCAAAGAAAAACCGAAGTGCGCCAAGTGTCTGAAGAACA
ACTGGGAGTGTCGCTACTCTCCCAAAACCAAAAGGTCTCCGCTGACTAGGGCACATCT
GACAGAAGTGGAATCAAGGCTAGAAAGACTGGAACAGCTATTTCTACTGATTTTTCCT
CGAGAAGACCTTGACATGATTTTGAAAATGGATTCTTTACAGGATATAAAAGCATTGTT
AACAGGATTATTTGTACAAGATAATGTGAATAAAGATGCCGTCACAGATAGATTGGCTT
CAGTGGAGACTGATATGCCTCTAACATTGAGACAGCATAGAATAAGTGCGACATCATCA
TCGGAAGAGAGTAGTAACAAAGGTCAAAGACAGTTGACTGTATAATTCACTCCTCAGG
TGCAGGCTGCCTATCAGAAGGTGGTGGCTGGTGTGGCCAATGCCCTGGCTCACAAATA
CCACTGAGATCTTTTTCCCTCTGCCAAAAATTATGGGGACATCATGAAGCCCCTTGAGC
ATCTGACTTCTGGCTAATAAAGGAAATTTATTTTCATTGCAATAGTGTGTTGGAATTTTT
TGTGTCTCTCACTCGGAAGGACATATGGGAGGGCAAATCATTTAAAACATCAGAATGA
GTATTTGGTTTAGAGTTTGGCAACATATGCCCATATGCTGGCTGCCATGAACAAAGGTT
GGCTATAAAGAGGTCATCAGTATATGAAACAGCCCCCTGCTGTCCATTCCTTATTCCATA
GAAAAGCCTTGACTTGAGGTTAGATTTTTTTTATATTTTGTTTTGTGTTATTTTTTTCTTT
AACATCCCTAAAATTTTCCTTACATGTTTTACTAGCCAGATTTTTCCTCCTCTCCTGACT
ACTCCCAGTCATAGCTGTCCCTCTTCTCTTATGGAGATCGGAGAAAGAGGTAAT (SEQ
ID NO:10).
 
GTAACTATAACGGTCGCTCCGAATTTCTCGAGTTAATTAAGATTACGCCAAGCTACG
GGCGGAGTACTGTCCTCCGAGCGGAGTACTGTCCTCCGAGCGGAGTACTGTCCTCCGA
GCGGAGTACTGTCCTCCGAGCGGAGTTCTGTCCTCCGAGCGGAGACTCTAGACTCCCT
ATCAGTGATAGAGATAGGCGTGTACGGTGGAGGCCTATATAAGCAGAGCTCGTTTAGT
GAACCGTCAGATCGCTCCCTATCAGTGATAGAGAATTCGACCGCCACCATGTCGGA
ATTCATGAGACATATTATCTGCCACGGAGGTGTTATTACCGAAGAAATGGCCGCCAGTC
TTTTGGACCAGCTGATCGAAGAGGTACTGGCTGATAATCTTCCACCTCCTAGCCATTTT
GAACCACCTACCCTTCACGAACTGTATGATTTAGACGTGACGGCCCCCGAAGATCCCA
ACGAGGAGGCGGTTTCGCAGATTTTTCCCGACTCTGTAATGTTGGCGGTGCAGGAAGG
GATTGACTTACTCACTTTTCCGCCGGCGCCCGGTTCTCCGGAGCCGCCTCACCTTTCCC
GGCAGCCCGAGCAGCCGGAGCAGAGAGCCTTGGGTCCGGTTTCTATGCCAAACCTTG
TACCGGAGGTGATCGATCTTACCTGCCACGAGGCTGGCTTTCCACCCAGTGACGACGA
GGATGAAGAGGGTGAGGAGTTTGTGTTAGATTATGTGGAGCACCCCGGGCACGGTTGC
AGGTCTTGTCATTATCACCGGAGGAATACGGGGGACCCAGATATTATGTGTTCGCTTTG
CTATATGAGGACCTGTGGCATGTTTGTCTACAGTAAGTGAAAATTATGGGCAGTGGGTG
 
ATAGAGTGGTGGGTTTGGTGTGGTAATTTTTTTTTAATTTTTACAGTTTTGTGGTTTAA
AGAATTTGTATTGTGATTTTTTTAAAAGGTCCTGTGTCTGAACCTGAGCCTGAGCCCG
AGCCAGAACCGGAGCCTGCAAGACCTACCCGCCGTCCTAAAATGGCGCCTGCTATCCT
GAGACGCCCGACATCACCTGTGTCTAGAGAATGCAATAGTAGTACGGATAGCTGTGAC
TCCGGTCCTTCTAACACACCTCCTGAGATACACCCGGTGGTCCCGCTGTGCCCCATTAA
ACCAGTTGCCGTGAGAGTTGGTGGGCGTCGCCAGGCTGTGGAATGTATCGAGGACTTG
CTTAACGAGCCTGGGCAACCTTTGGACTTGAGCTGTAAACGCCCCAGGCCACTCGAG
GGTACCGGGTCCGGAGCTACAAATTTTTCCCTCCTCAAACAGGCTGGAGATGTCGAAG
AAAATCCTGGGCCTACCGGTCCCATGTGGCTGCAGAGCCTGCTGCTCTTGGGCACTGT
GGCCTGCAGCATCTCTGCACCCGCCCGCTCGCCCAGCCCCAGCACGCAGCCCTGGGA
GCATGTGAATGCCATCCAGGAGGCCCGGCGTCTCCTGAACCTGAGTAGAGACACTGCT
GCTGAGATGAATGAAACAGTAGAAGTCATCTCAGAAATGTTTGACCTCCAGGAGCCGA
CCTGCCTACAGACCCGCCTGGAGCTGTACAAGCAGGGCCTGCGGGGCAGCCTCACCA
AGCTCAAGGGCCCCTTGACCATGATGGCCAGCCACTACAAGCAGCACTGCCCTCCAAC
CCCGGAAACTTCCTGTGCAACCCAGATTATCACCTTTGAAAGTTTCAAAGAGAACCTG
AAGGACTTTCTGCTTGTCATCCCCTTTGACTGCTGGGAGCCAGTCCAGGAGTGAAGAT
CTGAGGGCCGCGGCAGCCTGCTGACCTGCGGCGACGTGGAGGAAAACCCCGGCCCCG
GATCCATGAAACCAGTAACGTTATACGATGTCGCAGAGTATGCCGGTGTCTCTTATCAG
ACCGTTTCCCGCGTGGTGAACCAGGCCAGCCACGTTTCTGCGAAAACGCGGGAAAAA
GTGGAAGCGGCGATGGCGGAGCTGAATTACATTCCCAACCGCGTGGCACAACAACTG
GCGGGCAAACAGTCGTTGCTGATTGGCGTTGCCACCTCCAGTCTGGCCCTGCACGCGC
CGTCGCAAATTGTCGCGGCGATTAAATCTCGCGCCGATCAACTGGGTGCCAGCGTGGT
GGTGTCGATGGTAGAACGAAGCGGCGTCGAAGCCTGTAAAGCGGCGGTGCACAATCT
TCTCGCGCAACGCGTCAGTGGGCTGATCATTAACTATCCGCTGGATGACCAGGATGCCA
TTGCTGTGGAAGCTGCCTGCACTAATGTTCCGGCGTTATTTCTTGATGTCTCTGACCAG
ACACCCATCAACAGTATTATTTTCTCCCATGAAGACGGTACGCGACTGGGCGTGGAGC
ATCTGGTCGCATTGGGTCACCAGCAAATCGCGCTGTTAGCGGGCCCATTAAGTTCTGTC
TCGGCGCGTCTGCGTCTGGCTGGCTGGCATAAATATCTCACTCGCAATCAAATTCAGCC
GATAGCGGAACGGGAAGGCGACTGGAGTGCCATGTCCGGTTTTCAACAAACCATGCA
AATGCTGAATGAGGGCATCGTTCCCACTGCGATGCTGGTTGCCAACGATCAGATGGCG
CTGGGCGCAATGCGCGCCATTACCGAGTCCGGGCTGCGCGTTGGTGCGGATATCTCGG
TAGTGGGATACGACGATACCGAAGACAGCTCATGTTATATCCCGCCGTTAACCACCATC
 
AAACAGGATTTTCGCCTGCTGGGGCAAACCAGCGTGGACCGCTTGCTGCAACTCTCTC
AGGGCCAGGCGGTGAAGGGCAATCAGCTGTTGCCCGTCTCACTGGTGAAAAGAAAAA
CCACCCTGGCGCCCAATACGCAAACCGCCTCTCCCCGCGTTGGCCGATTCATTAATG
CAGCTGGCACGACAGGTTTCCCGACTGGAAAGCGGGCAGAGCAGCCTGAGGCCTCCT
AAGAAGAAGAGGAAGGTTGCGGCCGCTAACCAATGTGCAGACTACTGTTAACCAATG
TGCAGACTACTGTGAATTCTAACCAATGTGCAGACTACTGTTAACCAATGTGCAGACTA
CTGTAAGCTTCGAATTGAGTAGTGCTTTCTACTTTATGAGTAGTGCTTTCTACTTTATGA
GTAGTGCTTTCTACTTTATGAGTAGTGCTTTCTACTTTATGGTCGACAATACTAGTTAAG
AAATCGCCAAGCTACGGGCGGAGTACTGTCCTCCGAGCGGAGTACTGTCCTCCGAGCG
GAGTACTGTCCTCCGAGCGGAGTACTGTCCTCCGAGCGGAGTACTGTCCTCCGAGCGG
AGACTCTAGAAATTGTGAGCGGATAACAATTAGGCGTGTACGGTGGGAGGCCTATATA
AGCAGAGCTCGTTTAGTGAACCGTCAGATCGCCAGCTGACAAAATTGTGAGCGCTCAC
AATTACTAGAAGATCTTGAATTCGCCACCATGGCTAGATTAGATAAAAGTAAAGTGATT
AACAGCGCATTAGAGCTGCTTAATGAGGTCGGAATCGAAGGTTTAACAACCCGTAAAC
TCGCCCAGAAGCTAGGTGTAGAGCAGCCTACATTGTATTGGCATGTAAAAAATAAGCG
GGCTTTGCTCGACGCCTTAGCCATTGAGATGTTAGATAGGCACCATACTCACTTTTGCC
CTTTAGAAGGGGAAAGCTGGCAAGATTTTTTACGTAATAACGCTAAAAGTTTTAGATGT
GCTTTACTAAGTCATCGCGATGGAGCAAAAGTACATTTAGGTACACGGCCTACAGAAA
AACAGTATGAAACTCTCGAAAATCAATTAGCCTTTTTATGCCAACAAGGTTTTTCACTA
GAGAATGCATTATATGCACTCAGCGCTGTGGGGCATTTTACTTTAGGTTGCGTATTGGA
AGATCAAGAGCATCAAGTCGCTAAAGAAGAAAGGGAAACACCTACTACTGATAGTATG
CCGCCATTATTACGACAAGCTATCGAATTATTTGATCACCAAGGTGCAGAGCCAGCCTT
CTTATTCGGCCTTGAATTGATCATATGCGGATTAGAAAAACAACTTAAATGTGAAAGTG
GGTCGCCAAAAAAGAAGAGAAAGGTCGACGGCGGTGGTGCTTTGTCTCCTCAGCACT
CTGCTGTCACTCAAGGAAGTATCATCAAGAACAAGGAGGGCATGGATGCTAAGTCACT
AACTGCCTGGTCCCGGACACTGGTGACCTTCAAGGATGTATTTGTGGACTTCACCAGG
GAGGAGTGGAAGCTGCTGGACACTGCTCAGCAGATCGTGTACAGAAATGTGATGCTG
GAGAACTATAAGAACCTGGTTTCCTTGGGTTATCAGCTTACTAAGCCAGATGTGATCCT
CCGGTTGGAGAAGGGAGAAGAGCCCTGGCTGGTGGAGAGAGAAATTCACCAAGAGA
CCCATCCTGATTCAGAGACTGCATTTGAAATCAAATCATCAGTTTAATACAAGGCGGCC
GCAAATCAACATCAGTCTGATAAGCTATCAACATCAGTCTGATAAGCTATCAACATCAG
TCTGATAAGCTATCAACATCAGTCTGATAAGCTAAAGCTTCGAATTCTGATAATCAGCCA
 
TACCACATTTGTAGAGGTTTTACTTGCTTTAAAAAACCTCCCACACCTCCCCCTGAACC
TGAAACATAAAATGAATGCAATTGTTGTTGTTAACTTGTTTATTGCAGCTTATAATGGTT
ACAAATAAAGCAATAGCATCACAAATTTCACAAATAAAGCATTTTTTTCACTGCAGCTC
CAGATTGAATTATTTGCCTGTCATACAGCTAATAATTGACCATAAGACAATTAGATTTAA
ATTAGTTTTGAATCTTTCTAATACCAAAGTTCAGTTTACTGTTCCATGTTGCTTCTGAGT
GGCTTCACAGACTTATGAAAAAGTAAACGGAATCAGAATTACATCAATGCAAAAGCAT
TGCTGTGAACTCTGTACTTAGGACTAAACTTTGAGCAATAACACATATAGATTGAGGAT
TGTTTGCTGTTAGTATACAAACTCTGGTTCAAAGCTCCTCTTTATTGCTTGTCTTGGAAA
ATTTGCTGTTCTTCATGGTTTCTCTTTTCACTGCTATCTATTTTTCTCAACCACTCACATG
GCTACAAAAGCTTCCTGATTAATAATTACACTAAGTCAATAGGCATAGAGCCAGGACTG
TTTGGGTAAACTGGTCACTTTATCTTAAACTAAATATATCCAAAACTGAACATGTACTTA
GTTACTAAGTCTTTGACTTTATCTCATTCATACCACTCAGCTTTATCCAGGCCACTTATTT
GACAGTATTATTGCGAAAACTTCCTATCTAGAAGTTTGAGGAGAATATTTGTTATATTTG
CAAAATAAAATAAGTTTGCAAGTTTTTTTTCTGCCCCAAAGAGCTCTGTGTCCTTGA
ACATAAAATACAAATAACCGCTATGCTGTTAATTATTGACAAATGTCCCATTTTCAACCT
AAGGAAATACCATAAAGTAACAGATATACCAACAAAAGGTTACTAGTTAACAGGCATT
GCCTGAAAAGAGTATAAAAGAATTTCAGCATGATTTTCCATATTGTGCTTCCACCACTG
CCAATAACAAAATAACTAGCAAGGATCCGGCCACCATGGCCCCCCCGACCGATGTCAG
CCTGGGGGACGAGCTCCACTTAGACGGCGAGGACGTGGCGATGGCGCATGCCGACGC
GCTAGACGATTTCGATCTGGACATGTTGGGGGACGGGGATTCCCCGGGTCCGGGATTT
ACCCCCCACGACTCCGCCCCCTACGGCGCTCTGGATATGGCCGACTTCGAGTTTGAGC
AGATGTTTACCGATGCCCTTGGAATTGACGAGTACGGTGGGACGCGTATGAAGCTACT
GTCTTCTATCGAACAAGCATGCGATATTTGCCGACTTAAAAAGCTCAAGTGCTCCAAA
GAAAAACCGAAGTGCGCCAAGTGTCTGAAGAACAACTGGGAGTGTCGCTACTCTCCC
AAAACCAAAAGGTCTCCGCTGACTAGGGCACATCTGACAGAAGTGGAATCAAGGCTA
GAAAGACTGGAACAGCTATTTCTACTGATTTTTCCTCGAGAAGACCTTGACATGATTTT
GAAAATGGATTCTTTACAGGATATAAAAGCATTGTTAACAGGATTATTTGTACAAGATAA
TGTGAATAAAGATGCCGTCACAGATAGATTGGCTTCAGTGGAGACTGATATGCCTCTAA
CATTGAGACAGCATAGAATAAGTGCGACATCATCATCGGAAGAGAGTAGTAACAAAGG
TCAAAGACAGTTGACTGTATAATTCACTCCTCAGGTGCAGGCTGCCTATCAGAAGGTG
GTGGCTGGTGTGGCCAATGCCCTGGCTCACAAATACCACTGAGATCTTTTTCCCTCTGC
CAAAAATTATGGGGACATCATGAAGCCCCTTGAGCATCTGACTTCTGGCTAATAAAGG
 
AAATTTATTTTCATTGCAATAGTGTGTTGGAATTTTTTGTGTCTCTCACTCGGAAGGACA
TATGGGAGGGCAAATCATTTAAAACATCAGAATGAGTATTTGGTTTAGAGTTTGGCAAC
ATATGCCCATATGCTGGCTGCCATGAACAAAGGTTGGCTATAAAGAGGTCATCAGTATA
TGAAACAGCCCCCTGCTGTCCATTCCTTATTCCATAGAAAAGCCTTGACTTGAGGTTAG
ATTTTTTTTATATTTTGTTTTGTGTTATTTTTTTCTTTAACATCCCTAAAATTTTCCTTACA
TGTTTTACTAGCCAGATTTTTCCTCCTCTCCTGACTACTCCCAGTCATAGCTGTCCCTCT
TCTCTTATGGAGATCGGAGAAAGAGGTAAT (SEQ ID NO:11).
 
GTAACTATAACGGTCGCTCCGAATTTCTCGAGTTAATTAAGATTACGCCAAGCTACG
GGCGGAGTACTGTCCTCCGAGCGGAGTACTGTCCTCCGAGCGGAGTACTGTCCTCCGA
GCGGAGTACTGTCCTCCGAGCGGAGTTCTGTCCTCCGAGCGGAGACTCTAGACTCCCT
ATCAGTGATAGAGATAGGCGTGTACGGTGGGAGGCCTATATAAGCAGAGCTCGTTTAGT
GAACCGTCAGATCGCTCCCTATCAGTGATAGAGAATTCGACCGCCACCATGTCGGA
ATTCATGAGACATATTATCTGCCACGGAGGTGTTATTACCGAAGAAATGGCCGCCAGTC
TTTTGGACCAGCTGATCGAAGAGGTACTGGCTGATAATCTTCCACCTCCTAGCCATTTT
GAACCACCTACCCTTCACGAACTGTATGATTTAGACGTGACGGCCCCCGAAGATCCCA
ACGAGGAGGCGGTTTCGCAGATTTTTCCCGACTCTGTAATGTTGGCGGTGCAGGAAGG
GATTGACTTACTCACTTTTCCGCCGGCGCCCGGTTCTCCGGAGCCGCCTCACCTTTCCC
GGCAGCCCGAGCAGCCGGAGCAGAGAGCCTTGGGTCCGGTTTCTATGCCAAACCTTG
TACCGGAGGTGATCGATCTTACCTGCCACGAGGCTGGCTTTCCACCCAGTGACGACGA
GGATGAAGAGGGTGAGGAGTTTGTGTTAGATTATGTGGAGCACCCCGGGCACGGTTGC
AGGTCTTGTCATTATCACCGGAGGAATACGGGGGACCCAGATATTATGTGTTCGCTTTG
CTATATGAGGACCTGTGGCATGTTTGTCTACAGTAAGTGAAAATTATGGGCAGTGGGTG
ATAGAGTGGTGGGTTTGGTGTGGTAATTTTTTTTTAATTTTTACAGTTTTGTGGTTTAA
AGAATTTTGTATTGTGATTTTTTTAAAAGGTCCTGTGTCTGAACCTGAGCCTGAGCCCG
AGCCAGAACCGGAGCCTGCAAGACCTACCCGCCGTCCTAAAATGGCGCCTGCTATCCT
GAGACGCCCGACATCACCTGTGTCTAGAGAATGCAATAGTAGTACGGATAGCTGTGAC
TCCGGTCCTTCTAACACACCTCCTGAGATACACCCGGTGGTCCCGCTGTGCCCCATTAA
ACCAGTTGCCGTGAGAGTTGGTGGGCGTCGCCAGGCTGTGGAATGTATCGAGGACTTG
CTTAACGAGCCTGGGCAACCTTTGGACTTGAGCTGTAAACGCCCCAGGCCACTCGAG
GGTACCGGGTCCGGAGCTACAAATTTTCCCTCCTCAAACAGGCTGGAGATGTCGAAG
AAAATCCTGGGCCTACCGGTCCCATGTGGCTGCAGAATTTACTTTTCCTGGGCATTGTG
GTCTACAGCCTCTCAGCACCCACCCGCTCACCCATCACTGTCACCCGGCCTTGGAAGC
 
ATGTAGAGGCCATCAAAGAAGCCCTGAACCTCCTGGATGACATGCCTGTCACGTTGAA
TGAAGAGGTAGAAGTCGTCTCTAACGAGTTCTCCTTCAAGAAGCTAACATGTGTGCAG
ACCCGCCTGAAGATATTCGAGCAGGGTCTACGGGGCAATTTCACCAAACTCAAGGGCG
CCTTGAACATGACAGCCAGCTACTACCAGACATACTGCCCCCCAACTCCGGAAACGGA
CTGTGAAACACAAGTTACCACCTATGCGGATTTCATAGACAGCCTTAAAACCTTTCTGA
CTGATATCCCCTTTGAATGCAAAAAACCAGGCCAAAAATGAAGATCTGAGGCCGCGG
CAGCCTGCTGACCTGCGGCGACGTGGAGGAAAACCCCGGCCCCGGATCCATGAAACC
AGTAACGTTATACGATGTCGCAGAGTATGCCGGTGTCTCTTATCAGACCGTTTCCCGCG
TGGTGAACCAGGCCAGCCACGTTTCTGCGAAAACGCGGGAAAAAGTGGAAGCGGCG
ATGGCGGAGCTGAATTACATTCCCAACCGCGTGGCACAACAACTGGCGGGCAAACAG
TCGTTGCTGATTGGCGTTGCCACCTCCAGTCTGGCCCTGCACGCGCCGTCGCAAATTG
TCGCGGCGATTAAATCTCGCGCCGATCAACTGGGTGCCAGCGTGGTGGTGTCGATGGT
AGAACGAAGCGGCGTCGAAGCCTGTAAAGCGGCGGTGCACAATCTTCTCGCGCAACG
CGTCAGTGGGCTGATCATTAACTATCCGCTGGATGACCAGGATGCCATTGCTGTGGAAG
CTGCCTGCACTAATGTTCCGGCGTTATTTCTTGATGTCTCTGACCAGACACCCATCAAC
AGTATTATTTTCTCCCATGAAGACGGTACGCGACTGGGCGTGGAGCATCTGGTCGCATT
GGGTCACCAGCAAATCGCGCTGTTAGCGGGCCCATTAAGTTCTGTCTCGGCGCGTCTG
CGTCTGGCTGGCTGGCATAAATATCTCACTCGCAATCAAATTCAGCCGATAGCGGAACG
GGAAGGCGACTGGAGTGCCATGTCCGGTTTTCAACAAACCATGCAAATGCTGAATGAG
GGCATCGTTCCCACTGCGATGCTGGTTGCCAACGATCAGATGGCGCTGGGCGCAATGC
GCGCCATTACCGAGTCCGGGCTGCGCGTTGGTGCGGATATCTCGGTAGTGGGATACGA
CGATACCGAAGACAGCTCATGTTATATCCCGCCGTTAACCACCATCAAACAGGATTTTC
GCCTGCTGGGGCAAACCAGCGTGGACCGCTTGCTGCAACTCTCTCAGGGCCAGGCGG
TGAAGGGCAATCAGCTGTTGCCCGTCTCACTGGTGAAAAGAAAAACCACCCTGGCGC
CCAATACGCAAACCGCCTCTCCCCGCGCGTTGGCCGATTCATTAATGCAGCTGGCACG
ACAGGTTTCCCGACTGGAAAGCGGGCAGAGCAGCCTGAGGCCTCCTAAGAAGAAGA
GGAAGGTTGCGGCCGCTAACCAATGTGCAGACTACTGTTAACCAATGTGCAGACTACT
GTGAATTCTAACCAATGTGCAGACTACTGTTAACCAATGTGCAGACTACTGTAAGCTTC
GAATTGAGTAGTGCTTTCTACTTTATGAGTAGTGCTTTCTACTTTATGAGTAGTGCTTTC
TACTTTATGAGTAGTGCTTTCTACTTTATGGTCGACAATACTAGTTAAGAAATCGCCAAG
CTACGGGCGGAGTACTGTCCTCCGAGCGGAGTACTGTCCTCCGAGCGGAGTACTGTCC
TCCGAGCGGAGTACTGTCCTCCGAGCGGAGTACTGTCCTCCGAGCGGAGACTCTAGA
 
AATTGTGAGCGGATAACAATTTAGGCGTGTACGGTGGAGGCCTATATAAGCAGAGCT
CGTTTAGTGAACCGTCAGATCGCCAGCTGACAAAATTGTGAGCGCTCACAATTACTAG
AAGATCTTGAATTCGCCACCATGGCTAGATTAGATAAAAGTAAAGTGATTAACAGCGCA
TTAGAGCTGCTTAATGAGGTCGGAATCGAAGGTTTAACAACCCGTAAACTCGCCCAGA
AGCTAGGTGTAGAGCAGCCTACATTGTATTGGCATGTAAAAAATAAGCGGGCTTTGCTC
GACGCCTTAGCCATTGAGATGTTAGATAGGCACCATACTCACTTTTGCCCTTTAGAAGG
GGAAAGCTGGCAAGATTTTTTACGTAATAACGCTAAAAGTTTTAGATGTGCTTTACTAA
GTCATCGCGATGGAGCAAAAGTACATTTAGGTACACGGCCTACAGAAAAACAGTATGA
AACTCTCGAAAATCAATTAGCCTTTTTATGCCAACAAGGTTTTTCACTAGAGAATGCAT
TATATGCACTCAGCGCTGTGGGGCATTTTACTTTAGGTTGCGTATTGGAAGATCAAGAG
CATCAAGTCGCTAAAGAAGAAAGGGAAACACCTACTACTGATAGTATGCCGCCATTATT
ACGACAAGCTATCGAATTATTTGATCACCAAGGTGCAGAGCCAGCCTTCTTATTCGGCC
TTGAATTGATCATATGCGGATTAGAAAAACAACTTAAATGTGAAAGTGGGTCGCCAAA
AAAGAAGAGAAAGGTCGACGGCGGTGGTGCTTTGTCTCCTCAGCACTCTGCTGTCAC
TCAAGGAAGTATCATCAAGAACAAGGAGGGCATGGATGCTAAGTCACTAACTGCCTGG
TCCCGGACACTGGTGACCTTCAAGGATGTATTTGTGGACTTCACCAGGGAGGAGTGGA
AGCTGCTGGACACTGCTCAGCAGATCGTGTACAGAAATGTGATGCTGGAGAACTATAA
GAACCTGGTTTCCTTGGGTTATCAGCTTACTAAGCCAGATGTGATCCTCCGGTTGGAGA
AGGGAGAAGAGCCCTGGCTGGTGGAGAGAGAAATTCACCAAGAGACCCATCCTGATT
CAGAGACTGCATTTGAAATCAAATCATCAGTTTAATACAAGGCGGCCGCAAATCAACA
TCAGTCTGATAAGCTATCAACATCAGTCTGATAAGCTATCAACATCAGTCTGATAAGCTA
TCAACATCAGTCTGATAAGCTAAAGCTTCGAATTCTGATAATCAGCCATACCACATTTGT
AGAGGTTTTACTTGCTTTAAAAAACCTCCCACACCTCCCCCTGAACCTGAAACATAAA
ATGAATGCAATTGTTGTTGTTAACTTGTTTATTGCAGCTTATAATGGTTACAAATAAAGC
AATAGCATCACAAATTTCACAAATAAAGCATTTTTTTCACTGCAGCTCCAGATTGAATTA
TTTGCCTGTCATACAGCTAATAATTGACCATAAGACAATTAGATTTAAATTAGTTTTGAA
TCTTTCTAATACCAAAGTTCAGTTTACTGTTCCATGTTGCTTCTGAGTGGCTTCACAGA
CTTATGAAAAAGTAAACGGAATCAGAATTACATCAATGCAAAAGCATTGCTGTGAACT
CTGTACTTAGGACTAAACTTTGAGCAATAACACATATAGATTGAGGATTGTTTGCTGTTA
GTATACAAACTCTGGTTCAAAGCTCCTCTTTATTGCTTGTCTTGGAAAATTTGCTGTTCT
TCATGGTTTCTCTTTTCACTGCTATCTATTTTTCTCAACCACTCACATGGCTACAAAAGC
TTCCTGATTAATAATTACACTAAGTCAATAGGCATAGAGCCAGGACTGTTTGGGTAAAC
 
TGGTCACTTTATCTTAAACTAAATATATCCAAAACTGAACATGTACTTAGTTACTAAGTC
TTTGACTTTATCTCATTCATACCACTCAGCTTTATCCAGGCCACTTATTTGACAGTATTAT
TGCGAAAACTTCCTATCTAGAAGTTTGAGGAGAATATTTGTTATATTTGCAAAATAAAAT
AAGTTTGCAAGTTTTTTTTTCTGCCCCAAAGAGCTCTGTGTCCTTGAACATAAAATAC
AAATAACCGCTATGCTGTTAATTATTGACAAATGTCCCATTTTCAACCTAAGGAAATACC
ATAAAGTAACAGATATACCAACAAAAGGTTACTAGTTAACAGGCATTGCCTGAAAAGA
GTATAAAAGAATTTCAGCATGATTTTCCATATTGTGCTTCCACCACTGCCAATAACAAAA
TAACTAGCAAGGATCCGGCCACCATGGCCCCCCCGACCGATGTCAGCCTGGGGGACG
AGCTCCACTTAGACGGCGAGGACGTGGCGATGGCGCATGCCGACGCGCTAGACGATTT
CGATCTGGACATGTTGGGGGACGGGGATTCCCCGGGTCCGGGATTTACCCCCCACGAC
TCCGCCCCCTACGGCGCTCTGGATATGGCCGACTTCGAGTTTGAGCAGATGTTTACCGA
TGCCCTTGGAATTGACGAGTACGGTGGGACGCGTATGAAGCTACTGTCTTCTATCGAA
CAAGCATGCGATATTTGCCGACTTAAAAAGCTCAAGTGCTCCAAAGAAAAACCGAAG
TGCGCCAAGTGTCTGAAGAACAACTGGGAGTGTCGCTACTCTCCCAAAACCAAAAGG
TCTCCGCTGACTAGGGCACATCTGACAGAAGTGGAATCAAGGCTAGAAAGACTGGAA
CAGCTATTTCTACTGATTTTTCCTCGAGAAGACCTTGACATGATTTTGAAAATGGATTCT
TTACAGGATATAAAAGCATTGTTAACAGGATTATTTGTACAAGATAATGTGAATAAAGAT
GCCGTCACAGATAGATTGGCTTCAGTGGAGACTGATATGCCTCTAACATTGAGACAGCA
TAGAATAAGTGCGACATCATCATCGGAAGAGAGTAGTAACAAAGGTCAAAGACAGTT
GACTGTATAATTCACTCCTCAGGTGCAGGCTGCCTATCAGAAGGTGGTGGCTGGTGTG
GCCAATGCCCTGGCTCACAAATACCACTGAGATCTTTTTCCCTCTGCCAAAAATTATGG
GGACATCATGAAGCCCCTTGAGCATCTGACTTCTGGCTAATAAAGGAAATTTATTTTCA
TTGCAATAGTGTGTTGGAATTTTTTGTGTCTCTCACTCGGAAGGACATATGGGAGGGCA
AATCATTTAAAACATCAGAATGAGTATTTGGTTTAGAGTTTGGCAACATATGCCCATATG
CTGGCTGCCATGAACAAAGGTTGGCTATAAAGAGGTCATCAGTATATGAAACAGCCCC
CTGCTGTCCATTCCTTATTCCATAGAAAAGCCTTGACTTGAGGTTAGATTTTTTTTATATT
TTGTTTTGTGTTATTTTTTTCTTTAACATCCCTAAAATTTTCCTTACATGTTTTACTAGCC
AGATTTTTCCTCCTCTCCTGACTACTCCCAGTCATAGCTGTCCCTCTTCTCTTATGGAGA
TCGGAGAAAGAGGTAAT (SEQ ID NO:12).
 
GTAACTATAACGGTCGCTCCGAATTTCTCGAGTTAATTAAGATTACGCCAAGCTACG
GGCGGAGTACTGTCCTCCGAGCGGAGTACTGTCCTCCGAGCGGAGTACTGTCCTCCGA
GCGGAGTACTGTCCTCCGAGCGGAGTTCTGTCCTCCGAGCGGAGACTCTAGACTCCCT
 
ATCAGTGATAGAGATAGGCGTGTACGGTGGAGGCCTATATAAGCAGAGCTCGTTTAGT
GAACCGTCAGATCGCTCCCTATCAGTGATAGAGAGAATTCGACCGCCACCATGTCGGA
ATTCATGAGACATATTATCTGCCACGGAGGTGTTATTACCGAAGAAATGGCCGCCAGTC
TTTTGGACCAGCTGATCGAAGAGGTACTGGCTGATAATCTTCCACCTCCTAGCCATTTT
GAACCACCTACCCTTCACGAACTGTATGATTTAGACGTGACGGCCCCCGAAGATCCCA
ACGAGGAGGCGGTTTCGCAGATTTTCCCGACTCTGTAATGTTGGCGGTGCAGGAAGG
GATTGACTTACTCACTTTTCCGCCGGCGCCCGGTTCTCCGGAGCCGCCTCACCTTTCCC
GGCAGCCCGAGCAGCCGGAGCAGAGAGCCTTGGGTCCGGTTTCTATGCCAAACCTTG
TACCGGAGGTGATCGATCTTACCTGCCACGAGGCTGGCTTTCCACCCAGTGACGACGA
GGATGAAGAGGGTGAGGAGTTTGTGTTAGATTATGTGGAGCACCCCGGGCACGGTTGC
AGGTCTTGTCATTATCACCGGAGGAATACGGGGGACCCAGATATTATGTGTTCGCTTTG
CTATATGAGGACCTGTGGCATGTTTGTCTACAGTAAGTGAAAATTATGGGCAGTGGGTG
ATAGAGTGGTGGGTTTGGTGTGGTAATTTTTTTTTAATTTTTACAGTTTTGTGGTTTAA
AGAATTTTGTATTGTGATTTTTTTAAAAGGTCCTGTGTCTGAACCTGAGCCTGAGCCCG
AGCCAGAACCGGAGCCTGCAAGACCTACCCGCCGTCCTAAAATGGCGCCTGCTATCCT
GAGACGCCCGACATCACCTGTGTCTAGAGAATGCAATAGTAGTACGGATAGCTGTGAC
TCCGGTCCTTCTAACACACCTCCTGAGATACACCCGGTGGTCCCGCTGTGCCCCATTAA
ACCAGTTGCCGTGAGAGTTGGTGGGCGTCGCCAGGCTGTGGAATGTATCGAGGACTTG
CTTAACGAGCCTGGGCAACCTTTGGACTTGAGCTGTAAACGCCCCAGGCCACTCGAG
GGTACCGGGTCCGGAGCTACAAATTTTTCCCTCCTCAAACAGGCTGGAGATGTCGAAG
AAAATCCTGGGCCTACCGGTCCCATGAGTGTGCCCACTCAGGTCCTGGGGTTGCTGCT
GCTGTGGCTTACAGACGCTCGCTGCCAGGTGCAGCTGGTGGAGAGCGGCGGCGGCGT
GGTGCAGCCCGGCAGGAGCCTGAGGCTGGACTGCAAGGCCAGCGGCATCACCTTCAG
CAACAGCGGCATGCACTGGGTGAGGCAGGCCCCCGGCAAGGGCCTGGAGTGGGTGG
CCGTGATCTGGTACGACGGCAGCAAGAGGTACTACGCCGACAGCGTGAAGGGCAGGT
TCACCATCAGCAGGGACAACAGCAAGAACACCCTGTTCCTGCAGATGAACAGCCTGA
GGGCCGAGGACACCGCCGTGTACTACTGCGCCACCAACGACGACTACTGGGGCCAGG
GCACCCTGGTGACCGTGAGCAGCGGTGGAGGCGGTTCAGGCGGAGGTGGCTCTGGCG
GTGGCGGATCGGAGATCGTGCTGACCCAGAGCCCCGCCACCCTGAGCCTGAGCCCCG
GCGAGAGGGCCACCCTGAGCTGCAGGGCCAGCCAGAGCGTGAGCAGCTACCTGGCCT
GGTACCAGCAGAAGCCCGGCCAGGCCCCCAGGCTGCTGATCTACGACGCCAGCAACA
GGGCCACCGGCATCCCCGCCAGGTTCAGCGGCAGCGGCAGCGGCACCGACTTCACCC
 
TGACCATCAGCAGCCTGGAGCCCGAGGACTTCGCCGTGTACTACTGCCAGCAGAGCA
GCAACTGGCCCAGGACCTTCGGCCAGGGCACCAAGGTGGAGATCAAGAGATCTGAGG
GCCGCGGCAGCCTGCTGACCTGCGGCGACGTGGAGGAAAACCCCGGCCCCGGATCCA
TGAAACCAGTAACGTTATACGATGTCGCAGAGTATGCCGGTGTCTCTTATCAGACCGTT
TCCCGCGTGGTGAACCAGGCCAGCCACGTTTCTGCGAAAACGCGGGAAAAAGTGGAA
GCGGCGATGGCGGAGCTGAATTACATTCCCAACCGCGTGGCACAACAACTGGCGGGC
AAACAGTCGTTGCTGATTGGCGTTGCCACCTCCAGTCTGGCCCTGCACGCGCCGTCGC
AAATTGTCGCGGCGATTAAATCTCGCGCCGATCAACTGGGTGCCAGCGTGGTGGTGTC
GATGGTAGAACGAAGCGGCGTCGAAGCCTGTAAAGCGGCGGTGCACAATCTTCTCGC
GCAACGCGTCAGTGGGCTGATCATTAACTATCCGCTGGATGACCAGGATGCCATTGCTG
TGGAAGCTGCCTGCACTAATGTTCCGGCGTTATTTCTTGATGTCTCTGACCAGACACCC
ATCAACAGTATTATTTTCTCCCATGAAGACGGTACGCGACTGGGCGTGGAGCATCTGGT
CGCATTGGGTCACCAGCAAATCGCGCTGTTAGCGGGCCCATTAAGTTCTGTCTCGGCG
CGTCTGCGTCTGGCTGGCTGGCATAAATATCTCACTCGCAATCAAATTCAGCCGATAGC
GGAACGGGAAGGCGACTGGAGTGCCATGTCCGGTTTTCAACAAACCATGCAAATGCT
GAATGAGGGCATCGTTCCCACTGCGATGCTGGTTGCCAACGATCAGATGGCGCTGGGC
GCAATGCGCGCCATTACCGAGTCCGGGCTGCGCGTTGGTGCGGATATCTCGGTAGTGG
GATACGACGATACCGAAGACAGCTCATGTTATATCCCGCCGTTAACCACCATCAAACAG
GATTTTCGCCTGCTGGGGCAAACCAGCGTGGACCGCTTGCTGCAACTCTCTCAGGGCC
AGGCGGTGAAGGGCAATCAGCTGTTGCCCGTCTCACTGGTGAAAAGAAAAACCACCC
TGGCGCCCAATACGCAAACCGCCTCTCCCCGCGCGTTGGCCGATTCATTAATGCAGCT
GGCACGACAGGTTTCCCGACTGGAAAGCGGGCAGAGCAGCCTGAGGCCTCCTAAGAA
GAAGAGGAAGGTTGCGGCCGCTAACCAATGTGCAGACTACTGTTAACCAATGTGCAG
ACTACTGTGAATTCTAACCAATGTGCAGACTACTGTTAACCAATGTGCAGACTACTGTA
AGCTTCGAATTGAGTAGTGCTTTCTACTTTATGAGTAGTGCTTTCTACTTTATGAGTAGT
GCTTTCTACTTTATGAGTAGTGCTTTCTACTTTATGGTCGACAATACTAGTTAAGAAATC
GCCAAGCTACGGGCGGAGTACTGTCCTCCGAGCGGAGTACTGTCCTCCGAGCGGAGT
ACTGTCCTCCGAGCGGAGTACTGTCCTCCGAGCGGAGTACTGTCCTCCGAGCGGAGA
CTCTAGAAATTGTGAGCGGATAACAATTTAGGCGTGTACGGTGGGAGGCCTATATAAGC
AGAGCTCGTTTAGTGAACCGTCAGATCGCCAGCTGACAAAATTGTGAGCGCTCACAAT
TACTAGAAGATCTTGAATTCGCCACCATGGCTAGATTAGATAAAAGTAAAGTGATTAAC
AGCGCATTAGAGCTGCTTAATGAGGTCGGAATCGAAGGTTTAACAACCCGTAAACTCG
 
CCCAGAAGCTAGGTGTAGAGCAGCCTACATTGTATTGGCATGTAAAAAATAAGCGGGC
TTTGCTCGACGCCTTAGCCATTGAGATGTTAGATAGGCACCATACTCACTTTTGCCCTTT
AGAAGGGGAAAGCTGGCAAGATTTTTTACGTAATAACGCTAAAAGTTTTAGATGTGCT
TTACTAAGTCATCGCGATGGAGCAAAAGTACATTTAGGTACACGGCCTACAGAAAAAC
AGTATGAAACTCTCGAAAATCAATTAGCCTTTTTATGCCAACAAGGTTTTTCACTAGAG
AATGCATTATATGCACTCAGCGCTGTGGGGCATTTTACTTTAGGTTGCGTATTGGAAGAT
CAAGAGCATCAAGTCGCTAAAGAAGAAAGGGAAACACCTACTACTGATAGTATGCCGC
CATTATTACGACAAGCTATCGAATTATTTGATCACCAAGGTGCAGAGCCAGCCTTCTTAT
TCGGCCTTGAATTGATCATATGCGGATTAGAAAAACAACTTAAATGTGAAAGTGGGTC
GCCAAAAAAGAAGAGAAAGGTCGACGGCGGTGGTGCTTTGTCTCCTCAGCACTCTGC
TGTCACTCAAGGAAGTATCATCAAGAACAAGGAGGGCATGGATGCTAAGTCACTAACT
GCCTGGTCCCGGACACTGGTGACCTTCAAGGATGTATTTGTGGACTTCACCAGGGAGG
AGTGGAAGCTGCTGGACACTGCTCAGCAGATCGTGTACAGAAATGTGATGCTGGAGA
ACTATAAGAACCTGGTTTCCTTGGGTTATCAGCTTACTAAGCCAGATGTGATCCTCCGG
TTGGAGAAGGGAGAAGAGCCCTGGCTGGTGGAGAGAGAAATTCACCAAGAGACCCA
TCCTGATTCAGAGACTGCATTTGAAATCAAATCATCAGTTTAATACAAGGCGGCCGCAA
ATCAACATCAGTCTGATAAGCTATCAACATCAGTCTGATAAGCTATCAACATCAGTCTG
ATAAGCTATCAACATCAGTCTGATAAGCTAAAGCTTCGAATTCTGATAATCAGCCATACC
ACATTTGTAGAGGTTTTACTTGCTTTAAAAAACCTCCCACACCTCCCCCTGAACCTGAA
ACATAAAATGAATGCAATTGTTGTTGTTAACTTGTTTATTGCAGCTTATAATGGTTACAA
ATAAAGCAATAGCATCACAAATTTCACAAATAAAGCATTTTTTTCACTGCAGCTCCAGA
TTGAATTATTTGCCTGTCATACAGCTAATAATTGACCATAAGACAATTAGATTTAAATTA
GTTTTGAATCTTTCTAATACCAAAGTTCAGTTTACTGTTCCATGTTGCTTCTGAGTGGCT
TCACAGACTTATGAAAAAGTAAACGGAATCAGAATTACATCAATGCAAAAGCATTGCT
GTGAACTCTGTACTTAGGACTAAACTTTGAGCAATAACACATATAGATTGAGGATTGTT
TGCTGTTAGTATACAAACTCTGGTTCAAAGCTCCTCTTTATTGCTTGTCTTGGAAAATTT
GCTGTTCTTCATGGTTTCTCTTTTCACTGCTATCTATTTTTCTCAACCACTCACATGGCTA
CAAAAGCTTCCTGATTAATAATTACACTAAGTCAATAGGCATAGAGCCAGGACTGTTTG
GGTAAACTGGTCACTTTATCTTAAACTAAATATATCCAAAACTGAACATGTACTTAGTTA
CTAAGTCTTTGACTTTATCTCATTCATACCACTCAGCTTTATCCAGGCCACTTATTTGAC
AGTATTATTGCGAAAACTTCCTATCTAGAAGTTTGAGGAGAATATTTGTTATATTTGCAA
AATAAAATAAGTTTGCAAGTTTTTTTTTTCTGCCCCAAAGAGCTCTGTGTCCTTGAACA
 
TAAAATACAAATAACCGCTATGCTGTTAATTATTGACAAATGTCCCATTTTCAACCTAAG
GAAATACCATAAAGTAACAGATATACCAACAAAAGGTTACTAGTTAACAGGCATTGCCT
GAAAAGAGTATAAAAGAATTTCAGCATGATTTTCCATATTGTGCTTCCACCACTGCCAA
TAACAAAATAACTAGCAAGGATCCGGCCACCATGGCCCCCCGACCGATGTCAGCCTG
GGGACGAGCTCCACTTAGACGGCGAGGACGTGGCGATGGCGCATGCCGACGCGCTA
GACGATTTCGATCTGGACATGTTGGGGGACGGGGATTCCCCGGGTCCGGGATTACCC
CCCACGACTCCGCCCCCTACGGCGCTCTGGATATGGCCGACTTCGAGTTTGAGCAGAT
GTTTACCGATGCCCTTGGAATTGACGAGTACGGTGGGACGCGTATGAAGCTACTGTCTT
CTATCGAACAAGCATGCGATATTTGCCGACTTAAAAAGCTCAAGTGCTCCAAAGAAAA
ACCGAAGTGCGCCAAGTGTCTGAAGAACAACTGGGAGTGTCGCTACTCTCCCAAAAC
CAAAAGGTCTCCGCTGACTAGGGCACATCTGACAGAAGTGGAATCAAGGCTAGAAAG
ACTGGAACAGCTATTTCTACTGATTTTTCCTCGAGAAGACCTTGACATGATTTTGAAAA
TGGATTCTTTACAGGATATAAAAGCATTGTTAACAGGATTATTTGTACAAGATAATGTGA
ATAAAGATGCCGTCACAGATAGATTGGCTTCAGTGGAGACTGATATGCCTCTAACATTG
AGACAGCATAGAATAAGTGCGACATCATCATCGGAAGAGAGTAGTAACAAAGGTCAA
AGACAGTTGACTGTATAATTCACTCCTCAGGTGCAGGCTGCCTATCAGAAGGTGGTGG
CTGGTGTGGCCAATGCCCTGGCTCACAAATACCACTGAGATCTTTTTCCCTCTGCCAAA
AATTATGGGGACATCATGAAGCCCCTTGAGCATCTGACTTCTGGCTAATAAAGGAAATT
TATTTTCATTGCAATAGTGTGTTGGAATTTTTTGTGTCTCTCACTCGGAAGGACATATGG
GAGGGCAAATCATTTAAAACATCAGAATGAGTATTTGGTTTAGAGTTTGGCAACATATG
CCCATATGCTGGCTGCCATGAACAAAGGTTGGCTATAAAGAGGTCATCAGTATATGAAA
CAGCCCCCTGCTGTCCATTCCTTATTCCATAGAAAAGCCTTGACTTGAGGTTAGATTTTT
TTTATATTTTGTTTTGTGTTATTTTTTTCTTTAACATCCCTAAAATTTTCCTTACATGTTTT
ACTAGCCAGATTTTTCCTCCTCTCCTGACTACTCCCAGTCATAGCTGTCCCTCTTCTCTT
ATGGAGATCGGAGAAAGAGGTAAT (SEQ ID NO:13).
 
GTAACTATAACGGTCGCTCCGAATTTCTCGAGTTAATTAAGATTACGCCAAGCTACG
GGCGGAGTACTGTCCTCCGAGCGGAGTACTGTCCTCCGAGCGGAGTACTGTCCTCCGA
GCGGAGTACTGTCCTCCGAGCGGAGTTCTGTCCTCCGAGCGGAGACTCTAGACTCCCT
ATCAGTGATAGAGATAGGCGTGTACGGTGGGAGGCCTATATAAGCAGAGCTCGTTTAGT
GAACCGTCAGATCGCTCCCTATCAGTGATAGAGAGAATTCGACCGCCACCATGTCGGA
ATTCATGAGACATATTATCTGCCACGGAGGTGTTATTACCGAAGAAATGGCCGCCAGTC
TTTTGGACCAGCTGATCGAAGAGGTACTGGCTGATAATCTTCCACCTCCTAGCCATTTT
 
GAACCACCTACCCTTCACGAACTGTATGATTTAGACGTGACGGCCCCCGAAGATCCCA
ACGAGGAGGCGGTTTCGCAGATTTTCCCGACTCTGTAATGTTGGCGGTGCAGGAAGG
GATTGACTTACTCACTTTTCCGCCGGCGCCCGGTTCTCCGGAGCCGCCTCACCTTTCCC
GGCAGCCCGAGCAGCCGGAGCAGAGAGCCTTGGGTCCGGTTTCTATGCCAAACCTTG
TACCGGAGGTGATCGATCTTACCTGCCACGAGGCTGGCTTTCCACCCAGTGACGACGA
GGATGAAGAGGGTGAGGAGTTTGTGTTAGATTATGTGGAGCACCCCGGGCACGGTTGC
AGGTCTTGTCATTATCACCGGAGGAATACGGGGGACCCAGATATTATGTGTTCGCTTTG
CTATATGAGGACCTGTGGCATGTTTGTCTACAGTAAGTGAAAATTATGGGCAGTGGGTG
ATAGAGTGGTGGGTTTGGTGTGGTAATTTTTTTTTAATTTTTACAGTTTTGTGGTTTAA
AGAATTTTGTATTGTGATTTTTTTAAAAGGTCCTGTGTCTGAACCTGAGCCTGAGCCCG
AGCCAGAACCGGAGCCTGCAAGACCTACCCGCCGTCCTAAAATGGCGCCTGCTATCCT
GAGACGCCCGACATCACCTGTGTCTAGAGAATGCAATAGTAGTACGGATAGCTGTGAC
TCCGGTCCTTCTAACACACCTCCTGAGATACACCCGGTGGTCCCGCTGTGCCCCATTAA
ACCAGTTGCCGTGAGAGTTGGTGGGCGTCGCCAGGCTGTGGAATGTATCGAGGACTTG
CTTAACGAGCCTGGGCAACCTTTGGACTTGAGCTGTAAACGCCCCAGGCCACTCGAG
GGTACCGGGTCCGGAGCTACAAATTTTCCCTCCTCAAACAGGCTGGAGATGTCGAAG
AAAATCCTGGGCCTACCGGTCCCATGAGTGTGCCCACTCAGGTCCTGGGGTTGCTGCT
GCTGTGGCTTACAGACGCTCGCTGCGAGGTGCAGCTGGTGGAGAGCGGCGGCGGCCT
GGTGCAGCCCGGCGGCAGCCTGAGGCTGAGCTGCGCCGCCAGCGGCTTCACCTTCAG
CGACAGCTGGATCCACTGGGTGAGGCAGGCCCCCGGCAAGGGCCTGGAGTGGGTGGC
CTGGATCAGCCCCTACGGCGGCAGCACCTACTACGCCGACAGCGTGAAGGGCAGGTT
CACCATCAGCGCCGACACCAGCAAGAACACCGCCTACCTGCAGATGAACAGCCTGAG
GGCCGAGGACACCGCCGTGTACTACTGCGCCAGGAGGCACTGGCCCGGCGGCTTCGA
CTACTGGGGCCAGGGCACCCTGGTGACCGTGAGCAGCGGTGGAGGCGGTTCAGGCGG
AGGTGGCTCTGGCGGTGGCGGATCGGACATCCAGATGACCCAGAGCCCCAGCAGCCT
GAGCGCCAGCGTGGGCGACAGGGTGACCATCACCTGCAGGGCCAGCCAGGACGTGA
GCACCGCCGTGGCCTGGTACCAGCAGAAGCCCGGCAAGGCCCCCAAGCTGCTGATCT
ACAGCGCCAGCTTCCTGTACAGCGGCGTGCCCAGCAGGTTCAGCGGCAGCGGCAGCG
GCACCGACTTCACCCTGACCATCAGCAGCCTGCAGCCCGAGGACTTCGCCACCTACTA
CTGCCAGCAGTACCTGTACCACCCCGCCACCTTCGGCCAGGGCACCAAGGTGGAGAT
CAAGAGATCTGAGGGCCGCGGCAGCCTGCTGACCTGCGGCGACGTGGAGGAAAACCC
CGGCCCCGGATCCATGAAACCAGTAACGTTATACGATGTCGCAGAGTATGCCGGTGTCT
 
CTTATCAGACCGTTTCCCGCGTGGTGAACCAGGCCAGCCACGTTTCTGCGAAAACGCG
GGAAAAAGTGGAAGCGGCGATGGCGGAGCTGAATTACATTCCCAACCGCGTGGCACA
ACAACTGGCGGGCAAACAGTCGTTGCTGATTGGCGTTGCCACCTCCAGTCTGGCCCTG
CACGCGCCGTCGCAAATTGTCGCGGCGATTAAATCTCGCGCCGATCAACTGGGTGCCA
GCGTGGTGGTGTCGATGGTAGAACGAAGCGGCGTCGAAGCCTGTAAAGCGGCGGTGC
ACAATCTTCTCGCGCAACGCGTCAGTGGGCTGATCATTAACTATCCGCTGGATGACCAG
GATGCCATTGCTGTGGAAGCTGCCTGCACTAATGTTCCGGCGTTATTTCTTGATGTCTCT
GACCAGACACCCATCAACAGTATTATTTTCTCCCATGAAGACGGTACGCGACTGGGCG
TGGAGCATCTGGTCGCATTGGGTCACCAGCAAATCGCGCTGTTAGCGGGCCCATTAAG
TTCTGTCTCGGCGCGTCTGCGTCTGGCTGGCTGGCATAAATATCTCACTCGCAATCAAA
TTCAGCCGATAGCGGAACGGGAAGGCGACTGGAGTGCCATGTCCGGTTTTCAACAAA
CCATGCAAATGCTGAATGAGGGCATCGTTCCCACTGCGATGCTGGTTGCCAACGATCA
GATGGCGCTGGGCGCAATGCGCGCCATTACCGAGTCCGGGCTGCGCGTTGGTGCGGAT
ATCTCGGTAGTGGGATACGACGATACCGAAGACAGCTCATGTTATATCCCGCCGTTAAC
CACCATCAAACAGGATTTTCGCCTGCTGGGGCAAACCAGCGTGGACCGCTTGCTGCAA
CTCTCTCAGGGCCAGGCGGTGAAGGGCAATCAGCTGTTGCCCGTCTCACTGGTGAAA
AGAAAAACCACCCTGGCGCCCAATACGCAAACCGCCTCTCCCCGCGCGTTGGCCGATT
CATTAATGCAGCTGGCACGACAGGTTTCCCGACTGGAAAGCGGGCAGAGCAGCCTGA
GGCCTCCTAAGAAGAAGAGGAAGGTTGCGGCCGCTAACCAATGTGCAGACTACTGTT
AACCAATGTGCAGACTACTGTGAATTCTAACCAATGTGCAGACTACTGTTAACCAATGT
GCAGACTACTGTAAGCTTCGAATTGAGTAGTGCTTTCTACTTTATGAGTAGTGCTTTCTA
CTTTATGAGTAGTGCTTTCTACTTTATGAGTAGTGCTTTCTACTTTATGGTCGACAATACT
AGTTAAGAAATCGCCAAGCTACGGGCGGAGTACTGTCCTCCGAGCGGAGTACTGTCCT
CCGAGCGGAGTACTGTCCTCCGAGCGGAGTACTGTCCTCCGAGCGGAGTACTGTCCTC
CGAGCGGAGACTCTAGAAATTGTGAGCGGATAACAATTAGGCGTGTACGGTGGGAG
GCCTATATAAGCAGAGCTCGTTTAGTGAACCGTCAGATCGCCAGCTGACAAAATTGTG
AGCGCTCACAATTACTAGAAGATCTTGAATTCGCCACCATGGCTAGATTAGATAAAAGT
AAAGTGATTAACAGCGCATTAGAGCTGCTTAATGAGGTCGGAATCGAAGGTTTAACAA
CCCGTAAACTCGCCCAGAAGCTAGGTGTAGAGCAGCCTACATTGTATTGGCATGTAAA
AAATAAGCGGGCTTTGCTCGACGCCTTAGCCATTGAGATGTTAGATAGGCACCATACTC
ACTTTTGCCCTTTAGAAGGGGAAAGCTGGCAAGATTTTTTACGTAATAACGCTAAAAG
TTTTAGATGTGCTTTACTAAGTCATCGCGATGGAGCAAAAGTACATTTAGGTACACGGC
 
CTACAGAAAAACAGTATGAAACTCTCGAAAATCAATTAGCCTTTTATGCCAACAAGG
TTTTTCACTAGAGAATGCATTATATGCACTCAGCGCTGTGGGGCATTTTACTTTAGGTTG
CGTATTGGAAGATCAAGAGCATCAAGTCGCTAAAGAAGAAAGGGAAACACCTACTAC
TGATAGTATGCCGCCATTATTACGACAAGCTATCGAATTATTTGATCACCAAGGTGCAGA
GCCAGCCTTCTTATTCGGCCTTGAATTGATCATATGCGGATTAGAAAAACAACTTAAAT
GTGAAAGTGGGTCGCCAAAAAAGAAGAGAAAGGTCGACGGCGGTGGTGCTTTGTCTC
CTCAGCACTCTGCTGTCACTCAAGGAAGTATCATCAAGAACAAGGAGGGCATGGATGC
TAAGTCACTAACTGCCTGGTCCCGGACACTGGTGACCTTCAAGGATGTATTTGTGGAC
TTCACCAGGGAGGAGTGGAAGCTGCTGGACACTGCTCAGCAGATCGTGTACAGAAAT
GTGATGCTGGAGAACTATAAGAACCTGGTTTCCTTGGGTTATCAGCTTACTAAGCCAGA
TGTGATCCTCCGGTTGGAGAAGGGAGAAGAGCCCTGGCTGGTGGAGAGAGAAATTCA
CCAAGAGACCCATCCTGATTCAGAGACTGCATTTGAAATCAAATCATCAGTTTAATACA
AGGCGGCCGCAAATCAACATCAGTCTGATAAGCTATCAACATCAGTCTGATAAGCTATC
AACATCAGTCTGATAAGCTATCAACATCAGTCTGATAAGCTAAAGCTTCGAATTCTGAT
AATCAGCCATACCACATTTGTAGAGGTTTTACTTGCTTTAAAAAACCTCCCACACCTCC
CCCTGAACCTGAAACATAAAATGAATGCAATTGTTGTTGTTAACTTGTTTATTGCAGCT
TATAATGGTTACAAATAAAGCAATAGCATCACAAATTTCACAAATAAAGCATTTTTTTCA
CTGCAGCTCCAGATTGAATTATTTGCCTGTCATACAGCTAATAATTGACCATAAGACAAT
TAGATTTAAATTAGTTTTGAATCTTTCTAATACCAAAGTTCAGTTTACTGTTCCATGTTG
CTTCTGAGTGGCTTCACAGACTTATGAAAAAGTAAACGGAATCAGAATTACATCAATG
CAAAAGCATTGCTGTGAACTCTGTACTTAGGACTAAACTTTGAGCAATAACACATATAG
ATTGAGGATTGTTTGCTGTTAGTATACAAACTCTGGTTCAAAGCTCCTCTTTATTGCTTG
TCTTGGAAAATTTGCTGTTCTTCATGGTTTCTCTTTTCACTGCTATCTATTTTTCTCAACC
ACTCACATGGCTACAAAAGCTTCCTGATTAATAATTACACTAAGTCAATAGGCATAGAG
CCAGGACTGTTTGGGTAAACTGGTCACTTTATCTTAAACTAAATATATCCAAAACTGAA
CATGTACTTAGTTACTAAGTCTTTGACTTTATCTCATTCATACCACTCAGCTTTATCCAGG
CCACTTATTTGACAGTATTATTGCGAAAACTTCCTATCTAGAAGTTTGAGGAGAATATTT
GTTATATTTGCAAAATAAAATAAGTTTGCAAGTTTTTTTTTCTGCCCCAAAGAGCTCTG
TGTCCTTGAACATAAAATACAAATAACCGCTATGCTGTTAATTATTGACAAATGTCCCAT
TTTCAACCTAAGGAAATACCATAAAGTAACAGATATACCAACAAAAGGTTACTAGTTAA
CAGGCATTGCCTGAAAAGAGTATAAAAGAATTTCAGCATGATTTTCCATATTGTGCTTC
CACCACTGCCAATAACAAAATAACTAGCAAGGATCCGGCCACCATGGCCCCCCCGACC
 
GATGTCAGCCTGGGGGACGAGCTCCACTTAGACGGCGAGGACGTGGCGATGGCGCAT
GCCGACGCGCTAGACGATTTCGATCTGGACATGTTGGGGGACGGGGATTCCCCGGGTC
CGGGATTTACCCCCCACGACTCCGCCCCCTACGGCGCTCTGGATATGGCCGACTTCGA
GTTTGAGCAGATGTTTACCGATGCCCTTGGAATTGACGAGTACGGTGGGACGCGTATG
AAGCTACTGTCTTCTATCGAACAAGCATGCGATATTTGCCGACTTAAAAAGCTCAAGTG
CTCCAAAGAAAAACCGAAGTGCGCCAAGTGTCTGAAGAACAACTGGGAGTGTCGCTA
CTCTCCCAAAACCAAAAGGTCTCCGCTGACTAGGGCACATCTGACAGAAGTGGAATC
AAGGCTAGAAAGACTGGAACAGCTATTTCTACTGATTTTTCCTCGAGAAGACCTTGAC
ATGATTTTGAAAATGGATTCTTTACAGGATATAAAAGCATTGTTAACAGGATTATTTGTA
CAAGATAATGTGAATAAAGATGCCGTCACAGATAGATTGGCTTCAGTGGAGACTGATAT
GCCTCTAACATTGAGACAGCATAGAATAAGTGCGACATCATCATCGGAAGAGAGTAGT
AACAAAGGTCAAAGACAGTTGACTGTATAATTCACTCCTCAGGTGCAGGCTGCCTATC
AGAAGGTGGTGGCTGGTGTGGCCAATGCCCTGGCTCACAAATACCACTGAGATCTTTT
TCCCTCTGCCAAAAATTATGGGGACATCATGAAGCCCCTTGAGCATCTGACTTCTGGCT
AATAAAGGAAATTTATTTTCATTGCAATAGTGTGTTGGAATTTTTTGTGTCTCTCACTCG
GAAGGACATATGGGAGGGCAAATCATTTAAAACATCAGAATGAGTATTTGGTTTAGAG
TTTGGCAACATATGCCCATATGCTGGCTGCCATGAACAAAGGTTGGCTATAAAGAGGTC
ATCAGTATATGAAACAGCCCCCTGCTGTCCATTCCTTATTCCATAGAAAAGCCTTGACTT
GAGGTTAGATTTTTTTTATATTTTGTTTTGTGTTATTTTTTTCTTTAACATCCCTAAAATTT
TCCTTACATGTTTTACTAGCCAGATTTTTCCTCCTCTCCTGACTACTCCCAGTCATAGCT
GTCCCTCTTCTCTTATGGAGATCGGAGAAAGAGGTAAT (SEQ ID NO:14).
 wherein SEQ ID NO: 9 is the sequence of an EBFP-bearing nucleic acid carried by an adenoviral vector;
SEQ ID NO: 10 is the sequence of an hIL-2-bearing nucleic acid carried by an adenoviral vector;
SEQ ID NO: 11 is the sequence of an hGMCSF-bearing nucleic acid carried by an adenoviral vector;
SEQ ID NO: 12 is the sequence of an mGMCSF-bearing nucleic acid carried by an adenoviral vector;
SEQ ID NO: 13 is the sequence of an anti-PD-1 scFv-bearing nucleic acid carried by an adenoviral vector; and
SEQ ID NO: 14 is the sequence of an anti-PD-Ll scFv-bearing nucleic acid carried by an adenoviral vector.

In an embodiment of the present disclosure, the adenovirus is obtained by the following steps: removing the adenoviral E1 gene and a part of the adenoviral E3 gene associated with adenoviral replication and packaging from an Adenoviral vector; inserting the adenoviral E1A gene into a gene circuit by a stepwise Golden Gate method; and incorporating the gene circuit into the adenoviral vector by the Gateway or Gibson method. The method of obtaining adenovirus as described above enables rapidly engineering of a complex and large-fragment oncolytic adenoviral vector.

In a second aspect, the present disclosure in embodiments provides a recombinant virus. In an embodiment of the present disclosure, the recombinant virus comprises: a first nucleic acid molecule, comprising a tumor cell-specific promoter, the tumor cell-specific promoter being an alpha-fetoprotein-specific promoter; a second nucleic acid molecule, operably linked to the first nucleic acid molecule and encoding a transcriptional activator, the transcriptional activator being Gal4VP16; a third nucleic acid molecule, comprising a first recognition sequence of the transcriptional activator, the first recognition sequence being 5 × UAS; a fourth nucleic acid molecule, operably linked to the third nucleic acid molecule and comprising a first promoter and a first regulatory element, wherein the first promoter is a miniCMV, and the first regulatory element comprises a plurality of repeated tetO sequences, and at least one of the pluralities of repeated tetO sequences is set downstream of the first promoter; a fifth nucleic acid molecule, operably linked to the fourth nucleic acid molecule and encoding a first regulatory protein, the first regulatory protein being LacI, wherein the fifth nucleic acid molecule further comprises a sequence encoding a protein of interest, the protein of interest comprises a viral replication protein and an immune effector, wherein the immune effector is expressed in the form of an individual protein or a fusion protein, the viral replication protein and the immune effector are linked by a cleavable linker peptide, the protein of interest and the first regulatory protein are expressed in the form of a fusion protein, and the protein of interest and the first regulatory protein are linked by a cleavable linker peptide; a sixth nucleic acid molecule, comprising a second recognition sequence of the transcriptional activator, the second recognition sequence being 5 × UAS; a seventh nucleic acid molecule, operably linked to the sixth nucleic acid molecule and comprising a second promoter and a second regulatory element, wherein the second promoter is a miniCMV, the second regulatory element comprises a plurality of repeated LacO sequences, at least one of the pluralities of repeated LacO sequences is inserted downstream of the second promoter; an eighth nucleic acid molecule, operably linked to the seventh nucleic acid molecule and encoding a second regulatory protein, the second regulatory protein being tetR-KRAB; a ninth nucleic acid molecule, operably linked to the fifth nucleic acid molecule and configured to conditionally inhibit expression of the first regulatory protein, wherein the ninth nucleic acid molecule comprises a nucleic acid sequence specifically recognized by a first microRNA, the first microRNA being a normal cell-specific microRNA; and a tenth nucleic acid molecule, operably linked to the eighth nucleic acid molecule and configured to conditionally inhibit expression of the second regulatory protein, wherein the tenth nucleic acid molecule comprises a nucleic acid sequence specifically recognized by a second microRNA, the second microRNA being a tumor cell-specific microRNA, wherein the first regulatory element is adapted to inhibit the function of the first promoter by binding to the second regulatory protein, and the second regulatory element is adapted to inhibit the function of the second promoter by binding to the first regulatory protein. With the recombinant virus according to the embodiment of the present disclosure, the first regulatory protein LacI and the protein of interest are specifically expressed in tumor cells and the second regulatory protein tetR-KRAB is not expressed or low-expressed specifically in tumor cells under the coregulation of the alpha-fetoprotein-specific promoter, the ninth nucleic acid molecule and the tenth nucleic acid molecule, thus the tetR-KRAB- mediated inhibition mechanism on the first promoter miniCMV is released, therefore the first regulatory protein LacI and the protein of interest are effectively expressed under the control of the first promoter miniCMV, and the function of the second promoter miniCMV is effectively inhibited via the LacI-mediated inhibition mechanism, and the expression of tetR-KRAB is further inhibited. Furthermore, proteins like the target protein LacI are specifically expressed in tumor cells and the proteins like tetR-KRAB are not specifically expressed in tumor cells by using the recombinant virus according to the embodiment of the present disclosure, with high efficiency and specificity.

In embodiments of the present disclosure, the recombinant virus may further comprise at least one of additional technical features as follows.

In an embodiment of the present disclosure, the recombinant virus comprises at least one selected from the group consisting of a retrovirus, an adenovirus, a herpes virus and a vaccinia virus.

In an embodiment of the present disclosure, the recombinant virus is an adenovirus. As mentioned above, adenovirus as a gene therapy vector has advantages of a wide range of hosts, low pathogenicity to humans, infection and gene expression in proliferating and non-proliferating cells, high titer, homology to human genes, and no mutagenicity after insertion, as well as amplifying in a suspension and simultaneously expressing multiple genes.

In an embodiment of the present disclosure, the immune effector comprises at least one sequence selected from the group consisting of an inhibitory sequence that antagonizes PD-1 gene, an inhibitory sequence that antagonizes PD-L1 gene, an inhibitory sequence that antagonizes CTLA4 gene, an inhibitory sequence that antagonizes Tim-3 gene, IL-2, IL-12, IL-15 and GM-CSF. Optionally, the immune effector may be present in the form of a fusion protein.

In a third aspect, the present disclosure in embodiments provides a recombinant cell. In an embodiment of the present disclosure, the recombinant cell comprises the expression system as described above. The recombinant cell according to the embodiment of the present disclosure can effectively activate the systemic immune response in human body, and attack xenogeneic cells such as tumor cells, with high safety and specificity.

In embodiments of the present disclosure, the recombinant cell may further comprise at least one of additional technical features as follows.

In an embodiment of the present disclosure, at least a portion of the expression system is integrated into the genome of the recombinant cell. The expression system replicates as the recombinant cell genome replicates, where the expression system can still effectively regulate the expression of the protein of interest.

In a fourth aspect, the present disclosure in embodiments provides use of the aforementioned expression system, the aforementioned recombinant virus, and the aforementioned recombinant cells in the preparation of a medicament for the treatment of a cancer. The expression system described in the present disclosure allows the protein of interest to be specifically expressed in tumor cells, and the medicament in the embodiment exhibits stronger efficacy, specificity and safety on the treatment of cancer.

In embodiments of the present disclosure, the use as described above can further comprise at least one of additional technical features as follows.

In an embodiment of the present disclosure, the cancer comprises liver cancer, lung cancer, colorectal cancer, melanoma, breast cancer or prostate cancer. The inventors have found that the medicament in the embodiment has a more significant efficacy on the treatment of liver cancer, lung cancer, colorectal cancer, melanoma, breast cancer and prostate cancer.

In a fifth aspect, the present disclosure in embodiments provides a method of expressing a protein of interest by use of an expression system, wherein the expression system is the expression system as described above. In an embodiment of the present disclosure, the method comprises: (1) providing a fifth nucleic acid molecule which comprises a nucleic acid sequence encoding the protein of interest; and (2) inhibiting expression of a second regulatory protein by a tenth nucleic acid molecule so as to express the protein of interest. With the method of expressing a protein of interest as described above in the embodiment, the second regulatory protein-mediated inhibition mechanism on the first promoter is released, thus the protein of interest is effectively expressed in specific cells under the coaction of the cell-specific promoter and the second promoter.

In embodiments of the present disclosure, the above method can further comprise at least one of additional technical features as follows.

In an embodiment of the present disclosure, the expression is carried out in a cell. The cells can provide a microenvironment for the expression of the protein of interest, and the expression of the protein of interest in the cell is more efficient.

In an embodiment of the present disclosure, the tenth nucleic acid molecule comprises a nucleic acid sequence specifically recognized by a second microRNA, and the step (2) further comprises contacting the second microRNA with the tenth nucleic acid molecule.

In a sixth aspect, the present disclosure in embodiments provides a method of expressing a protein of interest by use of an expression system, wherein the expression system is the expression system as described above. In an embodiment of the present disclosure, the method comprises: (1) providing an eighth nucleic acid molecule which comprises a nucleic acid sequence encoding a first protein of interest, and providing a fifth nucleic acid molecule which comprises a nucleic acid sequence encoding a second protein of interest; and (2) expressing the first protein of interest or the second protein of interest by inhibiting expression of the first regulatory protein by the ninth nucleic acid molecule so as to express the first protein of interest; or inhibiting expression of the second regulatory protein by the tenth nucleic acid molecule so as to express the second protein of interest. With the method of expressing a protein of interest as described above in the embodiment, the second regulatory protein-mediated inhibition mechanism on the first promoter is released, thus the second protein of interest is effectively expressed in specific cells under the coaction of the cell-specific promoter and the first promoter; or the first regulatory protein-mediated inhibition mechanism on the second promoter is released, thus the first protein of interest is effectively expressed in specific cells under the coaction of the cell-specific promoter and the second promoter.

It should be noted that the fusion protein described herein refers to proteins that are co-transcribed under the control of a same promoter, including a fusion protein that is not linked between proteins, or a fusion protein that is linked by other linker peptides (such as GGGS or 2A sequences).

### BRIEF DESCRIPTION OF THE DRAWINGS

Figure 1 is a schematic diagram showing an initial structure of an adenovirus engineered by the Gateway system.
Figure 2 is a schematic diagram showing an initial structure of an adenovirus engineered by the Golden Gate system.
Figure 3 is a schematic diagram showing a mutually repressive switch according to an embodiment of the present disclosure.
Figure 4 is a graph showing the expression level of alpha-fetoprotein-specific promoter (hAFP) in cell lines Chang, HepG2, Huh7, Hep3B, PLC and Hepa1-6 by the fluorescence quantitative PCR method according to an embodiment of the present disclosure.
Figure 5 is a schematic diagram showing the modification of human AFP promoter according to an embodiment of the present disclosure.
Figure 6 is a graph showing the results of transient transfection of a transmission system of the modified AFP promoter to Chang and HepG2 according to an embodiment of the present disclosure.
Figure 7 is a schematic diagram showing microRNA detection systems according to an embodiment of the present disclosure.
Figure 8 is a graph showing the significantly different expression of microRNA markers in different cell lines according to an embodiment of the present disclosure.
Figure 9 is a schematic diagram of a mutually repressive switch based on LacI and tetR-KRAB genes according to an embodiment of the present disclosure.
Figure 10 is a graph showing the effective reversal of the mutually repressive switch in response to artificially synthesized shRNA signals according to an embodiment of the present disclosure.
Figure 11 is schematic diagrams showing the mutually repressive switch in response to different activators according to some embodiments of the present disclosure.
Figure 12 is a schematic diagram showing the first step of the CGGA (Cascade Golden-Gate and Gateway/Gibson Assemble method) according to an embodiment of the present disclosure.
Figure 13 is a schematic diagram showing the second step of the CGGA (Cascade Golden-Gate and Gateway/Gibson Assemble method) according to an embodiment of the present disclosure.
Figure 14 is a schematic diagram showing the third step of the CGGA (Cascade Golden-Gate and Gateway/Gibson Assemble method) according to an embodiment of the present disclosure.
Figure 15 is graphs showing the detection of adenovirus at a cellular level according to some embodiments of the present disclosure.
Figure 16 is a graph showing activities of cytokines (expressed by a plasmid vector) detected *in vitro* according to some embodiments of the present disclosure.
Figure 17 is graphs showing activities of cytokines (expressed by a viral vector) detected *in vitro* according to some embodiments of the present disclosure.
Figure 18 is schematic diagrams showing the treatment of nude mouse model bearing HepG2, Huh7 and Hepa1-6 respectively by using an oncolytic adenovirus according to some embodiments of the present disclosure.
Figure 19 is schematic diagrams showing the treatment of C57 mouse model bearing Hepa1-6 by using an oncolytic adenovirus according to an embodiment of the present disclosure.
Figure 20 is graphs showing improved expression of IFN-γ and Ki67 markers among the tumor filtrating T cells after the treatment with oncolytic adenovirus according to an embodiment of the present disclosure.
Figure 21 is a graph showing the re-challenge of mouse hepatoma Hepa1-6 cells on mouse after treatment according to an embodiment of the present disclosure.
Figure 22 shows a model of tumor-virus- effector system according to an embodiment of the present disclosure.
Figure 23 is a graph showing the minimal tumor size from an initial state to a final state over time under different initial tumor size, viral titer and viral replication rate during the simulation of the tumor-virus system according to an embodiment of the present disclosure.
Figure 24 is a graph showing the tumor size in the final state under the different inhibitory effects of immune cells on tumors and viruses when simulated by the tumor-virus- effector system according to an embodiment of the present disclosure.

### DETAILED DESCRIPTION

Embodiments of the present disclosure are described in detail below. The embodiments described below are illustrative only and are not to be construed as limiting the present disclosure.

### Expression system

In a first aspect of the present disclosure, provided in embodiments is an expression system. According to an embodiment of the present disclosure, the expression system comprises: a first nucleic acid molecule, incorporating a cell-specific promoter; a second nucleic acid molecule, operably linked to the first nucleic acid molecule and encoding a transcriptional activator; a third nucleic acid molecule, incorporating a first recognition sequence of the transcriptional activator; a fourth nucleic acid molecule, operably linked to the third nucleic acid molecule and incorporating a first promoter and a first regulatory element; a fifth nucleic acid molecule, operably linked to the fourth nucleic acid molecule and encoding a first regulatory protein; a sixth nucleic acid molecule, incorporating a second recognition sequence of the transcriptional activator; a seventh nucleic acid molecule, operably linked to the sixth nucleic acid molecule and incorporating a second promoter and a second regulatory element; an eighth nucleic acid molecule, operably linked to the seventh nucleic acid and encoding a second regulatory protein; and at least one selected from the group consisting of: a ninth nucleic acid molecule, operably linked to the fifth nucleic acid molecule and configured to conditionally inhibit expression of the first regulatory protein; a tenth nucleic acid molecule, operably linked to the eighth nucleic acid molecule and configured to conditionally inhibit expression of the second regulatory protein, wherein the first regulatory element is adapted to inhibit the function of the first promoter by binding to the second regulatory protein, and the second regulatory element is adapted to inhibit the function of the second promoter by binding to the first regulatory protein. With the expression system according to the embodiment of the present disclosure, the gene of interest can be specifically expressed under a specific cellular environment while being regulated in a mutual repression way, with strong control, high efficiency and specificity.

According to an embodiment of the present disclosure, the cell-specific promoter is a tumor cell-specific promoter. The tumor cell-specific promoter is at least one selected from the group consisting of an alpha-fetoprotein-specific promoter (AFP), a Survivin gene promoter (SUR), a human telomerase reverse transcriptase (hTERT) gene promoter, a cholecystokinin A receptor gene promoter (CCKAR promoter), a carcinoembryonic antigen promoter (CEA promoter), a proto-oncogene human epidermal growth factor receptor 2 promoter (Human epidermal growth factor receptor-2, HER2), a prostaglandin oxidase reductase 2 promoter (Cyclooxygenase 2, COX2), a chemokine receptor-4 (CXCR4), an E2F-1 gene promoter (E2F-1promoter), a mucin promoter (Mucin1MUC1), a prostate specific antigen (PSA), a human tyrosine related protein 1 (TRP1) and a tyrosinase (Tyr) promoter. The expression system in the embodiment can be initiated in the microenvironment of specific tumor cells under the control of the tumor cell-specific promoters described as above, thereby further enhancing the specificity of gene expression and regulation.

According to an embodiment of the present disclosure, the transcriptional activator is at least one selected from the group consisting of Gal4VP16, Gal4esn, Gal4VP64, dCas9-VP16, dCas9-VP64, dCas9-VPR, dCas9-VTR and rtTA. The yeast Gal4-UAS gene expression system is one of the most well understood transcriptional regulatory systems of eukaryotic cells, and the Gal4 gene encodes a transcriptional activator protein in yeast (*Saccharomyces cerevisiae*)*.* Gal4 can recognize a 17 bp of sequence in the upstream activation sequence (UAS) of gene promoter, that is, 5'-CGGRNNRCYNYNYNCNCCG-3' (R represents purine, Y represents pyrimidine, and N represents any deoxynucleotide). Gal4 interacts with Gal80 which binds to galactose metabolites. Gal4 comprises two domains (a DNA binding domain (N-terminal domain) and an activation domain (C-terminal domain)) and further comprises a zinc cluster domain (specifically a Zn(2)-Cys(6) dual core cluster domain), which usually functions in a form of a homodimer. The core sequence in the UAS sequence that binds to the GAL4 protein is a four-copy tandem repeat, each copy containing 17 base pairs (i.e. the sequence 5'-CGGAGTACTGTCGTGGG-3'). Gal4, present in mammalian cells, activates target genes only when UAS is recognized, thus initiating the transcription of downstream genes, with good specificity and inducibility. The activation domain of Gal4 protein can be replaced with the activation domain of herpes simplex virus VP16 protein or 4 copies of VP16 (VP64). The formed fusion transcription factor increases the transcriptional activation activity of Gal4 protein while maintaining the binding specificity of Gal4 protein. The tetracycline-inducing system is a bacterial-derived transcriptional activation system for eukaryotic cells, including tetracycline response elements and tetracycline-inducing proteins. The Tet response element (TRE) contains 7 repeats of 19-nucleotide tetracycline operon sequence (i.e. 5'-TCCCTATCAGTGATAGAGA-3'), which can be recognized by tetracycline-inducing proteins. The tetracycline-inducing system includes a tetracycline-inducing repression system and a tetracycline-inducing activation system. The tetracycline-inducing repression system comprises a tetracycline response element and a tetracycline repressor tetR. When tetracycline is absent, the expression of downstream genes is activated by the binding of the repressor to the response element; and when tetracycline is present, the repressor binds to tetracycline, thus inhibiting the expression of downstream genes. The tetracycline-inducing activation system comprises a tetracycline response element and a reverse tetracycline-controlled transactivator rtTA. When tetracycline is absent, rtTA cannot bind to the TRE element, thus cannot activate the expression of downstream genes; while when tetracycline is present, rtTA binds to TRE under the action of tetracycline, thus activating the expression of downstream genes.

Nuclease deactivated Cas9 (dCas9) is a mutant form of Cas9, which can recognize a target sequence via gRNA mediation, only with specific recognition and binding on DNA and without cleavage activity on target sequence. When dCas9 is fusion-expressed along with activation elements, the expression of target genes can be effectively activated.

According to an embodiment of the present disclosure, the first recognition sequence and the second recognition sequence are each independently selected from at least one of 5 × UAS, 7 × tetO and a target sequence of dCas9. 5 × UAS is 5 copies of response elements of Gal4VP16 regulatory system. The core sequence in the UAS sequence that binds to the Gal4 protein is a four-copy tandem repeat, each copy containing 17 base pairs (sequence 5'-CGGAGTACTGTCGTGGG-3'). 7 × tetO is 7 copies of TREs which are a response element of tetracycline regulatory system. The Tet response element (TRE) contains 7 repeats of 19-nucleotide tetracycline operon sequence (5'-TCCCTATCAGTGATAGAGA-3'), which can be recognized by tetracycline-inducing proteins.

According to an embodiment of the present disclosure, the first promoter and the second promoter are miniCMV. 5 × UAS-miniCMV is a synthetic inducible promoter. 5 × UAS is a recognition sequence of Gal4 protein, and miniCMV is a part of promoter sequence of Cytomegalovirus. TRE is also an inducible promoter. For 7 × tetO-miniCMV, tetO is a recognition sequence of the response element of tetracycline system.

According to an embodiment of the present disclosure, the first regulatory protein and the second regulatory protein are each independently selected from at least one of LacI, tetR, zinc finger, KRAB and dCas9-KRAB. Lactose repressor (LacI) binds to the major groove of lactose operon (LacO) through recognizing specific sequences via the helix-turn-helix element of its own DNA-binding domain, as well as tightly binding to base sequence on the minor groove of LacO via symmetry-related α-helix residues. Such a tight binding causes high affinity binding of RNA polymerase to promoter region and prevents DNA extension, thereby inhibiting mRNA transcription and expression of downstream genes. The tetracycline repressor (TetR) contains a conserved helix-turn-helix DNA binding region (forming a homodimer) which binds to the tetracycline operon (tetO), thus the transcriptional expression of downstream genes is inhibited. Zinc finger protein consists of a ring containing about 30 amino acids and a Zn²⁺ coordinated to 4 Cys proteins (or 2 Cys) and 2 His proteins on the ring, forming a structure in a shape of a finger. Zinc finger proteins are a class of proteins that play an important role in gene regulation. Zinc finger proteins can be mainly classified into C2H2 type, C4 type and C6 type depending to their conserved domains. Zinc finger can regulate gene expression, cell differentiation and embryonic development at transcriptional and translational levels by specifically binding to target sequences of DNA, RNA, DNA-RNA molecules, or to its own or other zinc finger proteins. The Krüppel associated box (KRAB) domain is a class of transcriptional repression regions which is present in nearly 400 zinc finger proteins in humans. KRAB generally consists of 75 amino acid residues and its smallest effective inhibition region consists of 45 amino acids, which plays its role through amphipathic helical connection between proteins. KRAB can be divided into two regions (A box and B box) which are encoded by different exons, where A box plays a central role in transcriptional repression, and B box enhances the transcriptional repression of A box.

Nuclease deactivated Cas9 (dCas9) is a mutant form of Cas9, which can recognize a target sequence via gRNA mediation, only with specific recognition and binding activities on DNA sequences and without cleavage activity on target sequences. When dCas9 is fusion-expressed along with repression regions, the expression of target genes can be effectively inhibited.

According to an embodiment of the present disclosure, the first regulatory element and the second regulatory element are each independently selected from at least one of a tetO, a LacO, a zinc finger target site, and a target sequence of dCas9. The tetracycline operon (tetO) can be recognized by tetracycline repressors to inhibit the expression of downstream genes. The lactose operon (LacO) can be recognized by lactose repressors (LacI) to inhibit the expression of downstream genes. The zinc finger target site can be recognized by zinc finger proteins to regulate the expression of downstream genes. The dCas9 target sequence can be recognized by dCas9-KRAB with the aid of a complementary gRNA to inhibit the expression of downstream genes.

According to an embodiment of the present disclosure, the first regulatory protein is LacI, and the second regulatory element comprises a plurality of repeated LacO sequences, at least one of the pluralities of repeated LacO sequences being set downstream of the second promoter. The expressed LacI can specifically bind to LacO sequence, thereby inhibiting the function of the second promoter. According to the LacI/LacO repression system of the embodiment of the present disclosure, Lactose repressor (LacI) binds to the major groove of lactose operon (LacO) through recognizing specific sequences via the helix-turn-helix element of its own DNA-binding domain, as well as tightly binds to base sequence on the minor groove of LacO via symmetry-related α-helix residues. Such a tight binding causes high affinity binding of RNA polymerase to promoter regions and prevents DNA extension, thereby inhibiting mRNA transcription and expression of downstream genes. According to an embodiment of the present disclosure, two sides of miniCMV sequence in 5 × UAS-miniCMV promoter are each inserted with LacO by the inventors, which can effectively inhibit the expression of downstream genes of the promoter as shown by experiments. However, the inventors do not exclude other repression systems which can be used at those locations.

According to an embodiment of the present disclosure, the second regulatory protein is tetR-KRAB, and the first regulatory element comprises a plurality of repeated tetO sequences, at least one of the pluralities of repeated tetO sequences being set downstream of the first promoter. According to the tetR-KRAB/tetO repression system in the embodiment of the present disclosure, the tetracycline repressor (TetR) contains a conserved helix-turn-helix DNA binding region (forming a homodimer), which binds to tetracycline operon (tetO) to inhibit the transcriptional expression of downstream genes. In this embodiment, two sides of miniCMV sequence in 5 × UAS-miniCMV promoter are each inserted with tetO by inventors, which can effectively inhibit the expression of downstream genes of the promoter as shown by experiments. The switch system comprising the first regulatory protein and the second regulatory protein can effectively respond to corresponding input signals, thus effectively distinguishing different cell lines. However, the inventors do not exclude other repression systems which can be used at those locations.

According to an embodiment of the present disclosure, at least one of the fifth nucleic acid molecule and the ninth nucleic acid molecule further comprises a sequence encoding a protein of interest. With the expression system according to the embodiment of the present disclosure, the protein of interest can be specifically expressed in a specific cellular microenvironment, while being regulated in a mutual repression way. According to a specific embodiment of the present disclosure, the expression system exhibits a significant increased specificity and regulation on the expression of the protein of interest.

According to an embodiment of the present disclosure, the fifth nucleic acid molecule comprises a sequence encoding the protein of interest, and the protein of interest comprises at least one selected from the group consisting of a viral replication and packaging protein, and an immune effector. The viral replication and packaging protein can effectively ensure the survival and replication of the expression system vector in a host. The expression of the immune effector can effectively activate the immune system in body, thereby promoting the immune killing to specific cells, such as tumor cells.

According to an embodiment of the present disclosure, the virus replication and packaging protein comprises at least one selected from the group consisting of an adenovirus E1 gene, an adenovirus E1A gene, an adenovirus E1B gene, an adenovirus E2 gene and an adenovirus E4 gene. According to the timing of transcription, adenoviral genes are mainly divided into early expression genes (E1 ∼ 4) and late expression genes (L1 ∼ 5). After the adenoviral genome enters the nucleus, the cellular transcription factor firstly binds to the enhancer located upstream of the E1A region, expressing E1A protein which regulates cellular metabolism and makes the viral DNA replicated in cells more easily. The E1A protein also can activate promoters of other early genes (E1B, E2A, E2B, E3 and E4), where E2B drives the expression of three additional transcriptional units of early genes involving in viral replication (i.e. a precursor terminal protein (pTP), a single-stranded DNA binding protein (ssDBP) and a DNA polymerase (DNA pol)). Expression products of these three genes are tightly bound to be a complex, which interacts with at least three cellular proteins to initiate viral genome replication. The expression products of E1 region gene are divided into E1A and E1B. E1A mainly consists of two components, that is 289R (or 13S) and 243R (or 12S). The main function of the E1A protein is in regulating cellular metabolism, thus making cells more susceptible to viral replication. E1B19K is homologous to the expression product of Bcl-2 gene, and can prevent cells from apoptosis or necrosis by inactivating and clearing Bax family members. The E1B55K gene product can down-regulate the transcription level of p53 gene, with other adenoviral genes (such as E4 or f6) involved. The E1B55K gene product is also involved in viral replication, transcription of viral late mRNAs, and transportation of viral RNAs. The expression products of E2 region gene are divided into E2A and E2B, where E2A is a DNA binding protein (DBP), and E2B mainly has two products (i.e. a precursor terminal protein (pTP) and a viral DNA polymerase (pol) respectively). Such three proteins at least interact with three intracellular factors to initiate adenoviral DNA replication as well as transcription and translation of viral late genes. The gene products of E4 region gene are usually referred as orf 1 to 6/7, mainly involving in metabolism of viral messenger RNA, as well as promoting viral DNA replication and shutting down protein synthesis in a host. Studies have found that some E4 products can bind to DNA-activated protein kinases to prevent viral DNA from concatenating. Since this kinase can activate p53 gene, it is believed that some products of E4 region gene can inhibit cell apoptosis. Many products of E1B and E4 genes are involved in antagonizing the E1A protein. For example, E4 inhibits the activation of E2F promoter via E1A.

According to an embodiment of the present disclosure, the immune effector comprises at least one sequence selected from the group consisting of an inhibitory sequence that antagonizes the PD-1 gene, an inhibitory sequence that antagonizes the PD-L1 gene, an inhibitory sequence that antagonizes the CTLA4 gene, an inhibitory sequence that antagonizes the Tim-3 gene, GM - CSF, IL-2, IL-12, IL-15 or a fusion expression form of these factors. Programmed death 1(programmed death receptor 1, PD-1) is an important immunosuppressive molecule belonging to a member of CD28 superfamily, which is originally cloned from apoptotic mouse T cell hybridoma 2B4.11. PD-1 is an important inhibitory molecule on the surface of T cells, and its intracellular domain comprises an immunoreceptor tyrosine inhibitory motif (ITIM) and an immunoreceptor tyrosine transfer motif (ITSM). ITSM mediates the recruitment of protein tyrosine phosphatase family phosphatase and the inhibition of T cell activation signals. Ligands of ITSM are PD-L1 and PD-L2, which mainly play a major role in inhibiting activation of T cells in the tumor microenvironment during immune responses of immune system. Programmed cell death 1 ligand (PD-L1), also known as a cluster of differentiation 274 (CD274) or a B7 homolog (B7 homolog 1, B7-H1), is a human protein in body that is encoded by CD274 gene. PD-L1 is expressed inductively in a variety of tumor cells and hematopoietic cells in tumor cell microenvironments, and its expression level is positively correlated with the malignancy of tumors. PD-1 antibody and PD-L1 antibody can block the binding of PD-1 to PD-L1. However, tumor cells in tissues can escape from immune system through the PD-1/PD-L1 pathway. Cytotoxic T lymphocyte associate protein-4, short for CTLA-4, is a type of co-stimulatory molecule expressed on the surface of T cells. CTLA-4 is capable of specifically binding to CD80/CD86 on the surface of APC to activate downstream signals during activation of T cells, similar to the function of CD28. Studies have found that cells that predominantly express CTLA-4 among T cells are regulatory T cells (Treg), which is a class of T cells that negatively regulate cellular immunity. In the absence of CTLA-4 receptor, mice exhibit T cell overexpression, thus accompanied with severe autoimmune diseases. The above results show that Treg needs CTLA-4 to exercise its function. In addition, CTLA-4 is also expressed in conT cells, which plays a role in inhibiting the signaling of T cell activation. T cell immunoglobulin and mucin-domain containing molecule (TIM) gene family is a novel gene family discovered by Mclntire in 2001 in search of mouse asthma susceptibility genes, which is further identified by genomic analysis and positional cloning. The TIM family gene contains immunoglobulin V region and mucin region. Tim-3 is an important member of the TIM family and expresses a negative regulatory molecule on the surface of activated Th1 cells. Currently, it is found that TIM3 is expressed in CD8⁺ T cells, Th17 cells, Treg, NK cells and other lymphocyte subsets. Granulocyte-macrophage Colony Stimulating Factor (GM-CSF) is a monomeric glycoprotein cytokine secreted by macrophages, T cells, NK cells and the like, which stimulates stem cells to produce granules cells (neutrophils, eosinophils, basophils and monocytes). GM-CSF also affects mature cells in the immune system, such as inhibiting the transfer of neutrophils and altering expression of receptor on the cell surface. This factor also can inhibit fungal infections by activating macrophages. Interleukin 2 (IL-2) can mediate cytokines between white blood cells and white blood cells as well as between white blood cells and other cells. IL-2 is mainly produced by activated T cells, and acts on local target cells in a manner of autocrine and paracrine, which is a major cytokine involving in immune response, thus exhibiting a significant immune effect. IL-2 can also promote T cell proliferation and produce cytokines, promote B cell proliferation and secrete Ig, activate macrophages and enhance activation and proliferation of NK cells. In addition, IL-2 also has a negative regulation effect, which can induce apoptosis of T cells which activate Ag, limit the intensity of immune response, and avoid obvious immune damage. Interleukin -12 (IL -12) is a kind of interleukin produced by dendritic cells, macrophages, neutrophils and human B lymphoblasts (nc-37) in response to antigen stimulation. IL-12, called T cell stimulating factor, participates in differentiation of naive cells into Th1 cells, and stimulates the production of interferon gamma (IFN-γ) and tumor necrosis factor alpha (TNF-α). IL-12 plays an important role in regulating the activities of natural killer cells and T lymphocytes. IL-12 mediates enhanced cytotoxic activity of NK cells and CD8+ cytotoxic T lymphocytes. IL-12 also has anti-angiogenic activity, indicating it can block the formation of new blood vessels. IL-12 increases the production of gamma-interferon (INF-γ) thus increasing the induction of protein 10 (IP-10 or CXCL10), where IP-10 mediates anti-angiogenesis. IL-12 has been tested as a potential anti-cancer drug because of its ability of inducing immune response and anti-angiogenesis. IL-15 is a factor recently discovered which can be produced by a variety of cells, such as activated monocytes-macrophages, epidermal cells, fibroblasts and the like. The molecular structure of IL-15 is much similar to that of IL-2, thus IL-15 is capable of exerting a biological activity similar to IL-2 based on the binding of β-chain and γ-chain of IL-2 receptor to target cells. IL-15 can induce proliferation and differentiation of B cells, which is the only cytokine that can partially substitute IL-2 to induce initial antibody production. IL-15 can stimulate proliferation of T cells and NK cells, induce LAK cell activity, and can also interact with IL-12 to synergistically stimulate NK cells so as to produce IFN-γ.

According to an embodiment of the present disclosure, the protein of interest and the first regulatory protein are co-expressed under the control of one same promoter, and the protein of interest and the first regulatory protein are linked by a cleavable linker peptide. The protein of interest and the first regulatory protein are regulated and expressed under the one same promoter, and are further cleaved at the linker peptide after expression. The protein of interest is separated from the first regulatory protein, and the protein of interest and the first regulatory protein function independently of each other.

According to an embodiment of the present disclosure, the ninth nucleic acid molecule and the tenth nucleic acid molecule independently inhibit expression of the first regulatory protein or the second regulatory protein, respectively, via RNA interference. MicroRNA is a specific microRNA expressed in different cellular microenvironments, and the ninth or tenth nucleic acid molecule is a specific target sequence of microRNA. The specific effect between the microRNA expressed in a specific microenvironment and its target sequence can be realized via RNA interference, thereby specifically regulating the expression of the first regulatory protein or the second regulatory protein. MicroRNAs (miRNAs) are a class of endogenous small RNAs having about 20-24 nucleotides, and several miRNAs can regulate one same gene. The expression of a gene can be finely regulated by a combination of several miRNAs. MicroRNA has many forms, including a pri-miRNA in an original form, which is of a length of about 300-1000 bases; a pre-miRNA, in a form of microRNA precursor, which is formed by processing of the pri-miRNA and is of a length of about 70-90 bases; and a mature miRNA of about 20 to 24 nt, which is generated by digestion of the pre-miRNA with Dicer. The RNA-induced silencing complex (RISC) inhibits the expression of target genes. The effect of microRNA-RISC on mRNAs of target genes has always been largely dependent on the complementation degree between the microRNA-RISC and the target gene transcript sequence, which is performed in three ways. In a first way, the mRNA molecules of target gene are cleaved, that is the miRNAs which are completely complementary to a target gene (having closely similar mechanism and function to those of siRNAs) are cleaved. In plants, most of miRNAs of target genes are cleaved by the method described above, and the molecules without poly(A) are added with Uracils (Us) at the 3' end and are rapidly degraded while the molecules with poly(A) can be stably present for a time period (such as Arabidopsis miR-171). Currently, one miRNA is found to be completely complementary to three potential target genes in plants, even although it is unclear whether these genes are targets of the miRNA. However, it is found for the first time that miRNA can be completely complementary to its potential targets, suggesting miRNA may have a mechanism similar as siRNA. In a second way, the translation of target gene is inhibited. miRNAs are not completely complement to target genes, thus inhibiting the translation of target genes, without affecting the stability of mRNAs. Such a miRNA is the most widely discovered miRNA, like nematode lin-4, however few miRNAs in plants inhibit target genes in this way. The third way refers to binding inhibition, that is incorporating the two mechanisms in the above two ways. When miRNAs are completely complementary to target genes, they are cleaved directly. When miRNAs are not completely complementary to target genes, they participate in regulation of gene expression.

According to an embodiment of the present disclosure, the ninth nucleic acid molecule comprises a nucleic acid sequence specifically recognized by a first microRNA, and the tenth nucleic acid molecule comprises a nucleic acid sequence that specifically recognized by a second microRNA, where the first microRNA is a normal cell-specific microRNA, and the second microRNA is an abnormal cell-specific microRNA. Further, the first regulatory protein is expressed in abnormal cells and is not expressed or low-expressed in normal cells, while the second regulatory protein is expressed in normal cells and is not expressed or low-expressed in abnormal cells.

According to still another specific embodiment of the present disclosure, the first microRNA comprises at least one selected from the group consisting of miR199a, miR95, miR125, miRamir25b, Let-7, miR143, miR145 and miR200C. The microRNA as described above is expressed in normal liver cells.

According to still another specific embodiment of the present disclosure, the second microRNA comprises at least one selected from the group consisting of miR21, miR223, miR224, miR221, miR18, miR214, miR146a and miR1792 expressed in hepatoma cells (HepG2, Huh7 and PLC). The second microRNA described above is a microRNA specifically expressed in a hepatoma cell. Further, the first regulatory protein is expressed in liver cancer cells and is not expressed or low-expressed in normal cells, while the second regulatory protein is expressed in normal cells and is not expressed or low-expressed in liver cancer cells.

According to an embodiment of the present disclosure, the first nucleic acid molecule and the second nucleic acid molecule are loaded in a first expression vector; the third nucleic acid molecule, the fourth nucleic acid molecule, the fifth nucleic acid molecule, and optionally the ninth nucleic acid molecule are loaded in a second expression vector; and the sixth nucleic acid molecule, the seventh nucleic acid molecule, the eighth nucleic acid molecule, and optionally the tenth nucleic acid molecule are loaded in a third expression vector. The first, second and third expression vectors serve as a loading vector for the expression system to regulate the specific expression of the gene of interest in a suitable microenvironment, such as a cell.

The selection of the expression vector is not particularly limited as long as the function of the expression system in a suitable microenvironment can be achieved. According to a particular embodiment of the present disclosure, the first expression vector, the second expression vector and the third expression vector are each independently selected from at least one of the following:
plasmids, viruses, stable cell lines, and other material carriers such as nano-materials, liposomes, molecular-conjugated vectors, naked DNAs, chromosomal vectors and polymers. The liposome carrier is a liposome-DNA complex formed by packaging DNA with an artificial bilayer phospholipid. The liposome carrier is non-toxic and non-antigenic, has a large capacity, and can prevent target gene from nuclease degradation due to the encapsulation of liposome carrier. Such a liposome carrier can be used alone or in combination with other carriers, and target gene can be introduced into a specific site by intravenously injecting the liposome carrier encapsulating the target gene, with disadvantages of short duration of expression, and unable to pass through cell membrane barrier. The molecular-conjugated vector consists of three parts: DNA, DNA binding factor and ligand.

According to an embodiment of the present disclosure, the virus comprises at least one selected from the group consisting of an adenovirus, a vaccinia virus and a retrovirus.

According to an embodiment of the present disclosure, the first expression vector, the second expression vector and the third expression vector are one same vector. According to a specific embodiment of the present disclosure, the components of the expression system in the present disclosure are loaded into the one same expression vector by the present inventors through the Cascade Golden-Gate Gibson/Gateway Assemble Method, thus effectively solving the problem of very low transfection efficiency when a plurality of large fragments are transfected.

According to an embodiment of the present disclosure, the one same vector is an adenovirus. Adenovirus as a gene therapy vector has the following advantages.
1) Adenovirus is useful for a wide range of hosts and has low pathogenicity to humans, thus adenoviral vector systems are widely used for the expression of human and non-human proteins. Further, adenoviruses can infect a range of mammalian cells and therefore can be useful in expression of recombinant proteins in most mammalian cells and tissues. It is particularly noted that adenoviruses are epitheliophilic, and most of human tumors are derived from epithelial cells. In addition, the replication genes and pathogenic genes of adenovirus are clear enough and the antibody against adenovirus has been prevalent in human populations (70-80% of adults having neutralizing antibodies against adenovirus). Humans produce slight self-limiting symptoms only after infected with wild-type adenovirus, and the treatment with ribavirin is effective.
2) Adenovirus can infect and express gene in proliferating and non-proliferating cells. Retrovirus can only infect proliferating cells, and DNA transfection cannot be performed in non-proliferating cells, thus cells are necessary to be kept in incubation. Adenovirus can infect almost all cell types, except for some lymphoma cells against adenovirus. Adenovirus is the best system for studying gene expression in non-proliferating primary cells, which allows direct comparison between the results obtained from transformed cells and primary cells.
3) Adenovirus can effectively proliferate and has a high titer. The adenovirus system can produce a titer from 10¹⁰ to 10¹¹ VP/mlL and can be up to 10¹³VP/mL after concentration, thus this feature makes it very suitable for gene therapy.
4) Adenovirus is homologous to human genes. Human viruses are generally used as vectors and human cells are used as hosts for adenoviral vector systems, which provide an ideal environment for accurate post-translational processing and proper folding of human proteins. Most of human proteins can achieve high expression levels and are fully functioning.
5) Adenovirus does not integrate into chromosomes and does not cause mutagenicity after insertion. Retrovirus will be randomly integrated into the host's chromosome(s), causing gene inactivation or oncogene activation. However, adenovirus does not integrate into the chromosome in almost all known cells except for egg cells, and thus does not interfere with other genes in hosts. The egg cell integrated with a single copy of adenovirus is a good system for producing transgenic animals with specific characteristics.
6) Adenovirus can be amplified in suspension culture medium. 293 cells can be adjusted for suspension culture, thus allowing large amplification of adenovirus. A large number of facts have shown that suspension of 293 cells can express recombinant proteins in a 1-20 L bioreactor.
7) Adenovirus has the ability of expressing multiple genes simultaneously. This is the first expression system which is designed to express multiple genes in a same cell line or tissue. The simplest method for construction of the expression system comprises inserting a double-expression cassette containing two genes into an adenoviral transfer vector, or co-transfecting a cell strain of interest with different recombinant viruses to express proteins respectively. Relative co-expression of individual recombinant proteins can be correctly estimated by determining the MOI ratio of different recombinant viruses.

According to a specific embodiment of the present disclosure, the adenovirus as described above was injected into a tumor mouse model by the present inventors, with significant inhibition to the growth of mouse tumor. The adenovirus as described above can be used as a safe and effective oncolytic virus vaccine so as to specifically kill related tumors in a safe and effective way.

According to an embodiment of the present disclosure, the adenovirus is obtained by steps: removing the adenoviral E1 gene and a part of adenoviral E3 gene associated with adenoviral replication and packaging from an adenoviral vector; inserting the adenoviral E1A gene into a gene circuit by a stepwise Golden Gate method; and incorporating the gene circuit into the adenoviral vector by the Gateway or Gibson method. Specifically, the gene circuit recognizing cancer cells is inserted into an adenoviral vector by the present inventors through three steps. First, marker elements associated with distinguishing of cancer cells from normal cells (such as a tumor-specific promoter, a gene circuit-related repression element, and a cancer cell-distinguishing miRNA recognition sequence) are constructed into a primary vector plasmid, thereby forming a primary element library, where two ends of the primary elements are each inserted with the recognition site of Esp3I so as to make multiple primary elements constructed into one secondary expression system by the Golden Gate method. Second, an expression system library is constructed, where multiple primary elements selected from the primary element library are assembled into three expression systems by the Golden Gate method, such three expression systems including a tumor-specific promoter system, a first regulatory element-mediated repression system and a second regulatory element-mediated repression system, with two ends of each expression system inserted with the recognition site of BsaI endonuclease. Third, a complete gene circuit is formed by randomly assembling the three expression systems through the Golden Gate method. Two ends of the gene circuit are each inserted with Gibson homologous sequence or Gateway recognition site for further experiments. Finally, the gene circuit is constructed into an adenoviral vector (with E1 gene and part of E3 gene removed) by the Gibson or Gateway method. The method of obtaining an adenovirus as described above ensures rapid modification of a complex oncolytic adenoviral vector with large fragments.

### Recombinant virus

In a second aspect of the present disclosure, provided in embodiments is a recombinant virus. According to an embodiment of the present disclosure, the recombinant virus comprises: a first nucleic acid molecule, comprising a tumor cell-specific promoter, the tumor cell-specific promoter being an alpha-fetoprotein-specific promoter; a second nucleic acid molecule, operably linked to the first nucleic acid molecule and encoding a transcriptional activator, the transcriptional activator being Gal4VP16; a third nucleic acid molecule, comprising a first recognition sequence of the transcriptional activator, the first recognition sequence being 5 × UAS; a fourth nucleic acid molecule, operably linked to the third nucleic acid molecule and comprising a first promoter and a first regulatory element, wherein the first promoter is a miniCMV, and the first regulatory element comprises a plurality of repeated tetO sequences, and at least one of the pluralities of repeated tetO sequences is set downstream of the first promoter; a fifth nucleic acid molecule, operably linked to the fourth nucleic acid molecule and encoding a first regulatory protein, the first regulatory protein being LacI, wherein the fifth nucleic acid molecule further comprises a sequence encoding a protein of interest, the protein of interest comprises a viral replication protein and an immune effector, wherein the immune effector and the viral replication protein are co-expressed, the immune effectors are linked by a cleavable linker peptide, the protein of interest and the first regulatory protein are regulated and co-expressed by one same promoter, and the protein of interest and the first regulatory protein are linked by a cleavable linker peptide; a sixth nucleic acid molecule, comprising a second recognition sequence of the transcriptional activator, the second recognition sequence being 5 × UAS; a seventh nucleic acid molecule, operably linked to the sixth nucleic acid molecule and comprising a second promoter and a second regulatory element, wherein the second promoter is a miniCMV, the second regulatory element comprises a plurality of repeated LacO sequences, at least one of the pluralities of repeated LacO sequences is inserted downstream of the second promoter; an eighth nucleic acid molecule, operably linked to the seventh nucleic acid molecule and encoding a second regulatory protein, the second regulatory protein being tetR-KRAB; a ninth nucleic acid molecule, operably linked to the fifth nucleic acid molecule and configured to conditionally inhibit expression of the first regulatory protein, wherein the ninth nucleic acid molecule comprises a nucleic acid sequence specifically recognized by a first microRNA, the first microRNA being a normal cell-specific microRNA; and a tenth nucleic acid molecule, operably linked to the eighth nucleic acid molecule and configured to conditionally inhibit expression of the second regulatory protein, wherein the tenth nucleic acid molecule comprises a nucleic acid sequence specifically recognized by a second microRNA, the second microRNA being a tumor cell-specific microRNA, wherein the first regulatory element is adapted to inhibit the function of the first promoter by binding to the second regulatory protein, and the second regulatory element is adapted to inhibit the function of the second promoter by binding to the first regulatory protein. With the recombinant virus according to the embodiment of the present disclosure, the first regulatory protein LacI and the protein of interest are specifically expressed in tumor cells and the second regulatory protein tetR-KRAB is not expressed or low-expressed specifically in tumor cells under the co-regulation of the alpha-fetoprotein-specific promoter, the ninth nucleic acid molecule and the tenth nucleic acid molecule, thus the tetR-KRAB- mediated inhibition mechanism on the first promoter miniCMV is released, therefore the first regulatory protein LacI and the protein of interest are effectively expressed under the control of the first promoter miniCMV, and the function of the second promoter miniCMV is effectively inhibited via the LacI-mediated inhibition mechanism, and the expression of tetR-KRAB is further inhibited. Further, proteins (like the protein of interest E1A and the first regulatory protein LacI) are more specifically expressed in tumor cells and the proteins like the second regulatory protein tetR-KRABs are not specifically expressed in tumor cells by using the recombinant virus according to the embodiment of the present disclosure, with high efficiency and specificity.

According to an embodiment of the present disclosure, the recombinant virus is at least one selected from the group consisting of a retrovirus, an adenovirus, a herpes virus and a vaccinia virus. (1) Retroviral vector has characteristics such as a similar structure and infection process to those of common retrovirus, stable expression of gene of interest in transformed target cells, no spread after infection of target cells, high efficiency of pseudovirus in infecting target cells, and no infection to non-proliferating cells. (2) Adenoviral vector has characteristics of a wide range of hosts, no dependence on host proliferation at expression of adenoviral protein, high virus titer, stable recombinant, no induction to tumor, high safty, no envelope protein, not easily inactivated by complement, direct application in body and no integration into chromosome. (3) Herpes simplex virus vector has characteristics of high titer, large capacity, infection into both proliferating and non-proliferating cells, non-integration, and long-term existence and stable expression.

According to an embodiment of the present disclosure, the recombinant virus is an adenovirus. As mentioned above, adenovirus as a gene therapy vector has many advantages.
1. Adenovirus is useful for a wide range of hosts and has low pathogenicity to humans, thus adenoviral vector systems are widely used for the expression of human and non-human proteins. Further, adenovirus can infect a range of mammalian cells and therefore can be useful in expression of recombinant proteins in most mammalian cells and tissues. It is particularly noted that adenoviruses are epitheliophilic, and most of human tumors are derived from epithelial cells. In addition, the replication genes and pathogenic genes of adenovirus are clear enough and the antibody against adenovirus has been prevalent in human populations (70-80% of adults having neutralizing antibodies against adenovirus). Humans produce slight self-limiting symptoms only after infected with wild-type adenovirus, and the treatment with ribavirin is effective.
2. Adenovirus can infect and express gene in proliferating and non-proliferating cells. Retrovirus can only infect proliferating cells, and DNA transfection cannot be performed in non-proliferating cells, thus cells are necessary to be kept in incubation. Adenovirus can infect almost all cell types, except for some lymphoma cells against adenovirus. Adenovirus is the best system for studying gene expression in non-proliferating primary cells, which allows direct comparison between the results obtained from transformed cells and primary cells.
3. Adenovirus can effectively proliferate and has a high titer. The adenovirus system can produce a titer from 10¹⁰ to 10¹¹ VP/mlL and can be up to 10¹³VP/mL after concentration, thus this feature makes it very suitable for gene therapy.
4. Adenovirus is homologous to human genes. Human viruses are generally used as vectors and human cells are used as hosts for adenoviral vector systems, which provide an ideal environment for accurate post-translational processing and proper folding of human proteins. Most of human proteins can achieve high expression level and are fully functioned.
5. Adenovirus does not integrate into chromosomes and does not cause mutagenicity after insertion. Retrovirus will be randomly integrated into the host's chromosome(s), causing gene inactivation or oncogene activation. However, adenovirus does not integrate into the chromosome in almost all known cells except for egg cells, and thus does not interfere with other genes in hosts. The egg cell integrated with a single copy of adenovirus is a good system for producing transgenic animals with specific characteristics.
6. Adenovirus can be amplified in suspension culture medium. 293 cells can be adjusted for suspension culture, thus allowing large amplification of adenovirus. A large number of facts have shown that suspension of 293 cells can express recombinant proteins in a 1-20 L bioreactor.
7. Adenovirus has the ability of expressing multiple genes simultaneously. This is the first expression system which is designed to express multiple genes in a same cell line or tissue. The simplest method for construction of the expression system comprises inserting a double-expression cassette containing two genes into an adenoviral transfer vector, or co-transfecting a cell strain of interest with different recombinant viruses to express proteins respectively. Relative co-expression of individual recombinant proteins can be correctly estimated by determining the MOI ratio of different recombinant viruses.

According to an embodiment of the present disclosure, the immune effector comprises at least one sequence selected from the group consisting of an inhibitory sequence that antagonizes PD-1 gene, an inhibitory sequence that antagonizes PD-L1 gene, an inhibitory sequence that antagonizes CTLA4 gene, an inhibitory sequence that antagonizes Tim-3 gene, IL-2, IL-12, IL-15 and GM-CSF, or a fusion expression form of these factors. Programmed death 1(programmed death receptor 1, PD-1) is an important immunosuppressive molecule belonging to a member of CD28 superfamily, which is originally cloned from apoptotic mouse T cell hybridoma 2B4.11. PD-1 is an important inhibitory molecule on the surface of T cells, and its intracellular domain comprises an immunoreceptor tyrosine inhibitory motif (ITIM) and an immunoreceptor tyrosine transfer motif (ITSM). ITSM mediates the recruitment of protein tyrosine phosphatase family phosphatase and the inhibition of T cell activation signals. Ligands of ITSM are PD-L1 and PD-L2, which mainly play a major role in suppressing activation of T cells in the tumor microenvironment during immune response of immune system. Programmed cell death 1 ligand (PD-L1), also known as cluster of differentiation 274 (CD274) or B7 homolog (B7 homolog 1, B7-H1), is a human protein in body that is encoded by CD274 gene. PD-L1 is expressed inductively in a variety of tumor cells and hematopoietic cells in tumor cell microenvironments, and its expression level is positively correlated with the malignancy of some tumors. PD-1 antibody and PD-L1 antibody can block the binding of PD-1 to PD-L1. However, tumor cells in tissues can escape from immune system through the PD-1/PD-L1 pathway. Cytotoxic T lymphocyte associate protein-4, short for CTLA-4, is a type of co-stimulatory molecule expressed on the surface of T cells. CTLA-4 is capable of specifically binding to CD80/CD86 on the surface of APC to activate downstream signals during activation of T cells, similar to the function of CD28. Studies have found that cells that predominantly express CTLA-4 among T cells are regulatory T cells (Treg), which is a class of T cells that negatively regulate cellular immunity. In the absence of CTLA-4 receptor, mice exhibit T cell overexpression, thus accompanied with severe autoimmune diseases. The above results show that Treg needs CTLA-4 to exercise its function. In addition, CTLA-4 is also expressed in conT cells, which plays a role in inhibiting the signaling of T cell activation. T cell immunoglobulin and mucin-domain containing molecule (TIM) gene family is a novel gene family discovered by Mclntire in 2001 in search of mouse asthma susceptibility genes, which is further identified by genomic analysis and positional cloning. The TIM family gene contains immunoglobulin V region and mucin region. TIM-3 is an important member of the TIM family and expresses a negative regulatory molecule on the surface of activated Th1 cells. Currently, it is found that TIM3 is expressed in CD8⁺T cells, Th17 cells, Treg, NK cells and other lymphocyte subsets. Granulocyte-macrophage Colony Stimulating Factor (GM-CSF) is a monomeric glycoprotein cytokine secreted by macrophages, T cells, NK cells and the like, which stimulates stem cells to produce granules cells (neutrophils, eosinophils, basophils and monocytes). GM-CSF also affects mature cells in the immune system, such as inhibiting the transfer of neutrophils and altering expression of receptor on the cell surface. This factor also can inhibit fungal infections by activating macrophages. Interleukin 2 (IL-2) can mediate cytokines between white blood cells and white blood cells as well as between white blood cells and other cells. IL-2 is mainly produced by activated T cells, and acts on local target cells in a manner of autocrine and paracrine, which is a major cytokine involving in immune response, thus exhibiting a significant immune effect. IL-2 can also promote T cell proliferation and produce cytokines, promote B cell proliferation and secrete Ig, activate macrophages and enhance activation and proliferation of NK cells. In addition, IL-2 also has a negative regulation effect, which can induce apoptosis of T cells which activate Ag, limit the intensity of immune response, and avoid obvious immune damage. Interleukin -12 (IL -12) is a kind of interleukin produced by dendritic cells, macrophages, neutrophils and human B lymphoblasts (nc-37) in response to antigen stimulation. IL-12, called T cell stimulating factor, participates in the differentiation of naive cells into Th1 cells, and stimulates the production of interferon gamma (IFN-γ) and tumor necrosis factor alpha (TNF-α). IL-12 plays an important role in regulating the activities of natural killer cells and T lymphocytes. IL-12 mediates enhanced cytotoxic activity of NK cells and CD8+ cytotoxic T lymphocytes. IL-12 also has anti-angiogenic activity, indicating it can block the formation of new blood vessels. IL-12 increases the production of gamma-interferon (INF-γ) thus increasing the induction of protein 10 (IP-10 or CXCL10), where IP-10 mediates anti-angiogenesis. IL-12 has been tested as a potential anti-cancer drug because of its ability of inducing immune response and anti-angiogenesis. IL-15 is a factor recently discovered which can be produced by a variety of cells, such as activated monocytes-macrophages, epidermal cells, fibroblasts and the like. The molecular structure of IL-15 is much similar to that of IL-2, thus IL-15 is capable of exerting a biological activity similar to IL-2 based on the binding of β-chain and γ-chain of IL-2 receptor to target cells. IL-15 can induce proliferation and differentiation of B cells, which is the only cytokine that can partially replace IL-2 to induce initial antibody production. IL-15 can stimulate proliferation of T cells and NK cells, induce LAK cell activity, and can also interact with IL-12 to synergistically stimulate NK cells so as to produce IFN-γ.

### Recombinant cell

In a third aspect of the present disclosure, provided in embodiments is a recombinant cell. According to an embodiment of the present disclosure, the recombinant cell comprises an expression system as described above. The recombinant cell according to the embodiment of the present disclosure can effectively activate the systemic immune response in human body, and attack xenogeneic cells such as tumor cells, with high safety and specificity.

According to an embodiment of the present disclosure, at least a portion of the expression system is integrated into the genome of the recombinant cell. The expression system replicates as the recombinant cell genome replicates, and the expression system can regulate the expression of proteins of interest constantly and effectively.

### Use in the preparation of a medicament

In a fourth aspect of the present disclosure, provided in embodiments is use of the aforementioned expression system, the aforementioned recombinant virus, and the aforementioned recombinant cell in the preparation of a medicament for treating a cancer. The expression system described in the present disclosure can specifically express a protein of interest in tumor cells. The drug according to the embodiment of the present disclosure can treat cancers in a more effective, specific and safer way. In this embodiment, the present inventors have designed a gene circuit in response to multiple targets to regulate the expression of the key gene E1A associated with adenoviral transcription according to the concept of synthetic biology. At the same time, such a co-expression can activate cytokine or antibody gene which is related to a systemic immune response. First, the gene circuit designed by the present inventors can regulate the packaging of adenoviruses at multiple levels, unlike traditional oncolytic adenoviruses. In the first step, a tumor-specific promoter is used by the present inventors to regulate the expression of the master switch Gal4VP16 in the gene circuit. In the second step, the target sequence of microRNA in the gene circuit can distinguish tumor cells from non-tumor cells in response to microRNA expression in different cell lines. In the third step, a stable mutually repressive switch is used in the designed gene circuit by the present inventors, which can efficiently respond to external input signals such as cell-specific promoters and microRNA signals, and the gene circuit can further enlarge the difference of input signals and be more efficient in distinguishing tumor cells from non-tumor cells. In the fourth step, the expression system in the adenoviral vector designed by the present inventors does not comprise the adenoviral E1B gene, where the E1B gene can interact with the P53 gene in normal cells to ensure the successful adenovirus proliferation in normal cells. The adenovirus without the E1B gene could not proliferate in normal cells expressing P53, while it can still efficiently proliferate in tumor cells lacking P53 gene. Therefore, the adenoviral vector in the embodiment is more specific and safer than the traditional oncolytic adenovirus through multi-level regulation. In addition, this engineered adenovirus is used as a vector to carry genes expressing a variety of cytokines and antibodies in the embodiment. Thus, the adenovirus not only exhibits the oncolytic effect, but also can activate the systemic immune response by using the co-expressed factors, with improved efficacy of oncolytic adenovirus.

Specifically, the present application constructs a gene circuit to regulate the specific expression and packaging of oncolytic adenovirus in hepatocellular carcinoma cells by synthetic biology means. Except for having oncolytic efficacy, the adenovirus constructed by the present inventors is also loaded with genes expressing cytokines which can activate immune response in the body. Such a cytokine once expressed can stimulate immune response inhibiting tumor growth to treat tumors. The expression system designed in the present application has great innovation and technical advantages as follows.

Efficient and safe: The gene circuit designed in this application can respond to biomarkers distinguishing normal cells from cancer cells at multi-levels and comprises a mutually repressive close-loop gene circuit to regulate the replication of adenovirus in different cells, thus is capable of responding to microenvironments in different cell lines more sensitively while reducing the effects of gene expression noise.

Efficient: The gene circuit in this application is loaded with genes expressing a variety of cytokines and/or antibodies, thus an effective immune response can be induced in the cells of interest.

Modularization of gene circuit: Biomarkers involved in the gene circuit are modularized to modules, which are each modelled into different module libraries containing biomarkers against different diseases, thus a disease-specific gene circuit can be constructed by assembling relative modules rapidly.

Accuracy: It is possible for inventors to replace different tumor markers based on rapid assembly technology, thus gene circuits directing different patients can be designed for precise treatment.

According to an embodiment of the present disclosure, the cancer comprises liver cancer, lung cancer, colorectal cancer, melanoma, breast cancer or prostate cancer. The inventors have found that the drug in the embodiment of the present disclosure has a more remarkable therapeutic effect on liver cancer, lung cancer, colorectal cancer, melanoma, breast cancer and prostate cancer.

According to a specific embodiment of the present disclosure, the inventors have found that the expression of microRNAs in different tumor cell lines is shown in Table 1. The upward arrow indicates microRNAs that are highly expressed in cancer cells compared to normal cells, while the downward arrow indicates microRNAs that are low-expressed in cancer cells compared to normal cells.

**Table 1:**

| | |
|---|---|
| Hepatocellular carcinoma | miR-21↑,18↑,224↑,199↓,195,200↓,125↓ |
| Lung cancer | Let-7↓, miR-17-92↑ |
| Breast cancer | miR125b↓, 145↓, 21↓, 155↓ |
| Brain tumor | miR-21↑, 221↑, 181↓, |
| Colorectal cancer | miR143↓, 145↓ |
| Lymphoma | miR155↑, miR-17-92↑ |
| Melanoma | miR21↑,221↑,214↑146a↑137↓200c↓let-7↓ |

### Method of expressing a protein of interest by using an expression system

In a fifth aspect of the present disclosure, provided in embodiments is a method of expressing a protein of interest by using an expression system. The expression system is the expression system as described above. According to an embodiment of the present disclosure, the method comprises: (1) providing a fifth nucleic acid molecule which comprises a nucleic acid sequence encoding the protein of interest; and (2) inhibiting expression of a second regulatory protein by a tenth nucleic acid molecule so as to express the protein of interest. With the method of expressing a protein of interest by using an expression system in the embodiment, the second regulatory protein-mediated inhibition mechanism on the first promoter is released, thus the protein of interest is effectively expressed in specific cells under the coaction of the cell-specific promoter and the first promoter.

According to an embodiment of the present disclosure, the expression is carried out in cells. The cells can provide a microenvironment for the expression of proteins of interest, thus the proteins of interest can be expressed in cells more efficiently.

According to an embodiment of the present disclosure, the tenth nucleic acid molecule comprises a nucleic acid sequence specifically recognized by the second microRNA, and the method further comprises contacting the second microRNA with the tenth nucleic acid molecule in the step (2). MicroRNAs (miRNAs) are a class of endogenous small RNAs having about 20-24 nucleotides, and several miRNAs can regulate one same gene. The expression of a gene can be finely regulated by a combination of several miRNAs. MicroRNA has many forms, including a pri-miRNA in an original form, which is of a length of about 300-1000 bases; a pre-miRNA, in a form of microRNA precursor, which is formed by processing of the pri-miRNA and is of a length of about 70-90 bases; and a mature miRNA of about 20 to 24 nt, which is generated by digestion of the pre-miRNA with Dicer. The RNA-induced silencing complex (RISC) inhibits the expression of target genes. The effect of microRNA-RISC on target gene mRNA has always been largely dependent on the complementation degree between the microRNA-RISC and the target gene transcript sequence, which is performed in three ways. In a first way, the mRNA molecules of target gene are cleaved, that is the miRNAs which are completely complementary to target gene (having closely similar mechanism and function to those of siRNAs) are cleaved. In plants, most of miRNAs of target genes are cleaved by the method described above, and the molecules without poly(A) are added with Uracils (Us) at the 3' end and are rapidly degraded while the molecules with poly(A) can be stably present for a time period (such as Arabidopsis miR-171). Currently, one miRNA is found to be completely complementary to three potential target genes in plants, even although it is unclear whether these genes are targets of the miRNA. However, it is found for the first time that miRNA can be completely complementary to its potential targets, suggesting miRNA may have a mechanism similar as siRNA. In a second way, the translation of target gene is inhibited. MiRNAs are not completely complement to target genes, thus inhibiting the translation of target genes, without affecting the stability of mRNAs. Such a miRNA is the most widely discovered miRNA, like nematode lin-4, however few miRNAs in plants inhibit target genes in this way. The third way refers to binding inhibition, that is incorporating the two mechanisms in the above two ways. When miRNAs are completely complementary to target genes, they are cleaved directly. When miRNAs are not completely complementary to target genes, they participate in regulation of gene expression.

In a sixth aspect of the present disclosure, provided in embodiments is a method of expressing a protein of interest by using an expression system. The expression system is the expression system as described above. According to an embodiment of the present disclosure, the method comprises: (1) providing an eighth nucleic acid molecule which comprises a nucleic acid sequence encoding a first protein of interest, and providing a fifth nucleic acid molecule which comprises a nucleic acid sequence encoding a second protein of interest; and (2) inhibiting expression of the first regulatory protein by the ninth nucleic acid molecule so as to express the first protein of interest; or inhibiting expression of the second regulatory protein by the tenth nucleic acid molecule so as to express the second protein of interest. With the method of expressing a protein of interest by using an expression system in the embodiment, the second regulatory protein-mediated inhibition mechanism on the first promoter is released, thus the second protein of interest is effectively expressed in specific cells under the coaction of the cell-specific promoter and the first promoter, or the first regulatory protein-mediated inhibition mechanism on the second promoter is released, thus the first protein of interest is effectively expressed in specific cells under the coaction of the cell-specific promoter and the second promoter.

Experimental embodiments of the present disclosure are described in detail below. The embodiments described below are illustrative only and are not to be construed as limiting the present disclosure. Where specific techniques or conditions are not indicated in the examples, they are carried out according to the techniques or conditions described in the literature in the art or in accordance with the product specifications. The used reagents or instruments not indicated by the manufacturer are all conventional products that can be obtained commercially.

### Experimental materials and methods

### 1. Cell culture and transfection

HEK293 (293-H) cell line was purchased from Invitrogen Co., Ltd. Human hepatoma cell line HepG2, normal liver cells Chang and Hepa1-6 were purchased from ATCC. Human hepatoma cell line Huh7 was purchased from BeNa Co., Ltd. The cells were cultured in high-glucose Dulbecco's Modified Eagle's Medium containing 4.5 g/L glucose, 0.045 unit/mL penicillin, 0.045 g/mL streptomycin and 10% FBS (purchased from the Invitrogen Co., Ltd.) (i.e. DMEM complete medium) at 37°C, 100% humidity and 5% CO₂ concentration.

Cell transfection was conducted as follows. About 7.5 × 10⁴ HEK293 cells in 0.5 mL of the high-glucose DMEM complete medium were seeded into individual wells of 24-well plates (Falcon) and incubated for 24 hours, followed by replacing the medium with fresh DMEM complete medium and addition of Attractene transfection reagent (Qiagen) or Lipofectamine LTX (Life Technologies) for transfection. Specifically, 1.5 µL of Attractene was added into an amount of mixed DNA plasmids, stood at room temperature for 15 minutes, and then added into corresponding wells if Attractene was used. However, if Lipofectamine LTX was used, an appropriate amount of Plus reagent was firstly added into mixed DNA plasmids and stood at room temperature for 5 minutes, then 1.25 µL of Lipofectamine LTX was added and stood at room temperature for another 25 minutes prior to transferred into the 24-well plates. pDT7004 (pUBI-linker-NOS), which contains a maize ubiquitin promoter (UBI) followed by a NOS terminator with no protein-coding sequences between UBI and NOS, was used to ensure that the amount of plasmid DNA in different experiment groups was equal. Cells after transfection were cultured for 48 hours before flow cytometry analysis or some experiments related to cytokine detection.

### 2. Adenoviral vector

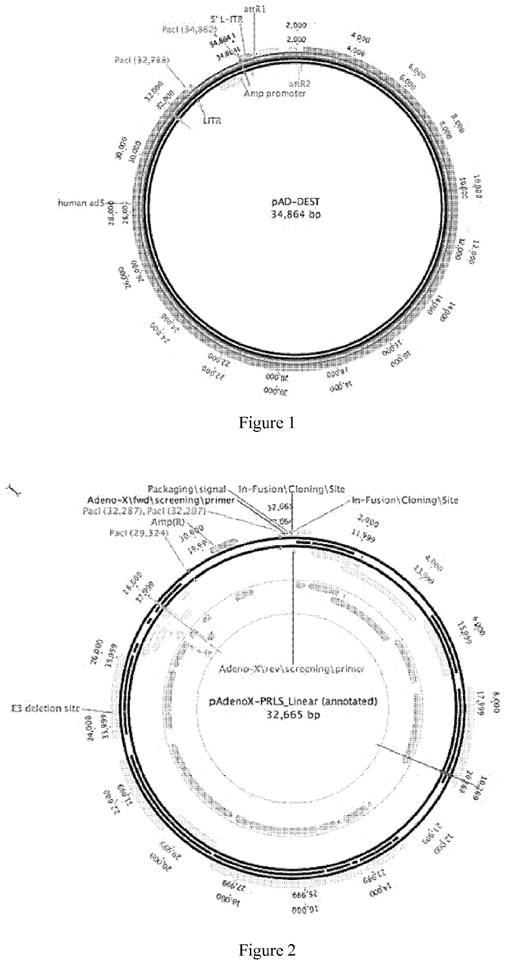

Two initial adenoviral vector sequences are used in this example, that is ViraPower™ Adenoviral Gateway™ Expression Kit (K49300 Invitrogen) and Adeno-X-Adenoviral System 3 (632266 Clontech). The structures of the two adenoviral vectors are shown in Figure 1 and Figure 2 respectively.

### 3. Plasmid construction

The plasmids related to truncated AFP promoter derivatives were constructed. AFP enhancer and AFP promoter sequences were directly obtained from the genome of hepatoma cell line under specifically designed PCR primers via PCR amplification. Point mutations in the AFP promoter sequence were obtained by using the overlap extension PCR method. The AFP enhancer and AFP promoter sequences were constructed into an entry clone, with a specifically designed recognition sequence of LR Clonase at both ends of the entry clone. The specific promoter and the activator gene Gal4VP16 were constructed into one same vector by the LR reaction. The target sequence of microRNA was inserted into the CMV-EYFP vector (XbaI/SalI) through primer annealing, enzyme digestion and ligation. The primary element library of the expression system was constructed through the Golden Gate method. The type IIs restriction enzyme (such as BsaI, BbsI, BsmBI, SapI and the like) is a specific class of restriction enzyme where its recognition site is a non-palindromic and symmetrical sequence and its cleavage site is located outside the recognition site, which is different from the type II restriction enzyme most commonly used in molecular biology where its recognition site is a palindromic and symmetrical sequence and its cleavage site overlaps with the recognition sequence. Thus, when two fragments digested by the type IIs restriction enzyme are ligated in subsequent digestion and ligation reactions, they will not be cleaved again due to the absence of previous recognition sites, thus achieving the effect of welding. For the Golden Gate cloning method, different cleavage site sequences are artificially designed outside the recognition sequences because of such a characteristic of the type IIs restriction enzyme, by which one same type IIs restriction enzyme is capable of generating different sticky ends, thereby ensuring of assembling multiple fragments at one time, and thus overcoming the deficiency of traditional multi-fragment assembly such as use of multiple different restriction enzymes. The biggest feature of this Golden Gate cloning method is in that no additional "scar" is introduced and thus achieving the seamless ligation of DNA fragments, compared with the traditional digestion-ligation method. The type IIs restriction enzyme used in the primary element library in this example is Esp3I. A second logic circuit library of the expression system was also constructed by the Golden Gate method, with the type IIs restriction enzyme BsaI used. The second logic circuit was then inserted into the adenoviral vector sequence of the ViraPower™ Adenoviral Gateway™ Expression Kit by the Gateway method, or inserted into the adenoviral vector sequence of Adeno-X Adenoviral System 3 by Gibson cloning. The Gateway technique is based on the well-studied phage λ site-specific recombination system (attB × attP → attL × attR), which consists of BP and LR reactions. The BP reaction is a recombination reaction between an attB DNA fragment or an expression clone and an attP donor vector, thus creating an entry clone. The LR reaction is a recombination reaction between an attL entry clone and an attR destination vector, which is used to insert a sequence of interest into one or more destination vectors in parallel reactions. The present inventors employed the LR reaction in this example. Gibson assembly technique, also known as "Gibson thermostatic one-step assembly method", is a DNA assembly method created by Gibson et al. at the J. CraigVenter Institute in the United States of America, where a plurality of DNA fragments having overlapping end sequences are assembled *in vitro* under the synergistic effect of T5 exonuclease, DNA polymerase and ligase. Among them, T5 exonuclease has 5'→3' exonuclease activity, and can cleave the DNA fragment having overlapping end sequences from the 5' end of one strand of the DNA fragment, thus generating an overhanging end having the overlapping sequence at the 3' end of the complementary strand which is specifically annealed at 50°C (T5 exonuclease is gradually inactivated at this temperature), and finally a complete DNA molecule is formed in the presence of DNA polymerase and Taq ligase, achieving seamless assembly.

### 4. Cellular RNA extraction, reverse transcription and quantitative PCR reaction

Cellular RNA was extracted by the Trizol method. 1 mL of Trizol was added into 5 × 10⁶ cells pelleted by centrifugation, followed by repeatedly pipetting with a pipette or vigorous shaking to lyse cells. After standing at room temperature of 15-30 °C for 5 minutes, 0.2 mL of chloroform was added, and the mixture was shaken vigorously for 15 seconds, kept at room temperature for another 2 to 3 minutes, and centrifuged at 12,000 × g and 4°C for 15 minutes. 0.5 mL of isopropanol was added into the separated upper aqueous layer, left at room temperature for 10 minutes, and centrifuged at 12,000 × g and 4 °C for 10 minutes, followed by adding 1 mL of 75% ethanol, vortexed and centrifugation at 7500 ×g and 4 °C for 5 minutes. The supernatant was discarded, and the precipitated RNA was naturally dried at room temperature. The dried RNA was dissolved in RNase-free water. The reverse transcription experiment was performed by using the QuantiTect Rev. Transcription Kit (Cat No./ID: 205310 QIAGEN). The quantitative PCR primers are qAFP for: CAAAGCTGAAAATGCAGTTGAATG (SEQ ID NO: 15), qAFP rev: TTCCCCATCCTGCAGACAATCC (SEQ ID NO: 16), GAPDH for: AGAAGGCTGGGGCTCATTTG (SEQ ID NO: 17), and GAPDH rev: AGGGGCCATCCACAGTCTTC (SEQ ID NO: 18). Fluorescent dye is PowerUp™ SYBR®Green Master Mix (A25741Thermo Fisher).

### 5. Packaging, purification and titration of Adenovirus

The endotoxin-free recombinant adenoviral plasmid with high purity was extracted. HEK293 cells were transfected with the linearized adenovirus DNA which was digested by PacI overnight, followed by replacing the culture medium with the complete medium. Cells along with 1 mL of culture medium were collected into a 15 mL centrifuge tube after one week of culture (fresh medium added depending on the cell growth state during the culture), which were then lysed with three consecutive freeze-thaw cycles at 37°C via liquid nitrogen, and centrifuged at 2000 rpm for 5 minutes. The supernatant was obtained as the primary virus solution. After three consecutive generations of transfection, the obtained adenoviruses were seeded into 10 of 15 cm plates for amplification, and further purified by the combination of CsCl density gradient centrifugation and dialysis. The CsCl density gradient was prepared by adding 2 mL of a CsCl solution in a density of 1.4 g/mL, further adding 3 mL of a CsCl solution in a density of 1.3 g/mL slowly, and then adding 5 mL of the virus suspension. After centrifugation at 20000 rpm and 4°C for 10 hours, the adenoviruses concentrated in the CsCl solution in a density between 1.3 g/mL and 1.4 g/mL were collected into dialysis bags which have been boiled with 10 mM EDTA-Na₂ for 10 minutes before use. The dialysis buffer is prepared by using 50 g sucrose, 10 mL 1 M Tris-HCl and 2 mL 1 M MgCl₂, made up to 1 L with water and adjusted to pH 8.0. After stirring at 4°C overnight and changing the dialysis buffer twice, the adenovirus was collected for determination of virus titer. The viral titer was detected by quantifying the viral DNA extracted from the infected cells. Specifically, about 2 × 10⁶ HeLa cells/well were seeded into 6-well tissue culture plates (with the cell coverage rate reaching up 90-100% after one day of incubation) and infected with 5 µL of the virus stock diluted with 500 µL of serum-free medium in duplicate, followed by incubation at 37°C for 3 to 6 hours, removal of culture medium, and washed twice with 1 mL PBS. The cells were lysed by adding 500 µL of fresh NP-40 lysis buffer (including 0.65% NP-40 (Calbiochem, Billerica, MA, USA), 150 mM NaCl, 10 mM Tris, pH 8.0), incubating at room temperature for 5-10 minutes with pipetting up and down 10-15 times for fully lysis, and then centrifuging at 2000 × g for 3 minutes for virus precipitation. After the supernatant was discarded, 1 mL of NP-40 lysis buffer was added followed by vortexed briefly and centrifugation at 2000 × g for 3 minutes. The supernatant was discarded again and the precipitate was suspended in 200 µL of PBS. During the DNA isolation and purification, DNAs were extracted by using the DNeasy Blood & Tissue Kit (QIAgen, Valencia, CA, USA) for subsequent quantitative reactions. The quantitative primers are L2 Forward Primer: TTGTGGTTCTTGCAGATATGGC (SEQ ID NO: 19), and L2 Reverse Primer: TCGGAATCCCGGCACC (SEQ ID NO: 20).

A standard curve was prepared. The PCR L2 target sequence was constructed into T vectors, which were diluted to 1 × 10⁹ to 1 × 10² copy/µL as a standard sequence. The dilution formula is shown as: [Plasmid concentration (µg/µL) × Avogadro's number × 10-6] / [number of nucleotides × 1bp molecular weight] = particle copies/µL

### 6. Cell experiment

Adenovirus was detected for its recognition and killing abilities to different cells. Chang cells (1 × 10⁴ cells/well), and HepG2 and Huh7 cells (5 × 10⁴ cells/well respectively) were seeded into 96-well plates and infected with adenoviral vectors at seven Multiplicity of Infection (MOI) gradients in ten-fold dilutions from 10² to 10⁻⁴, with cell viability measured by the MTS method after six days of observation.

### 7. MTS detection

The number of cells was measured by the MTS method. 20 µL of MTS/PMS mixture (Promega) was added to individual wells of 96-well plates containing the cells to be tested, then mixed, and incubated at 37°C for 1 to 4 hours. The plate was shaken for 10 seconds after color development to get uniform color. The absorbance (OD value) at a wavelength of 490 nm was measured by using a microplate reader. A standard curve was established. The number of cells of interest was detected by reference of the standard curve.

### 8. Cytokine detection

The biological activity of cytokines can be detected via proliferation of cytokine-dependent cell line. The biological activities of IL-2, mGM-CSF and hGM-CSF were detected by using CTLL-2, FDC-P1 and TF-1 cell lines, respectively. After collected, 1 × 10⁴ cytokine-dependent cells to be tested were seeded in 96-well plates, infected with the sample containing the cytokine to be tested, and thermostatically cultured at 37°C for 24 hours or 48 hours. The amplified cells were measured by the MTS method. A dose-proliferation standard curve was established. The bioactivity of cytokine in the measured sample was estimated by using the standard curve.

### 9. Co-immunoprecipitation Assay

Co-immunoprecipitation was performed as follows. An appropriate amount of protein lysis buffer (Biyuntian Co., Ltd.) was added into the collected cells to be tested, and centrifuged to remove cell debris. A small amount of the protein lysis buffer was left for subsequent Western blot analysis. The remaining protein lysis buffer plus 10-30 µL of protein A/G-agarose beads conjugated with corresponding tag antibody (Thermo Fisher Scientific Co., Ltd.) was added into the obtained cell lysate, and incubated at 4°C overnight with slightly shaking, then centrifuged to remove the supernatant. After the precipitated beads were washed three times, the antibody and captured protein were eluted from the agarose beads by using an eluent, followed by addition of SDS-PAGE loading buffer (Shanghai Shenggong Co., Ltd.) and boiled. The enrichment of related proteins was detected by the Western blot method.

### 10. Animal studies

The experimental animals were purchased from HuafuKang Co., Ltd. or Weitonglihua Co., Ltd. The tumor model was constructed by using 6-week old male or female mice, kept in SPF environment. For the human tumor model, six-week old nude mice were subcutaneously injected with 1 × 10⁷ human hepatoma cells HepG2 in matrigel (BD Co., Ltd.) on the right flank. For the mouse tumor model, 1 × 10⁶ mouse hepatoma cells Hepa1-6 were subcutaneously injected into the right flank of six-week old wild-type C57BL/6J mice. Subcutaneous tumor size was measured by vernier calipers every 3 days, and calculated by using the formula: Volume = 0.5 × length × width². After tumor volume exceeded 100 mm³, oncolytic adenoviruses or control treatment was administrated. Tumor-bearing mice were euthanized after the tumor size reached 15 mm in diameter, according to the requirements of animal experiment ethics.

### Experimental results

### 1. Construction of mutually repressive switch in response to various biomarkers to distinguish cancer cells from non-cancer cells by use of synthetic biology

Related studies in recent years have shown that oncolytic viruses are very effective for treating a cancer, thus many clinical studies on oncolytic adenovirus have been developed. According to the existing clinical research results, there is a relatively high proportion of leakage on oncolytic adenovirus, so how to improve the safety of oncolytic adenovirus in clinical stage is an important issue. Based on the analysis of existing oncolytic adenoviruses, most oncolytic adenoviruses are regulated depending on a single biomarker, such as a tumor-specific promoter that regulates the expression of gene E1 key to adenoviral replication. However, only one single biomarker may cause a relatively high probability of leakage. Therefore, the present inventors have designed a mutually repressive switch in response to a variety of biomarkers and to regulate the expression of adenovirus E1A gene at various levels in this example, thereby improving the specificity of the oncolytic adenovirus on recognition and enhancing safety.

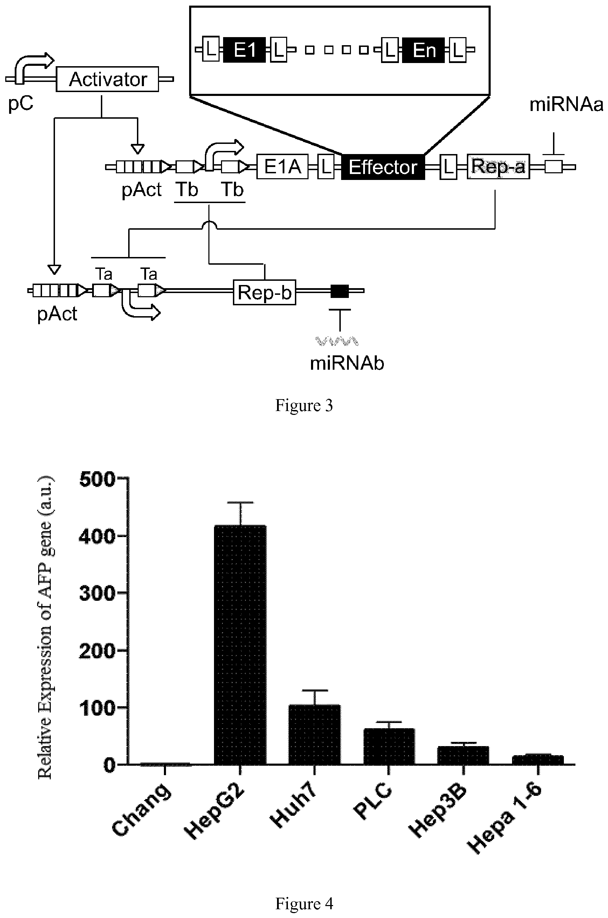

As shown in Figure 3, the mutually repressive switch has the following features. The tumor specific promoter (pC) regulates the master activator of the mutually repressive switch. The master activator acts on the activator response promoter (pAct) to regulate the expression of downstream genes. The first side of the mutually repressive switch expresses the adenovirus packaging-related E1A gene, effectors and the repressor a (Rep-a), as well as a target sequence of microRNA a (miRNA a) highly expressed in non-tumor cells, which further comprises a recognition sequence of repressor b expressed on the second side and a target sequence of microRNA b (miRNA b) highly expressed in tumor cells, and where the recognition sequence of the repressor b is located at two sides of the first promoter in the first side respectively. The second side of the mutually repressive switch expresses the repressor b (Rep-b), which further comprises a recognition sequence of the repressor a expressed on the first side, and where the recognition sequence of the repressor a is located at two sides of the second promoter in the second side respectively. In tumor cells, the tumor specific promoter (pC) initiates the expression of the whole system. The repressor b is degraded via RNA interference due to the high expression of miRNA b and the low expression of miRNA a in tumor cells, thus the inhibition of the repressor b to the gene expression on the first side is eliminated. Besides, the high expression of repressor a can further inhibit the expression of repressor b, thus allowing the mutually repressive switch to switch into the state of E1A expression quickly and efficiently, thereby initiating the packaging of adenovirus and the expression of effectors. Conversely, when the mutually repressive switch enters non-tumor cells, E1A and effectors cannot be expressed due to the combination of the tumor-specific promoter, miRNA markers and the logic circuit, thereby effectively closing the packaging process of adenovirus, and ensuring the specificity and safety of this logic circuit. Therefore, such a mutually repressive switch can respond to a variety of input signals and distinguish target cells from non-target cells at transcriptional and post-transcriptional levels. In addition, the present expression system proposed by the inventors can express the E1A gene only, with the E1B gene removed, thus improving the recognition ability of oncolytic virus to target cells from the viewpoint of complementation of functional defects. Therefore, the expression system designed by the inventors is capable of distinguishing target cells at multiple levels, with effectively improved adenovirus safety.

### 2. Verification and analysis of biomarkers distinguishing hepatoma cells from normal cells

### 2.1 Construction of hepatoma-specific alpha-fetoprotein (AFP) promoter

Alpha-fetoprotein (αFP or AFP) is mainly synthesized in liver of a fetus, which is not expressed in a normal adult, however it will be expressed again when liver cells become cancerous, and the amount of AFP in serum will increase dramatically with the progress of liver cancer. At present, alpha-fetoprotein is a specific clinical indicator for the diagnosis of primary liver cancer, thus the inventors selected the alpha-fetoprotein promoter as a promoter marker for distinguishing hepatoma cells from non-hepatoma cells in the example.

The inventors further detected the specific expression of AFP by experiments. First, the expression of human AFP gene in a series of non-hepatoma cells and hepatoma cells was detected by the qPCR method. As shown in Figure 4, RNAs of Chang, HepG2, Huh7, PLC, Hep3B and Hepa1-6 cell lines were respectively extracted, and the expression level of AFP gene in these cells was detected by the quantitative PCR method, where Chang cells are liver cells of a normal human, and HepG2, Huh7, PLC and Hep3B cells are human hepatoma cells, and Hepa1-6 cells are mouse hepatoma cells. The results show that the human AFP gene is highly expressed in the HepG2 and Huh7 cell lines, and low-expressed in the PLC and Hep3B cell lines, as well as slightly expressed in the mouse Hepa1-6 hepatoma cells, suggesting that the AFP promoter can be employed by the inventors as a promoter marker in the hepatoma cells due to its specific expression.

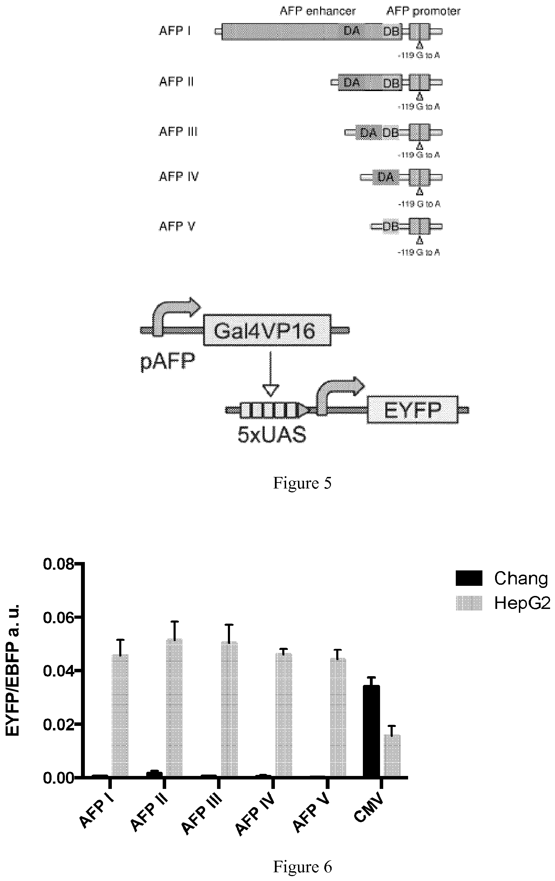

The currently widely used AFP promoter consists of an enhancer and a promoter. The enhancer contains two sequences which can activate the promoter, i.e. domain A (DA) and domain B (DB). In order to increase the efficiency of the promoter, the inventors mutated guanine (G) at position -119 of the promoter to adenine (A). In addition, the enhancer region was truncated to different extent by the inventors. As shown in Figure 5, AFP I comprises an enhancer sequence of AFP in a length of 1.8 K (as shown in SEQ ID NO: 21); AFP II comprises an activation region A (DA), an activation region B (DB) and a sequence between the two activation regions (as shown in SEQ ID NO: 22); AFP III comprises an activation region A (DA) and an activation region B (DB) only (as shown in SEQ ID NO: 23); AFP IV comprises an activation region A (DA) only (as shown in SEQ ID NO: 24); and AFP V comprises an activation region B (DB) only (as shown in SEQ ID NO: 25). To detect the expression efficiency and specificity of the AFP promoter, these five AFP promoter reporter system plasmids were transiently transfected into normal hepatocytes Chang and hepatoma cells HepG2 respectively. The results in Figure 6 show that the five AFP promoters are all highly expressed in hepatoma cell lines, even exceeding expression level of the commonly expressed CMV promoter. Based on the analysis of specificity, other four promoters except for AFP II promoter are not obviously leaked in Chang cells, with slight expression of the AFP II reporter gene in Chang cells. In consideration of the length of sequence to be inserted, and the efficiency and specificity of a promoter, the inventors selected the AFP III promoter to regulate the present mutually repressive switch in subsequent experiments.

TTTAGAAATATGGGGGTAGGGGTGGTGGGGCCTGGATAAAGCTGAGTGGTAT
GAATGAGTTAGAAATATGGGGGTAGGGGTGGTGGTAATTCTGTTTCTCCCCATAG
GTGAGATAAGCATTGGGTTAAATGTGCTTTCTCTCTCTCCCTCTCCTTTCTTAAGAATTA
AGGGACAGACTATGGGCTGGAGGACTTTGAGGATGTCTGTCTCATAACACTTGGGTTG
TATCTGTTCTATGGGGCTTGTTTTAAGCTTGGCAACTTGCAACAGGGTTCACTGACTTT
CTCCCCAGGCCCAAGGTACTGTCCTCTTTTCATATCTGTTTTGGGGCCTCTGGGGCTTG
AATATCTGAGAAAATATAAACATTTCAATAATGTTCTGTGGTGAGATGAGTATGAGAGA
TGTGTCATTCATTTGTATCAATGAATGAATGAGGACAATTAGTGTATAAATCCTTAGTAC
AACAATCTGAGGGTAGGGGTGGTACTATTCAATTTCTATTTATAAAGATACTTATTTCTAT
TTATTTATGCTTGTGACAAATGTTTTGTTCGGGACCACAGGAATCACAAAGATGAGTCT
TTGAATTTAAGAAGTTAATGGTCCAGGAATAATTACATAGCTTACAAATGACTATGATAT
ACCATCAAACAAGAGGTTCCATGAGAAAATAATCTGAAAGGTTTAATAAGTTGTCAAA
GGTGAGAGGGCTCTTCTCTAGCTAGAGACTAATCAGAAATACATTCAGGGATAATTATT
TGAATAGACCTTAAGGGTTGGGTACATTTTGTTCAAGCATTGATGGAGAAGGAGAGTG
AATATTTGAAAACATTTCAACTAACCAACCACCCAATCCAACAAACAAAAAATGAAAA
GAATCTCAGAAACAGTGAGATAAGAGAAGGAATTTTCTCACAACCCACACGTATAGCT
CAACTGCTCTGAAGAAGTATATATCTAATATTTAACACTAACATCATGCTAATAATGATAA
TAATTACTGTCATTTTTTAATGTCTATAAGTACCAGGCATTTAGAAGATATTATTCCATTTA
TATATCAAAATAAACTTGAGGGGATAGATCATTTTCATGATATATGAGAAAAATTAAAAA
TCAGATTGAATTATTTGCCTGTCATACAGCTAATAATTGACCATAAGACAATTAGATTTA
AATTAGTTTTGAATCTTTCTAATACCAAAGTTCAGTTTACTGTTCCATGTTGCTTCTGAG
TGGCTTCACAGACTTATGAAAAAGTAAACGGAATCAGAATTACATCAATGCAAAAGCA
TTGCTGTGAACTCTGTACTTAGGACTAAACTTTGAGCAATAACACATATAGATTGAGGA
TTGTTTGCTGTTAGTATACAAACTCTGGTTCAAAGCTCCTCTTTATTGCTTGTCTTGGAA
AATTTGCTGTTCTTCATGGTTTCTCTTTTCACTGCTATCTATTTTTCTCAACCACTCACAT
GGCTACAATAACTGTCTGCAAGCTTATGATTCCCAAATATCTATCTCTAGCCTCAATCTT
GTTCCAGAAGATAAAAAGTAGTATTCAAATGCACATCAACGTCTCCACTTGGAGGGCT
TAAAGACGTTTCAACATACAAACCGGGGAGTTTTGCCTGGAATGTTTCCTAAAATGTG

TCCTGTAGCACATAGGGTCCTCTTGTTCCTTAAAATCTAATTACTTTTAGCCCAGTGCTC
ATCCCACCTATGGGGAGATGAGAGTGAAAAGGGAGCCTGATTAATAATTACACTAAGT
CAATAGGCATAGAGCCAGGACTGTTTGGGTAAACTGGTCACTTTATCTTAAACTAAATA
TATCCAAAACTGAACATGTACTTAGTTACTAAGTCTTTGACTTTATCTCATTCATACCAC
TCAGCTTTATCCAGGCCTCTAGAAGTTTGAGGAGAATATTTGTTATATTTGCAAAATAAA
ATAAGTTTGCAAGTTTTTTTTTCTGCCCCAAAGAGCTCTGTGTCCTTGAACATAAAAT
ACAAATAACCGCTATGCTGTTAATTATTGACAAATGTCCCATTTTCAACCTAAGGAAATA
CCATAAAGTAACAGATATACCAACAAAAGGTTACTAGTTAACAGGCATTGCCTGAAAA
GAGTATAAAAGAATTTCAGCATGATTTTCCATATTGTGCTTCCACCACTGCCAATAACA
AAATAACTAGCAA (SEQ ID NO:21).

CAGATTGAATTATTTGCCTGTCATACAGCTAATAATTGACCATAAGACAATTAGATT
TAAATTAGTTTTGAATCTTTCTAATACCAAAGTTCAGTTTACTGTTCCATGTTGCTTCTG
AGTGGCTTCACAGACTTATGAAAAAGTAAACGGAATCAGAATTACATCAATGCAAAAG
CATTGCTGTGAACTCTGTACTTAGGACTAAACTTTGAGCAATAACACATATAGATTGAG
GATTGTTTGCTGTTAGTATACAAACTCTGGTTCAAAGCTCCTCTTTATTGCTTGTCTTGG
AAAATTTGCTGTTCTTCATGGTTTCTCTTTTCACTGCTATCTATTTTTCTCAACCACTCAC
ATGGCTACAATAACTGTCTGCAAGCTTATGATTCCCAAATATCTATCTCTAGCCTCAATC
TTGTTCCAGAAGATAAAAAGTAGTATTCAAATGCACATCAACGTCTCCACTTGGAGGG
CTTAAAGACGTTTCAACATACAAACCGGGGAGTTTTGCCTGGAATGTTTCCTAAAATGT
GTCCTGTAGCACATAGGGTCCTCTTGTTCCTTAAAATCTAATTACTTTTAGCCCAGTGCT
CATCCCACCTATGGGGAGATGAGAGTGAAAAGGGAGCCTGATTAATAATTACACTAAG
TCAATAGGCATAGAGCCAGGACTGTTTGGGTAAACTGGTCACTTTATCTTAAACTAAAT
ATATCCAAAACTGAACATGTACTTAGTTACTAAGTCTTTGACTTTATCTCATTCATACCA
CTCAGCTTTATCCAGGCCACTTATTTGACAGTATTATTGCGAAAACTTCCTATCTAGAAG
TTTGAGGAGAATATTTGTTATATTTGCAAAATAAAATAAGTTTGCAAGTTTTTTTTTCT
GCCCCAAAGAGCTCTGTGTCCTTGAACATAAAATACAAATAACCGCTATGCTGTTAATT
ATTGACAAATGTCCCATTTTCAACCTAAGGAAATACCATAAAGTAACAGATATACCAAC
AAAAGGTTACTAGTTAACAGGCATTGCCTGAAAAGAGTATAAAAGAATTTCAGCATGA
TTTTCCATATTGTGCTTCCACCACTGCCAATAACAAAATAACTAGCAA (SEQ ID NO:22).

CAGATTGAATTATTTGCCTGTCATACAGCTAATAATTGACCATAAGACAATTAGATT
TAAATTAGTTTTGAATCTTTCTAATACCAAAGTTCAGTTTACTGTTCCATGTTGCTTCTG
AGTGGCTTCACAGACTTATGAAAAAGTAAACGGAATCAGAATTACATCAATGCAAAAG

CATTGCTGTGAACTCTGTACTTAGGACTAAACTTTGAGCAATAACACATATAGATTGAG
GATTGTTTGCTGTTAGTATACAAACTCTGGTTCAAAGCTCCTCTTTATTGCTTGTCTTGG
AAAATTTGCTGTTCTTCATGGTTTCTCTTTTCACTGCTATCTATTTTTCTCAACCACTCAC
ATGGCTACAAAAGCTTCCTGATTAATAATTACACTAAGTCAATAGGCATAGAGCCAGGA
CTGTTTGGGTAAACTGGTCACTTTATCTTAAACTAAATATATCCAAAACTGAACATGTAC
TTAGTTACTAAGTCTTTGACTTTATCTCATTCATACCACTCAGCTTTATCCAGGCCACTTA
TTTGACAGTATTATTGCGAAAACTTCCTATCTAGAAGTTTGAGGAGAATATTTGTTATAT
TTGCAAAATAAAATAAGTTTGCAAGTTTTTTTTTCTGCCCCAAAGAGCTCTGTGTCCT
TGAACATAAAATACAAATAACCGCTATGCTGTTAATTATTGACAAATGTCCCATTTTCAA
CCTAAGGAAATACCATAAAGTAACAGATATACCAACAAAAGGTTACTAGTTAACAGGC
ATTGCCTGAAAAGAGTATAAAAGAATTTCAGCATGATTTTCCATATTGTGCTTCCACCA
CTGCCAATAACAAAATAACTAGCAA (SEQ ID NO:23).

CAGATTGAATTATTTGCCTGTCATACAGCTAATAATTGACCATAAGACAATTAGATT
TAAATTAGTTTTGAATCTTTCTAATACCAAAGTTCAGTTTACTGTTCCATGTTGCTTCTG
AGTGGCTTCACAGACTTATGAAAAAGTAAACGGAATCAGAATTACATCAATGCAAAAG
CATTGCTGTGAACTCTGTACTTAGGACTAAACTTTGAGCAATAACACATATAGATTGAG
GATTGTTTGCTGTTAGTATACAAACTCTGGTTCAAAGCTCCTCTTTATTGCTTGTCTTGG
AAAATTTGCTGTTCTTCATGGTTTCTCTTTTCACTGCTATCTATTTTTCTCAACCACTCAC
ATGGCTACAAAAGCTTTCTAGAAGTTTGAGGAGAATATTTGTTATATTTGCAAAATAAA
ATAAGTTTGCAAGTTTTTTTTTCTGCCCCAAAGAGCTCTGTGTCCTTGAACATAAAAT
ACAAATAACCGCTATGCTGTTAATTATTGACAAATGTCCCATTTTCAACCTAAGGAAATA
CCATAAAGTAACAGATATACCAACAAAAGGTTACTAGTTAACAGGCATTGCCTGAAAA
GAGTATAAAAGAATTTCAGCATGATTTTCCATATTGTGCTTCCACCACTGCCAATAACA
AAATAACTAGCAA (SEQ ID NO:24).

CCTGATTAATAATTACACTAAGTCAATAGGCATAGAGCCAGGACTGTTTGGGTAAA
CTGGTCACTTTATCTTAAACTAAATATATCCAAAACTGAACATGTACTTAGTTACTAAGT
CTTTGACTTTATCTCATTCATACCACTCAGCTTTATCCAGGCCACTTATTGACAGTATTA
TTGCGAAAACTTCCTATCTAGAAGTTTGAGGAGAATATTGTTATATTGCAAAATAAAA
TAAGTTTGCAAGTTTTTTTTTCTGCCCCAAAGAGCTCTGTGTCCTTGAACATAAAATA
CAAATAACCGCTATGCTGTTAATTATTGACAAATGTCCCATTTTCAACCTAAGGAAATAC
CATAAAGTAACAGATATACCAACAAAAGGTTACTAGTTAACAGGCATTGCCTGAAAAG
AGTATAAAAGAATTTCAGCATGATTTTCCATATTGTGCTTCCACCACTGCCAATAACAA

AATAACTAGCAA (SEQ ID NO:25).

### 2.2 Construction of microRNA (miRNA) fluorescent plasmid reporter system for detection of expression levels of different cell lines

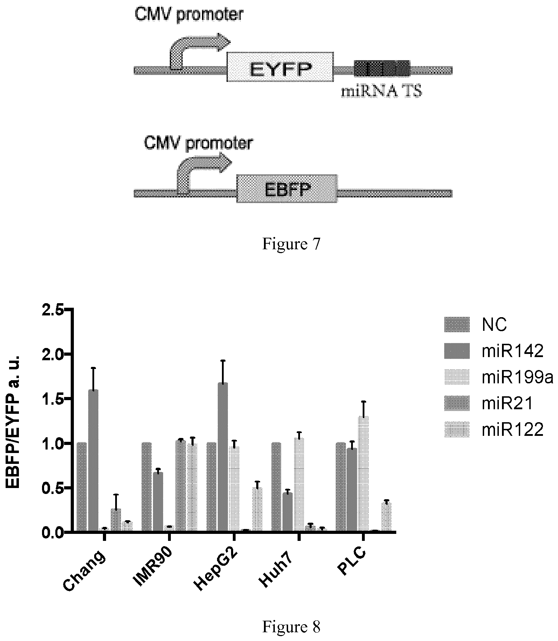

Recent research shows that the formation and growth of a tumor will greatly change the expression level of microRNA (miRNA). As shown in Table 1, various microRNAs are highly expressed in different cancer cells. The inventors selected miR199a as a microRNA marker to characterize the high expression of normal hepatocytes, and correspondingly selected miR21 and miR122 as microRNA markers to characterize the high expression of hepatoma cells based on the analysis of the known research results. First, the inventors have designed and constructed a microRNA fluorescent plasmid reporter system. As shown in Figure 7, target sequences of microRNAs were inserted into the 3' untranslated region of commonly-expressed EYFP by the inventors, with another commonly-expressed EBFP as a fluorescence control. Several different cell lines were transfected with the EYFP and EBFP fluorescent plasmids respectively to detect the expression of the microRNA target sequences. The experimental results are shown in Figure 8, indicating that miR199a is highly expressed in normal hepatocytes compared to hepatoma cell lines, while miR21 exhibits significantly increased expression level in hepatoma cell lines, thus the miR199a and the miR21 can be used as effective markers of the mutually repressive switch designed by the inventors to distinguish hepatoma cells from normal cells.

### 2.3 Mutually repressive switch can effectively respond to microRNA input signals and stably function in different cell lines

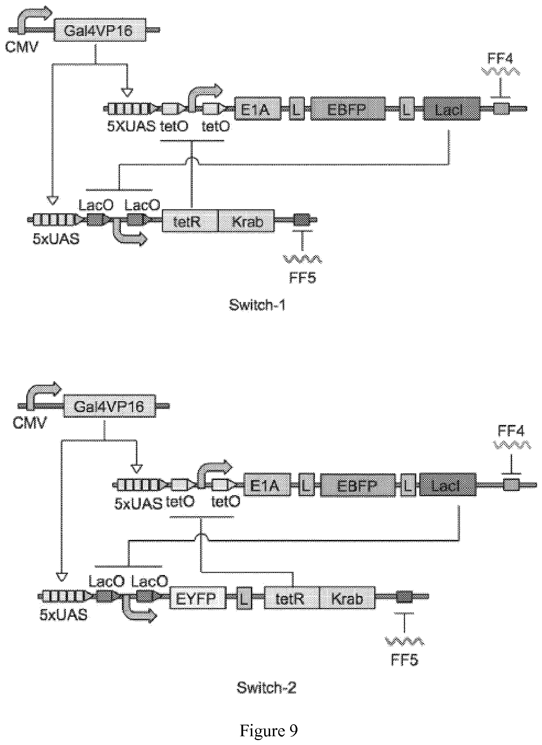

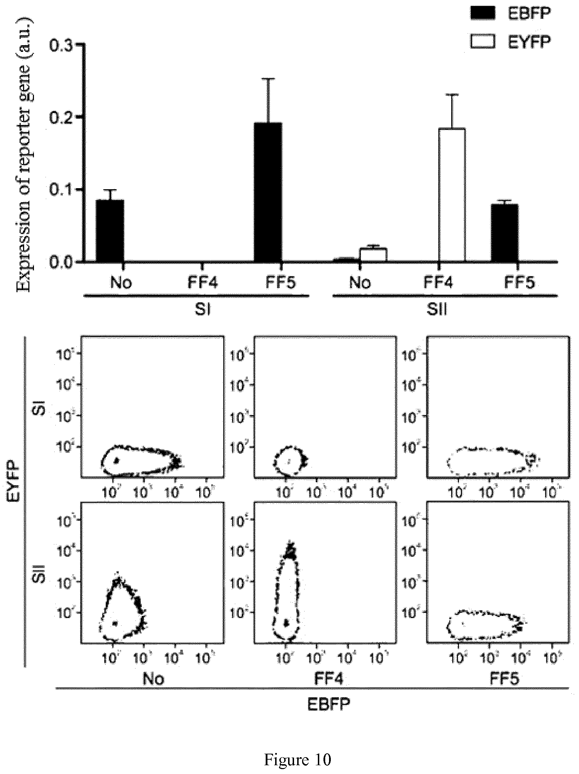

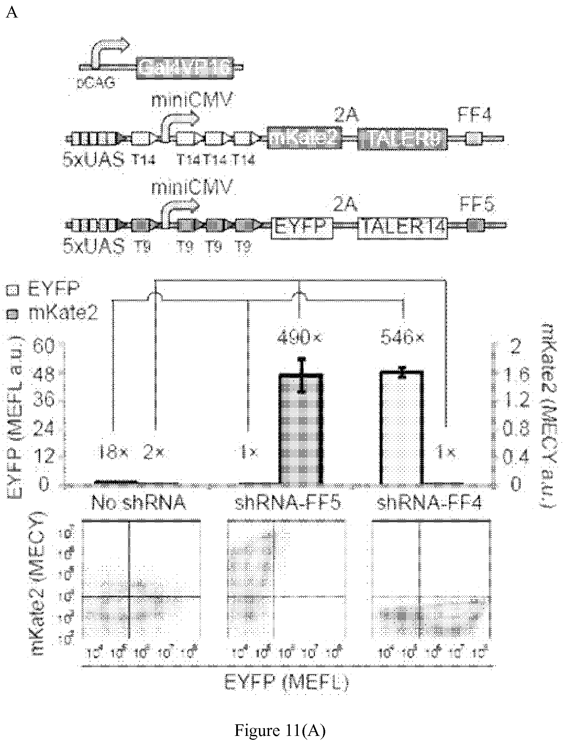

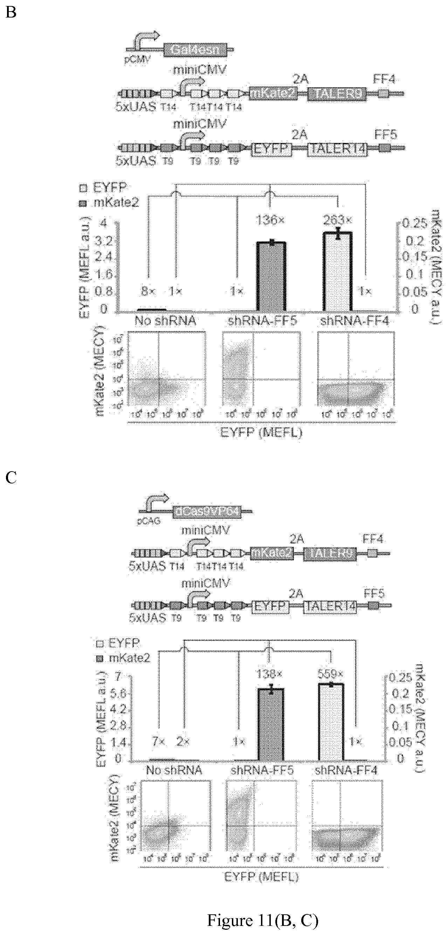

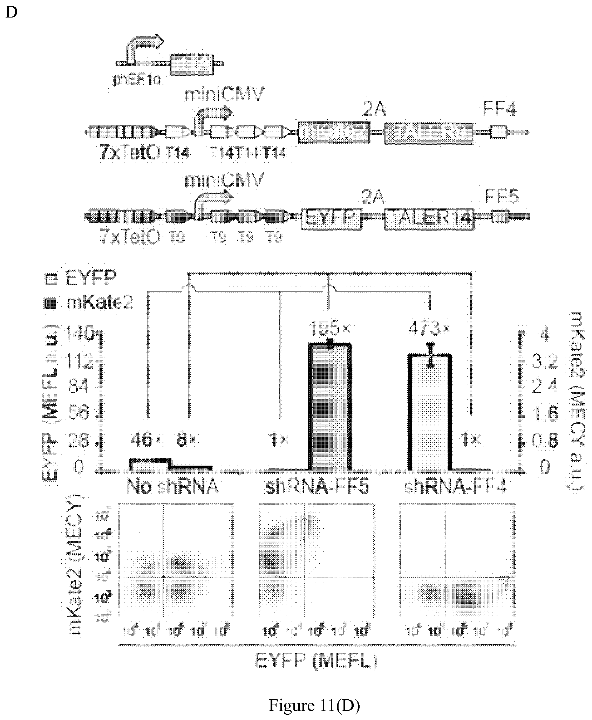

Because of limited packaging capacity of the oncolytic adenovirus (only 8 K foreign genes can be packaged at most), the inventors have selected short and effective inhibition elements as much as possible in this example. The inventors selected LacI and tetR-KRAB in this example. As shown in Figure 9, the inventors constructed two logic circuits depending on whether the EYFP gene is co-expressed on the tetR-KRAB side, that is Switch I (SI) which does not express EYFP, and Switch II (SII) which expresses EYFP. The two switches were transiently transfected into HEK293 cells individually and each were cotransfected with shRNA-FF4 and shRNA-FF5 respectively. As shown in Figure 10, the present two switches both can efficiently reverse under different input signals. The SI expresses at a higher level on the LacI side, but exhibits a higher leakage level on the tetR-KRAB side than SII. The inventors after comprehensive consideration have chosen the SI system as a backbone for constructing the present oncolytic adenovirus carrying different effectors in subsequent experiments, since the SI switch system is capable of responding to different input signals and efficiently expressing the E1 gene in cells of interest. Based on the previous results in laboratory, the inventors tested the regulation effects of different activators on the mutually repressive switch SI. As shown in Figure 11, the inventors tested the regulation efficiency of activators Gal4VP16, Gal4esn, dCas9-VP64 and rtTA. The experimental results show that the gene circuit can effectively respond to those different activators, proving the stability of such a gene circuit.

### 2.4 Construction of oncolytic adenovirus by using the CGGA method

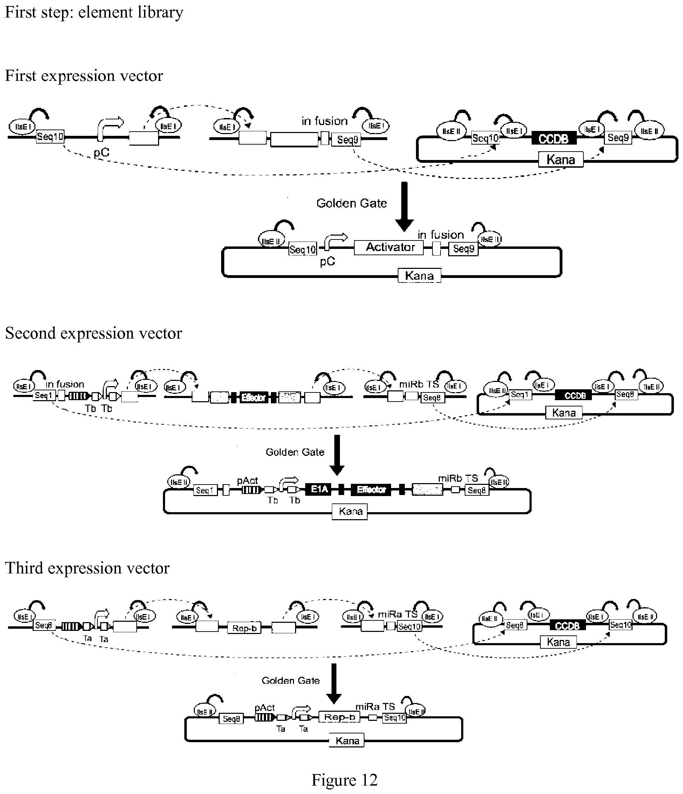

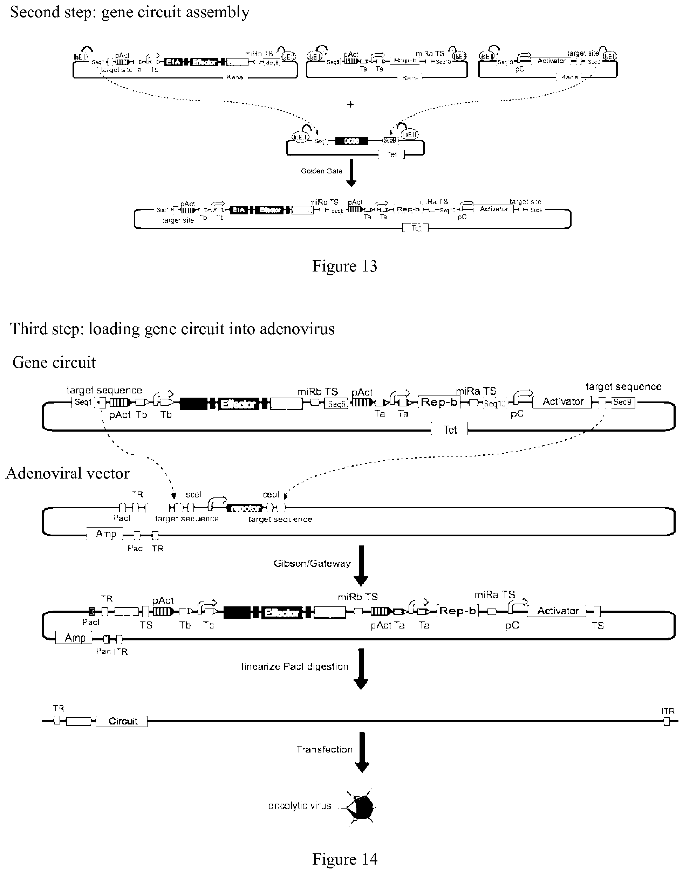

A large amount of clinical information indicates that the expression of tumor markers varies greatly among different individuals. Therefore, different markers may be chosen by the inventors for different individuals. In order to achieve the precise treatment for individual patients, the inventors need to prepare a variety of oncolytic adenoviruses carrying different biomarkers. However, it is very difficult to rapidly construct these oncolytic adenoviruses through traditional enzyme digestion and ligation method. In the present example, the inventors designed and constructed a three-stage library, and rapidly modified oncolytic adenovirus by the CGGA method (Cascade Golden-Gate Gateway/Gibson Assemble method). First, marker elements associated with distinguishing of cancer cells from normal cells (such as a tumor-specific promoter, a logic circuit-related repression element, and a cancer cell-distinguishing miRNA recognition sequence) are constructed on a primary plasmid, thereby forming a primary element library, as shown in Figure 12. Two ends of the primary elements are each inserted with the recognition site of EI (a restriction enzyme of which recognition site and cleavage site are different, such as Esp3I and BsaI), so as to construct multiple primary elements into one secondary expression system by the Golden Gate method. An expression system is constructed as follow. Specifically, multiple primary elements are selected from the primary element library and are assembled into three expression systems by the Golden Gate method, such three expression systems including a tumor-specific promoter system, a first regulatory element-mediated repression system and a second regulatory element-mediated repression system. Then, a complete logic circuit is formed by randomly assembling the three expression systems by the Golden Gate method, as shown in Figure 13. Two ends of the logic circuit are each inserted with Gibson homologous sequence or Gateway recognition site (target sequence)for further experiments. Finally, the logic circuit is constructed into an adenoviral vector (with E1 and part of E3 removed) by the Gibson or Gateway method, as shown in Figure 14. The engineered adenoviral vector is linearized by PacI digestion, and then transfected into a specific cell line so as to package adenoviral particles. Thus, one or more tumor biomarkers can be replaced by the inventors.

### 2.5 Detection of oncolytic adenovirus on effectively killing of hepatoma cells at cellular level

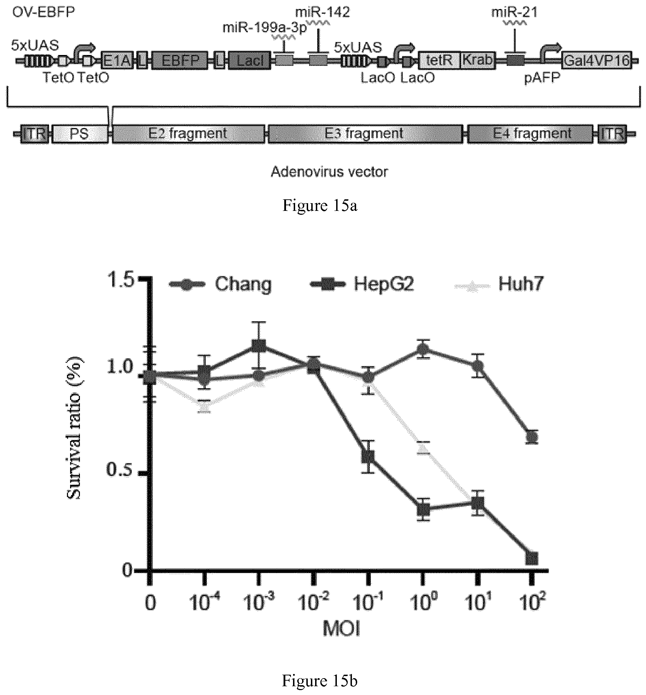

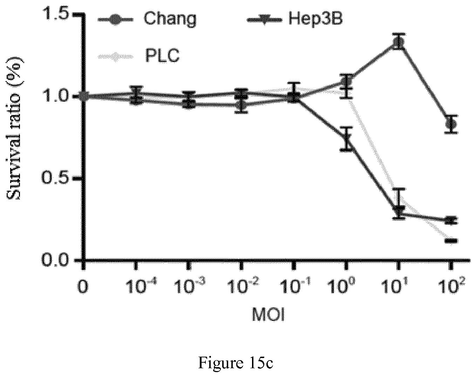

In order to detect whether the mutually repressive switch in the oncolytic adenovirus constructed by the inventors can effectively distinguish and kill hepatoma cells, the inventors designed an *in vitro* cell killing assay for oncolytic adenovirus. Different cell lines were infected with the oncolytic adenovirus in different multiplicity of infection, where Chang cells are normal liver cells, and Huh7 and HepG2 cells are human hepatoma cell lines. The viability of cells was measured by the MTS method after 6 days of incubation. As shown in Figure 15, it is found that the hepatoma cells show severe death due to significant cytotoxicity when the multiplicity of infection is 100, 10 and 1 respectively, and the hepatoma cells exhibit slight cell death at the multiplicity of infection of 0.1. Correspondingly, Chang cells are in normal growth state without cell death when the multiplicity of infection is 10 or less; while they exhibit a slight cytotoxic response at the multiplicity of infection of 100, with enlarged cell volume and slowed cell growth. This data indicates that the packaged adenovirus within a specific range of multiplicity of infection can effectively distinguish and kill hepatoma cells, without interference of growth and proliferation of normal liver cells.

### 2.6 In vitro detection of expression level of effector carried by oncolytic adenovirus

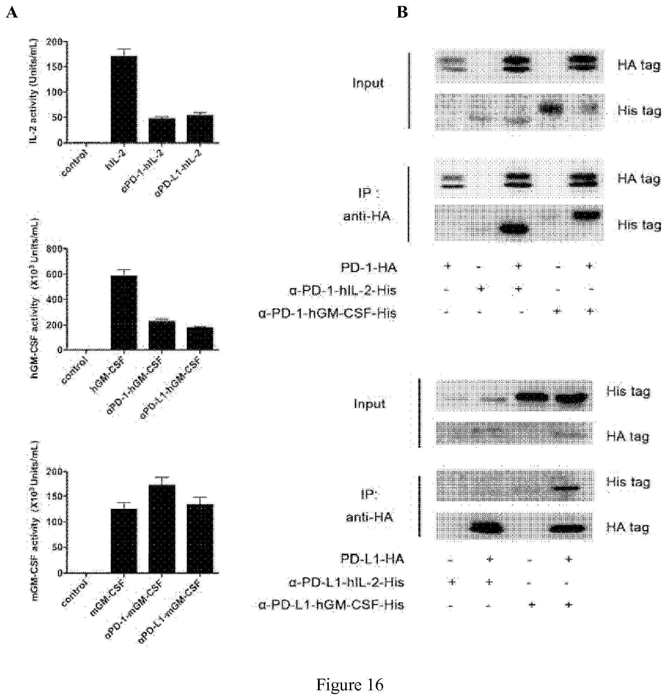

The adenoviral vectors currently used in clinical practice are human Ad2 or Ad5 type adenoviruses which are widely present in nature. Further, neutralizing antibodies that antagonize these adenoviruses have been known to exist in the human body. Thus, clinical data showed a poor treatment effect for the tumor treatment relying on the oncolytic effect of adenovirus alone. Therefore, the inventors in the example used the oncolytic adenovirus as a delivery system to carry effector genes to tumor sites, so as to cause a systemic immune response. As shown in Figure 16, the inventors first constructed these effector genes into a frequently-expressed plasmid vector, and detected the activity of these effectors by transient transfection. The results demonstrate that the effector genes constructed by the inventors can efficiently express immune factors having biological function, and repressors of immunological checkpoint genes. The inventors further constructed these effector genes on an oncolytic adenoviral vector, thus obtaining the oncolytic adenovirus which can express these effectors after packaging and purification. After infection of the hepatoma cells HepG2, the inventors extracted the supernatant to detect the activity of immune factors in the supernatant.

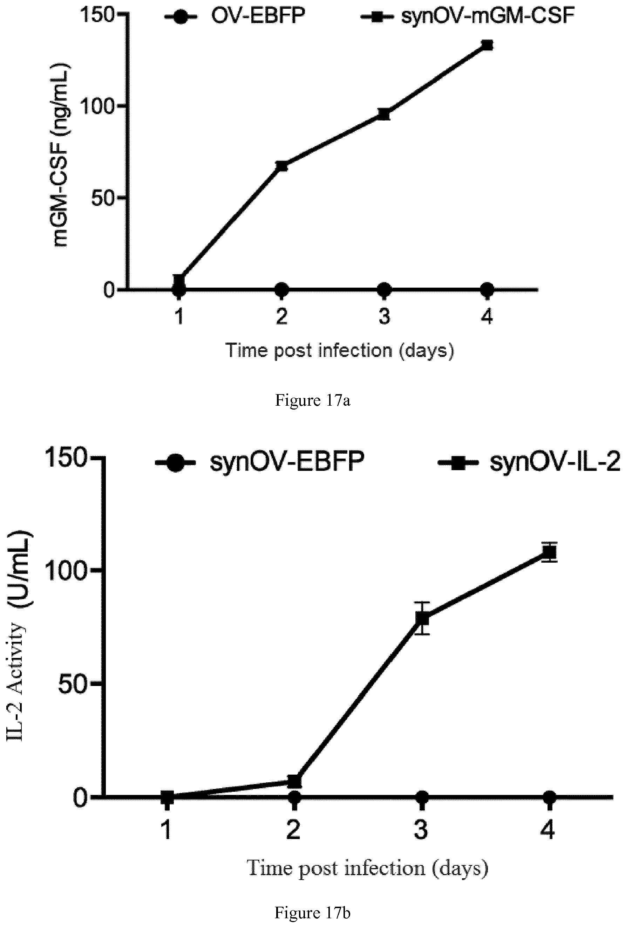

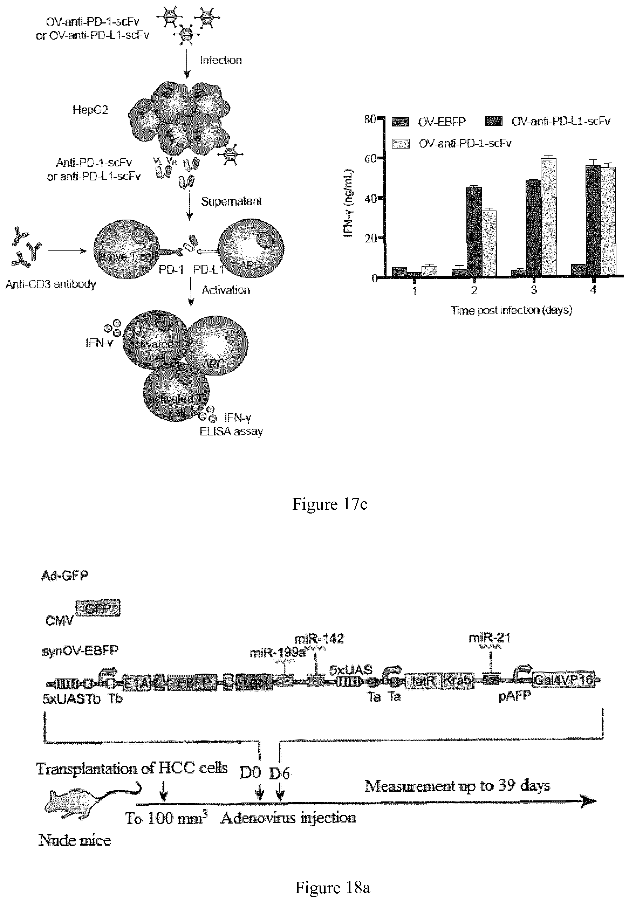

In the meantime, the activity of the effectors expressed by the effector genes in the adenoviral vector has been also detected by the inventors. As shown in Figure 17, the effectors expressed by the adenovirus are effectively enhanced over time. The three cytokines are detected by the method as follows. In Figure 17(a), 3 × 10⁶ HepG2 cells were seeded into 12-well plates, infected with synOV-EBFP and synOV-mGM-SCF at the multiplicity of infection of 10 respectively, and collected the supernatant at day 1, 2, 3 and 4 post-infection. The mouse GM-CSF content in the supernatant was detected by an ELISA kit. In Figure 17(b), 3 × 10⁶ HepG2 cells were seeded into 12-well plates, infected with synOV-EBFP and synOV-hIL-2 at the multiplicity of infection of 10 respectively, and collected the supernatant at day 1, 2, 3 and 4 post-infection. The supernatant was added into a medium containing IL-2 dependent cells, and the IL-2 content in the supernatant was detected by the MTS method. In Figure 17(c), 3 × 10⁶ HepG2 cells were seeded into 12-well plates, infected with synOV-EBFP, synOV-anti-PD-1 scFv and synOV-anti-PD-L1 scFv at the multiplicity of infection of 10 respectively, and collected the supernatant at day 1, 2, 3 and 4 post-infection. The supernatant obtained was added into a spleen cell system (formed by addition of 2 µg/mL anti-mouse CD3-antibody in isolated spleen cells for T cell activation) at a dilution ratio of 1:10, and the IFN-γ production in the spleen cell system was detected by the ELISA method after 48 hours of incubation.

### 2.7 Detection of therapeutic effect of oncolytic adenovirus by animal studies

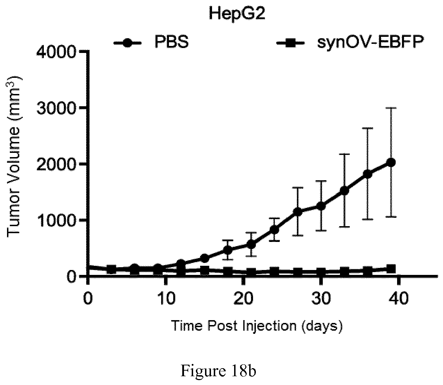

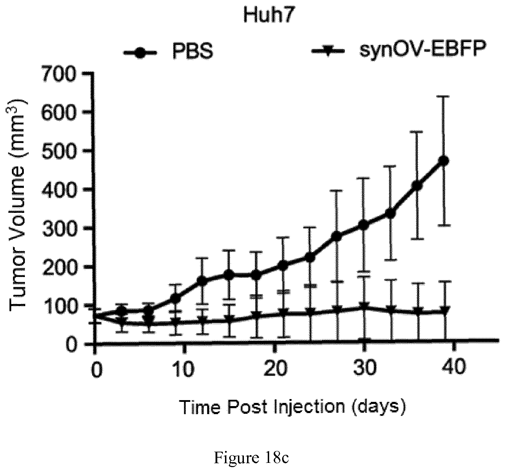

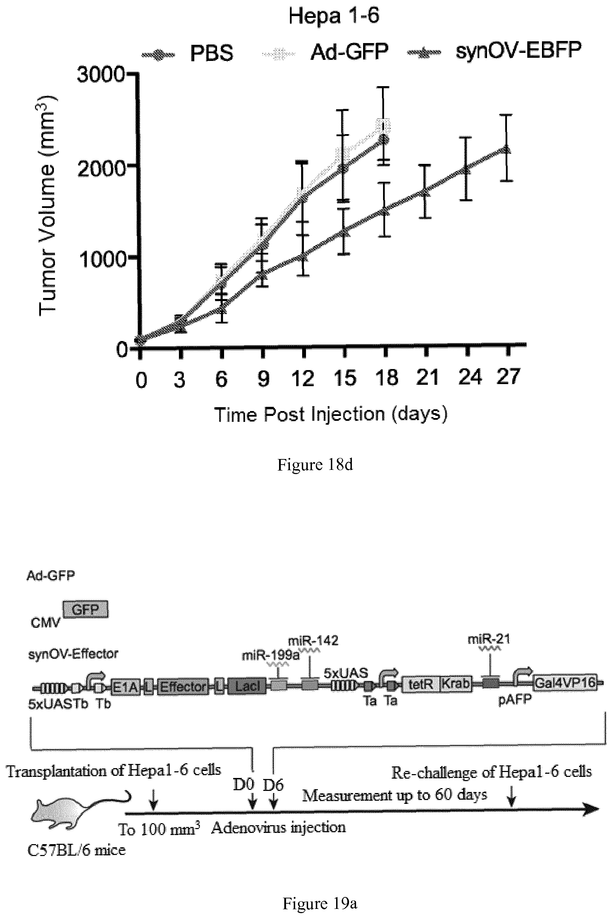

As shown in Figure 18, 1 × 10⁷ HepG2, Huh7 and Hepa1-6 hepatoma cells were subcutaneously transplanted into the right flank of nude mice, with measurement of subcutaneous tumor volume every three days. HepG2 and Huh7 cells are human hepatoma cells, and Hepa1-6 cells are mouse hepatoma cells. After tumor volume exceeded 100 mm³, mice were divided randomly into two groups, where the treatment group was intratumorally injected with 1 × 10⁹ oncolytic adenovirus and the control group was injected with PBS. The change of tumor volume after treatment was continuously observed. It can be seen that the tumor in the control group injected with PBS continues to grow, with increasingly increased tumor volume, while the tumor in the treatment group is significantly inhibited after the injection of oncolytic adenovirus, indicating that the oncolytic adenovirus can inhibit the tumor growth *in vivo* through lysis of tumor cells, thereby exhibiting a significant therapeutic effect on the formed tumor. In the treatment of mouse liver cancer, the inventors used a non-oncolytic viruse (the second generation adenoviral vector, GFP gene-bearing virus, Ad-GFP) as another virus control. It can be seen from the experimental results that the tumors in the PBS and Ad-GFP treatment groups continue growing, while the oncolytic virus treatment groups can inhibit the growth of tumors to some extent and slightly inhibit the growth of tumor. It is probably resulted from a faster growth rate of Hepa1-6 cells than HepG2 and Huh7 cells, as well as a relatively-low expression level of AFP in the Hepa1-6 cells. Therefore, the inventors employed the immune effector-carrying oncolytic adenovirus to treat the Hepa1-6 tumor model in subsequent experiments, which achieved greatly effective results.

After two weeks or one month of treatment of the tumor-bearing mice with oncolytic adenovirus, tissue samples of various organs of the mice were taken, and the virus DNA was extracted after homogenization. The viral titer was detected by the qPCR method. It is found that the viruses are only replicated in the tumor cells and do not spread into other tissues after virus infection. The results demonstrate that oncolytic adenovirus *in vivo* has a strong specificity and is safe to normal tissue cells.

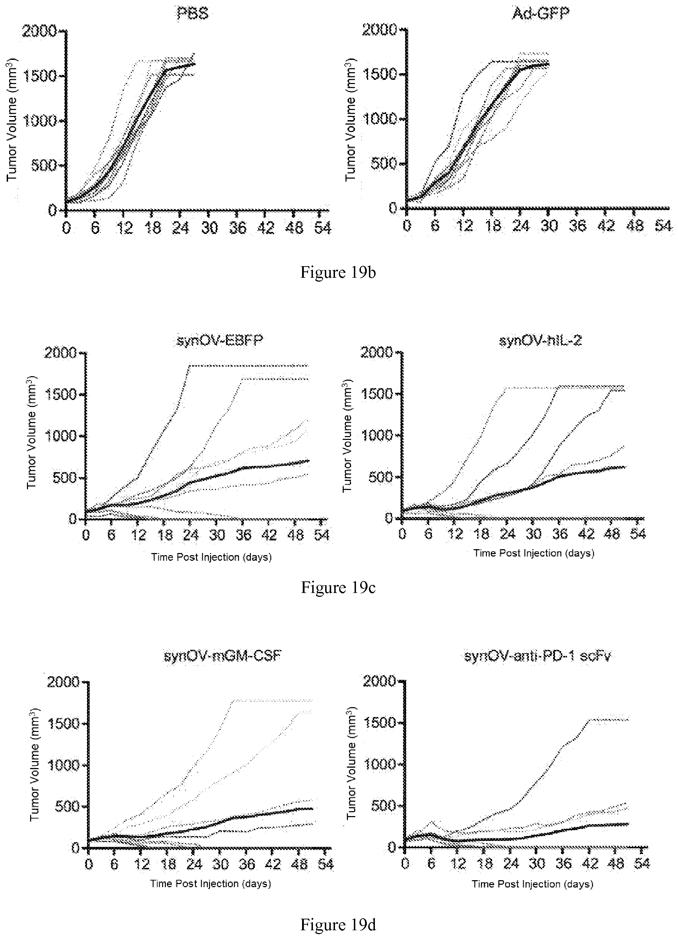

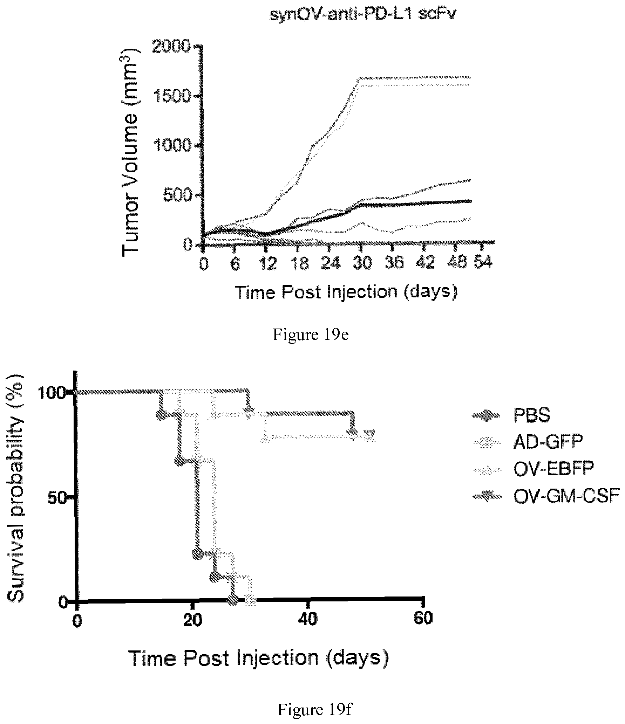

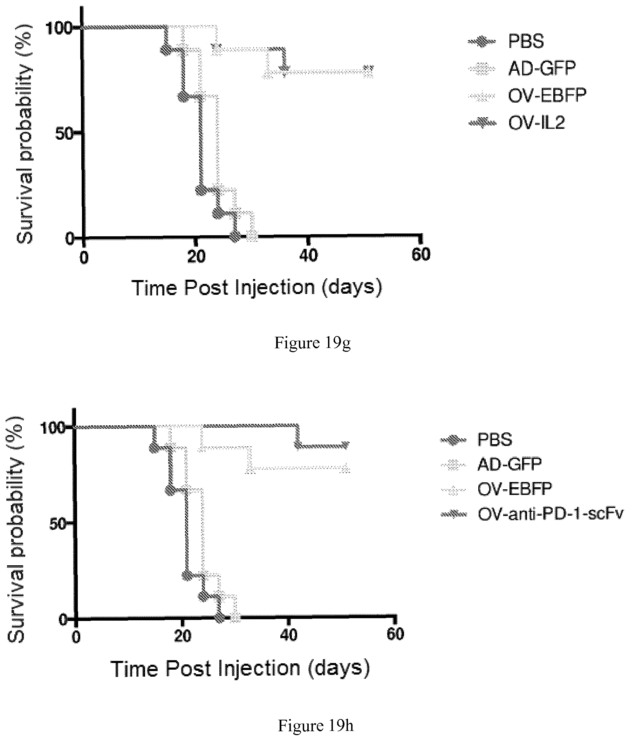

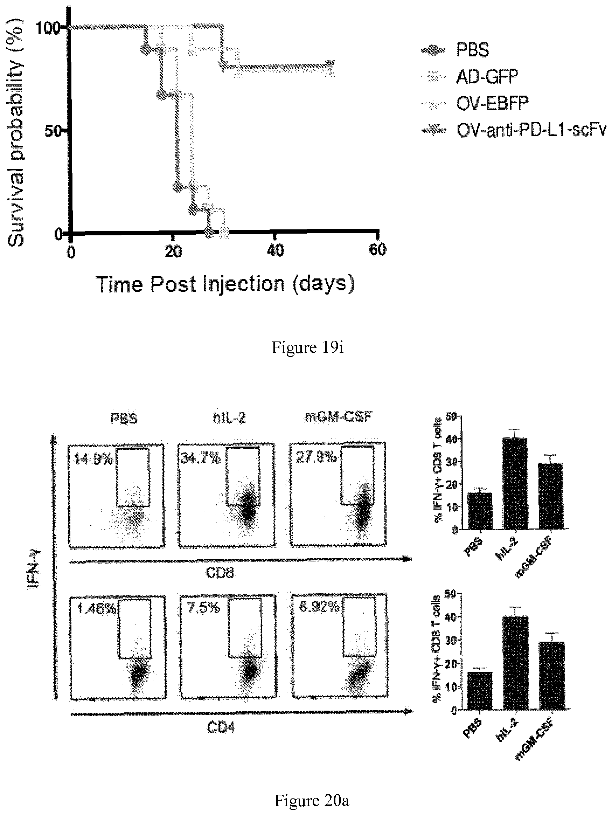

The therapeutic effect of the present oncolytic adenovirus on a C57 mouse model with an immune system is further demonstrated. As shown in Figure 19, 1 × 10⁶ mouse hepatoma cells Hepa1-6 were subcutaneously injected into the right flank of wild-type C57BL/6J mice. After the tumor volume reached 100 mm³, 1 × 10⁹ oncolytic adenoviruses expressing different cytokines were intratumorally injected into each group, with PBS injection as a negative control. The tumor volume after treatment was observed, and the survival time of the tumor-bearing mice was analyzed by the Kaplan-Meier method. The experimental results demonstrate that oncolytic adenovirus can still inhibit the tumor growth and significantly prolong the survival time of tumor-bearing mice with an intact immune system. Some tumor-bearing mice were cured after treatment, with totally disappeared tumor. A month after the initial treatment with oncolytic adenovirus, the mice survived were re-challenged with 1 × 10⁶ mouse hepatoma cells Hepa1-6. It is found that a tumor mass cannot be formed when the one same mouse was transplanted with the tumor cells same as those firstly injected at a site away from the first transplantation site. These results demonstrate that the mice treated with oncolytic virus have generated specific immunity against tumor cells in body, which can resist the same tumor cells from growing again in body.

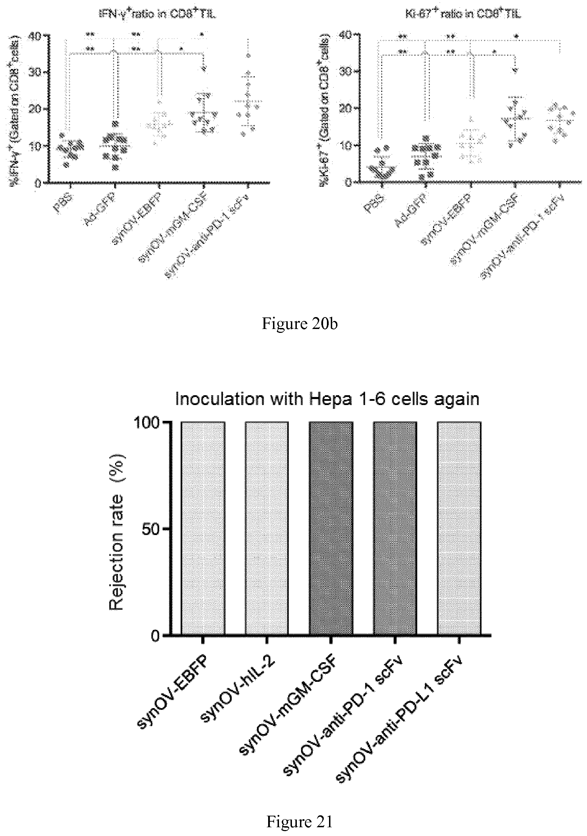

Tumors of tumor-bearing mice were taken, fixed, sectioned and HE stained for detection of lymphocyte infiltration in tumor tissues. It can be found that lymphocyte infiltration in tumor tissues of mice which is treated with oncolytic adenovirus is significantly increased compared with the PBS control group, demonstrating that the treatment of oncolytic adenovirus can recruit more lymphocytes in tumor tissues. These results indicated that expression of immune stimulators can promote the cytotoxic T cell response during oncolytic virus therapies. The infiltrating lymphocytes in tumors were isolated and purified, and the phenotype of the infiltrating T cells in mouse tumors was detected by the flow cytometry. It is found that a greater proportion of Ki-67⁺ cells among infiltrating T cells in tumors of mice treated with oncolytic virus, which indicates treatment with oncolytic adenovirus promotes the proliferation of T cells inside the tumor and triggers a stronger immune response. Tumor-infiltrating lymphocytes were added to RPMI 1640 medium containing PMA (20 ng/mL) and Ionomycin (1 µg/ml L), cultured at 37°C for 4 hours in the presence of Brevedlin A, followed by fixation and staining of cells, and detecting the expression of γ-interferon in CD4⁺ and CD8⁺ T cells by flow cytometry. We found a higher expression of γ-interferon in T cells in tumors of the oncolytic adenovirus treatment group, which changed the immune microenvironment in tumor and promoted the killing of tumor cells, as shown in Figure 20(a) and Figure 20(b).

### Systemic anti-tumor immune response by treatment of oncolytic adenovirus

C57BL/6 mice were subcutaneously injected with mouse hepatoma cells Hepa-1-6 on both left and right flanks. After the formation of tumor mass, the oncolytic adenoviruses expressing cytokine hIL-2, mGM-CSF and anti-PD-1 scFv respectively were injected into the tumor on the right flank, with PBS and AdGFP injections as controls. The tumor growth was measured continuously. The infiltrating lymphocytes in tumors on the left and right flanks of the tumor-bearing mice after 14 days of treatment were isolated and purified, and the phenotype of infiltrating T cells in the tumors was detected by the flow cytometry method. We found a higher proportion of T cells expressing Ki-67⁺ T cells or interferon-y are present not only in the tumor injected with oncolytic adenovirus on the right flank but also in the tumor on the left flank without oncolytic adenovirus injection, compared with the control groups. It is demonstrated that the immune response caused by the treatment of oncolytic adenovirus can not only function on the tumor in the injection site, but also on the tumor in the distal side. This suggests that the oncolytic adenovirus not only has therapeutic effect on the tumor in the injection site, but also exhibits therapeutic effect on the tumor in distal metastasis.

At the same time, in order to detect whether the treatment of oncolytic adenovirus has generated an effective systemic immune response in survived mice, Hepa1-6 was subcutaneously injected again into the mice after treatment by the inventors. It is found that the rejection rate of all the mice is 100%, as shown in Figure 21.

### 2.8 Modeling of the treatment process of oncolytic adenovirus

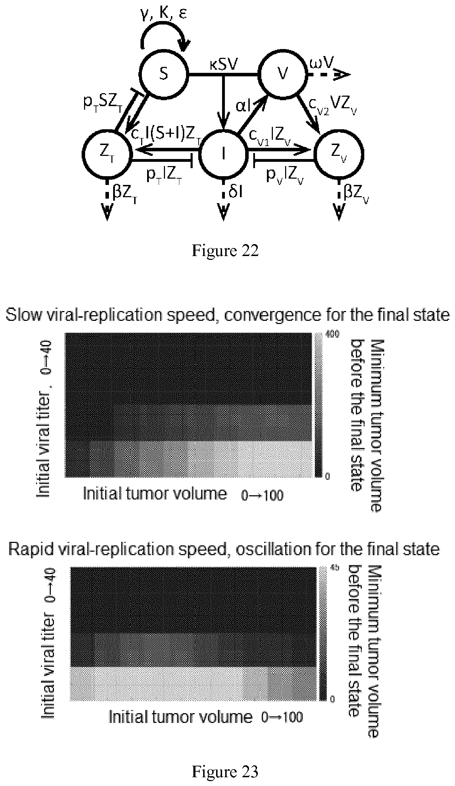

The treatment process of oncolytic adenovirus is abstracted into a tumor-virus-immune system, including uninfected tumor cells, infected tumor cells, free viruses, oncolytic adenovirus antigen activated-immune cells and tumor antigen activated-immune cells, such five components represented by S, I, V, Z_{V} and Z_{T} respectively, as shown in Figure 22. The model assumes that the growth of uninfected tumor cells conforms to the generalized logistic growth model, where γ indicates the growth rate, K indicates the carrying capacity and ε indicates the non-linearity. Uninfected tumor cells can be infected with free viruses and transformed into infected tumor cells at a rate of κ. The infected tumor cells will lyse at a rate of δ, and release large amount of free viruses at a rate of α. The free virus naturally decays in the environment at a rate of ω. Z_{V} will be increased by the activation of oncolytic adenovirus or the surface antigen of tumor cells infected with viruses at a rate of c_{V1} and c_{V2} respectively, while it will inhibit the production of the infected tumor cells at a rate of p_{V}. Z_{T} will be increased by the activation of antigens released by lysed tumor cells at a rate of c_{T}, which will inhibit the production of the infected and uninfected tumor cells at a rate of p_{T}. Z_{V} and Z_{T} will naturally decay in the environment at a rate of β.

Without considering the immune system, the final state of the tumor-virus system will be converted from a state of convergence to oscillation with the increase of the virus replication speed. The simulations show the change of the minimal tumor volume in the tumor-virus system from an initial state to a final state over time under different initial virus titer, initial tumor size and viral replication rate. It can be seen from the simulation results that increase of initial viral titer contributes to the control of tumor size. In practical applications, the appropriate initial administration dose can be chose in combination of the initial tumor size and administration dose limit to clear tumors at predicted time points or provide other adjuvant therapies, as shown in Figure 23.

### Tumor size in the tumor-virus-immune system at the final state during the duration of simulation under different inhibition effects of immune cells on tumors and viruses

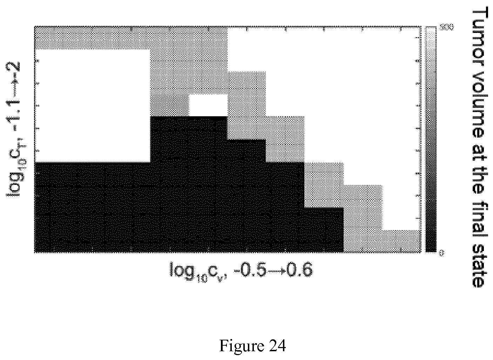

It can be seen from the simulation results that the system exhibits several dynamic behaviors including tending to complete tumor elimination, tumor growth towards saturation, stable tumor volume, and unstable tumor volume. In general, stronger the inhibition effect of immune cells on tumors is, more tumors are cleared by the system, while stronger the inhibition effect of immune cells on viruses is, more tumors in the system are out of control. However, the system exhibits non-monotonic changes on the tendencies of stability and non-stability of tumor, as shown in Figure 24.

In the specification of the present disclosure, the terms "an embodiment", "some embodiments", "a specific embodiment", "an example", "a specific example", "some examples" and the like are intended to refer to particular features, structures, materials or characteristics described by way of example or embodiment are contained in at least one embodiment or example of the disclosure. In this specification, the schematic representation of the above terms does not necessarily refer to the same embodiment or example. Moreover, the particular features, structures, materials or characteristics described may be combined in any suitable manner in one or more embodiments or examples. In addition, various embodiments or examples described in the specification and features of various embodiments or examples may be combined by skilled in the art without contradiction.

Although embodiments of the present disclosure have been described in detail, it will be understood the embodiments are illustrative and are not to be construed as limiting the scope of the disclosure. Changes, modifications, substitutions and variations can be made in these embodiments by those skilled in the art within the scope of the present disclosure.

## Claims

1. An expression system comprising:
a first nucleic acid molecule, incorporating a cell-specific promoter;
a second nucleic acid molecule, operably linked to the first nucleic acid molecule and encoding a transcriptional activator;
a third nucleic acid molecule, incorporating a first recognition sequence of the transcriptional activator;
a fourth nucleic acid molecule, operably linked to the third nucleic acid molecule and incorporating a first promoter and a first regulatory element;
a fifth nucleic acid molecule, operably linked to the fourth nucleic acid molecule and encoding a first regulatory protein;
a sixth nucleic acid molecule, incorporating a second recognition sequence of the transcriptional activator;
a seventh nucleic acid molecule, operably linked to the sixth nucleic acid molecule and incorporating a second promoter and a second regulatory element;
an eighth nucleic acid molecule, operably linked to the seventh nucleic acid and encoding a second regulatory protein; and
at least one selected from the group consisting of:
a ninth nucleic acid molecule, operably linked to the fifth nucleic acid molecule and configured to conditionally inhibit expression of the first regulatory protein;
a tenth nucleic acid molecule, operably linked to the eighth nucleic acid molecule and configured to conditionally inhibit expression of the second regulatory protein,
wherein
the first regulatory element is adapted to inhibit the function of the first promoter by binding to the second regulatory protein, and
the second regulatory element is adapted to inhibit the function of the second promoter by binding to the first regulatory protein.

2. The expression system according to claim 1, wherein the cell-specific promoter is a tumor cell-specific promoter, and the tumor cell-specific promoter is at least one selected from the group consisting of an alpha-fetoprotein-specific promoter, a Survivin promoter, a human telomerase reverse transcriptase gene promoter, a cholecystokinin A receptor gene promoter, a carcinoembryonic antigen promoter, a proto-oncogene human epidermal growth factor receptor 2 promoter, a prostaglandin endoxygenase reductase 2 promoter, a chemokine receptor-4, an E2F-1 gene promoter, a mucin promoter, a prostate specific antigen, a human tyrosinase-related protein 1, and a tyrosinase promoter.

3. The expression system according to claim 1, wherein the transcriptional activator is at least one selected from the group consisting of Gal4VP16, Gal4vp64, dCas9-VPR, dCas9-VP64, dCas9-VP16, dCas9-VTR, and rtTA.

4. The expression system according to claim 1, wherein the first recognition sequence and the second recognition sequence are each independently selected from at least one of 5 × UAS, 7 × tetO and a target sequence of dCas9.

5. The expression system according to claim 1, wherein the first promoter and the second promoter are each independently selected from a miniCMV and a TATA box.

6. The expression system according to claim 1, wherein the first regulatory protein and the second regulatory protein are each independently selected from at least one of Lacl, tetR, zinc finger, KRAB, tetR-KRAB and dCas9-KRAB.

7. The expression system according to claim 6, wherein the first regulatory element and the second regulatory element are each independently selected from at least one of a tetO, a LacO, a zinc finger target site, and a target sequence of dCas9.

8. The expression system according to claim 7, wherein the first regulatory protein is LacI, and the second regulatory element comprises a plurality of repeated LacO sequences, wherein at least one of the pluralities of repeated LacO sequences is set downstream of the second promoter.

9. The expression system according to claim 6, wherein the second regulatory protein is tetR-KRAB, and the first regulatory element comprises a plurality of repeated tetO sequences, wherein at least one of the pluralities of repeated tetO sequences is set downstream of the first promoter.

10. The expression system according to claim 1, wherein at least one of the fifth nucleic acid molecule and the ninth nucleic acid molecule further comprises a sequence encoding a protein of interest.

11. The expression system according to claim 10, wherein the fifth nucleic acid molecule comprises a sequence encoding the protein of interest, and the protein of interest comprises at least one selected from the group consisting of a viral replication and packaging protein and an immune effector.

12. The expression system according to claim 11, wherein the virus replication and packaging protein comprises at least one selected from the group consisting of an adenovirus E1 gene, an adenovirus E1A gene, an adenovirus E1B gene, an adenovirus E2 gene and an adenovirus E4 gene.

13. The expression system according to claim 11, wherein the immune effector comprises at least one sequence selected from the group consisting of an inhibitory sequence that antagonizes PD-1 gene, an inhibitory sequence that antagonizes PD-L1 gene, an inhibitory sequence that antagonizes CTLA4 gene, an inhibitory sequence that antagonizes Tim-3 gene, GM-CSF, IL-2, IL-12, and IL-15.

14. The expression system according to claim 10, wherein the protein of interest and the first regulatory protein are expressed in a form of a fusion protein, and the protein of interest and the first regulatory protein are linked by a cleavable linker peptide.

15. The expression system according to claim 1, wherein the ninth nucleic acid molecule and the tenth nucleic acid molecule independently inhibit expression of the first regulatory protein or the second regulatory protein, respectively, by RNA interference.

16. The expression system according to claim 15, wherein the ninth nucleic acid molecule comprises a nucleic acid sequence specifically recognized by a first microRNA, the tenth nucleic acid molecule comprises a nucleic acid sequence specifically recognized by a second microRNA, the first microRNA is a normal cell-specific microRNA, and the second microRNA is an abnormal cell-specific microRNA.

17. The expression system according to claim 16, wherein the first microRNA comprises at least one selected from the group consisting of miR199a, miR95, miR125, miR25b, Let-7, miR143, miR145, and miR200C.

18. The expression system according to claim 16, wherein the second microRNA comprises at least one selected from the group consisting of miR21, miR223, miR224, miR221, miR18, miR214, miR146a, and miR1792.

19. The expression system according to claim 1, wherein the first nucleic acid molecule and the second nucleic acid molecule are loaded in a first expression vector; the third nucleic acid molecule, the fourth nucleic acid molecule, the fifth nucleic acid molecule and optionally the ninth nucleic acid molecule are loaded in a second expression vector; and the sixth nucleic acid molecule, the seventh nucleic acid molecule, the eighth nucleic acid molecule and optionally the tenth nucleic acid molecules are loaded in a third expression vector.

20. The expression system according to claim 19, wherein the first expression vector, the second expression vector and the third expression vector are each independently selected from at least one of a plasmid, a virus, a nanomaterial, a liposome, a molecularly coupled vector, naked DNA, a chromosomal vector, and a polymer.

21. The expression system according to claim 20, wherein the virus comprises at least one selected from the group consisting of an adenovirus, a vaccinia virus, a herpes virus and a retrovirus.

22. The expression system according to claim 19, wherein the first expression vector, the second expression vector and the third expression vector are loaded in one same vector.

23. The expression system according to claim 22, wherein the one same vector is an adenoviral vector.

24. The expression system according to claim 19, wherein the first expression vector comprises: BsaI, AFP III, Gal4VP16 and BsaI from the 5' end to the 3' end,
optionally the first expression vector carries a nucleic acid having the nucleotide sequence shown in SEQ ID NO: 1.

25. The expression system according to claim 19, wherein the second expression vector comprises: BsaI, 5 × UAS, tetO, miniCMV, tetO, E1A, 2A, an immune effect factor, LacI, microRNA199a specific recognition sequence and BsaI from the 5' end to the 3' end,
optionally the second expression vector carries a nucleic acid having the nucleotide sequence selected from the group consisting of SEQ ID NOs: 2-7.

26. The expression system according to claim 19, wherein the third expression vector comprises: BsaI, 5 × UAS, LacO, miniCMV, LacO, tetR-KRAB, microRNA21 specific recognition sequence and BsaI from the 5' end to the 3' end,
optionally the third expression vector carries a nucleic acid having the nucleotide sequence set forth in SEQ ID NO: 8.

27. The expression system according to claim 23, wherein the adenovirus comprises: a first inverted terminal repeat sequence, a packaging signal, AFPIII, Gal4VP16, 5 × UAS, tetO, miniCMV, tetO, E1A, 2A, an immune effector, 2A, LacI, microRNA 199a specific recognition sequence, 5 × UAS, LacO, miniCMV, LacO, tetR-KRAB, microRNA 21 specific recognition sequence, an adenovirus E2 gene region, an adenovirus E3 gene region, an adenovirus E4 gene region, and a second inverted terminal repeat sequence from the 5' end to the 3' end,
optionally the adenoviral vector carries a nucleic acid having the nucleotide sequences selected from the group consisting of SEQ ID NOs: 9-14.

28. The expression system according to claim 27, wherein the adenovirus is obtained by the steps of:
removing the adenoviral E1 gene and a part of the adenoviral E3 gene associated with adenoviral replication and packaging from an adenoviral vector,
inserting the adenoviral E1A gene into a gene circuit by the stepwise Golden Gate method, and
incorporating the gene circuit into the adenoviral vector by the Gateway or Gibson method.

29. A recombinant virus comprising:
a first nucleic acid molecule, incorporating a tumor cell-specific promoter, and the tumor cell-specific promoter being an alpha-fetoprotein-specific promoter;
a second nucleic acid molecule, operably linked to the first nucleic acid molecule and encoding a transcriptional activator, the transcriptional activator being Gal4VP16;
a third nucleic acid molecule, incorporating a first recognition sequence of the transcriptional activator, the first recognition sequence being 5 × UAS;
a fourth nucleic acid molecule, operably linked to the third nucleic acid molecule and incorporating a first promoter and a first regulatory element, wherein the first promoter is a miniCMV, the first regulatory element comprises a plurality of repeated tetO sequences, and at least one of the pluralities of repeated tetO sequences is set downstream of the first promoter;
a fifth nucleic acid molecule, operably linked to the fourth nucleic acid molecule and encoding a first regulatory protein, the first regulatory protein being LacI,
wherein
the fifth nucleic acid molecule further comprises a sequence encoding a protein of interest, the protein of interest comprises a viral replication protein and an immune effector, the immune effector is expressed in a form of an individual protein or a fusion protein, the viral replication protein and the immune effector are linked by a cleavable linker peptide,
the protein of interest and the first regulatory protein are expressed in a form of a fusion protein, and the protein of interest and the first regulatory protein are linked by a cleavable linker peptide;
a sixth nucleic acid molecule, incorporating a second recognition sequence of the transcriptional activator, the second recognition sequence being 5 × UAS;
a seventh nucleic acid molecule, operably linked to the sixth nucleic acid molecule and incorporating a second promoter and a second regulatory element, wherein the second promoter is miniCMV, the second regulatory element comprises a plurality of repeated LacO sequences, and at least one of the pluralities of repeated LacO sequences is inserted downstream of the second promoter;
an eighth nucleic acid molecule, operably linked to the seventh nucleic acid molecule and encoding a second regulatory protein, the second regulatory protein being tetR-KRAB;
a ninth nucleic acid molecule, operably linked to the fifth nucleic acid molecule and configured to conditionally inhibit expression of the first regulatory protein, wherein the ninth nucleic acid molecule comprises a nucleic acid sequence specifically recognized by a first microRNA, and the first microRNA is a normal cell-specific microRNA; and
a tenth nucleic acid molecule, operably linked to the eighth nucleic acid molecule and configured to conditionally inhibit expression of the second regulatory protein, wherein the tenth nucleic acid molecule comprises a nucleic acid sequence specifically recognized by a second microRNA, and the second microRNA is a tumor cell-specific microRNA,
wherein
the first regulatory element is adapted to inhibit the function of the first promoter by binding to the second regulatory protein, and
the second regulatory element is adapted to inhibit the function of the second promoter by binding to the first regulatory protein.

30. The recombinant virus according to claim 29, wherein the recombinant virus comprises at least one selected from the group consisting of an adenovirus, a vaccinia virus, a retrovirus and a herpes virus.

31. The recombinant virus according to claim 29, wherein the recombinant virus is an adenovirus.

32. The recombinant virus according to claim 29, wherein the immune effector comprises at least one sequence selected from the group consisting of an inhibitory sequence that antagonizes PD-1 gene, an inhibitory sequence that antagonizes PD-L1 gene, an inhibitory sequence that antagonizes CTLA4 gene, an inhibitory sequence that antagonizes Tim-3 gene, IL-2, IL-12, IL-15 and GM-CSF.

33. A recombinant cell comprising the expression system according to any one of claims 1 to 28.

34. The recombinant cell according to claim 33, wherein at least a portion of the expression system is integrated into the genome of the recombinant cell.

35. Use of the expression system according to any one of claims 1 to 28, the recombinant virus according to any one of claims 29 to 32, and the recombinant cell according to claim 33 or 34 in the preparation of a medicament for treating a cancer.

36. The use according to claim 35, wherein the cancer comprises liver cancer, lung cancer, colorectal cancer, melanoma, breast cancer or prostate cancer.

37. A method of expressing a protein of interest by using an expression system according to any one of claims 1 to 28, the method comprises steps of:
(1) providing a fifth nucleic acid molecule which comprises a nucleic acid sequence encoding the protein of interest; and
(2) inhibiting expression of a second regulatory protein by a tenth nucleic acid molecule so as to express the protein of interest.

38. The method according to claim 37, wherein the expression is carried out in a cell.

39. The method according to claim 37, wherein the tenth nucleic acid molecule comprises a nucleic acid sequence specifically recognized by a second microRNA, and
the method further comprises contacting the second microRNA with the tenth nucleic acid molecule in the step (2).

40. A method of expressing a protein of interest by using an expression system according to any one of claims 1 to 28, the method comprises steps of:
(1) providing an eighth nucleic acid molecule which comprises a nucleic acid sequence encoding a first protein of interest, and providing a fifth nucleic acid molecule which comprises a nucleic acid sequence encoding a second protein of interest; and
(2) inhibiting expression of the first regulatory protein by the ninth nucleic acid molecule so as to express the first protein of interest; or
inhibiting expression of the second regulatory protein by the tenth nucleic acid molecule so as to express the second protein of interest.


SEQUENCE LISTING

<110>  Tsinghua University
       Beijing Syngentech Co., LTD.

<120>  Programmable Oncolytic Virus Vaccine System and Application Thereof

<130>  PIDC1168718P

<160>  25

<170>  PatentIn version 3.3

<210>  1
<211>  2126
<212>  DNA
<213>  Artificial

<220>
<223>  A nucleotide sequence of a nucleic acid carried by the first
expression vector

<400>  1
ggtctctgct ccagattgaa ttatttgcct gtcatacagc taataattga ccataagaca      60

attagattta aattagtttt gaatctttct aataccaaag ttcagtttac tgttccatgt     120

tgcttctgag tggcttcaca gacttatgaa aaagtaaacg gaatcagaat tacatcaatg     180

caaaagcatt gctgtgaact ctgtacttag gactaaactt tgagcaataa cacatataga     240

ttgaggattg tttgctgtta gtatacaaac tctggttcaa agctcctctt tattgcttgt     300

cttggaaaat ttgctgttct tcatggtttc tcttttcact gctatctatt tttctcaacc     360

actcacatgg ctacaaaagc ttcctgatta ataattacac taagtcaata ggcatagagc     420

caggactgtt tgggtaaact ggtcacttta tcttaaacta aatatatcca aaactgaaca     480

tgtacttagt tactaagtct ttgactttat ctcattcata ccactcagct ttatccaggc     540

cacttatttg acagtattat tgcgaaaact tcctatctag aagtttgagg agaatatttg     600

ttatatttgc aaaataaaat aagtttgcaa gttttttttt tctgccccaa agagctctgt     660

gtccttgaac ataaaataca aataaccgct atgctgttaa ttattgacaa atgtcccatt     720

ttcaacctaa ggaaatacca taaagtaaca gatataccaa caaaaggtta ctagttaaca     780

ggcattgcct gaaaagagta taaaagaatt tcagcatgat tttccatatt gtgcttccac     840

cactgccaat aacaaaataa ctagcaagga tccggccacc atggcccccc cgaccgatgt     900

cagcctgggg gacgagctcc acttagacgg cgaggacgtg gcgatggcgc atgccgacgc     960

gctagacgat ttcgatctgg acatgttggg ggacggggat tccccgggtc cgggatttac    1020

cccccacgac tccgccccct acggcgctct ggatatggcc gacttcgagt ttgagcagat    1080

gtttaccgat gcccttggaa ttgacgagta cggtgggacg cgtatgaagc tactgtcttc    1140

tatcgaacaa gcatgcgata tttgccgact taaaaagctc aagtgctcca aagaaaaacc    1200

gaagtgcgcc aagtgtctga agaacaactg ggagtgtcgc tactctccca aaaccaaaag 1260

gtctccgctg actagggcac atctgacaga agtggaatca aggctagaaa gactggaaca 1320

gctatttcta ctgatttttc ctcgagaaga ccttgacatg attttgaaaa tggattcttt 1380

acaggatata aaagcattgt taacaggatt atttgtacaa gataatgtga ataaagatgc 1440

cgtcacagat agattggctt cagtggagac tgatatgcct ctaacattga gacagcatag 1500

aataagtgcg acatcatcat cggaagagag tagtaacaaa ggtcaaagac agttgactgt 1560

ataattcact cctcaggtgc aggctgccta tcagaaggtg gtggctggtg tggccaatgc 1620

cctggctcac aaataccact gagatctttt tccctctgcc aaaaattatg gggacatcat 1680

gaagcccctt gagcatctga cttctggcta ataaaggaaa tttattttca ttgcaatagt 1740

gtgttggaat tttttgtgtc tctcactcgg aaggacatat gggagggcaa atcatttaaa 1800

acatcagaat gagtatttgg tttagagttt ggcaacatat gcccatatgc tggctgccat 1860

gaacaaaggt tggctataaa gaggtcatca gtatatgaaa cagccccctg ctgtccattc 1920

cttattccat agaaaagcct tgacttgagg ttagattttt tttatatttt gttttgtgtt 1980

atttttttct ttaacatccc taaaattttc cttacatgtt ttactagcca gatttttcct 2040

cctctcctga ctactcccag tcatagctgt ccctcttctc ttatggagat cggagaaaga 2100

ggtaatttaa ttaagtcgat gagacc 2126

<210> 2
<211> 3510
<212> DNA
<213> Artificial

<220>
<223> BsaI-5¡ÁUAS-tetO-miniCMV-tetO-E1A-2A-EBFP-2A-LacI-miR199a target
      site-BsaI sequence

<400> 2
ggtctctcta tttaattaag taactataac ggtcgctccg aatttctcga gttaattaag 60

attacgccaa gctacgggcg gagtactgtc ctccgagcgg agtactgtcc tccgagcgga 120

gtactgtcct ccgagcggag tactgtcctc cgagcggagt tctgtcctcc gagcggagac 180

tctagactcc ctatcagtga tagagatagg cgtgtacggt gggaggccta tataagcaga 240

gctcgtttag tgaaccgtca gatcgctccc tatcagtgat agagagaatt cgaccgccac 300

catgtcggaa ttcatgagac atattatctg ccacggaggt gttattaccg aagaaatggc 360

cgccagtctt ttggaccagc tgatcgaaga ggtactggct gataatcttc cacctcctag 420

ccattttgaa ccacctaccc ttcacgaact gtatgattta gacgtgacgg cccccgaaga 480

tcccaacgag gaggcggttt cgcagatttt tcccgactct gtaatgttgg cggtgcagga 540

agggattgac ttactcactt ttccgccggc gcccggttct ccggagccgc ctcacctttc 600

ccggcagccc gagcagccgg agcagagagc cttgggtccg gtttctatgc caaaccttgt 660
accggaggtg atcgatctta cctgccacga ggctggcttt ccacccagtg acgacgagga 720
tgaagagggt gaggagtttg tgttagatta tgtggagcac cccgggcacg gttgcaggtc 780
ttgtcattat caccggagga atacggggga cccagatatt atgtgttcgc tttgctatat 840
gaggacctgt ggcatgtttg tctacagtaa gtgaaaatta tgggcagtgg gtgatagagt 900
ggtgggtttg gtgtggtaat ttttttttta atttttacag ttttgtggtt taaagaattt 960
tgtattgtga tttttttaaa aggtcctgtg tctgaacctg agcctgagcc cgagccagaa 1020
ccggagcctg caagacctac ccgccgtcct aaaatggcgc ctgctatcct gagacgcccg 1080
acatcacctg tgtctagaga atgcaatagt agtacggata gctgtgactc cggtccttct 1140
aacacacctc ctgagataca cccggtggtc ccgctgtgcc ccattaaacc agttgccgtg 1200
agagttggtg ggcgtcgcca ggctgtggaa tgtatcgagg acttgcttaa cgagcctggg 1260
caacctttgg acttgagctg taaacgcccc aggccactcg agggtaccgg gtccggagct 1320
acaaattttt ccctcctcaa acaggctgga gatgtcgaag aaaatcctgg gcctaccggt 1380
cccatggtga gcaagggcga ggagctgttc accggggtgg tgcccatcct ggtcgagctg 1440
gacggcgacg taaacggcca caagttcagc gtgaggggcg agggcgaggg cgatgccacc 1500
aacggcaagc tgaccctgaa gttcatctgc accaccggca agctgcccgt gccctggccc 1560
accctcgtga ccaccctgag ccacggcgtg cagtgcttcg cccgctaccc cgaccacatg 1620
aagcagcacg acttcttcaa gtccgccatg cccgaaggct acgtccagga gcgcaccatc 1680
ttcttcaagg acgacggcac ctacaagacc cgcgccgagg tgaagttcga gggcgacacc 1740
ctagtgaacc gcatcgagct gaagggcgtc gacttcaagg aggacggcaa catcctgggg 1800
cacaagctgg agtacaactt caacagccac aacatctata tcatggccgt caagcagaag 1860
aacggcatca aggtgaactt caagatccgc cacaacgtgg aggacggcag cgtgcagctc 1920
gccgaccact accagcagaa cacccccatc ggcgacggcc ccgtgctgct gcccgacagc 1980
cactacctga gcacccagtc cgtgctgagc aaagacccca acgagaagcg cgatcacatg 2040
gtcctgctgg agttccgcac cgccgccggg atcactctcg gcatggacga gctgtacaag 2100
agatctgagg gccgcggcag cctgctgacc tgcggcgacg tggaggaaaa ccccggcccc 2160
ggatccatga aaccagtaac gttatacgat gtcgcagagt atgccggtgt ctcttatcag 2220
accgtttccc gcgtggtgaa ccaggccagc cacgtttctg cgaaaacgcg ggaaaaagtg 2280
gaagcggcga tggcggagct gaattacatt cccaaccgcg tggcacaaca actggcgggc 2340
aaacagtcgt tgctgattgg cgttgccacc tccagtctgg ccctgcacgc gccgtcgcaa 2400
attgtcgcgg cgattaaatc tcgcgccgat caactgggtg ccagcgtggt ggtgtcgatg 2460
gtagaacgaa gcggcgtcga agcctgtaaa gcggcggtgc acaatcttct cgcgcaacgc 2520

gtcagtgggc tgatcattaa ctatccgctg gatgaccagg atgccattgc tgtggaagct 2580

gcctgcacta atgttccggc gttatttctt gatgtctctg accagacacc catcaacagt 2640

attattttct cccatgaaga cggtacgcga ctgggcgtgg agcatctggt cgcattgggt 2700

caccagcaaa tcgcgctgtt agcgggccca ttaagttctg tctcggcgcg tctgcgtctg 2760

gctggctggc ataaatatct cactcgcaat caaattcagc cgatagcgga acgggaaggc 2820

gactggagtg ccatgtccgg ttttcaacaa accatgcaaa tgctgaatga gggcatcgtt 2880

cccactgcga tgctggttgc caacgatcag atggcgctgg gcgcaatgcg cgccattacc 2940

gagtccgggc tgcgcgttgg tgcggatatc tcggtagtgg gatacgacga taccgaagac 3000

agctcatgtt atatcccgcc gttaaccacc atcaaacagg attttcgcct gctggggcaa 3060

accagcgtgg accgcttgct gcaactctct cagggccagg cggtgaaggg caatcagctg 3120

ttgcccgtct cactggtgaa aagaaaaacc accctggcgc ccaatacgca aaccgcctct 3180

ccccgcgcgt tggccgattc attaatgcag ctggcacgac aggtttcccg actggaaagc 3240

gggcagagca gcctgaggcc tcctaagaag aagaggaagg ttgcggccgc taaccaatgt 3300

gcagactact gttaaccaat gtgcagacta ctgtgaattc taaccaatgt gcagactact 3360

gttaaccaat gtgcagacta ctgtaagctt cgaattgagt agtgctttct actttatgag 3420

tagtgctttc tactttatga gtagtgcttt ctactttatg agtagtgctt tctactttat 3480

ggtcgacaat actagttaag aaatgagacc 3510

<210> 3
<211> 3242
<212> DNA
<213> Artificial

<220>
<223> BsaI-5¡ÁUAS-tetO-miniCMV-tetO-E1A-2A-hIL-2-2A-LacI-miR199a target
 site-BsaI sequence

<400> 3
ggtctctcta tttaattaag taactataac ggtcgctccg aatttctcga gttaattaag 60

attacgccaa gctacgggcg gagtactgtc ctccgagcgg agtactgtcc tccgagcgga 120

gtactgtcct ccgagcggag tactgtcctc cgagcggagt tctgtcctcc gagcggagac 180

tctagactcc ctatcagtga tagagatagg cgtgtacggt gggaggccta tataagcaga 240

gctcgtttag tgaaccgtca gatcgctccc tatcagtgat agagagaatt cgaccgccac 300

catgtcggaa ttcatgagac atattatctg ccacggaggt gttattaccg aagaaatggc 360

cgccagtctt ttggaccagc tgatcgaaga ggtactggct gataatcttc cacctcctag 420

ccattttgaa ccacctaccc ttcacgaact gtatgattta gacgtgacgg cccccgaaga 480

tcccaacgag gaggcggttt cgcagatttt tcccgactct gtaatgttgg cggtgcagga 540

agggattgac ttactcactt ttccgccggc gcccggttct ccggagccgc ctcacctttc 600

ccggcagccc gagcagccgg agcagagagc cttgggtccg gtttctatgc caaaccttgt 660

accggaggtg atcgatctta cctgccacga ggctggcttt ccacccagtg acgacgagga 720

tgaagagggt gaggagtttg tgttagatta tgtggagcac cccgggcacg gttgcaggtc 780

ttgtcattat caccggagga atacggggga cccagatatt atgtgttcgc tttgctatat 840

gaggacctgt ggcatgtttg tctacagtaa gtgaaaatta tgggcagtgg gtgatagagt 900

ggtgggtttg gtgtggtaat ttttttttta atttttacag ttttgtggtt taaagaattt 960

tgtattgtga tttttttaaa aggtcctgtg tctgaacctg agcctgagcc cgagccagaa 1020

ccggagcctg caagacctac ccgccgtcct aaaatggcgc ctgctatcct gagacgcccg 1080

acatcacctg tgtctagaga atgcaatagt agtacggata gctgtgactc cggtccttct 1140

aacacacctc ctgagataca cccggtggtc ccgctgtgcc ccattaaacc agttgccgtg 1200

agagttggtg ggcgtcgcca ggctgtggaa tgtatcgagg acttgcttaa cgagcctggg 1260

caacctttgg acttgagctg taaacgcccc aggccactcg aggagggccg cggcagcctg 1320

ctgacctgcg gcgacgtgga ggaaaacccc ggccccaccg gtcaccatgg atgtacagga 1380

tgcaactcct gtcttgcatt gcactaagtc ttgcacttgt cacaaacagt gcacctactt 1440

caagttctac aaagaaaaca cagctacaac tggagcattt actgctggat ttacagatga 1500

ttttgaatgg aattaataat tacaagaatc ccaaactcac cgcgatgctc acagctaagt 1560

ttgccatgcc caagaaggcc acagaactga aacatcttca gtgtctagaa gaagcactca 1620

aacctctgga ggaagtgcta aatttagctc aaagcaaaaa ctttcactta agacccaggg 1680

acttaatcag caatatcaac gtaatagttc tggaactaaa gggatctgaa acaacattca 1740

tgtgtgaata tgctgatgag acagcaacca ttgtagaatt tctgaacaga tggattacct 1800

tttgtcaaag catcatctca acactgactt gaagatctga gggccgcggc agcctgctga 1860

cctgcggcga cgtggaggaa aaccccggcc ccggatccat gaaaccagta acgttatacg 1920

atgtcgcaga gtatgccggt gtctcttatc agaccgtttc ccgcgtggtg aaccaggcca 1980

gccacgtttc tgcgaaaacg cgggaaaaag tggaagcggc gatggcggag ctgaattaca 2040

ttcccaaccg cgtggcacaa caactggcgg gcaaacagtc gttgctgatt ggcgttgcca 2100

cctccagtct ggccctgcac gcgccgtcgc aaattgtcgc ggcgattaaa tctcgcgccg 2160

atcaactggg tgccagcgtg gtggtgtcga tggtagaacg aagcggcgtc gaagcctgta 2220

aagcggcggt gcacaatctt ctcgcgcaac gcgtcagtgg gctgatcatt aactatccgc 2280

tggatgacca ggatgccatt gctgtggaag ctgcctgcac taatgttccg gcgttatttc 2340

ttgatgtctc tgaccagaca cccatcaaca gtattatttt ctcccatgaa gacggtacgc 2400

gactgggcgt ggagcatctg gtcgcattgg gtcaccagca aatcgcgctg ttagcgggcc 2460

cattaagttc tgtctcggcg cgtctgcgtc tggctggctg gcataaatat ctcactcgca 2520

atcaaattca gccgatagcg gaacgggaag gcgactggag tgccatgtcc ggttttcaac 2580

aaaccatgca aatgctgaat gagggcatcg ttcccactgc gatgctggtt gccaacgatc 2640

agatggcgct gggcgcaatg cgcgccatta ccgagtccgg gctgcgcgtt ggtgcggata 2700

tctcggtagt gggatacgac gataccgaag acagctcatg ttatatcccg ccgttaacca 2760

ccatcaaaca ggattttcgc ctgctggggc aaaccagcgt ggaccgcttg ctgcaactct 2820

ctcagggcca ggcggtgaag ggcaatcagc tgttgcccgt ctcactggtg aaaagaaaaa 2880

ccaccctggc gcccaatacg caaaccgcct ctccccgcgc gttggccgat tcattaatgc 2940

agctggcacg acaggtttcc cgactggaaa gcgggcagag cagcctgagg cctcctaaga 3000

agaagaggaa ggttgcggcc gctaaccaat gtgcagacta ctgttaacca atgtgcagac 3060

tactgtgaat tctaaccaat gtgcagacta ctgttaacca atgtgcagac tactgtaagc 3120

ttcgaattga gtagtgcttt ctactttatg agtagtgctt tctactttat gagtagtgct 3180

ttctacttta tgagtagtgc tttctacttt atggtcgaca atactagtta agaaatgaga 3240

cc 3242

<210> 4
<211> 3233
<212> DNA
<213> Artificial

<220>
<223> BsaI-5¡ÁUAS-tetO-miniCMV-tetO-E1A-2A-hGMCSF-2A-LacI-miR199a
      target site-BsaI sequence

<400> 4
ggtctctcta tttaattaag taactataac ggtcgctccg aatttctcga gttaattaag 60

attacgccaa gctacggcgg gagtactgtc ctccgagcgg agtactgtcc tccgagcgga 120

gtactgtcct ccgagcggag tactgtcctc cgagcggagt tctgtcctcc gagcggagac 180

tctagactcc ctatcagtga tagagatagg cgtgtacggt gggaggccta tataagcaga 240

gctcgtttag tgaaccgtca gatcgctccc tatcagtgat agagagaatt cgaccgccac 300

catgtcggaa ttcatgagac atattatctg ccacggaggt gttattaccg aagaaatggc 360

cgccagtctt ttggaccagc tgatcgaaga ggtactggct gataatcttc cacctcctag 420

ccattttgaa ccacctaccc ttcacgaact gtatgattta gacgtgacgg cccccgaaga 480

tcccaacgag gaggcggttt cgcagatttt tcccgactct gtaatgttgg cggtgcagga 540

agggattgac ttactcactt ttccgccggc gcccggttct ccggagccgc ctcacctttc 600

ccggcagccc gagcagccgg agcagagagc cttgggtccg gtttctatgc caaaccttgt 660

accggaggtg atcgatctta cctgccacga ggctggcttt ccacccagtg acgacgagga 720

tgaagagggt gaggagtttg tgttagatta tgtggagcac cccgggcacg gttgcaggtc 780

ttgtcattat caccggagga atacggggga cccagatatt atgtgttcgc tttgctatat 840

gaggacctgt ggcatgtttg tctacagtaa gtgaaaatta tgggcagtgg gtgatagagt 900

ggtgggtttg gtgtggtaat ttttttttta atttttacag ttttgtggtt taaagaattt 960

tgtattgtga tttttttaaa aggtcctgtg tctgaacctg agcctgagcc cgagccagaa 1020

ccggagcctg caagacctac ccgccgtcct aaaatggcgc ctgctatcct gagacgcccg 1080

acatcacctg tgtctagaga atgcaatagt agtacggata gctgtgactc cggtccttct 1140

aacacacctc ctgagataca cccggtggtc ccgctgtgcc ccattaaacc agttgccgtg 1200

agagttggtg ggcgtcgcca ggctgtggaa tgtatcgagg acttgcttaa cgagcctggg 1260

caacctttgg acttgagctg taaacgcccc aggccactcg agggtaccgg gtccggagct 1320

acaaattttt ccctcctcaa acaggctgga gatgtcgaag aaaatcctgg gcctaccggt 1380

caccatggat gtggctgcag agcctgctgc tcttgggcac tgtggcctgc agcatctctg 1440

cacccgcccg ctcgcccagc cccagcacgc agccctggga gcatgtgaat gccatccagg 1500

aggcccggcg tctcctgaac ctgagtagag acactgctgc tgagatgaat gaaacagtag 1560

aagtcatctc agaaatgttt gacctccagg agccgacctg cctacagacc cgcctggagc 1620

tgtacaagca gggcctgcgg ggcagcctca ccaagctcaa gggccccttg accatgatgg 1680

ccagccacta caagcagcac tgccctccaa ccccggaaac ttcctgtgca acccagatta 1740

tcacctttga aagtttcaaa gagaacctga aggactttct gcttgtcatc ccctttgact 1800

gctgggagcc agtccaggag tgaagatctg agggccgcgg cagcctgctg acctgcggcg 1860

acgtggagga aaaccccggc cccggatcca tgaaaccagt aacgttatac gatgtcgcag 1920

agtatgccgg tgtctcttat cagaccgttt cccgcgtggt gaaccaggcc agccacgttt 1980

ctgcgaaaac gcgggaaaaa gtggaagcgg cgatggcgga gctgaattac attcccaacc 2040

gcgtggcaca acaactggcg ggcaaacagt cgttgctgat tggcgttgcc acctccagtc 2100

tggccctgca cgcgccgtcg caaattgtcg cggcgattaa atctcgcgcc gatcaactgg 2160

gtgccagcgt ggtggtgtcg atggtagaac gaagcggcgt cgaagcctgt aaagcggcgg 2220

tgcacaatct tctcgcgcaa cgcgtcagtg ggctgatcat taactatccg ctggatgacc 2280

aggatgccat tgctgtggaa gctgcctgca ctaatgttcc ggcgttattt cttgatgtct 2340

ctgaccagac acccatcaac agtattattt tctcccatga agacggtacg cgactgggcg 2400

tggagcatct ggtcgcattg ggtcaccagc aaatcgcgct gttagcgggc ccattaagtt 2460

ctgtctcggc gcgtctgcgt ctggctggct ggcataaata tctcactcgc aatcaaattc 2520

agccgatagc ggaacgggaa ggcgactgga gtgccatgtc cggttttcaa caaaccatgc 2580

aaatgctgaa tgagggcatc gttcccactg cgatgctggt tgccaacgat cagatggcgc 2640

tgggcgcaat gcgcgccatt accgagtccg ggctgcgcgt tggtgcggat atctcggtag 2700

tgggatacga cgataccgaa gacagctcat gttatatccc gccgttaacc accatcaaac 2760

aggattttcg cctgctgggg caaaccagcg tggaccgctt gctgcaactc tctcagggcc 2820

aggcggtgaa gggcaatcag ctgttgcccg tctcactggt gaaaagaaaa accaccctgg 2880

cgcccaatac gcaaaccgcc tctccccgcg cgttggccga ttcattaatg cagctggcac 2940

gacaggtttc ccgactggaa agcgggcaga gcagcctgag gcctcctaag aagaagagga 3000

aggttgcggc cgctaaccaa tgtgcagact actgttaacc aatgtgcaga ctactgtgaa 3060

ttctaaccaa tgtgcagact actgttaacc aatgtgcaga ctactgtaag cttcgaattg 3120

agtagtgctt tctactttat gagtagtgct ttctacttta tgagtagtgc tttctacttt 3180

atgagtagtg ctttctactt tatggtcgac aatactagtt aagaaatgag acc 3233

<210> 5
<211> 3224
<212> DNA
<213> Artificial

<220>
<223> BsaI-5¡ÁUAS-tetO-miniCMV-tetO-E1A-2A- mGMCSF -2A-LacI-miR199a
      target site-BsaI sequence

<400> 5
ggtctctcta tttaattaag taactataac ggtcgctccg aatttctcga gttaattaag 60

attacgccaa gctacgggcg gagtactgtc ctccgagcgg agtactgtcc tccgagcgga 120

gtactgtcct ccgagcggag tactgtcctc cgagcggagt tctgtcctcc gagcggagac 180

tctagactcc ctatcagtga tagagatagg cgtgtacggt gggaggccta tataagcaga 240

gctcgtttag tgaaccgtca gatcgctccc tatcagtgat agagagaatt cgaccgccac 300

catgtcggaa ttcatgagac atattatctg ccacggaggt gttattaccg aagaaatggc 360

cgccagtctt ttggaccagc tgatcgaaga ggtactggct gataatcttc cacctcctag 420

ccattttgaa ccacctaccc ttcacgaact gtatgattta gacgtgacgg cccccgaaga 480

tcccaacgag gaggcggttt cgcagatttt tcccgactct gtaatgttgg cggtgcagga 540

agggattgac ttactcactt ttccgccggc gcccggttct ccggagccgc ctcacctttc 600

ccggcagccc gagcagccgg agcagagagc cttgggtccg gtttctatgc caaaccttgt 660

accggaggtg atcgatctta cctgccacga ggctggcttt ccacccagtg acgacgagga 720

tgaagagggt gaggagtttg tgttagatta tgtggagcac cccgggcacg gttgcaggtc 780

ttgtcattat caccggagga atacggggga cccagatatt atgtgttcgc tttgctatat 840

gaggacctgt ggcatgtttg tctacagtaa gtgaaaatta tgggcagtgg gtgatagagt 900

ggtgggtttg gtgtggtaat ttttttttta atttttacag ttttgtggtt taaagaattt 960

tgtattgtga tttttttaaa aggtcctgtg tctgaacctg agcctgagcc cgagccagaa 1020

ccggagcctg caagacctac ccgccgtcct aaaatggcgc ctgctatcct gagacgcccg 1080

acatcacctg tgtctagaga atgcaatagt agtacggata gctgtgactc cggtccttct 1140

aacacacctc ctgagataca cccggtggtc ccgctgtgcc ccattaaacc agttgccgtg 1200

agagttggtg ggcgtcgcca ggctgtggaa tgtatcgagg acttgcttaa cgagcctggg 1260

caacctttgg acttgagctg taaacgcccc aggccactcg agggtaccgg gtccggagct 1320

acaaattttt ccctcctcaa acaggctgga gatgtcgaag aaaatcctgg gcctaccggt 1380

caccatggat gtggctgcag aatttacttt tcctgggcat tgtggtctac agcctctcag 1440

cacccacccg ctcacccatc actgtcaccc ggccttggaa gcatgtagag gccatcaaag 1500

aagccctgaa cctcctggat gacatgcctg tcacgttgaa tgaagaggta gaagtcgtct 1560

ctaacgagtt ctccttcaag aagctaacat gtgtgcagac ccgcctgaag atattcgagc 1620

agggtctacg gggcaatttc accaaactca agggcgcctt gaacatgaca gccagctact 1680

accagacata ctgcccccca actccggaaa cggactgtga aacacaagtt accacctatg 1740

cggatttcat agacagcctt aaaacctttc tgactgatat cccctttgaa tgcaaaaaac 1800

caggccaaaa atgaagatct gagggccgcg gcagcctgct gacctgcggc gacgtggagg 1860

aaaaccccgg ccccggatcc atgaaaccag taacgttata cgatgtcgca gagtatgccg 1920

gtgtctctta tcagaccgtt tcccgcgtgg tgaaccaggc cagccacgtt tctgcgaaaa 1980

cgcgggaaaa agtggaagcg gcgatggcgg agctgaatta cattcccaac cgcgtggcac 2040

aacaactggc gggcaaacag tcgttgctga ttggcgttgc cacctccagt ctggccctgc 2100

acgcgccgtc gcaaattgtc gcggcgatta aatctcgcgc cgatcaactg ggtgccagcg 2160

tggtggtgtc gatggtagaa cgaagcggcg tcgaagcctg taaagcggcg gtgcacaatc 2220

ttctcgcgca acgcgtcagt gggctgatca ttaactatcc gctggatgac caggatgcca 2280

ttgctgtgga agctgcctgc actaatgttc cggcgttatt tcttgatgtc tctgaccaga 2340

cacccatcaa cagtattatt ttctcccatg aagacggtac gcgactgggc gtggagcatc 2400

tggtcgcatt gggtcaccag caaatcgcgc tgttagcggg cccattaagt tctgtctcgg 2460

cgcgtctgcg tctggctggc tggcataaat atctcactcg caatcaaatt cagccgatag 2520

cggaacggga aggcgactgg agtgccatgt ccggttttca acaaaccatg caaatgctga 2580

atgagggcat cgttcccact gcgatgctgg ttgccaacga tcagatggcg ctgggcgcaa 2640

tgcgcgccat taccgagtcc gggctgcgcg ttggtgcgga tatctcggta gtgggatacg 2700

acgataccga agacagctca tgttatatcc cgccgttaac caccatcaaa caggattttc 2760

gcctgctggg gcaaaccagc gtggaccgct tgctgcaact ctctcagggc caggcggtga 2820

agggcaatca gctgttgccc gtctcactgg tgaaaagaaa aaccaccctg gcgcccaata 2880

cgcaaaccgc ctctccccgc gcgttggccg attcattaat gcagctggca cgacaggttt 2940

cccgactgga aagcgggcag agcagcctga ggcctcctaa gaagaagagg aaggttgcgg 3000

ccgctaacca atgtgcagac tactgttaac caatgtgcag actactgtga attctaacca 3060

atgtgcagac tactgttaac caatgtgcag actactgtaa gcttcgaatt gagtagtgct 3120

ttctacttta tgagtagtgc tttctacttt atgagtagtg ctttctactt tatgagtagt 3180

gctttctact ttatggtcga caatactagt taagaaatga gacc 3224

<210> 6
<211> 3558
<212> DNA
<213> Artificial

<220>
<223> BsaI-5¡ÁUAS-tetO-miniCMV-tetO-E1A-2A- anti-PD-1scFv
 -2A-LacI-miR199a target site-BsaI sequence

<400> 6
ggtctctcta tttaattaag taactataac ggtcgctccg aatttctcga gttaattaag 60

attacgccaa gctacgggcg gagtactgtc ctccgagcgg agtactgtcc tccgagcgga 120

gtactgtcct ccgagcggag tactgtcctc cgagcggagt tctgtcctcc gagcggagac 180

tctagactcc ctatcagtga tagagatagg cgtgtacggt gggaggccta tataagcaga 240

gctcgtttag tgaaccgtca gatcgctccc tatcagtgat agagagaatt cgaccgccac 300

catgtcggaa ttcatgagac atattatctg ccacggaggt gttattaccg aagaaatggc 360

cgccagtctt ttggaccagc tgatcgaaga ggtactggct gataatcttc cacctcctag 420

ccattttgaa ccacctaccc ttcacgaact gtatgattta gacgtgacgg cccccgaaga 480

tcccaacgag gaggcggttt cgcagatttt tcccgactct gtaatgttgg cggtgcagga 540

agggattgac ttactcactt ttccgccggc gcccggttct ccggagccgc ctcacctttc 600

ccggcagccc gagcagccgg agcagagagc cttgggtccg gtttctatgc caaaccttgt 660

accggaggtg atcgatctta cctgccacga ggctggcttt ccacccagtg acgacgagga 720

tgaagagggt gaggagtttg tgttagatta tgtggagcac cccgggcacg gttgcaggtc 780

ttgtcattat caccggagga atacggggga cccagatatt atgtgttcgc tttgctatat 840

gaggacctgt ggcatgtttg tctacagtaa gtgaaaatta tgggcagtgg gtgatagagt 900

ggtgggtttg gtgtggtaat ttttttttta atttttacag ttttgtggtt taaagaattt 960

tgtattgtga tttttttaaa aggtcctgtg tctgaacctg agcctgagcc cgagccagaa 1020

ccggagcctg caagacctac ccgccgtcct aaaatggcgc ctgctatcct gagacgcccg 1080

acatcacctg tgtctagaga atgcaatagt agtacggata gctgtgactc cggtccttct 1140

aacacacctc ctgagataca cccggtggtc ccgctgtgcc ccattaaacc agttgccgtg 1200

agagttggtg ggcgtcgcca ggctgtggaa tgtatcgagg acttgcttaa cgagcctggg 1260

caacctttgg acttgagctg taaacgcccc aggccactcg agggtaccgg gtccggagct 1320

acaaattttt ccctcctcaa acaggctgga gatgtcgaag aaaatcctgg gcctaccggt 1380

cccatgagtg tgcccactca ggtcctgggg ttgctgctgc tgtggcttac agacgctcgc 1440

tgccaggtgc agctggtgga gagcggcggc ggcgtggtgc agcccggcag gagcctgagg 1500

ctggactgca aggccagcgg catcaccttc agcaacagcg gcatgcactg ggtgaggcag 1560

gcccccggca agggcctgga gtgggtggcc gtgatctggt acgacggcag caagaggtac 1620

tacgccgaca gcgtgaaggg caggttcacc atcagcaggg acaacagcaa gaacaccctg 1680

ttcctgcaga tgaacagcct gagggccgag gacaccgccg tgtactactg cgccaccaac 1740

gacgactact ggggccaggg caccctggtg accgtgagca gcggtggagg cggttcaggc 1800

ggaggtggct ctggcggtgg cggatcggag atcgtgctga cccagagccc cgccaccctg 1860

agcctgagcc ccggcgagag ggccaccctg agctgcaggg ccagccagag cgtgagcagc 1920

tacctggcct ggtaccagca gaagcccggc caggccccca ggctgctgat ctacgacgcc 1980

agcaacaggg ccaccggcat ccccgccagg ttcagcggca gcggcagcgg caccgacttc 2040

accctgacca tcagcagcct ggagcccgag gacttcgccg tgtactactg ccagcagagc 2100

agcaactggc ccaggacctt cggccagggc accaaggtgg agatcaagag atctgagggc 2160

cgcggcagcc tgctgacctg cggcgacgtg gaggaaaacc ccggccccgg atccatgaaa 2220

ccagtaacgt tatacgatgt cgcagagtat gccggtgtct cttatcagac cgtttcccgc 2280

gtggtgaacc aggccagcca cgtttctgcg aaaacgcggg aaaaagtgga agcggcgatg 2340

gcggagctga attacattcc caaccgcgtg gcacaacaac tggcgggcaa acagtcgttg 2400

ctgattggcg ttgccacctc cagtctggcc ctgcacgcgc cgtcgcaaat tgtcgcggcg 2460

attaaatctc gcgccgatca actgggtgcc agcgtggtgg tgtcgatggt agaacgaagc 2520

ggcgtcgaag cctgtaaagc ggcggtgcac aatcttctcg cgcaacgcgt cagtgggctg 2580

atcattaact atccgctgga tgaccaggat gccattgctg tggaagctgc ctgcactaat 2640

gttccggcgt tatttcttga tgtctctgac cagacaccca tcaacagtat tattttctcc 2700

catgaagacg gtacgcgact gggcgtggag catctggtcg cattgggtca ccagcaaatc 2760

gcgctgttag cgggcccatt aagttctgtc tcggcgcgtc tgcgtctggc tggctggcat 2820

aaatatctca ctcgcaatca aattcagccg atagcggaac gggaaggcga ctggagtgcc 2880

atgtccggtt ttcaacaaac catgcaaatg ctgaatgagg gcatcgttcc cactgcgatg 2940

ctggttgcca acgatcagat ggcgctgggc gcaatgcgcg ccattaccga gtccgggctg 3000

cgcgttggtg cggatatctc ggtagtggga tacgacgata ccgaagacag ctcatgttat 3060

atcccgccgt taaccaccat caaacaggat tttcgcctgc tggggcaaac cagcgtggac 3120

cgcttgctgc aactctctca gggccaggcg gtgaagggca atcagctgtt gcccgtctca 3180

ctggtgaaaa gaaaaaccac cctggcgccc aatacgcaaa ccgcctctcc ccgcgcgttg 3240

gccgattcat taatgcagct ggcacgacag gtttcccgac tggaaagcgg gcagagcagc 3300

ctgaggcctc ctaagaagaa gaggaaggtt gcggccgcta accaatgtgc agactactgt 3360

taaccaatgt gcagactact gtgaattcta accaatgtgc agactactgt taaccaatgt 3420

gcagactact gtaagcttcg aattgagtag tgctttctac tttatgagta gtgctttcta 3480

ctttatgagt agtgctttct actttatgag tagtgctttc tactttatgg tcgacaatac 3540

tagttaagaa atgagacc 3558

<210> 7
<211> 3573
<212> DNA
<213> Artificial

<220>
<223> BsaI-5¡ÁUAS-tetO-miniCMV-tetO-E1A-2A- anti-PD-L1scFv
 -2A-LacI-miR199a target site-BsaI sequence

<400> 7
ggtctctcta tttaattaag taactataac ggtcgctccg aatttctcga gttaattaag 60

attacgccaa gctacgggcg gagtactgtc ctccgagcgg agtactgtcc tccgagcgga 120

gtactgtcct ccgagcggag tactgtcctc cgagcggagt tctgtcctcc gagcggagac 180

tctagactcc ctatcagtga tagagatagg cgtgtacggt gggaggccta tataagcaga 240

gctcgtttag tgaaccgtca gatcgctccc tatcagtgat agagagaatt cgaccgccac 300

catgtcggaa ttcatgagac atattatctg ccacggaggt gttattaccg aagaaatggc 360

cgccagtctt ttggaccagc tgatcgaaga ggtactggct gataatcttc cacctcctag 420

ccattttgaa ccacctaccc ttcacgaact gtatgattta gacgtgacgg cccccgaaga 480

tcccaacgag gaggcggttt cgcagatttt tcccgactct gtaatgttgg cggtgcagga 540

agggattgac ttactcactt ttccgccggc gcccggttct ccggagccgc ctcacctttc 600

ccggcagccc gagcagccgg agcagagagc cttgggtccg gtttctatgc caaaccttgt 660

accggaggtg atcgatctta cctgccacga ggctggcttt ccacccagtg acgacgagga 720

tgaagagggt gaggagtttg tgttagatta tgtggagcac cccgggcacg gttgcaggtc 780

ttgtcattat caccggagga atacggggga cccagatatt atgtgttcgc tttgctatat 840

gaggacctgt ggcatgtttg tctacagtaa gtgaaaatta tgggcagtgg gtgatagagt 900

ggtgggtttg gtgtggtaat ttttttttta atttttacag ttttgtggtt taaagaattt 960

tgtattgtga tttttttaaa aggtcctgtg tctgaacctg agcctgagcc cgagccagaa 1020

ccggagcctg caagacctac ccgccgtcct aaaatggcgc ctgctatcct gagacgcccg 1080

acatcacctg tgtctagaga atgcaatagt agtacggata gctgtgactc cggtccttct 1140

aacacacctc ctgagataca cccggtggtc ccgctgtgcc ccattaaacc agttgccgtg 1200

agagttggtg ggcgtcgcca ggctgtggaa tgtatcgagg acttgcttaa cgagcctggg 1260

caacctttgg acttgagctg taaacgcccc aggccactcg agggtaccgg gtccggagct 1320

acaaattttt ccctcctcaa acaggctgga gatgtcgaag aaaatcctgg gcctaccggt 1380

cccatgagtg tgcccactca ggtcctgggg ttgctgctgc tgtggcttac agacgctcgc 1440

tgcgaggtgc agctggtgga gagcggcggc ggcctggtgc agcccggcgg cagcctgagg 1500

ctgagctgcg ccgccagcgg cttcaccttc agcgacagct ggatccactg ggtgaggcag 1560

gcccccggca agggcctgga gtgggtggcc tggatcagcc cctacggcgg cagcacctac 1620

tacgccgaca gcgtgaaggg caggttcacc atcagcgccg acaccagcaa gaacaccgcc 1680

tacctgcaga tgaacagcct gagggccgag gacaccgccg tgtactactg cgccaggagg 1740

cactggcccg gcggcttcga ctactggggc cagggcaccc tggtgaccgt gagcagcggt 1800

ggaggcggtt caggcggagg tggctctggc ggtggcggat cggacatcca gatgacccag 1860

agccccagca gcctgagcgc cagcgtgggc gacagggtga ccatcacctg cagggccagc 1920

caggacgtga gcaccgccgt ggcctggtac cagcagaagc ccggcaaggc ccccaagctg 1980

ctgatctaca gcgccagctt cctgtacagc ggcgtgccca gcaggttcag cggcagcggc 2040

agcggcaccg acttcaccct gaccatcagc agcctgcagc ccgaggactt cgccacctac 2100

tactgccagc agtacctgta ccaccccgcc accttcggcc agggcaccaa ggtggagatc 2160

aagagatctg agggccgcgg cagcctgctg acctgcggcg acgtggagga aaaccccggc 2220

cccggatcca tgaaaccagt aacgttatac gatgtcgcag agtatgccgg tgtctcttat 2280

cagaccgttt cccgcgtggt gaaccaggcc agccacgttt ctgcgaaaac gcgggaaaaa 2340

gtggaagcgg cgatggcgga gctgaattac attcccaacc gcgtggcaca acaactggcg 2400

ggcaaacagt cgttgctgat tggcgttgcc acctccagtc tggccctgca cgcgccgtcg 2460

caaattgtcg cggcgattaa atctcgcgcc gatcaactgg gtgccagcgt ggtggtgtcg 2520

atggtagaac gaagcggcgt cgaagcctgt aaagcggcgg tgcacaatct tctcgcgcaa 2580

cgcgtcagtg ggctgatcat taactatccg ctggatgacc aggatgccat tgctgtggaa 2640

gctgcctgca ctaatgttcc ggcgttattt cttgatgtct ctgaccagac acccatcaac 2700

agtattattt tctcccatga agacggtacg cgactgggcg tggagcatct ggtcgcattg 2760

ggtcaccagc aaatcgcgct gttagcgggc ccattaagtt ctgtctcggc gcgtctgcgt 2820

ctggctggct ggcataaata tctcactcgc aatcaaattc agccgatagc ggaacgggaa 2880

ggcgactgga gtgccatgtc cggttttcaa caaaccatgc aaatgctgaa tgagggcatc 2940

gttcccactg cgatgctggt tgccaacgat cagatggcgc tgggcgcaat gcgcgccatt 3000

accgagtccg ggctgcgcgt tggtgcggat atctcggtag tgggatacga cgataccgaa 3060

gacagctcat gttatatccc gccgttaacc accatcaaac aggattttcg cctgctgggg 3120

caaaccagcg tggaccgctt gctgcaactc tctcagggcc aggcggtgaa gggcaatcag 3180

ctgttgcccg tctcactggt gaaaagaaaa accaccctgg cgcccaatac gcaaaccgcc 3240

tctccccgcg cgttggccga ttcattaatg cagctggcac gacaggtttc ccgactggaa 3300

agcgggcaga gcagcctgag gcctcctaag aagaagagga aggttgcggc cgctaaccaa 3360

tgtgcagact actgttaacc aatgtgcaga ctactgtgaa ttctaaccaa tgtgcagact 3420

actgttaacc aatgtgcaga ctactgtaag cttcgaattg agtagtgctt tctactttat 3480

gagtagtgct ttctacttta tgagtagtgc tttctacttt atgagtagtg ctttctactt 3540

tatggtcgac aatactagtt aagaaatgag acc 3573

<210> 8
<211> 1594
<212> DNA
<213> Artificial

<220>
<223> A nucleotide sequence of a nucleic acid carried by the third
expression vector

<400> 8
ggtctccgaa atcgccaagc tacgggcgga gtactgtcct ccgagcggag tactgtcctc 60

cgagcggagt actgtcctcc gagcggagta ctgtcctccg agcggagtac tgtcctccga 120

gcggagactc tagaaattgt gagcggataa caatttaggc gtgtacggtg ggaggcctat 180

ataagcagag ctcgtttagt gaaccgtcag atcgccagct gacaaaattg tgagcgctca 240

caattactag aagatcttga attcgccacc atggctagat tagataaaag taaagtgatt 300

aacagcgcat tagagctgct taatgaggtc ggaatcgaag gtttaacaac ccgtaaactc 360

gcccagaagc taggtgtaga gcagcctaca ttgtattggc atgtaaaaaa taagcgggct 420

ttgctcgacg ccttagccat tgagatgtta gataggcacc atactcactt ttgcccttta 480

gaaggggaaa gctggcaaga ttttttacgt aataacgcta aaagttttag atgtgcttta 540

ctaagtcatc gcgatggagc aaaagtacat ttaggtacac ggcctacaga aaaacagtat 600

gaaactctcg aaaatcaatt agccttttta tgccaacaag gtttttcact agagaatgca 660

ttatatgcac tcagcgctgt ggggcatttt actttaggtt gcgtattgga agatcaagag 720

catcaagtcg ctaaagaaga aagggaaaca cctactactg atagtatgcc gccattatta 780

cgacaagcta tcgaattatt tgatcaccaa ggtgcagagc cagccttctt attcggcctt 840

gaattgatca tatgcggatt agaaaaacaa cttaaatgtg aaagtgggtc gccaaaaaag 900

aagagaaagg tcgacggcgg tggtgctttg tctcctcagc actctgctgt cactcaagga 960

agtatcatca agaacaagga gggcatggat gctaagtcac taactgcctg gtcccggaca 1020

ctggtgacct tcaaggatgt atttgtggac ttcaccaggg aggagtggaa gctgctggac 1080

actgctcagc agatcgtgta cagaaatgtg atgctggaga actataagaa cctggtttcc 1140

ttgggttatc agcttactaa gccagatgtg atcctccggt tggagaaggg agaagagccc 1200

tggctggtgg agagagaaat tcaccaagag acccatcctg attcagagac tgcatttgaa 1260

atcaaatcat cagtttaata caaggcggcc gcaaatcaac atcagtctga taagctatca 1320

acatcagtct gataagctat caacatcagt ctgataagct atcaacatca gtctgataag 1380

ctaaagcttc gaattctgat aatcagccat accacatttg tagaggtttt acttgcttta 1440

aaaaacctcc cacacctccc cctgaacctg aaacataaaa tgaatgcaat tgttgttgtt 1500

aacttgttta ttgcagctta taatggttac aaataaagca atagcatcac aaatttcaca 1560

aataaagcat ttttttcact gcagctctga gacc 1594

<210> 9
<211> 7155
<212> DNA
<213> Artificial

<220>
<223> A nucleotide sequence of an EBFP-bearing nucleic acid carried by an adenoviral vector

<400> 9
gtaactataa cggtcgctcc gaatttctcg agttaattaa gattacgcca agctacgggc 60

ggagtactgt cctccgagcg gagtactgtc ctccgagcgg agtactgtcc tccgagcgga 120

gtactgtcct ccgagcggag ttctgtcctc cgagcggaga ctctagactc cctatcagtg 180

atagagatag gcgtgtacgg tgggaggcct atataagcag agctcgttta gtgaaccgtc 240

agatcgctcc ctatcagtga tagagagaat tcgaccgcca ccatgtcgga attcatgaga 300

catattatct gccacggagg tgttattacc gaagaaatgg ccgccagtct tttggaccag 360

ctgatcgaag aggtactggc tgataatctt ccacctccta gccattttga accacctacc 420

cttcacgaac tgtatgattt agacgtgacg gcccccgaag atcccaacga ggaggcggtt 480

tcgcagattt ttcccgactc tgtaatgttg gcggtgcagg aagggattga cttactcact 540

tttccgccgg cgcccggttc tccggagccg cctcaccttt cccggcagcc cgagcagccg 600

gagcagagag ccttgggtcc ggtttctatg ccaaaccttg taccggaggt gatcgatctt 660

acctgccacg aggctggctt tccacccagt gacgacgagg atgaagaggg tgaggagttt 720

gtgttagatt atgtggagca ccccgggcac ggttgcaggt cttgtcatta tcaccggagg 780
aatacggggg acccagatat tatgtgttcg ctttgctata tgaggacctg tggcatgttt 840
gtctacagta agtgaaaatt atgggcagtg ggtgatagag tggtgggttt ggtgtggtaa 900
tttttttttt aatttttaca gttttgtggt ttaaagaatt ttgtattgtg atttttttaa 960
aaggtcctgt gtctgaacct gagcctgagc ccgagccaga accggagcct gcaagaccta 1020
cccgccgtcc taaaatggcg cctgctatcc tgagacgccc gacatcacct gtgtctagag 1080
aatgcaatag tagtacggat agctgtgact ccggtccttc taacacacct cctgagatac 1140
acccggtggt cccgctgtgc cccattaaac cagttgccgt gagagttggt gggcgtcgcc 1200
aggctgtgga atgtatcgag gacttgctta acgagcctgg gcaacctttg gacttgagct 1260
gtaaacgccc caggccactc gagggtaccg ggtccggagc tacaaatttt tccctcctca 1320
aacaggctgg agatgtcgaa gaaaatcctg ggcctaccgg tcccatggtg agcaagggcg 1380
aggagctgtt caccggggtg gtgcccatcc tggtcgagct ggacggcgac gtaaacggcc 1440
acaagttcag cgtgaggggc gagggcgagg gcgatgccac caacggcaag ctgaccctga 1500
agttcatctg caccaccggc aagctgcccg tgccctggcc caccctcgtg accaccctga 1560
gccacggcgt gcagtgcttc gcccgctacc ccgaccacat gaagcagcac gacttcttca 1620
agtccgccat gcccgaaggc tacgtccagg agcgcaccat cttcttcaag gacgacggca 1680
cctacaagac ccgcgccgag gtgaagttcg agggcgacac cctagtgaac cgcatcgagc 1740
tgaagggcgt cgacttcaag gaggacggca acatcctggg gcacaagctg gagtacaact 1800
tcaacagcca caacatctat atcatggccg tcaagcagaa gaacggcatc aaggtgaact 1860
tcaagatccg ccacaacgtg gaggacggca gcgtgcagct cgccgaccac taccagcaga 1920
acacccccat cggcgacggc cccgtgctgc tgcccgacag ccactacctg agcacccagt 1980
ccgtgctgag caaagacccc aacgagaagc gcgatcacat ggtcctgctg gagttccgca 2040
ccgccgccgg gatcactctc ggcatggacg agctgtacaa gagatctgag ggccgcggca 2100
gcctgctgac ctgcggcgac gtggaggaaa accccggccc cggatccatg aaaccagtaa 2160
cgttatacga tgtcgcagag tatgccggtg tctcttatca gaccgtttcc cgcgtggtga 2220
accaggccag ccacgtttct gcgaaaacgc gggaaaaagt ggaagcggcg atggcggagc 2280
tgaattacat tcccaaccgc gtggcacaac aactggcggg caaacagtcg ttgctgattg 2340
gcgttgccac ctccagtctg gccctgcacg cgccgtcgca aattgtcgcg gcgattaaat 2400
ctcgcgccga tcaactgggt gccagcgtgg tggtgtcgat ggtagaacga agcggcgtcg 2460
aagcctgtaa agcggcggtg cacaatcttc tcgcgcaacg cgtcagtggg ctgatcatta 2520
actatccgct ggatgaccag gatgccattg ctgtggaagc tgcctgcact aatgttccgg 2580
cgttatttct tgatgtctct gaccagacac ccatcaacag tattattttc tcccatgaag 2640

acggtacgcg actgggcgtg gagcatctgg tcgcattggg tcaccagcaa atcgcgctgt 2700

tagcgggccc attaagttct gtctcggcgc gtctgcgtct ggctggctgg cataaatatc 2760

tcactcgcaa tcaaattcag ccgatagcgg aacgggaagg cgactggagt gccatgtccg 2820

gttttcaaca aaccatgcaa atgctgaatg agggcatcgt tcccactgcg atgctggttg 2880

ccaacgatca gatggcgctg ggcgcaatgc gcgccattac cgagtccggg ctgcgcgttg 2940

gtgcggatat ctcggtagtg ggatacgacg ataccgaaga cagctcatgt tatatcccgc 3000

cgttaaccac catcaaacag gattttcgcc tgctggggca aaccagcgtg gaccgcttgc 3060

tgcaactctc tcagggccag gcggtgaagg gcaatcagct gttgcccgtc tcactggtga 3120

aaagaaaaac caccctggcg cccaatacgc aaaccgcctc tccccgcgcg ttggccgatt 3180

cattaatgca gctggcacga caggtttccc gactggaaag cgggcagagc agcctgaggc 3240

ctcctaagaa gaagaggaag gttgcggccg ctaaccaatg tgcagactac tgttaaccaa 3300

tgtgcagact actgtgaatt ctaaccaatg tgcagactac tgttaaccaa tgtgcagact 3360

actgtaagct tcgaattgag tagtgctttc tactttatga gtagtgcttt ctactttatg 3420

agtagtgctt tctactttat gagtagtgct ttctacttta tggtcgacaa tactagttaa 3480

gaaatcgcca agctacgggc ggagtactgt cctccgagcg gagtactgtc ctccgagcgg 3540

agtactgtcc tccgagcgga gtactgtcct ccgagcggag tactgtcctc cgagcggaga 3600

ctctagaaat tgtgagcgga taacaattta ggcgtgtacg gtgggaggcc tatataagca 3660

gagctcgttt agtgaaccgt cagatcgcca gctgacaaaa ttgtgagcgc tcacaattac 3720

tagaagatct tgaattcgcc accatggcta gattagataa aagtaaagtg attaacagcg 3780

cattagagct gcttaatgag gtcggaatcg aaggtttaac aacccgtaaa ctcgcccaga 3840

agctaggtgt agagcagcct acattgtatt ggcatgtaaa aaataagcgg gctttgctcg 3900

acgccttagc cattgagatg ttagataggc accatactca cttttgccct ttagaagggg 3960

aaagctggca agatttttta cgtaataacg ctaaaagttt tagatgtgct ttactaagtc 4020

atcgcgatgg agcaaaagta catttaggta cacggcctac agaaaaacag tatgaaactc 4080

tcgaaaatca attagccttt ttatgccaac aaggtttttc actagagaat gcattatatg 4140

cactcagcgc tgtggggcat tttactttag gttgcgtatt ggaagatcaa gagcatcaag 4200

tcgctaaaga agaaagggaa acacctacta ctgatagtat gccgccatta ttacgacaag 4260

ctatcgaatt atttgatcac caaggtgcag agccagcctt cttattcggc cttgaattga 4320

tcatatgcgg attagaaaaa caacttaaat gtgaaagtgg gtcgccaaaa aagaagagaa 4380

aggtcgacgg cggtggtgct ttgtctcctc agcactctgc tgtcactcaa ggaagtatca 4440

tcaagaacaa ggagggcatg gatgctaagt cactaactgc ctggtcccgg acactggtga 4500

ccttcaagga tgtatttgtg gacttcacca gggaggagtg gaagctgctg gacactgctc 4560
agcagatcgt gtacagaaat gtgatgctgg agaactataa gaacctggtt tccttgggtt 4620
atcagcttac taagccagat gtgatcctcc ggttggagaa gggagaagag ccctggctgg 4680
tggagagaga aattcaccaa gagacccatc ctgattcaga gactgcattt gaaatcaaat 4740
catcagttta atacaaggcg gccgcaaatc aacatcagtc tgataagcta tcaacatcag 4800
tctgataagc tatcaacatc agtctgataa gctatcaaca tcagtctgat aagctaaagc 4860
ttcgaattct gataatcagc cataccacat ttgtagaggt tttacttgct ttaaaaaacc 4920
tcccacacct ccccctgaac ctgaaacata aaatgaatgc aattgttgtt gttaacttgt 4980
ttattgcagc ttataatggt tacaaataaa gcaatagcat cacaaatttc acaaataaag 5040
catttttttc actgcagctc cagattgaat tatttgcctg tcatacagct aataattgac 5100
cataagacaa ttagatttaa attagttttg aatctttcta ataccaaagt tcagtttact 5160
gttccatgtt gcttctgagt ggcttcacag acttatgaaa aagtaaacgg aatcagaatt 5220
acatcaatgc aaaagcattg ctgtgaactc tgtacttagg actaaacttt gagcaataac 5280
acatatagat tgaggattgt ttgctgttag tatacaaact ctggttcaaa gctcctcttt 5340
attgcttgtc ttggaaaatt tgctgttctt catggtttct cttttcactg ctatctattt 5400
ttctcaacca ctcacatggc tacaaaagct tcctgattaa taattacact aagtcaatag 5460
gcatagagcc aggactgttt gggtaaactg gtcactttat cttaaactaa atatatccaa 5520
aactgaacat gtacttagtt actaagtctt tgactttatc tcattcatac cactcagctt 5580
tatccaggcc acttatttga cagtattatt gcgaaaactt cctatctaga agtttgagga 5640
gaatatttgt tatatttgca aaataaaata agtttgcaag tttttttttt ctgccccaaa 5700
gagctctgtg tccttgaaca taaaatacaa ataaccgcta tgctgttaat tattgacaaa 5760
tgtcccattt tcaacctaag gaaataccat aaagtaacag atataccaac aaaaggttac 5820
tagttaacag gcattgcctg aaaagagtat aaaagaattt cagcatgatt ttccatattg 5880
tgcttccacc actgccaata acaaaataac tagcaaggat ccggccacca tggccccccc 5940
gaccgatgtc agcctggggg acgagctcca cttagacggc gaggacgtgg cgatggcgca 6000
tgccgacgcg ctagacgatt tcgatctgga catgttgggg gacggggatt ccccgggtcc 6060
gggatttacc ccccacgact ccgcccccta cggcgctctg gatatggccg acttcgagtt 6120
tgagcagatg tttaccgatg cccttggaat tgacgagtac ggtgggacgc gtatgaagct 6180
actgtcttct atcgaacaag catgcgatat ttgccgactt aaaaagctca agtgctccaa 6240
agaaaaaccg aagtgcgcca agtgtctgaa gaacaactgg gagtgtcgct actctcccaa 6300
aaccaaaagg tctccgctga ctagggcaca tctgacagaa gtggaatcaa ggctagaaag 6360
actggaacag ctatttctac tgatttttcc tcgagaagac cttgacatga ttttgaaaat 6420

ggattcttta caggatataa aagcattgtt aacaggatta tttgtacaag ataatgtgaa 6480

taaagatgcc gtcacagata gattggcttc agtggagact gatatgcctc taacattgag 6540

acagcataga ataagtgcga catcatcatc ggaagagagt agtaacaaag gtcaaagaca 6600

gttgactgta taattcactc ctcaggtgca ggctgcctat cagaaggtgg tggctggtgt 6660

ggccaatgcc ctggctcaca aataccactg agatcttttt ccctctgcca aaaattatgg 6720

ggacatcatg aagccccttg agcatctgac ttctggctaa taaaggaaat ttattttcat 6780

tgcaatagtg tgttggaatt ttttgtgtct ctcactcgga aggacatatg ggagggcaaa 6840

tcatttaaaa catcagaatg agtatttggt ttagagtttg gcaacatatg cccatatgct 6900

ggctgccatg aacaaaggtt ggctataaag aggtcatcag tatatgaaac agccccctgc 6960

tgtccattcc ttattccata gaaaagcctt gacttgaggt tagatttttt ttatattttg 7020

ttttgtgtta tttttttctt taacatccct aaaattttcc ttacatgttt tactagccag 7080

atttttcctc ctctcctgac tactcccagt catagctgtc cctcttctct tatggagatc 7140

ggagaaagag gtaat 7155

<210> 10
<211> 6900
<212> DNA
<213> Artificial

<220>
<223> A nucleotide sequence of an hIL-2-bearing nucleic acid carried by an adenoviral vector

<400> 10
gtaactataa cggtcgctcc gaatttctcg agttaattaa gattacgcca agctacgggc 60

ggagtactgt cctccgagcg gagtactgtc ctccgagcgg agtactgtcc tccgagcgga 120

gtactgtcct ccgagcggag ttctgtcctc cgagcggaga ctctagactc cctatcagtg 180

atagagatag gcgtgtacgg tgggaggcct atataagcag agctcgttta gtgaaccgtc 240

agatcgctcc ctatcagtga tagagagaat tcgaccgcca ccatgtcgga attcatgaga 300

catattatct gccacggagg tgttattacc gaagaaatgg ccgccagtct tttggaccag 360

ctgatcgaag aggtactggc tgataatctt ccacctccta gccattttga accacctacc 420

cttcacgaac tgtatgattt agacgtgacg gcccccgaag atcccaacga ggaggcggtt 480

tcgcagattt ttcccgactc tgtaatgttg gcggtgcagg aagggattga cttactcact 540

tttccgccgg cgcccggttc tccggagccg cctcaccttt cccggcagcc cgagcagccg 600

gagcagagag ccttgggtcc ggtttctatg ccaaaccttg taccggaggt gatcgatctt 660

acctgccacg aggctggctt tccacccagt gacgacgagg atgaagaggg tgaggagttt 720

gtgttagatt atgtggagca ccccgggcac ggttgcaggt cttgtcatta tcaccggagg 780

aatacggggg acccagatat tatgtgttcg ctttgctata tgaggacctg tggcatgttt 840

gtctacagta agtgaaaatt atgggcagtg ggtgatagag tggtgggttt ggtgtggtaa 900

tttttttttt aatttttaca gttttgtggt ttaaagaatt ttgtattgtg atttttttaa 960

aaggtcctgt gtctgaacct gagcctgagc ccgagccaga accggagcct gcaagaccta 1020

cccgccgtcc taaaatggcg cctgctatcc tgagacgccc gacatcacct gtgtctagag 1080

aatgcaatag tagtacggat agctgtgact ccggtccttc taacacacct cctgagatac 1140

acccggtggt cccgctgtgc cccattaaac cagttgccgt gagagttggt gggcgtcgcc 1200

aggctgtgga atgtatcgag gacttgctta acgagcctgg gcaacctttg gacttgagct 1260

gtaaacgccc caggccactc gagggtaccg ggtccggagc tacaaatttt tccctcctca 1320

aacaggctgg agatgtcgaa gaaaatcctg ggcctaccgg tcccatgtac aggatgcaac 1380

tcctgtcttg cattgcacta agtcttgcac ttgtcacaaa cagtgcacct acttcaagtt 1440

ctacaaagaa aacacagcta caactggagc atttactgct ggatttacag atgattttga 1500

atggaattaa taattacaag aatcccaaac tcaccgcgat gctcacagct aagtttgcca 1560

tgcccaagaa ggccacagaa ctgaaacatc ttcagtgtct agaagaagca ctcaaacctc 1620

tggaggaagt gctaaattta gctcaaagca aaaactttca cttaagaccc agggacttaa 1680

tcagcaatat caacgtaata gttctggaac taaagggatc tgaaacaaca ttcatgtgtg 1740

aatatgctga tgagacagca accattgtag aatttctgaa cagatggatt accttttgtc 1800

aaagcatcat ctcaacactg acttgaagat ctgagggccg cggcagcctg ctgacctgcg 1860

gcgacgtgga ggaaaacccc ggccccggat ccatgaaacc agtaacgtta tacgatgtcg 1920

cagagtatgc cggtgtctct tatcagaccg tttcccgcgt ggtgaaccag gccagccacg 1980

tttctgcgaa aacgcgggaa aaagtggaag cggcgatggc ggagctgaat tacattccca 2040

accgcgtggc acaacaactg gcgggcaaac agtcgttgct gattggcgtt gccacctcca 2100

gtctggccct gcacgcgccg tcgcaaattg tcgcggcgat taaatctcgc gccgatcaac 2160

tgggtgccag cgtggtggtg tcgatggtag aacgaagcgg cgtcgaagcc tgtaaagcgg 2220

cggtgcacaa tcttctcgcg caacgcgtca gtgggctgat cattaactat ccgctggatg 2280

accaggatgc cattgctgtg gaagctgcct gcactaatgt tccggcgtta tttcttgatg 2340

tctctgacca gacacccatc aacagtatta ttttctccca tgaagacggt acgcgactgg 2400

gcgtggagca tctggtcgca ttgggtcacc agcaaatcgc gctgttagcg ggcccattaa 2460

gttctgtctc ggcgcgtctg cgtctggctg gctggcataa atatctcact cgcaatcaaa 2520

ttcagccgat agcggaacgg gaaggcgact ggagtgccat gtccggtttt caacaaacca 2580

tgcaaatgct gaatgagggc atcgttccca ctgcgatgct ggttgccaac gatcagatgg 2640

cgctgggcgc aatgcgcgcc attaccgagt ccgggctgcg cgttggtgcg gatatctcgg 2700
tagtgggata cgacgatacc gaagacagct catgttatat cccgccgtta accaccatca 2760
aacaggattt tcgcctgctg gggcaaacca gcgtggaccg cttgctgcaa ctctctcagg 2820
gccaggcggt gaagggcaat cagctgttgc ccgtctcact ggtgaaaaga aaaaccaccc 2880
tggcgcccaa tacgcaaacc gcctctcccc gcgcgttggc cgattcatta atgcagctgg 2940
cacgacaggt ttcccgactg gaaagcgggc agagcagcct gaggcctcct aagaagaaga 3000
ggaaggttgc ggccgctaac caatgtgcag actactgtta accaatgtgc agactactgt 3060
gaattctaac caatgtgcag actactgtta accaatgtgc agactactgt aagcttcgaa 3120
ttgagtagtg ctttctactt tatgagtagt gctttctact ttatgagtag tgctttctac 3180
tttatgagta gtgctttcta ctttatggtc gacaatacta gttaagaaat cgccaagcta 3240
cgggcggagt actgtcctcc gagcggagta ctgtcctccg agcggagtac tgtcctccga 3300
gcggagtact gtcctccgag cggagtactg tcctccgagc ggagactcta gaaattgtga 3360
gcggataaca atttaggcgt gtacggtggg aggcctatat aagcagagct cgtttagtga 3420
accgtcagat cgccagctga caaaattgtg agcgctcaca attactagaa gatcttgaat 3480
tcgccaccat ggctagatta gataaaagta aagtgattaa cagcgcatta gagctgctta 3540
atgaggtcgg aatcgaaggt ttaacaaccc gtaaactcgc ccagaagcta ggtgtagagc 3600
agcctacatt gtattggcat gtaaaaaata agcgggcttt gctcgacgcc ttagccattg 3660
agatgttaga taggcaccat actcactttt gccctttaga aggggaaagc tggcaagatt 3720
ttttacgtaa taacgctaaa agttttagat gtgctttact aagtcatcgc gatggagcaa 3780
aagtacattt aggtacacgg cctacagaaa aacagtatga aactctcgaa aatcaattag 3840
cctttttatg ccaacaaggt ttttcactag agaatgcatt atatgcactc agcgctgtgg 3900
ggcattttac tttaggttgc gtattggaag atcaagagca tcaagtcgct aaagaagaaa 3960
gggaaacacc tactactgat agtatgccgc cattattacg acaagctatc gaattatttg 4020
atcaccaagg tgcagagcca gccttcttat tcggccttga attgatcata tgcggattag 4080
aaaaacaact taaatgtgaa agtgggtcgc caaaaaagaa gagaaaggtc gacggcggtg 4140
gtgctttgtc tcctcagcac tctgctgtca ctcaaggaag tatcatcaag aacaaggagg 4200
gcatggatgc taagtcacta actgcctggt cccggacact ggtgaccttc aaggatgtat 4260
ttgtggactt caccagggag gagtggaagc tgctggacac tgctcagcag atcgtgtaca 4320
gaaatgtgat gctggagaac tataagaacc tggtttcctt gggttatcag cttactaagc 4380
cagatgtgat cctccggttg gagaagggag aagagccctg gctggtggag agagaaattc 4440
accaagagac ccatcctgat tcagagactg catttgaaat caaatcatca gtttaataca 4500
aggcggccgc aaatcaacat cagtctgata agctatcaac atcagtctga taagctatca 4560

acatcagtct gataagctat caacatcagt ctgataagct aaagcttcga attctgataa 4620

tcagccatac cacatttgta gaggttttac ttgctttaaa aaacctccca cacctccccc 4680

tgaacctgaa acataaaatg aatgcaattg ttgttgttaa cttgtttatt gcagcttata 4740

atggttacaa ataaagcaat agcatcacaa atttcacaaa taaagcattt ttttcactgc 4800

agctccagat tgaattattt gcctgtcata cagctaataa ttgaccataa gacaattaga 4860

tttaaattag ttttgaatct ttctaatacc aaagttcagt ttactgttcc atgttgcttc 4920

tgagtggctt cacagactta tgaaaaagta aacggaatca gaattacatc aatgcaaaag 4980

cattgctgtg aactctgtac ttaggactaa actttgagca ataacacata tagattgagg 5040

attgtttgct gttagtatac aaactctggt tcaaagctcc tctttattgc ttgtcttgga 5100

aaatttgctg ttcttcatgg tttctctttt cactgctatc tatttttctc aaccactcac 5160

atggctacaa aagcttcctg attaataatt acactaagtc aataggcata gagccaggac 5220

tgtttgggta aactggtcac tttatcttaa actaaatata tccaaaactg aacatgtact 5280

tagttactaa gtctttgact ttatctcatt cataccactc agctttatcc aggccactta 5340

tttgacagta ttattgcgaa aacttcctat ctagaagttt gaggagaata tttgttatat 5400

ttgcaaaata aaataagttt gcaagttttt tttttctgcc ccaaagagct ctgtgtcctt 5460

gaacataaaa tacaaataac cgctatgctg ttaattattg acaaatgtcc cattttcaac 5520

ctaaggaaat accataaagt aacagatata ccaacaaaag gttactagtt aacaggcatt 5580

gcctgaaaag agtataaaag aatttcagca tgattttcca tattgtgctt ccaccactgc 5640

caataacaaa ataactagca aggatccggc caccatggcc cccccgaccg atgtcagcct 5700

gggggacgag ctccacttag acggcgagga cgtggcgatg gcgcatgccg acgcgctaga 5760

cgatttcgat ctggacatgt tgggggacgg ggattccccg ggtccgggat ttacccccca 5820

cgactccgcc ccctacggcg ctctggatat ggccgacttc gagtttgagc agatgtttac 5880

cgatgccctt ggaattgacg agtacggtgg gacgcgtatg aagctactgt cttctatcga 5940

acaagcatgc gatatttgcc gacttaaaaa gctcaagtgc tccaaagaaa aaccgaagtg 6000

cgccaagtgt ctgaagaaca actgggagtg tcgctactct cccaaaacca aaaggtctcc 6060

gctgactagg gcacatctga cagaagtgga atcaaggcta gaaagactgg aacagctatt 6120

tctactgatt tttcctcgag aagaccttga catgattttg aaaatggatt ctttacagga 6180

tataaaagca ttgttaacag gattatttgt acaagataat gtgaataaag atgccgtcac 6240

agatagattg gcttcagtgg agactgatat gcctctaaca ttgagacagc atagaataag 6300

tgcgacatca tcatcggaag agagtagtaa caaaggtcaa agacagttga ctgtataatt 6360

cactcctcag gtgcaggctg cctatcagaa ggtggtggct ggtgtggcca atgccctggc 6420

tcacaaatac cactgagatc tttttccctc tgccaaaaat tatggggaca tcatgaagcc 6480

ccttgagcat ctgacttctg gctaataaag gaaatttatt ttcattgcaa tagtgtgttg 6540

gaattttttg tgtctctcac tcggaaggac atatgggagg gcaaatcatt taaaacatca 6600

gaatgagtat ttggtttaga gtttggcaac atatgcccat atgctggctg ccatgaacaa 6660

aggttggcta taaagaggtc atcagtatat gaaacagccc cctgctgtcc attccttatt 6720

ccatagaaaa gccttgactt gaggttagat tttttttata ttttgttttg tgttattttt 6780

ttctttaaca tccctaaaat tttccttaca tgttttacta gccagatttt tcctcctctc 6840

ctgactactc ccagtcatag ctgtccctct tctcttatgg agatcggaga aagaggtaat 6900

<210> 11
<211> 6873
<212> DNA
<213> Artificial

<220>
<223> A nucleotide sequence of an hGMCSF-bearing nucleic acid carried by an adenoviral vector

<400> 11
gtaactataa cggtcgctcc gaatttctcg agttaattaa gattacgcca agctacgggc 60

ggagtactgt cctccgagcg gagtactgtc ctccgagcgg agtactgtcc tccgagcgga 120

gtactgtcct ccgagcggag ttctgtcctc cgagcggaga ctctagactc cctatcagtg 180

atagagatag gcgtgtacgg tgggaggcct atataagcag agctcgttta gtgaaccgtc 240

agatcgctcc ctatcagtga tagagagaat tcgaccgcca ccatgtcgga attcatgaga 300

catattatct gccacggagg tgttattacc gaagaaatgg ccgccagtct tttggaccag 360

ctgatcgaag aggtactggc tgataatctt ccacctccta gccattttga accacctacc 420

cttcacgaac tgtatgattt agacgtgacg gcccccgaag atcccaacga ggaggcggtt 480

tcgcagattt ttcccgactc tgtaatgttg gcggtgcagg aagggattga cttactcact 540

tttccgccgg cgcccggttc tccggagccg cctcaccttt cccggcagcc cgagcagccg 600

gagcagagag ccttgggtcc ggtttctatg ccaaaccttg taccggaggt gatcgatctt 660

acctgccacg aggctggctt tccacccagt gacgacgagg atgaagaggg tgaggagttt 720

gtgttagatt atgtggagca ccccgggcac ggttgcaggt cttgtcatta tcaccggagg 780

aatacggggg acccagatat tatgtgttcg ctttgctata tgaggacctg tggcatgttt 840

gtctacagta agtgaaaatt atgggcagtg ggtgatagag tggtgggttt ggtgtggtaa 900

tttttttttt aatttttaca gttttgtggt ttaaagaatt ttgtattgtg atttttttaa 960

aaggtcctgt gtctgaacct gagcctgagc ccgagccaga accggagcct gcaagaccta 1020

cccgccgtcc taaaatggcg cctgctatcc tgagacgccc gacatcacct gtgtctagag 1080

aatgcaatag tagtacggat agctgtgact ccggtccttc taacacacct cctgagatac 1140
acccggtggt cccgctgtgc cccattaaac cagttgccgt gagagttggt gggcgtcgcc 1200
aggctgtgga atgtatcgag gacttgctta acgagcctgg gcaacctttg gacttgagct 1260
gtaaacgccc caggccactc gagggtaccg ggtccggagc tacaaatttt tccctcctca 1320
aacaggctgg agatgtcgaa gaaaatcctg ggcctaccgg tcccatgtgg ctgcagagcc 1380
tgctgctctt gggcactgtg gcctgcagca tctctgcacc cgcccgctcg cccagcccca 1440
gcacgcagcc ctgggagcat gtgaatgcca tccaggaggc ccggcgtctc ctgaacctga 1500
gtagagacac tgctgctgag atgaatgaaa cagtagaagt catctcagaa atgtttgacc 1560
tccaggagcc gacctgccta cagacccgcc tggagctgta caagcagggc ctgcggggca 1620
gcctcaccaa gctcaagggc cccttgacca tgatggccag ccactacaag cagcactgcc 1680
ctccaacccc ggaaacttcc tgtgcaaccc agattatcac ctttgaaagt ttcaaagaga 1740
acctgaagga ctttctgctt gtcatcccct ttgactgctg ggagccagtc caggagtgaa 1800
gatctgaggg ccgcggcagc ctgctgacct gcggcgacgt ggaggaaaac cccggccccg 1860
gatccatgaa accagtaacg ttatacgatg tcgcagagta tgccggtgtc tcttatcaga 1920
ccgtttcccg cgtggtgaac caggccagcc acgtttctgc gaaaacgcgg gaaaaagtgg 1980
aagcggcgat ggcggagctg aattacattc ccaaccgcgt ggcacaacaa ctggcgggca 2040
aacagtcgtt gctgattggc gttgccacct ccagtctggc cctgcacgcg ccgtcgcaaa 2100
ttgtcgcggc gattaaatct cgcgccgatc aactgggtgc cagcgtggtg gtgtcgatgg 2160
tagaacgaag cggcgtcgaa gcctgtaaag cggcggtgca caatcttctc gcgcaacgcg 2220
tcagtgggct gatcattaac tatccgctgg atgaccagga tgccattgct gtggaagctg 2280
cctgcactaa tgttccggcg ttatttcttg atgtctctga ccagacaccc atcaacagta 2340
ttattttctc ccatgaagac ggtacgcgac tgggcgtgga gcatctggtc gcattgggtc 2400
accagcaaat cgcgctgtta gcgggcccat taagttctgt ctcggcgcgt ctgcgtctgg 2460
ctggctggca taaatatctc actcgcaatc aaattcagcc gatagcggaa cgggaaggcg 2520
actggagtgc catgtccggt tttcaacaaa ccatgcaaat gctgaatgag ggcatcgttc 2580
ccactgcgat gctggttgcc aacgatcaga tggcgctggg cgcaatgcgc gccattaccg 2640
agtccgggct gcgcgttggt gcggatatct cggtagtggg atacgacgat accgaagaca 2700
gctcatgtta tatcccgccg ttaaccacca tcaaacagga ttttcgcctg ctggggcaaa 2760
ccagcgtgga ccgcttgctg caactctctc agggccaggc ggtgaagggc aatcagctgt 2820
tgcccgtctc actggtgaaa agaaaaacca ccctggcgcc caatacgcaa accgcctctc 2880
cccgcgcgtt ggccgattca ttaatgcagc tggcacgaca ggtttcccga ctggaaagcg 2940
ggcagagcag cctgaggcct cctaagaaga agaggaaggt tgcggccgct aaccaatgtg 3000

cagactactg ttaaccaatg tgcagactac tgtgaattct aaccaatgtg cagactactg 3060
ttaaccaatg tgcagactac tgtaagcttc gaattgagta gtgctttcta ctttatgagt 3120
agtgctttct actttatgag tagtgctttc tactttatga gtagtgcttt ctactttatg 3180
gtcgacaata ctagttaaga aatcgccaag ctacgggcgg agtactgtcc tccgagcgga 3240
gtactgtcct ccgagcggag tactgtcctc cgagcggagt actgtcctcc gagcggagta 3300
ctgtcctccg agcggagact ctagaaattg tgagcggata acaatttagg cgtgtacggt 3360
gggaggccta tataagcaga gctcgtttag tgaaccgtca gatcgccagc tgacaaaatt 3420
gtgagcgctc acaattacta gaagatcttg aattcgccac catggctaga ttagataaaa 3480
gtaaagtgat taacagcgca ttagagctgc ttaatgaggt cggaatcgaa ggtttaacaa 3540
cccgtaaact cgcccagaag ctaggtgtag agcagcctac attgtattgg catgtaaaaa 3600
ataagcgggc tttgctcgac gccttagcca ttgagatgtt agataggcac catactcact 3660
tttgcccttt agaaggggaa agctggcaag attttttacg taataacgct aaaagtttta 3720
gatgtgcttt actaagtcat cgcgatggag caaaagtaca tttaggtaca cggcctacag 3780
aaaaacagta tgaaactctc gaaaatcaat tagccttttt atgccaacaa ggtttttcac 3840
tagagaatgc attatatgca ctcagcgctg tggggcattt tactttaggt tgcgtattgg 3900
aagatcaaga gcatcaagtc gctaaagaag aaagggaaac acctactact gatagtatgc 3960
cgccattatt acgacaagct atcgaattat ttgatcacca aggtgcagag ccagccttct 4020
tattcggcct tgaattgatc atatgcggat tagaaaaaca acttaaatgt gaaagtgggt 4080
cgccaaaaaa gaagagaaag gtcgacggcg gtggtgcttt gtctcctcag cactctgctg 4140
tcactcaagg aagtatcatc aagaacaagg agggcatgga tgctaagtca ctaactgcct 4200
ggtcccggac actggtgacc ttcaaggatg tatttgtgga cttcaccagg gaggagtgga 4260
agctgctgga cactgctcag cagatcgtgt acagaaatgt gatgctggag aactataaga 4320
acctggtttc cttgggttat cagcttacta agccagatgt gatcctccgg ttggagaagg 4380
gagaagagcc ctggctggtg gagagagaaa ttcaccaaga gacccatcct gattcagaga 4440
ctgcatttga aatcaaatca tcagtttaat acaaggcggc cgcaaatcaa catcagtctg 4500
ataagctatc aacatcagtc tgataagcta tcaacatcag tctgataagc tatcaacatc 4560
agtctgataa gctaaagctt cgaattctga taatcagcca taccacattt gtagaggttt 4620
tacttgcttt aaaaaacctc ccacacctcc ccctgaacct gaaacataaa atgaatgcaa 4680
ttgttgttgt taacttgttt attgcagctt ataatggtta caaataaagc aatagcatca 4740
caaatttcac aaataaagca tttttttcac tgcagctcca gattgaatta tttgcctgtc 4800
atacagctaa taattgacca taagacaatt agatttaaat tagttttgaa tctttctaat 4860

accaaagttc agtttactgt tccatgttgc ttctgagtgg cttcacagac ttatgaaaaa 4920

gtaaacggaa tcagaattac atcaatgcaa aagcattgct gtgaactctg tacttaggac 4980

taaactttga gcaataacac atatagattg aggattgttt gctgttagta tacaaactct 5040

ggttcaaagc tcctctttat tgcttgtctt ggaaaatttg ctgttcttca tggtttctct 5100

tttcactgct atctattttt ctcaaccact cacatggcta caaaagcttc ctgattaata 5160

attacactaa gtcaataggc atagagccag gactgtttgg gtaaactggt cactttatct 5220

taaactaaat atatccaaaa ctgaacatgt acttagttac taagtctttg actttatctc 5280

attcatacca ctcagcttta tccaggccac ttatttgaca gtattattgc gaaaacttcc 5340

tatctagaag tttgaggaga atatttgtta tatttgcaaa ataaaataag tttgcaagtt 5400

ttttttttct gccccaaaga gctctgtgtc cttgaacata aaatacaaat aaccgctatg 5460

ctgttaatta ttgacaaatg tcccattttc aacctaagga aataccataa agtaacagat 5520

ataccaacaa aaggttacta gttaacaggc attgcctgaa aagagtataa aagaatttca 5580

gcatgatttt ccatattgtg cttccaccac tgccaataac aaaataacta gcaaggatcc 5640

ggccaccatg gcccccccga ccgatgtcag cctgggggac gagctccact tagacggcga 5700

ggacgtggcg atggcgcatg ccgacgcgct agacgatttc gatctggaca tgttggggga 5760

cggggattcc ccgggtccgg gatttacccc ccacgactcc gccccctacg gcgctctgga 5820

tatggccgac ttcgagtttg agcagatgtt taccgatgcc cttggaattg acgagtacgg 5880

tgggacgcgt atgaagctac tgtcttctat cgaacaagca tgcgatattt gccgacttaa 5940

aaagctcaag tgctccaaag aaaaaccgaa gtgcgccaag tgtctgaaga acaactggga 6000

gtgtcgctac tctcccaaaa ccaaaaggtc tccgctgact agggcacatc tgacagaagt 6060

ggaatcaagg ctagaaagac tggaacagct atttctactg atttttcctc gagaagacct 6120

tgacatgatt ttgaaaatgg attctttaca ggatataaaa gcattgttaa caggattatt 6180

tgtacaagat aatgtgaata aagatgccgt cacagataga ttggcttcag tggagactga 6240

tatgcctcta acattgagac agcatagaat aagtgcgaca tcatcatcgg aagagagtag 6300

taacaaaggt caaagacagt tgactgtata attcactcct caggtgcagg ctgcctatca 6360

gaaggtggtg gctggtgtgg ccaatgccct ggctcacaaa taccactgag atctttttcc 6420

ctctgccaaa aattatgggg acatcatgaa gccccttgag catctgactt ctggctaata 6480

aaggaaattt attttcattg caatagtgtg ttggaatttt ttgtgtctct cactcggaag 6540

gacatatggg agggcaaatc atttaaaaca tcagaatgag tatttggttt agagtttggc 6600

aacatatgcc catatgctgg ctgccatgaa caaaggttgg ctataaagag gtcatcagta 6660

tatgaaacag ccccctgctg tccattcctt attccataga aaagccttga cttgaggtta 6720

gatttttttt atattttgtt ttgtgttatt tttttcttta acatccctaa aattttcctt 6780

acatgtttta ctagccagat ttttcctcct ctcctgacta ctcccagtca tagctgtccc 6840

tcttctctta tggagatcgg agaaagaggt aat 6873

<210> 12
<211> 6864
<212> DNA
<213> Artificial

<220>
<223> A nucleotide sequence of an mGMCSF-bearing nucleic acid carried by an adenoviral vector

<400> 12
gtaactataa cggtcgctcc gaatttctcg agttaattaa gattacgcca agctacgggc 60

ggagtactgt cctccgagcg gagtactgtc ctccgagcgg agtactgtcc tccgagcgga 120

gtactgtcct ccgagcggag ttctgtcctc cgagcggaga ctctagactc cctatcagtg 180

atagagatag gcgtgtacgg tgggaggcct atataagcag agctcgttta gtgaaccgtc 240

agatcgctcc ctatcagtga tagagagaat tcgaccgcca ccatgtcgga attcatgaga 300

catattatct gccacggagg tgttattacc gaagaaatgg ccgccagtct tttggaccag 360

ctgatcgaag aggtactggc tgataatctt ccacctccta gccattttga accacctacc 420

cttcacgaac tgtatgattt agacgtgacg gcccccgaag atcccaacga ggaggcggtt 480

tcgcagattt ttcccgactc tgtaatgttg gcggtgcagg aagggattga cttactcact 540

tttccgccgg cgcccggttc tccggagccg cctcaccttt cccggcagcc cgagcagccg 600

gagcagagag ccttgggtcc ggtttctatg ccaaaccttg taccggaggt gatcgatctt 660

acctgccacg aggctggctt tccacccagt gacgacgagg atgaagaggg tgaggagttt 720

gtgttagatt atgtggagca ccccgggcac ggttgcaggt cttgtcatta tcaccggagg 780

aatacggggg acccagatat tatgtgttcg ctttgctata tgaggacctg tggcatgttt 840

gtctacagta agtgaaaatt atgggcagtg ggtgatagag tggtgggttt ggtgtggtaa 900

tttttttttt aatttttaca gttttgtggt ttaaagaatt ttgtattgtg atttttttaa 960

aaggtcctgt gtctgaacct gagcctgagc ccgagccaga accggagcct gcaagaccta 1020

cccgccgtcc taaaatggcg cctgctatcc tgagacgccc gacatcacct gtgtctagag 1080

aatgcaatag tagtacggat agctgtgact ccggtccttc taacacacct cctgagatac 1140

acccggtggt cccgctgtgc cccattaaac cagttgccgt gagagttggt gggcgtcgcc 1200

aggctgtgga atgtatcgag gacttgctta acgagcctgg gcaacctttg gacttgagct 1260

gtaaacgccc caggccactc gagggtaccg ggtccggagc tacaaatttt tccctcctca 1320

aacaggctgg agatgtcgaa gaaaatcctg ggcctaccgg tcccatgtgg ctgcagaatt 1380

tacttttcct gggcattgtg gtctacagcc tctcagcacc cacccgctca cccatcactg 1440

tcacccggcc ttggaagcat gtagaggcca tcaaagaagc cctgaacctc ctggatgaca 1500

tgcctgtcac gttgaatgaa gaggtagaag tcgtctctaa cgagttctcc ttcaagaagc 1560

taacatgtgt gcagacccgc ctgaagatat tcgagcaggg tctacggggc aatttcacca 1620

aactcaaggg cgccttgaac atgacagcca gctactacca gacatactgc cccccaactc 1680

cggaaacgga ctgtgaaaca caagttacca cctatgcgga tttcatagac agccttaaaa 1740

cctttctgac tgatatcccc tttgaatgca aaaaaccagg ccaaaaatga agatctgagg 1800

gccgcggcag cctgctgacc tgcggcgacg tggaggaaaa ccccggcccc ggatccatga 1860

aaccagtaac gttatacgat gtcgcagagt atgccggtgt ctcttatcag accgtttccc 1920

gcgtggtgaa ccaggccagc cacgtttctg cgaaaacgcg ggaaaaagtg gaagcggcga 1980

tggcggagct gaattacatt cccaaccgcg tggcacaaca actggcgggc aaacagtcgt 2040

tgctgattgg cgttgccacc tccagtctgg ccctgcacgc gccgtcgcaa attgtcgcgg 2100

cgattaaatc tcgcgccgat caactgggtg ccagcgtggt ggtgtcgatg gtagaacgaa 2160

gcggcgtcga agcctgtaaa gcggcggtgc acaatcttct cgcgcaacgc gtcagtgggc 2220

tgatcattaa ctatccgctg gatgaccagg atgccattgc tgtggaagct gcctgcacta 2280

atgttccggc gttatttctt gatgtctctg accagacacc catcaacagt attattttct 2340

cccatgaaga cggtacgcga ctgggcgtgg agcatctggt cgcattgggt caccagcaaa 2400

tcgcgctgtt agcgggccca ttaagttctg tctcggcgcg tctgcgtctg gctggctggc 2460

ataaatatct cactcgcaat caaattcagc cgatagcgga acgggaaggc gactggagtg 2520

ccatgtccgg ttttcaacaa accatgcaaa tgctgaatga gggcatcgtt cccactgcga 2580

tgctggttgc caacgatcag atggcgctgg gcgcaatgcg cgccattacc gagtccgggc 2640

tgcgcgttgg tgcggatatc tcggtagtgg gatacgacga taccgaagac agctcatgtt 2700

atatcccgcc gttaaccacc atcaaacagg attttcgcct gctggggcaa accagcgtgg 2760

accgcttgct gcaactctct cagggccagg cggtgaaggg caatcagctg ttgcccgtct 2820

cactggtgaa aagaaaaacc accctggcgc ccaatacgca aaccgcctct ccccgcgcgt 2880

tggccgattc attaatgcag ctggcacgac aggtttcccg actggaaagc gggcagagca 2940

gcctgaggcc tcctaagaag aagaggaagg ttgcggccgc taaccaatgt gcagactact 3000

gttaaccaat gtgcagacta ctgtgaattc taaccaatgt gcagactact gttaaccaat 3060

gtgcagacta ctgtaagctt cgaattgagt agtgctttct actttatgag tagtgctttc 3120

tactttatga gtagtgcttt ctactttatg agtagtgctt tctactttat ggtcgacaat 3180

actagttaag aaatcgccaa gctacgggcg gagtactgtc ctccgagcgg agtactgtcc 3240

tccgagcgga gtactgtcct ccgagcggag tactgtcctc cgagcggagt actgtcctcc 3300

gagcggagac tctagaaatt gtgagcggat aacaatttag gcgtgtacgg tgggaggcct 3360
atataagcag agctcgttta gtgaaccgtc agatcgccag ctgacaaaat tgtgagcgct 3420
cacaattact agaagatctt gaattcgcca ccatggctag attagataaa agtaaagtga 3480
ttaacagcgc attagagctg cttaatgagg tcggaatcga aggtttaaca acccgtaaac 3540
tcgcccagaa gctaggtgta gagcagccta cattgtattg gcatgtaaaa aataagcggg 3600
ctttgctcga cgccttagcc attgagatgt tagataggca ccatactcac ttttgccctt 3660
tagaagggga aagctggcaa gattttttac gtaataacgc taaaagtttt agatgtgctt 3720
tactaagtca tcgcgatgga gcaaaagtac atttaggtac acggcctaca gaaaaacagt 3780
atgaaactct cgaaaatcaa ttagcctttt tatgccaaca aggtttttca ctagagaatg 3840
cattatatgc actcagcgct gtggggcatt ttactttagg ttgcgtattg gaagatcaag 3900
agcatcaagt cgctaaagaa gaaagggaaa cacctactac tgatagtatg ccgccattat 3960
tacgacaagc tatcgaatta tttgatcacc aaggtgcaga gccagccttc ttattcggcc 4020
ttgaattgat catatgcgga ttagaaaaac aacttaaatg tgaaagtggg tcgccaaaaa 4080
agaagagaaa ggtcgacggc ggtggtgctt tgtctcctca gcactctgct gtcactcaag 4140
gaagtatcat caagaacaag gagggcatgg atgctaagtc actaactgcc tggtcccgga 4200
cactggtgac cttcaaggat gtatttgtgg acttcaccag ggaggagtgg aagctgctgg 4260
acactgctca gcagatcgtg tacagaaatg tgatgctgga gaactataag aacctggttt 4320
ccttgggtta tcagcttact aagccagatg tgatcctccg gttggagaag ggagaagagc 4380
cctggctggt ggagagagaa attcaccaag agacccatcc tgattcagag actgcatttg 4440
aaatcaaatc atcagtttaa tacaaggcgg ccgcaaatca acatcagtct gataagctat 4500
caacatcagt ctgataagct atcaacatca gtctgataag ctatcaacat cagtctgata 4560
agctaaagct tcgaattctg ataatcagcc ataccacatt tgtagaggtt ttacttgctt 4620
taaaaaacct cccacacctc cccctgaacc tgaaacataa aatgaatgca attgttgttg 4680
ttaacttgtt tattgcagct tataatggtt acaaataaag caatagcatc acaaatttca 4740
caaataaagc atttttttca ctgcagctcc agattgaatt atttgcctgt catacagcta 4800
ataattgacc ataagacaat tagatttaaa ttagttttga atctttctaa taccaaagtt 4860
cagtttactg ttccatgttg cttctgagtg gcttcacaga cttatgaaaa agtaaacgga 4920
atcagaatta catcaatgca aaagcattgc tgtgaactct gtacttagga ctaaactttg 4980
agcaataaca catatagatt gaggattgtt tgctgttagt atacaaactc tggttcaaag 5040
ctcctcttta ttgcttgtct tggaaaattt gctgttcttc atggtttctc ttttcactgc 5100
tatctatttt tctcaaccac tcacatggct acaaaagctt cctgattaat aattacacta 5160
agtcaatagg catagagcca ggactgtttg ggtaaactgg tcactttatc ttaaactaaa 5220

tatatccaaa actgaacatg tacttagtta ctaagtcttt gactttatct cattcatacc 5280

actcagcttt atccaggcca cttatttgac agtattattg cgaaaacttc ctatctagaa 5340

gtttgaggag aatatttgtt atatttgcaa aataaaataa gtttgcaagt tttttttttc 5400

tgccccaaag agctctgtgt ccttgaacat aaaatacaaa taaccgctat gctgttaatt 5460

attgacaaat gtcccatttt caacctaagg aaataccata aagtaacaga tataccaaca 5520

aaaggttact agttaacagg cattgcctga aaagagtata aaagaatttc agcatgattt 5580

tccatattgt gcttccacca ctgccaataa caaaataact agcaaggatc cggccaccat 5640

ggcccccccg accgatgtca gcctggggga cgagctccac ttagacggcg aggacgtggc 5700

gatggcgcat gccgacgcgc tagacgattt cgatctggac atgttggggg acggggattc 5760

cccgggtccg ggatttaccc cccacgactc cgccccctac ggcgctctgg atatggccga 5820

cttcgagttt gagcagatgt ttaccgatgc ccttggaatt gacgagtacg gtgggacgcg 5880

tatgaagcta ctgtcttcta tcgaacaagc atgcgatatt tgccgactta aaaagctcaa 5940

gtgctccaaa gaaaaaccga agtgcgccaa gtgtctgaag aacaactggg agtgtcgcta 6000

ctctcccaaa accaaaaggt ctccgctgac tagggcacat ctgacagaag tggaatcaag 6060

gctagaaaga ctggaacagc tatttctact gatttttcct cgagaagacc ttgacatgat 6120

tttgaaaatg gattctttac aggatataaa agcattgtta acaggattat ttgtacaaga 6180

taatgtgaat aaagatgccg tcacagatag attggcttca gtggagactg atatgcctct 6240

aacattgaga cagcatagaa taagtgcgac atcatcatcg gaagagagta gtaacaaagg 6300

tcaaagacag ttgactgtat aattcactcc tcaggtgcag gctgcctatc agaaggtggt 6360

ggctggtgtg gccaatgccc tggctcacaa ataccactga gatctttttc cctctgccaa 6420

aaattatggg gacatcatga agccccttga gcatctgact tctggctaat aaaggaaatt 6480

tattttcatt gcaatagtgt gttggaattt tttgtgtctc tcactcggaa ggacatatgg 6540

gagggcaaat catttaaaac atcagaatga gtatttggtt tagagtttgg caacatatgc 6600

ccatatgctg gctgccatga acaaaggttg gctataaaga ggtcatcagt atatgaaaca 6660

gccccctgct gtccattcct tattccatag aaaagccttg acttgaggtt agattttttt 6720

tatattttgt tttgtgttat ttttttcttt aacatcccta aaattttcct tacatgtttt 6780

actagccaga tttttcctcc tctcctgact actcccagtc atagctgtcc ctcttctctt 6840

atggagatcg gagaaagagg taat 6864

<210> 13
<211> 7203
<212> DNA
<213> Artificial

<220>
<223>  A nucleotide sequence of an anti-PD-1scFv-bearing nucleic acid
carried by an adenoviral vector

<400>  13
gtaactataa cggtcgctcc gaatttctcg agttaattaa gattacgcca agctacgggc      60

ggagtactgt cctccgagcg gagtactgtc ctccgagcgg agtactgtcc tccgagcgga     120

gtactgtcct ccgagcggag ttctgtcctc cgagcggaga ctctagactc cctatcagtg     180

atagagatag gcgtgtacgg tgggaggcct atataagcag agctcgttta gtgaaccgtc     240

agatcgctcc ctatcagtga tagagagaat tcgaccgcca ccatgtcgga attcatgaga     300

catattatct gccacggagg tgttattacc gaagaaatgg ccgccagtct tttggaccag     360

ctgatcgaag aggtactggc tgataatctt ccacctccta gccattttga accacctacc     420

cttcacgaac tgtatgattt agacgtgacg gcccccgaag atcccaacga ggaggcggtt     480

tcgcagattt ttcccgactc tgtaatgttg gcggtgcagg aagggattga cttactcact     540

tttccgccgg cgcccggttc tccggagccg cctcaccttt cccggcagcc cgagcagccg     600

gagcagagag ccttgggtcc ggtttctatg ccaaaccttg taccggaggt gatcgatctt     660

acctgccacg aggctggctt tccacccagt gacgacgagg atgaagaggg tgaggagttt     720

gtgttagatt atgtggagca ccccgggcac ggttgcaggt cttgtcatta tcaccggagg     780

aatacggggg acccagatat tatgtgttcg ctttgctata tgaggacctg tggcatgttt     840

gtctacagta agtgaaaatt atgggcagtg ggtgatagag tggtgggttt ggtgtggtaa     900

tttttttttt aatttttaca gttttgtggt ttaaagaatt ttgtattgtg atttttttaa     960

aaggtcctgt gtctgaacct gagcctgagc ccgagccaga accggagcct gcaagaccta    1020

cccgccgtcc taaaatggcg cctgctatcc tgagacgccc gacatcacct gtgtctagag    1080

aatgcaatag tagtacggat agctgtgact ccggtccttc taacacacct cctgagatac    1140

acccggtggt cccgctgtgc cccattaaac cagttgccgt gagagttggt gggcgtcgcc    1200

aggctgtgga atgtatcgag gacttgctta acgagcctgg gcaacctttg gacttgagct    1260

gtaaacgccc caggccactc gagggtaccg ggtccggagc tacaaatttt tccctcctca    1320

aacaggctgg agatgtcgaa gaaaatcctg ggcctaccgg tcccatgagt gtgcccactc    1380

aggtcctggg gttgctgctg ctgtggctta cagacgctcg ctgccaggtg cagctggtgg    1440

agagcggcgg cggcgtggtg cagcccggca ggagcctgag gctggactgc aaggccagcg    1500

gcatcacctt cagcaacagc ggcatgcact gggtgaggca ggcccccggc aagggcctgg    1560

agtgggtggc cgtgatctgg tacgacggca gcaagaggta ctacgccgac agcgtgaagg    1620

gcaggttcac catcagcagg gacaacagca agaacaccct gttcctgcag atgaacagcc    1680

tgagggccga ggacaccgcc gtgtactact gcgccaccaa cgacgactac tggggccagg    1740

gcaccctggt gaccgtgagc agcggtggag gcggttcagg cggaggtggc tctggcggtg 1800
gcggatcgga gatcgtgctg acccagagcc ccgccaccct gagcctgagc cccggcgaga 1860
gggccaccct gagctgcagg gccagccaga gcgtgagcag ctacctggcc tggtaccagc 1920
agaagcccgg ccaggccccc aggctgctga tctacgacgc cagcaacagg gccaccggca 1980
tccccgccag gttcagcggc agcggcagcg gcaccgactt caccctgacc atcagcagcc 2040
tggagcccga ggacttcgcc gtgtactact gccagcagag cagcaactgg cccaggacct 2100
tcggccaggg caccaaggtg gagatcaaga gatctgaggg ccgcggcagc ctgctgacct 2160
gcggcgacgt ggaggaaaac cccggccccg gatccatgaa accagtaacg ttatacgatg 2220
tcgcagagta tgccggtgtc tcttatcaga ccgtttcccg cgtggtgaac caggccagcc 2280
acgtttctgc gaaaacgcgg gaaaaagtgg aagcggcgat ggcggagctg aattacattc 2340
ccaaccgcgt ggcacaacaa ctggcgggca aacagtcgtt gctgattggc gttgccacct 2400
ccagtctggc cctgcacgcg ccgtcgcaaa ttgtcgcggc gattaaatct cgcgccgatc 2460
aactgggtgc cagcgtggtg gtgtcgatgg tagaacgaag cggcgtcgaa gcctgtaaag 2520
cggcggtgca caatcttctc gcgcaacgcg tcagtgggct gatcattaac tatccgctgg 2580
atgaccagga tgccattgct gtggaagctg cctgcactaa tgttccggcg ttatttcttg 2640
atgtctctga ccagacaccc atcaacagta ttattttctc ccatgaagac ggtacgcgac 2700
tgggcgtgga gcatctggtc gcattgggtc accagcaaat cgcgctgtta gcgggcccat 2760
taagttctgt ctcggcgcgt ctgcgtctgg ctggctggca taaatatctc actcgcaatc 2820
aaattcagcc gatagcggaa cgggaaggcg actggagtgc catgtccggt tttcaacaaa 2880
ccatgcaaat gctgaatgag ggcatcgttc ccactgcgat gctggttgcc aacgatcaga 2940
tggcgctggg cgcaatgcgc gccattaccg agtccgggct gcgcgttggt gcggatatct 3000
cggtagtggg atacgacgat accgaagaca gctcatgtta tatcccgccg ttaaccacca 3060
tcaaacagga ttttcgcctg ctggggcaaa ccagcgtgga ccgcttgctg caactctctc 3120
agggccaggc ggtgaagggc aatcagctgt tgcccgtctc actggtgaaa agaaaaacca 3180
ccctggcgcc caatacgcaa accgcctctc cccgcgcgtt ggccgattca ttaatgcagc 3240
tggcacgaca ggtttcccga ctggaaagcg ggcagagcag cctgaggcct cctaagaaga 3300
agaggaaggt tgcggccgct aaccaatgtg cagactactg ttaaccaatg tgcagactac 3360
tgtgaattct aaccaatgtg cagactactg ttaaccaatg tgcagactac tgtaagcttc 3420
gaattgagta gtgctttcta ctttatgagt agtgctttct actttatgag tagtgctttc 3480
tactttatga gtagtgcttt ctactttatg gtcgacaata ctagttaaga aatcgccaag 3540
ctacgggcgg agtactgtcc tccgagcgga gtactgtcct ccgagcggag tactgtcctc 3600
cgagcggagt actgtcctcc gagcggagta ctgtcctccg agcggagact ctagaaattg 3660

tgagcggata acaatttagg cgtgtacggt gggaggccta tataagcaga gctcgtttag 3720

tgaaccgtca gatcgccagc tgacaaaatt gtgagcgctc acaattacta gaagatcttg 3780

aattcgccac catggctaga ttagataaaa gtaaagtgat taacagcgca ttagagctgc 3840

ttaatgaggt cggaatcgaa ggtttaacaa cccgtaaact cgcccagaag ctaggtgtag 3900

agcagcctac attgtattgg catgtaaaaa ataagcgggc tttgctcgac gccttagcca 3960

ttgagatgtt agataggcac catactcact tttgcccttt agaaggggaa agctggcaag 4020

attttttacg taataacgct aaaagtttta gatgtgcttt actaagtcat cgcgatggag 4080

caaaagtaca tttaggtaca cggcctacag aaaaacagta tgaaactctc gaaaatcaat 4140

tagccttttt atgccaacaa ggtttttcac tagagaatgc attatatgca ctcagcgctg 4200

tggggcattt tactttaggt tgcgtattgg aagatcaaga gcatcaagtc gctaaagaag 4260

aaagggaaac acctactact gatagtatgc cgccattatt acgacaagct atcgaattat 4320

ttgatcacca aggtgcagag ccagccttct tattcggcct tgaattgatc atatgcggat 4380

tagaaaaaca acttaaatgt gaaagtgggt cgccaaaaaa gaagagaaag gtcgacggcg 4440

gtggtgcttt gtctcctcag cactctgctg tcactcaagg aagtatcatc aagaacaagg 4500

agggcatgga tgctaagtca ctaactgcct ggtcccggac actggtgacc ttcaaggatg 4560

tatttgtgga cttcaccagg gaggagtgga agctgctgga cactgctcag cagatcgtgt 4620

acagaaatgt gatgctggag aactataaga acctggtttc cttgggttat cagcttacta 4680

agccagatgt gatcctccgg ttggagaagg gagaagagcc ctggctggtg gagagagaaa 4740

ttcaccaaga gacccatcct gattcagaga ctgcatttga aatcaaatca tcagtttaat 4800

acaaggcggc cgcaaatcaa catcagtctg ataagctatc aacatcagtc tgataagcta 4860

tcaacatcag tctgataagc tatcaacatc agtctgataa gctaaagctt cgaattctga 4920

taatcagcca taccacattt gtagaggttt tacttgcttt aaaaaacctc ccacacctcc 4980

ccctgaacct gaaacataaa atgaatgcaa ttgttgttgt taacttgttt attgcagctt 5040

ataatggtta caaataaagc aatagcatca caaatttcac aaataaagca tttttttcac 5100

tgcagctcca gattgaatta tttgcctgtc atacagctaa taattgacca taagacaatt 5160

agatttaaat tagttttgaa tctttctaat accaaagttc agtttactgt tccatgttgc 5220

ttctgagtgg cttcacagac ttatgaaaaa gtaaacggaa tcagaattac atcaatgcaa 5280

aagcattgct gtgaactctg tacttaggac taaactttga gcaataacac atatagattg 5340

aggattgttt gctgttagta tacaaactct ggttcaaagc tcctctttat tgcttgtctt 5400

ggaaaatttg ctgttcttca tggtttctct tttcactgct atctattttt ctcaaccact 5460

cacatggcta caaaagcttc ctgattaata attacactaa gtcaataggc atagagccag 5520

gactgtttgg gtaaactggt cactttatct taaactaaat atatccaaaa ctgaacatgt 5580

acttagttac taagtctttg actttatctc attcatacca ctcagcttta tccaggccac 5640

ttatttgaca gtattattgc gaaaacttcc tatctagaag tttgaggaga atatttgtta 5700

tatttgcaaa ataaaataag tttgcaagtt ttttttttct gccccaaaga gctctgtgtc 5760

cttgaacata aaatacaaat aaccgctatg ctgttaatta ttgacaaatg tcccattttc 5820

aacctaagga aataccataa agtaacagat ataccaacaa aaggttacta gttaacaggc 5880

attgcctgaa aagagtataa aagaatttca gcatgatttt ccatattgtg cttccaccac 5940

tgccaataac aaaataacta gcaaggatcc ggccaccatg gcccccccga ccgatgtcag 6000

cctgggggac gagctccact tagacggcga ggacgtggcg atggcgcatg ccgacgcgct 6060

agacgatttc gatctggaca tgttggggga cggggattcc ccgggtccgg gatttacccc 6120

ccacgactcc gccccctacg gcgctctgga tatggccgac ttcgagtttg agcagatgtt 6180

taccgatgcc cttggaattg acgagtacgg tgggacgcgt atgaagctac tgtcttctat 6240

cgaacaagca tgcgatattt gccgacttaa aaagctcaag tgctccaaag aaaaaccgaa 6300

gtgcgccaag tgtctgaaga acaactggga gtgtcgctac tctcccaaaa ccaaaaggtc 6360

tccgctgact agggcacatc tgacagaagt ggaatcaagg ctagaaagac tggaacagct 6420

atttctactg atttttcctc gagaagacct tgacatgatt ttgaaaatgg attctttaca 6480

ggatataaaa gcattgttaa caggattatt tgtacaagat aatgtgaata aagatgccgt 6540

cacagataga ttggcttcag tggagactga tatgcctcta acattgagac agcatagaat 6600

aagtgcgaca tcatcatcgg aagagagtag taacaaaggt caaagacagt tgactgtata 6660

attcactcct caggtgcagg ctgcctatca gaaggtggtg gctggtgtgg ccaatgccct 6720

ggctcacaaa taccactgag atctttttcc ctctgccaaa aattatgggg acatcatgaa 6780

gccccttgag catctgactt ctggctaata aaggaaattt attttcattg caatagtgtg 6840

ttggaatttt ttgtgtctct cactcggaag gacatatggg agggcaaatc atttaaaaca 6900

tcagaatgag tatttggttt agagtttggc aacatatgcc catatgctgg ctgccatgaa 6960

caaaggttgg ctataaagag gtcatcagta tatgaaacag ccccctgctg tccattcctt 7020

attccataga aaagccttga cttgaggtta gatttttttt atattttgtt ttgtgttatt 7080

tttttcttta acatccctaa aattttcctt acatgtttta ctagccagat ttttcctcct 7140

ctcctgacta ctcccagtca tagctgtccc tcttctctta tggagatcgg agaaagaggt 7200

aat 7203

<210> 14
<211> 7218
<212> DNA
<213> Artificial

<220>
<223>  A nucleotide sequence of an anti-PD-L1scFv-bearing nucleic acid
carried by an adenoviral vector

<400>  14
gtaactataa cggtcgctcc gaatttctcg agttaattaa gattacgcca agctacgggc      60

ggagtactgt cctccgagcg gagtactgtc ctccgagcgg agtactgtcc tccgagcgga     120

gtactgtcct ccgagcggag ttctgtcctc cgagcggaga ctctagactc cctatcagtg     180

atagagatag gcgtgtacgg tgggaggcct atataagcag agctcgttta gtgaaccgtc     240

agatcgctcc ctatcagtga tagagagaat tcgaccgcca ccatgtcgga attcatgaga     300

catattatct gccacggagg tgttattacc gaagaaatgg ccgccagtct tttggaccag     360

ctgatcgaag aggtactggc tgataatctt ccacctccta gccattttga accacctacc     420

cttcacgaac tgtatgattt agacgtgacg gcccccgaag atcccaacga ggaggcggtt     480

tcgcagattt ttcccgactc tgtaatgttg gcggtgcagg aagggattga cttactcact     540

tttccgccgg cgcccggttc tccggagccg cctcaccttt cccggcagcc cgagcagccg     600

gagcagagag ccttgggtcc ggtttctatg ccaaaccttg taccggaggt gatcgatctt     660

acctgccacg aggctggctt tccacccagt gacgacgagg atgaagaggg tgaggagttt     720

gtgttagatt atgtggagca ccccgggcac ggttgcaggt cttgtcatta tcaccggagg     780

aatacggggg acccagatat tatgtgttcg ctttgctata tgaggacctg tggcatgttt     840

gtctacagta agtgaaaatt atgggcagtg ggtgatagag tggtgggttt ggtgtggtaa     900

tttttttttt aatttttaca gttttgtggt ttaaagaatt ttgtattgtg atttttttaa     960

aaggtcctgt gtctgaacct gagcctgagc ccgagccaga accggagcct gcaagaccta    1020

cccgccgtcc taaaatggcg cctgctatcc tgagacgccc gacatcacct gtgtctagag    1080

aatgcaatag tagtacggat agctgtgact ccggtccttc taacacacct cctgagatac    1140

acccggtggt cccgctgtgc cccattaaac cagttgccgt gagagttggt gggcgtcgcc    1200

aggctgtgga atgtatcgag gacttgctta acgagcctgg gcaacctttg gacttgagct    1260

gtaaacgccc caggccactc gagggtaccg ggtccggagc tacaaatttt tccctcctca    1320

aacaggctgg agatgtcgaa gaaaatcctg ggcctaccgg tcccatgagt gtgcccactc    1380

aggtcctggg gttgctgctg ctgtggctta cagacgctcg ctgcgaggtg cagctggtgg    1440

agagcggcgg cggcctggtg cagcccggcg gcagcctgag gctgagctgc gccgccagcg    1500

gcttcacctt cagcgacagc tggatccact gggtgaggca ggcccccggc aagggcctgg    1560

agtgggtggc ctggatcagc ccctacggcg gcagcaccta ctacgccgac agcgtgaagg    1620

gcaggttcac catcagcgcc gacaccagca agaacaccgc ctacctgcag atgaacagcc    1680

tgagggccga ggacaccgcc gtgtactact gcgccaggag gcactggccc ggcggcttcg    1740

actactgggg ccagggcacc ctggtgaccg tgagcagcgg tggaggcggt tcaggcggag 1800

gtggctctgg cggtggcgga tcggacatcc agatgaccca gagccccagc agcctgagcg 1860

ccagcgtggg cgacagggtg accatcacct gcagggccag ccaggacgtg agcaccgccg 1920

tggcctggta ccagcagaag cccggcaagg cccccaagct gctgatctac agcgccagct 1980

tcctgtacag cggcgtgccc agcaggttca gcggcagcgg cagcggcacc gacttcaccc 2040

tgaccatcag cagcctgcag cccgaggact tcgccaccta ctactgccag cagtacctgt 2100

accaccccgc caccttcggc cagggcacca aggtggagat caagagatct gagggccgcg 2160

gcagcctgct gacctgcggc gacgtggagg aaaaccccgg ccccggatcc atgaaaccag 2220

taacgttata cgatgtcgca gagtatgccg gtgtctctta tcagaccgtt tcccgcgtgg 2280

tgaaccaggc cagccacgtt tctgcgaaaa cgcgggaaaa agtggaagcg gcgatggcgg 2340

agctgaatta cattcccaac cgcgtggcac aacaactggc gggcaaacag tcgttgctga 2400

ttggcgttgc cacctccagt ctggccctgc acgcgccgtc gcaaattgtc gcggcgatta 2460

aatctcgcgc cgatcaactg ggtgccagcg tggtggtgtc gatggtagaa cgaagcggcg 2520

tcgaagcctg taaagcggcg gtgcacaatc ttctcgcgca acgcgtcagt gggctgatca 2580

ttaactatcc gctggatgac caggatgcca ttgctgtgga agctgcctgc actaatgttc 2640

cggcgttatt tcttgatgtc tctgaccaga cacccatcaa cagtattatt ttctcccatg 2700

aagacggtac gcgactgggc gtggagcatc tggtcgcatt gggtcaccag caaatcgcgc 2760

tgttagcggg cccattaagt tctgtctcgg cgcgtctgcg tctggctggc tggcataaat 2820

atctcactcg caatcaaatt cagccgatag cggaacggga aggcgactgg agtgccatgt 2880

ccggttttca acaaaccatg caaatgctga atgagggcat cgttcccact gcgatgctgg 2940

ttgccaacga tcagatggcg ctgggcgcaa tgcgcgccat taccgagtcc gggctgcgcg 3000

ttggtgcgga tatctcggta gtgggatacg acgataccga agacagctca tgttatatcc 3060

cgccgttaac caccatcaaa caggattttc gcctgctggg gcaaaccagc gtggaccgct 3120

tgctgcaact ctctcagggc caggcggtga agggcaatca gctgttgccc gtctcactgg 3180

tgaaaagaaa aaccaccctg gcgcccaata cgcaaaccgc ctctccccgc gcgttggccg 3240

attcattaat gcagctggca cgacaggttt cccgactgga aagcgggcag agcagcctga 3300

ggcctcctaa gaagaagagg aaggttgcgg ccgctaacca atgtgcagac tactgttaac 3360

caatgtgcag actactgtga attctaacca atgtgcagac tactgttaac caatgtgcag 3420

actactgtaa gcttcgaatt gagtagtgct ttctacttta tgagtagtgc tttctacttt 3480

atgagtagtg ctttctactt tatgagtagt gctttctact ttatggtcga caatactagt 3540

taagaaatcg ccaagctacg ggcggagtac tgtcctccga gcggagtact gtcctccgag 3600

cggagtactg tcctccgagc ggagtactgt cctccgagcg gagtactgtc ctccgagcgg 3660
agactctaga aattgtgagc ggataacaat ttaggcgtgt acggtgggag gcctatataa 3720
gcagagctcg tttagtgaac cgtcagatcg ccagctgaca aaattgtgag cgctcacaat 3780
tactagaaga tcttgaattc gccaccatgg ctagattaga taaaagtaaa gtgattaaca 3840
gcgcattaga gctgcttaat gaggtcggaa tcgaaggttt aacaacccgt aaactcgccc 3900
agaagctagg tgtagagcag cctacattgt attggcatgt aaaaaataag cgggctttgc 3960
tcgacgcctt agccattgag atgttagata ggcaccatac tcacttttgc cctttagaag 4020
gggaaagctg gcaagatttt ttacgtaata acgctaaaag ttttagatgt gctttactaa 4080
gtcatcgcga tggagcaaaa gtacatttag gtacacggcc tacagaaaaa cagtatgaaa 4140
ctctcgaaaa tcaattagcc tttttatgcc aacaaggttt ttcactagag aatgcattat 4200
atgcactcag cgctgtgggg cattttactt taggttgcgt attggaagat caagagcatc 4260
aagtcgctaa agaagaaagg gaaacaccta ctactgatag tatgccgcca ttattacgac 4320
aagctatcga attatttgat caccaaggtg cagagccagc cttcttattc ggccttgaat 4380
tgatcatatg cggattagaa aaacaactta aatgtgaaag tgggtcgcca aaaaagaaga 4440
gaaaggtcga cggcggtggt gctttgtctc ctcagcactc tgctgtcact caaggaagta 4500
tcatcaagaa caaggagggc atggatgcta agtcactaac tgcctggtcc cggacactgg 4560
tgaccttcaa ggatgtattt gtggacttca ccagggagga gtggaagctg ctggacactg 4620
ctcagcagat cgtgtacaga aatgtgatgc tggagaacta taagaacctg gtttccttgg 4680
gttatcagct tactaagcca gatgtgatcc tccggttgga gaagggagaa gagccctggc 4740
tggtggagag agaaattcac caagagaccc atcctgattc agagactgca tttgaaatca 4800
aatcatcagt ttaatacaag gcggccgcaa atcaacatca gtctgataag ctatcaacat 4860
cagtctgata agctatcaac atcagtctga taagctatca acatcagtct gataagctaa 4920
agcttcgaat tctgataatc agccatacca catttgtaga ggttttactt gctttaaaaa 4980
acctcccaca cctccccctg aacctgaaac ataaaatgaa tgcaattgtt gttgttaact 5040
tgtttattgc agcttataat ggttacaaat aaagcaatag catcacaaat ttcacaaata 5100
aagcattttt ttcactgcag ctccagattg aattatttgc ctgtcataca gctaataatt 5160
gaccataaga caattagatt taaattagtt ttgaatcttt ctaataccaa agttcagttt 5220
actgttccat gttgcttctg agtggcttca cagacttatg aaaaagtaaa cggaatcaga 5280
attacatcaa tgcaaaagca ttgctgtgaa ctctgtactt aggactaaac tttgagcaat 5340
aacacatata gattgaggat tgtttgctgt tagtatacaa actctggttc aaagctcctc 5400
tttattgctt gtcttggaaa atttgctgtt cttcatggtt tctcttttca ctgctatcta 5460
tttttctcaa ccactcacat ggctacaaaa gcttcctgat taataattac actaagtcaa 5520

taggcataga gccaggactg tttgggtaaa ctggtcactt tatcttaaac taaatatatc 5580

caaaactgaa catgtactta gttactaagt ctttgacttt atctcattca taccactcag 5640

ctttatccag gccacttatt tgacagtatt attgcgaaaa cttcctatct agaagtttga 5700

ggagaatatt tgttatattt gcaaaataaa ataagtttgc aagttttttt tttctgcccc 5760

aaagagctct gtgtccttga acataaaata caaataaccg ctatgctgtt aattattgac 5820

aaatgtccca ttttcaacct aaggaaatac cataaagtaa cagatatacc aacaaaaggt 5880

tactagttaa caggcattgc ctgaaaagag tataaaagaa tttcagcatg attttccata 5940

ttgtgcttcc accactgcca ataacaaaat aactagcaag gatccggcca ccatggcccc 6000

cccgaccgat gtcagcctgg gggacgagct ccacttagac ggcgaggacg tggcgatggc 6060

gcatgccgac gcgctagacg atttcgatct ggacatgttg ggggacgggg attccccggg 6120

tccgggattt accccccacg actccgcccc ctacggcgct ctggatatgg ccgacttcga 6180

gtttgagcag atgtttaccg atgcccttgg aattgacgag tacggtggga cgcgtatgaa 6240

gctactgtct tctatcgaac aagcatgcga tatttgccga cttaaaaagc tcaagtgctc 6300

caaagaaaaa ccgaagtgcg ccaagtgtct gaagaacaac tgggagtgtc gctactctcc 6360

caaaaccaaa aggtctccgc tgactagggc acatctgaca gaagtggaat caaggctaga 6420

aagactggaa cagctatttc tactgatttt tcctcgagaa gaccttgaca tgattttgaa 6480

aatggattct ttacaggata taaaagcatt gttaacagga ttatttgtac aagataatgt 6540

gaataaagat gccgtcacag atagattggc ttcagtggag actgatatgc ctctaacatt 6600

gagacagcat agaataagtg cgacatcatc atcggaagag agtagtaaca aaggtcaaag 6660

acagttgact gtataattca ctcctcaggt gcaggctgcc tatcagaagg tggtggctgg 6720

tgtggccaat gccctggctc acaaatacca ctgagatctt tttccctctg ccaaaaatta 6780

tggggacatc atgaagcccc ttgagcatct gacttctggc taataaagga aatttatttt 6840

cattgcaata gtgtgttgga attttttgtg tctctcactc ggaaggacat atgggagggc 6900

aaatcattta aaacatcaga atgagtattt ggtttagagt ttggcaacat atgcccatat 6960

gctggctgcc atgaacaaag gttggctata aagaggtcat cagtatatga aacagccccc 7020

tgctgtccat tccttattcc atagaaaagc cttgacttga ggttagattt tttttatatt 7080

ttgttttgtg ttattttttt ctttaacatc cctaaaattt tccttacatg ttttactagc 7140

cagatttttc ctcctctcct gactactccc agtcatagct gtccctcttc tcttatggag 7200

atcggagaaa gaggtaat 7218

<210> 15
<211> 24
<212> DNA

<213>  Artificial

<220>
<223>  qAFP forward primer

<400>  15
caaagctgaa aatgcagttg aatg                                              24


<210>  16
<211>  22
<212>  DNA
<213>  Artificial

<220>
<223>  qAFP reverse primer

<400>  16
ttccccatcc tgcagacaat cc                                                22


<210>  17
<211>  20
<212>  DNA
<213>  Artificial

<220>
<223>  GAPDH forward primer

<400>  17
agaaggctgg ggctcatttg                                                   20


<210>  18
<211>  20
<212>  DNA
<213>  Artificial

<220>
<223>  GAPDH reverse primer

<400>  18
aggggccatc cacagtcttc                                                   20


<210>  19
<211>  22
<212>  DNA
<213>  Artificial

<220>
<223>  L2 forward primer

<400>  19
ttgtggttct tgcagatatg gc                                                22


<210>  20
<211>  16
<212>  DNA
<213>  Artificial

<220>

<223>  L2 reverse primer

<400>  20
tcggaatccc ggcacc                                                        16


<210>  21
<211>  2203
<212>  DNA
<213>  Artificial

<220>
<223>  AFPI sequence

<400>  21
tttagaaata tgggggtagg ggtggtggtg gggcctggat aaagctgagt ggtatgaatg      60

agttagaaat atgggggtag gggtggtggt ggtaattctg ttttctcccc ataggtgaga     120

taagcattgg gttaaatgtg ctttctctct ctccctctcc tttcttaaga attaagggac     180

agactatggg ctggaggact ttgaggatgt ctgtctcata acacttgggt tgtatctgtt     240

ctatggggct tgttttaagc ttggcaactt gcaacagggt tcactgactt tctccccagg     300

cccaaggtac tgtcctcttt tcatatctgt tttggggcct ctggggcttg aatatctgag     360

aaaatataaa catttcaata atgttctgtg gtgagatgag tatgagagat gtgtcattca     420

tttgtatcaa tgaatgaatg aggacaatta gtgtataaat ccttagtaca acaatctgag     480

ggtaggggtg gtactattca atttctattt ataaagatac ttatttctat ttatttatgc     540

ttgtgacaaa tgttttgttc gggaccacag gaatcacaaa gatgagtctt tgaatttaag     600

aagttaatgg tccaggaata attacatagc ttacaaatga ctatgatata ccatcaaaca     660

agaggttcca tgagaaaata atctgaaagg tttaataagt tgtcaaaggt gagagggctc     720

ttctctagct agagactaat cagaaataca ttcagggata attatttgaa tagaccttaa     780

gggttgggta cattttgttc aagcattgat ggagaaggag agtgaatatt tgaaaacatt     840

tcaactaacc aaccacccaa tccaacaaac aaaaaatgaa aagaatctca gaaacagtga     900

gataagagaa ggaattttct cacaacccac acgtatagct caactgctct gaagaagtat     960

atatctaata tttaacacta acatcatgct aataatgata ataattactg tcatttttta    1020

atgtctataa gtaccaggca tttagaagat attattccat ttatatatca aaataaactt    1080

gaggggatag atcattttca tgatatatga gaaaaattaa aaatcagatt gaattatttg    1140

cctgtcatac agctaataat tgaccataag acaattagat ttaaattagt tttgaatctt    1200

tctaatacca aagttcagtt tactgttcca tgttgcttct gagtggcttc acagacttat    1260

gaaaaagtaa acggaatcag aattacatca atgcaaaagc attgctgtga actctgtact    1320

taggactaaa ctttgagcaa taacacatat agattgagga ttgtttgctg ttagtataca    1380

aactctggtt caaagctcct ctttattgct tgtcttggaa aatttgctgt tcttcatggt    1440

ttctcttttc actgctatct atttttctca accactcaca tggctacaat aactgtctgc 1500

aagcttatga ttcccaaata tctatctcta gcctcaatct tgttccagaa gataaaaagt 1560

agtattcaaa tgcacatcaa cgtctccact tggagggctt aaagacgttt caacatacaa 1620

accggggagt tttgcctgga atgtttccta aaatgtgtcc tgtagcacat agggtcctct 1680

tgttccttaa aatctaatta cttttagccc agtgctcatc ccacctatgg ggagatgaga 1740

gtgaaaaggg agcctgatta ataattacac taagtcaata ggcatagagc caggactgtt 1800

tgggtaaact ggtcacttta tcttaaacta aatatatcca aaactgaaca tgtacttagt 1860

tactaagtct ttgactttat ctcattcata ccactcagct ttatccaggc ctctagaagt 1920

ttgaggagaa tatttgttat atttgcaaaa taaaataagt ttgcaagttt tttttttctg 1980

ccccaaagag ctctgtgtcc ttgaacataa aatacaaata accgctatgc tgttaattat 2040

tgacaaatgt cccattttca acctaaggaa ataccataaa gtaacagata taccaacaaa 2100

aggttactag ttaacaggca ttgcctgaaa agagtataaa agaatttcag catgattttc 2160

catattgtgc ttccaccact gccaataaca aaataactag caa 2203

<210> 22
<211> 1113
<212> DNA
<213> Artificial

<220>
<223> AFPII sequence

<400> 22
cagattgaat tatttgcctg tcatacagct aataattgac cataagacaa ttagatttaa 60

attagttttg aatctttcta ataccaaagt tcagtttact gttccatgtt gcttctgagt 120

ggcttcacag acttatgaaa aagtaaacgg aatcagaatt acatcaatgc aaaagcattg 180

ctgtgaactc tgtacttagg actaaacttt gagcaataac acatatagat tgaggattgt 240

ttgctgttag tatacaaact ctggttcaaa gctcctcttt attgcttgtc ttggaaaatt 300

tgctgttctt catggtttct cttttcactg ctatctattt ttctcaacca ctcacatggc 360

tacaataact gtctgcaagc ttatgattcc caaatatcta tctctagcct caatcttgtt 420

ccagaagata aaaagtagta ttcaaatgca catcaacgtc tccacttgga gggcttaaag 480

acgtttcaac atacaaaccg gggagttttg cctggaatgt ttcctaaaat gtgtcctgta 540

gcacataggg tcctcttgtt ccttaaaatc taattacttt tagcccagtg ctcatcccac 600

ctatggggag atgagagtga aaagggagcc tgattaataa ttacactaag tcaataggca 660

tagagccagg actgtttggg taaactggtc actttatctt aaactaaata tatccaaaac 720

tgaacatgta cttagttact aagtctttga ctttatctca ttcataccac tcagctttat 780

ccaggccact tatttgacag tattattgcg aaaacttcct atctagaagt ttgaggagaa 840

tatttgttat atttgcaaaa taaaataagt ttgcaagttt tttttttctg ccccaaagag 900

ctctgtgtcc ttgaacataa aatacaaata accgctatgc tgttaattat tgacaaatgt 960

cccattttca acctaaggaa ataccataaa gtaacagata taccaacaaa aggttactag 1020

ttaacaggca ttgcctgaaa agagtataaa agaatttcag catgattttc catattgtgc 1080

ttccaccact gccaataaca aaataactag caa 1113

<210> 23
<211> 856
<212> DNA
<213> Artificial

<220>
<223> AFPIII sequence

<400> 23
cagattgaat tatttgcctg tcatacagct aataattgac cataagacaa ttagatttaa 60

attagttttg aatctttcta ataccaaagt tcagtttact gttccatgtt gcttctgagt 120

ggcttcacag acttatgaaa aagtaaacgg aatcagaatt acatcaatgc aaaagcattg 180

ctgtgaactc tgtacttagg actaaacttt gagcaataac acatatagat tgaggattgt 240

ttgctgttag tatacaaact ctggttcaaa gctcctcttt attgcttgtc ttggaaaatt 300

tgctgttctt catggtttct cttttcactg ctatctattt ttctcaacca ctcacatggc 360

tacaaaagct tcctgattaa taattacact aagtcaatag gcatagagcc aggactgttt 420

gggtaaactg gtcactttat cttaaactaa atatatccaa aactgaacat gtacttagtt 480

actaagtctt tgactttatc tcattcatac cactcagctt tatccaggcc acttatttga 540

cagtattatt gcgaaaactt cctatctaga agtttgagga gaatatttgt tatatttgca 600

aaataaaata agtttgcaag tttttttttt ctgccccaaa gagctctgtg tccttgaaca 660

taaaatacaa ataaccgcta tgctgttaat tattgacaaa tgtcccattt tcaacctaag 720

gaaataccat aaagtaacag atataccaac aaaaggttac tagttaacag gcattgcctg 780

aaaagagtat aaaagaattt cagcatgatt ttccatattg tgcttccacc actgccaata 840

acaaaataac tagcaa 856

<210> 24
<211> 663
<212> DNA
<213> Artificial

<220>
<223> AFPIV sequence

<400> 24
cagattgaat tatttgcctg tcatacagct aataattgac cataagacaa ttagatttaa 60

attagttttg aatctttcta ataccaaagt tcagtttact gttccatgtt gcttctgagt 120

ggcttcacag acttatgaaa aagtaaacgg aatcagaatt acatcaatgc aaaagcattg 180

ctgtgaactc tgtacttagg actaaacttt gagcaataac acatatagat tgaggattgt 240

ttgctgttag tatacaaact ctggttcaaa gctcctcttt attgcttgtc ttggaaaatt 300

tgctgttctt catggtttct cttttcactg ctatctattt ttctcaacca ctcacatggc 360

tacaaaagct ttctagaagt ttgaggagaa tatttgttat atttgcaaaa taaaataagt 420

ttgcaagttt tttttttctg ccccaaagag ctctgtgtcc ttgaacataa aatacaaata 480

accgctatgc tgttaattat tgacaaatgt cccattttca acctaaggaa ataccataaa 540

gtaacagata taccaacaaa aggttactag ttaacaggca ttgcctgaaa agagtataaa 600

agaatttcag catgattttc catattgtgc ttccaccact gccaataaca aaataactag 660

caa 663

<210> 25
<211> 485
<212> DNA
<213> Artificial

<220>
<223> AFPV sequence

<400> 25
cctgattaat aattacacta agtcaatagg catagagcca ggactgtttg ggtaaactgg 60

tcactttatc ttaaactaaa tatatccaaa actgaacatg tacttagtta ctaagtcttt 120

gactttatct cattcatacc actcagcttt atccaggcca cttatttgac agtattattg 180

cgaaaacttc ctatctagaa gtttgaggag aatatttgtt atatttgcaa aataaaataa 240

gtttgcaagt tttttttttc tgccccaaag agctctgtgt ccttgaacat aaaatacaaa 300

taaccgctat gctgttaatt attgacaaat gtcccatttt caacctaagg aaataccata 360

aagtaacaga tataccaaca aaaggttact agttaacagg cattgcctga aaagagtata 420

aaagaatttc agcatgattt tccatattgt gcttccacca ctgccaataa caaaataact 480

agcaa 485